# EUROPEAN PATENT SPECIFICATION

(11) **EP 1 539 964 B1**
(45) Date of publication and mention of the grant of the patent: **27.12.2006**
(21) Application number: 03794818.9
(22) Date of filing: 10.09.2003
(51) Int. Cl.: C12N 15/62

(54) **COLLECTIN-COMPLEMENT ACTIVATING PROTEIN CHIMERAS**
COLLECTIN-KOMPLEMENT AKTIVIERENDE PROTEINCHIMÄREN
PROTEINES CHIMERES ACTIVANT LA COLLECTINE DU COMPLEMENT

(30) Priority: 10.09.2002 DK 200201328
(43) Date of publication of application: 15.06.2005
(73) Proprietor: Natlmmune A/S, 2100 Kobenhavn (DK)
(72) Inventor: KONGERSLEV, Leif, DK-3460 Birkeroed (DK); WEILGUNY, Dietmar, DK-2830 Virum (DK); MATTHIESEN, Finn, DK-2700 Bronshoj (DK)
(74) Representative: HOEIBERG A/S
(86) International application number: PCT/DK2003/000585
(87) International publication number: WO 2004/024925

(56) References cited:
- WO-A-00/70043
- WO-A-01/40451
- WO-A-02/06460
- WO-A-99/10001
- WO-A-99/37676
- US-B1- 6 337 193
- MATSUSHITA MISAO ET AL: "The role of ficolins in innate immunity." IMMUNOBIOLOGY. GERMANY SEP 2002, vol. 205, no. 4-5, September 2002 (2002-09), pages 490-497, XP002270462 ISSN: 0171-2985
- FUJITA TEIZO: "Evolution of the lectin-complement pathway and its role in innate immunity." NATURE REVIEWS. IMMUNOLOGY. ENGLAND MAY 2002, vol. 2, no. 5, May 2002 (2002-05), pages 346-353, XP002270463 ISSN: 1474-1733
- MATSUSHITA M ET AL: "Cutting edge: complement-activating complex of ficolin and mannose-binding lectin-associated serine protease." JOURNAL OF IMMUNOLOGY (BALTIMORE, MD.: 1950) UNITED STATES 1 MAR 2000, vol. 164, no. 5, 1 March 2000 (2000-03-01), pages 2281-2284, XP002270464 ISSN: 0022-1767
- JINHUA LU ET AL: "Collectins and ficolins: sugar pattern recognition molecules of the mammalian innate immune system" BIOCHIMICA ET BIOPHYSICA ACTA, vol. 1572, 2002, pages 387-400, XP002270465
- MATSUSHITA MISAO ET AL: "Activation of the lectin complement pathway by H-ficolin (Hakata antigen)." JOURNAL OF IMMUNOLOGY (BALTIMORE, MD.: 1950) UNITED STATES 1 APR 2002, vol. 168, no. 7, 1 April 2002 (2002-04-01), pages 3502-3506, XP002270466 ISSN: 0022-1767

## Description

### Field of invention

The present invention relates to a fusion protein capable of activating the complement system, methods for producing said fusion protein as well as pharmaceutial composition comprising said fusion protein and methods for treating diseases, in particular infections, with said fusion protein.

### Background of invention

Animals have developed different complex strategies to protect themselves against infections. The immune responses can be divided into to main groups, the adaptive immune response, in which an adaptation has taken place and in which cells play a dominant part and the innate immune response, which is available instantly and which primarily is based on molecules present in the body fluids. The innate immune system is operational at time of birth, in contrast to the adaptive immune defence which only during infancy obtains its full power of protecting the body (Janeway *et al.,* 1999).

Bacteria entering the body at mucosal surfaces or through broken skin are immediately recognised by collectins, a family of soluble proteins that recognise distinctive carbohydrate configurations that are present on the surfaces of microbes and absent from the cells of the multicellular organism. Collectins thus belong to the large and diverse group of pattern recognition receptors of the innate immune system. In humans, three collectins are known, although others may exist: cows for example have more. Collectins target the particles to which they bind either for uptake by phagocytes or for activation of the complement cascade, and in these ways can mediate their destruction.

Collectins all exhibit the following architecture: they have an N-terminal cysteine-rich region that appears to form inter-chain disulfide bonds, followed by a collagen-like region, an α-helical coiled-coil region and finally a C-type lectin domain which is the pattern-recognizing region and is referred to as the carbohydrate recognition domain (CRD). The name collectin is derived from the presence of both collagen and lectin domains. The α-helical coiled-coil region initiates trimerisation of the individual poly-petides to form collagen triple coils, thereby generating collectin subunits each consisting of 3 individual polypeptides, whereas the N-terminal region mediates formation of oligomers of subunits. Different collectins exhibit distinctive higher order structures, typically either tetramers of subunits or hexamers of subunits. The grouping of large numbers of binding domains allows collectins to bind with high avidity to microbial cell walls, despite a relatively low intrinsic affinity of each individual CRD for carbohydrates.

C-type CRDs are found in proteins with a widespread occurrence, both in phylogenetic and functional perspective. The different CRDs of the different collectins enable them to recognise a range of distinct microbial surface components exposed on different microorganisms. The terminal CRDs are distributed in such a way that all three domain target surfaces that present binding sites has a spacing of approximately 53 Å (Sheriff *et al.,* 1994; Weis & Drickamer, 1994). This property of pattern recognition' may contribute further to the selectively binding of microbial surfaces. The collagenous region or possibly the N-terminal tails of the collectins, are recognised by specific receptors on phagocytes, and is the binding site for associated proteases that are activated to initiate the complement cascade upon binding of the CRD domain to a target.

Mannan-binding lectin (MBL) also termed mannose-binding lectin or mannose binding protein is a collectin which has gained great interest as an important part of the innate immune system. MBL binds to specific carbohydrate structures found on the surface of a range of microorganisms including bacteria, yeast, parasitic protozoa and viruses, and has been found to exhibit antibacterial activity through killing mediated by activation of the terminal, lytic complement components or through promotion of phagocytosis. MBL deficiency is associated with susceptibility to frequent infections by a variety of microorganisms in childhood, and possibly also in adults.

The CRD of MBL recognises preferentially hexoses with equatorial 3- and 4-OH groups, such as mannose, glucose, *N*-acetylmannosamin and *N*-acetyl glucoseamin while carbohydrates which do not fulfil this sterical requirement, such as galactose and D-fucose, are not bound (Weis *et al.,* 1992). The carbohydrate selectivity is obviously an important aspect of the self/non-self discrimination by MBL and is probably mediated by the difference in prevalence of mannose and *N*-acetyl glucoseamin residues on microbial surfaces, one example being the high content of mannose in the cell wall of yeasts such as *Saccharomyces cerevisiae* and *Candida albicans.* Carbohydrate structures in glycosylation of mammalian proteins are usually completed with sialic acid, which prevents binding of MBL to these oligomeric carbohydrates and thus prevents MBL recognition of 'self' surfaces. Also, the trimeric structure of each MBL subunit may be of importance for target recognition.

Complement is a group of proteins present in blood plasma and tissue fluid that aids the body's defences following an infection. The complement system is being activated through at least three distinct pathways, designated the classical pathway, the alternative pathway, and the MBLectin pathway (Janeway *et al.,* 1999). The classical pathway is initiated when complement factor 1 (C1) recognises surface-bound immunoglobulin. The C1 complex is composed of two proteolytic enzymes, C1r and C1s, and a non-enzymatic part, C1q, which contains immunoglobulin-recognising domains. C1q and MBL shares structural features, both molecules having a bouquet-like appearance when visualised by electron microscopy. Also, like C1 q, MBL is found in complex with two proteolytic enzymes, the mannan-binding lectin associated proteases (MASP). The three pathways all generate complement factor 3 (C3) convertase, which ensures the binding of C3b to the surface of the activating surface, *i.e.* the targeted microbial pathogen. Conversion of C3 into surface bound C3b is pivotal in the process of eliminating the microbial pathogen by phagocytosis or lysis (Janeway *et al.,* 1999).

Certain O-antigen specific oligosaccharides of *Salmonella* have been reported to activate complement in C4-deficient guinea-pig serum and *Salmonella* serogroup C was later shown to react with MBL and hence activate complement by the MBLectin pathway, which is also termed the MBL pathway of complement activation or the lectin pathway.

It has for some time been speculated that the innate immune system may collaborate with the adaptive immune system in the generation of specific immune responses as exemplified by the antibody response after infection or vaccination. Fearon's group have shown that the attachment of the C3d fragment of complement factor C3 onto a protein antigen through fusion by gene technology can in crease the immunogenecity of the antigen 1000 fold or more. Practical applications of this technique, or any number of modifications, are still awaited.

The importance of the complement system for normal immune responses was first suggested by Pepys, who found impaired antibody responses to sheep erythrocytes, a thymus-dependent antigen, in mice that were C3-depleted with cobra venom factor. The idea of a link between innate and adaptive immunity was supported by reports demonstrating reduced primary antibody responses to thymus dependent antigens and impaired IgM to IgG switching in patients and experimental animals with deficiencies of C4, C2 and C3. The mechanism may involve the generation of C3-derived ligands for binding of antigen or antigen-containing complexes to complement receptors on B lymphocytes or antigen-presenting cells. Thus, blocking of CR1 (CD35) and CR2 (CD21) in mice with specific anti CR1 and anti-CR2 antibodies or with soluble receptor protein reduced antibody responses to immunisation and experiments with CR1 and CR2-deficient knock-out mice show the requirement of these receptors for responses to thymus-dependent antigens. In addition, patients with leucocyte adhesion deficiency, who lack the CD11/CD18 adhesion molecule CR3, demonstrate impaired antibody responses and failure to switch from IgM to IgG. The C3-derived fragment C3d, a specific CR2 ligand, as mentioned above, show a strong dose-dependent adjuvant effect.

Deficiencies of the classical complement pathway (C1, C2, C4 and C3) are associated with infections by encapsulated bacteria. The main reason for this is probably the reduced efficiency of opsonic and bactericidal defence mechanisms caused by complement dysfunction. However, impaired immune responses to polysaccharide antigens might also be considered. The influence of complement on responses to thymus-independent antigens has not been extensively studied, and the available information is contradictory. Thus, low antibody responses to thymus-independent antigens have been clearly documented in C3-depleted mice and C3-deficient dogs. On the other hand, some reports find that C3-deficient patients appear to respond normally to immunisation with polysaccharide vaccines.

Ficolins, like MBL, are lectins that contain a collagen-like domain. Unlike MBL, however, they have a fibrinogen-like domain, which is similar to fibrinogen β- and γ-chains. Ficolins also forms oligomers of structural subunits, each of which is composed of three identical 35 kDa polypeptides. Each subunit is composed of an amino-terminal, cysteine-rich region; a collagen-like domain that consists of tandem repeats of Gly-Xaa-Yaa triplet sequences (where Xaa and Yaa represent any amino acid); a neck region; and a fibrinogen-like domain. The oligomers of ficolins comprises two or more subunits, especially a tetrameric form of ficolin has been observed.

Some of the ficolins triggers the activation of the complement system substantially in similar way as done by MBL. This triggering of the complement system results in the activation of novel serine proteases (MASPs) as described above.

The fibrinogen-like domain of several lectins has a similar function to the CRD of C-type lectins including MBL, and hereby function as pattern-recognition receptors to discriminate pathogens from self.

Serum ficolins have a common binding specificity for GlcNAc (N-acetyl-glucosamine), elastin or GaINAc (N-acetyl-galactosamine). The fibrinogen-like domain is responsible for the carbohydrate binding. In human serum, two types of ficolin, known as L-ficolin (P35, ficolin L, ficolin 2 or hucolin) and H-ficolin (Hakata antigen, ficolin 3 or thermolabile b2-macroglycoprotein), have been identified, and both of them have lectin activity. L-ficolin recognises GlcNAc and H-ficolin recognises GaINAc. Another ficolin known as M-ficolin (P35-related protein, Ficolin 1 or Ficolin A) is not considered to be a serum protein and is found in leucocytes and in the lungs. L-ficolin and H-ficolin activate the lectin-complement pathway in association with MASPs. M-Ficolin, L-ficolin and H-ficolin has calcium-independent lectin activity.

MASPs (MBL-associated serine proteases) comprising MASP-1, MASP-2 and MASP-3 are proteolytic enzymes that are responsible for activation of the lectin pathway. The overall structure of MASPs resembles that of the two proteolytic components of the first factor in the classical complement pathway, C 1r and C1s. The lectin pathway is initiated when MBL or a ficolin associated with MASP-1, MASP-2, MASP-3 and sMAP binds to a carbohydrate structure of the surfaces of e.g. bacteria, yeast, parasitic protoxoa, viruses. MASP-2 is the enzyme component that - like C1s in the classical pathway - cleaves the complement components C4 and C2 to form the C3 convertase C4bC2a, which is common to both the lectin- and classical-pathway activation routes.

MASP-1, MASP-2, MASP-3 and sMAP are encoded by two genes; sMAP is a truncated form of MASP-2, and MASP-3 is produced from the MASP-1 gene by alternative splicing. The MASP-1 gene has an H-chain-encoding region that is common to MASP-1 and MASP-3, which is followed by tandem repeats of protease-domain-encoding regions that are specific to MASP-3 and MASP-1.

The MASP family can be divided into two phylogenetic lineages - TCN-type and AGY-type lineages. The TCN-type lineage, which includes MASP-1, has a TCN codon (where N denotes A, G, C or T) that encodes the active-site serine, the presence of a histidine-loop disulphide bridge and split exons. By contrast, the AGY-type lineage, which includes MASP-2, MASP-3, C1r and C1s, is characterised by an AGY codon (where Y denotes C or T) that encodes the active-site serine, the absence of a histidine-loop and a single exon.

MASP-1, MASP-2, MASP-3, C1r and C1s consist or six domains: two C1r/C1s/Uegf/bone morphogenetic protein 1 (CUB) domains; an epidermal growth factor (EGF)-like domain; two complement control protein (CCP) domains or short consensus repeats (SCRs), and a serine-protease domain. Histidine (H), aspartic acid (D) and serine (S) residues are essential for the formation of the active centre in the serine-protease domain. Only MASP-1 has two additional cysteine residues in a light chain, which form a histidine.loop disulphide bridge (S-S), as is found in trypsin and.chymotrypsin. On binding of MBL and ficolins to carbohydrate on the surface of a pathogen, the pro-enzyme form of a MASP is cleaved between the second CCP and the protease domain, which results in an active form that consists of two poly-peptides - heavy and light chains (also known as A and B chains).

### Summary of invention

The present invention relates to fusion proteins capable of activating the complement system. Accordingly, the present invention relates to a fusion protein comprising a first polypeptide sequence derived from a lectin complement pathway activating protein (=complement activating protein) or a functional homologue at least 70% identical to said lectin-complement activating pathway protein, wherein said first polypeptide sequence is capable of activating the lectin-complement pathway; and a second polypeptide sequence derived from a collectin or a functional homologue at least 70% identical to said collectin, wherein said second polypeptide sequence is capable of associating with one or more carbohydrates; wherein said complement activating protein is not a collectin.

The fusion protein is suitable for use in treatment consisting of creation, reconstitution, enhancing and/or stimulating the opsonic and/or bactericidal activity of the complement system, i.e. enhancing the ability of the immune defence to recognise and kill microbial pathogens, and accordingly, the invention relates to a medicament comprising the fusion protein.

Also, in another aspect the invention relates to the use of the fusion protein as defined above for the preparation of a medicament for the treatment of a clinical condition in an individual in need thereof comprising administering to said individual the fusion protein as defined above.

In another aspect the invention relates to the use of the fusion protein as defined above for the preparation of a medicament for the treatment or prophylaxis of a clinical condition, such as infection, in an individual in need thereof comprising administering to said individual a the fusion protein a first polypeptide sequence derived from a protein capable of forming oligomers of structural units; and a second polypeptide sequence derived from a mannose binding lectin (MBL, wherein said first polypeptide sequence and said second peptide sequence is not derived from the same protein, and said fusion protein is capable of associating with mannose-associated serine protease (MASP). The first polypeptide sequence is preferably derived from a protein capable of forming tetramers, pentamers, and/or hexamers of a structural unit. In a preferred embodiment the first polypeptide sequence and the second polypeptide sequence are as described below.

Furthermore the invention relates to a method for producing the fusion protein, as well as an isolated nucleic acid sequence encoding the fusion protein, a vector comprising the sequence and a cell comprising the vector.

### Drawings

Figure 1 shows the sequence of L ficolin
Figure 2 shows the sequence of MBL
Figure 3 shows an example of a fusion protein
Figures 4-8 show plasmids as described in Example 1
Figure 9 shows an alignment of fusion proteins described in Example 1
Figure 10 shows two Western blots as discussed in Example 2.

### Definitions

Collectins: A family of structurally related, carbohydrate-recognising proteins of innate immunity, including mannan-binding lectin (MBL) and surfactant proteins A and D. The name refers to the presence of a collagen-like region and a C-type lectin domain.

Complement: A group of proteins present in blood plasma and tissue fluid that aids the body's defences following an infection. Complement is involved in destroying foreign cells and attracting phagocytes to the area of conflict in the body.

Conjugated: An association formed between two compounds for example between an immunogenic determinant and a collectin and/or collectin homologue or between an immunogenic determinant and a saccharide. The association may be a physical association generated e.g. by the formation of a chemical bond, such as e.g. a co-valent bond.

CRD: Carbohydrate recognition domain, a C-type lectin domain that is found at the C-terminus of collectins.

Immunogenic determinant: A molecule, or a part thereof, containing one or more epitopes that will stimulate the immune system of a host organism to make a secretory, humoral and/or cellular antigen-specific response, or a DNA molecule which is capable of producing such an immunogen in a vertebrate.

Immune response: Response to a immunogenic composition comprising an immunogenic determinant. An immune response involves the development in the host of a cellular- and/or humoral immune response to the administered composition or vaccine in question. An immune response generally involves the action of one or more of i) the antibodies raised, ii) B cells, iii) helper T cells, iv) suppressor T cells, v) cytotoxic T cells and iv) complement directed specifically or unspecifically to an immunogenic determinant present in an administered immunogenic composition.

Lectin: Proteins that specifically bind carbohydrates.

MASP: Mannose-associated serine protease

MBL: Mannan-binding lectin or mannose-binding lectin.

Subunit complex=structural unit complex of three individual fusion proteins, like the subunit complex discussed above for MBL and ficolins.

### Detailed description of the invention

An object of the present invention is to provide a fusion protein capable of activating the complement system in order to aid in preventing or treating diseases, in particular infectious diseases.

The fusion protein is composed of
a first polypeptide sequence derived from a lectin-complement pathway activating protein (=complement activating protein) or from a functional homologue at least 70% identical to said lectin-complement activating pathway protein, wherein said first polypeptipe sequence is capable of activating the lectin-complement pathway; and
a second polypeptide sequence derived from a collectin or from a functional homologue at least 70% identical to said collectin, wherein said second polypeptide sequence is capable of associating with one or more carbohydrates;
wherein said complement activating protein is not a collectin.

By combining a polypeptide sequence derived from a lectin-complement pathway activating protein and a polypeptide sequence derived from a collectin it is possible to design a fusion protein having binding affinity for a variety of carbohydrates, preferably bacterial and viral carbohydrates and at the same time having complement system activating activity.

### First polypeptide sequence

The first polypeptide sequence may be derived from any lectin-complement pathway activating protein. Said lectin-complement pathway activating protein may be naturally occurring lectin-complement pathway activating protein as well as variants or homologues to said lectin-complement pathway activating proteins, wherein said variants or homologues have maintained the lectin-complement pathway activating activity.

It is preferred that the fusion protein is capable of forming subunit complexes, each consisting of 3 individual fusion proteins as defined above.

Also the first polypeptide sequence is preferably capable of forming oligomeric complexes with the first polypeptide sequence of another fusion protein, wherein said another fusion protein may be identical to the first fusion protein. Thereby an oligomeric complex of two or more fusion proteins or two or more subunit complexes may be provided, said oligomeric complex having a higher binding avidity for bacterial or viral carbohydrates than the monomeric fusion protein. In a preferred embodiment the oligomeric complex is a dimeric subunit complex, more preferably a trimeric subunit complex, more preferably a tetrameric subunit complex.

In a preferred embodiment the lectin-complement pathway activating protein is a ficolin as defined above. Said ficolin may be L-ficolin, H-ficolin or M-ficolin or variants or homologues thereof. In a preferred embodiment the lectiri-complement pathway activating protein is L-ficolin.

In another embodiment the first polypeptide sequence comprises the fibrinogen domain of the lectin, and/or the neck region of a lectin, such as a ficolin or a homologue or a variant thereof.

When the first polypeptide sequence is derived from ficolin or from a variant or a homologue of ficolin, it is preferred that the first polypeptide sequence comprises the collagen-like domain from ficolin or from a variant or homologue of ficolin. In another embodiment it is preferred that the first polypeptide sequence comprises the Cystein rich domain from ficolin or from a variant or homologue of ficolin. It is even more preferred that the first polypeptide sequence comprises the collagen-like domain and the Cystein rich domain from ficolin or from a variant or homologue of ficolin.

It is more preferred that the first polypeptide sequence comprises the collagen-like domain from L-ficolin or from a variant or homologue of L-ficolin. In another embodiment it is more preferred that the first polypeptide sequence comprises the Cystein rich domain from L-ficolin or from a variant or homologue of L-ficolin. It is even more preferred that the first polypeptide sequence comprises the N-terminal region of L-ficolin including two Cystein amino acid residues.

It is even more preferred that the first polypeptide sequence comprises the collagen-like domain and the Cystein rich domain from L-ficolin or from a variant or homologue of L-ficolin.

In a particular preferred embodiment the ficolin has one of the sequences listed below with reference to their database and accession No. For each of the sequences the Cystein rich region and the collagen-like region is described.

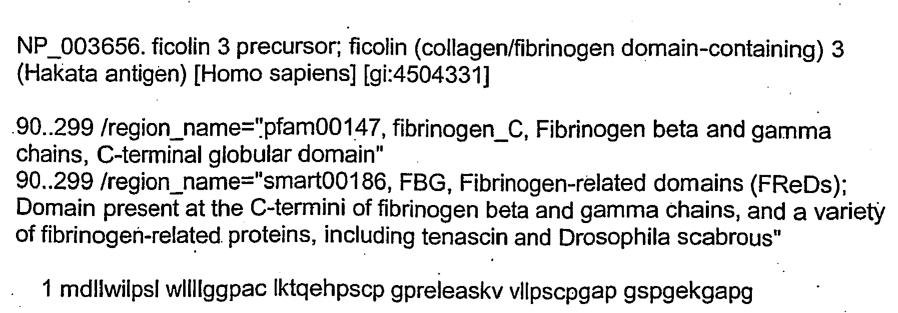

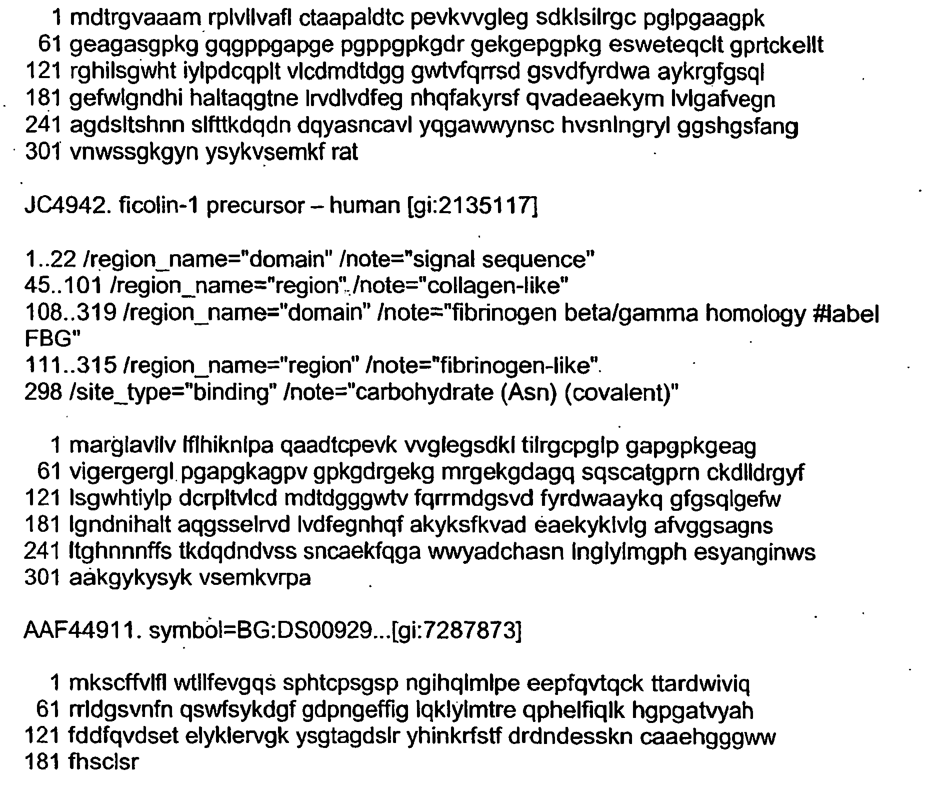

The first polypeptide preferably comprises at least 10, such as at least 12, for example at least 15, such as at least 20, for example at least 25, such as at least 30, for example at least 35, such as at least 40, for example at least 50 consecutive amino acid residues of the complement activating protein or of a variant or a homologue to said protein. Such a variant or homologue is preferably at least 70%, such as 80%, for example 90%, such as 95% identical to the complement activating protein.

The first polypeptide sequence of the fusion protein is preferably capable of activating the lectin-complement pathway when bound directly or indirectly to a target, such as a bacteria or a virus. In a preferred embodiment the first polypeptide sequence is capable of associating with at least one MASP protein, such as a MASP protein selected from the group consisting of MASP-1, MASP-2 and MASP-3 or functional homologues or variants hereof. In particular the first polypeptide is capable of associating with said at least one MASP protein when being part of the fusion protein. Thereby the first polypeptide sequence is capable of providing the fusion protein with complement system activating activity.

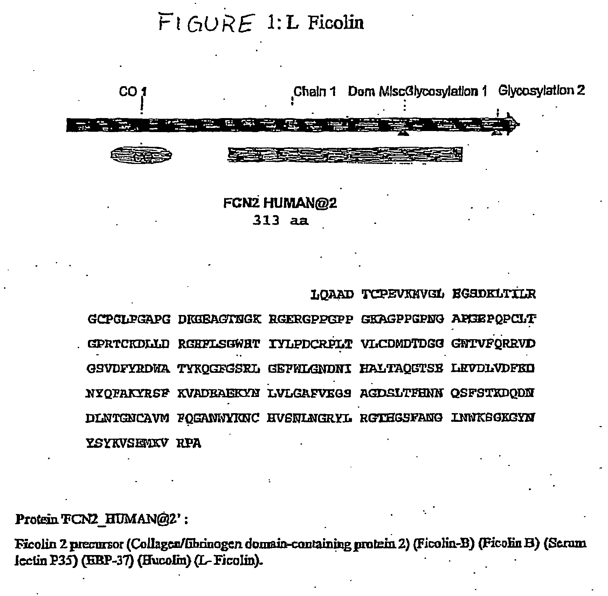

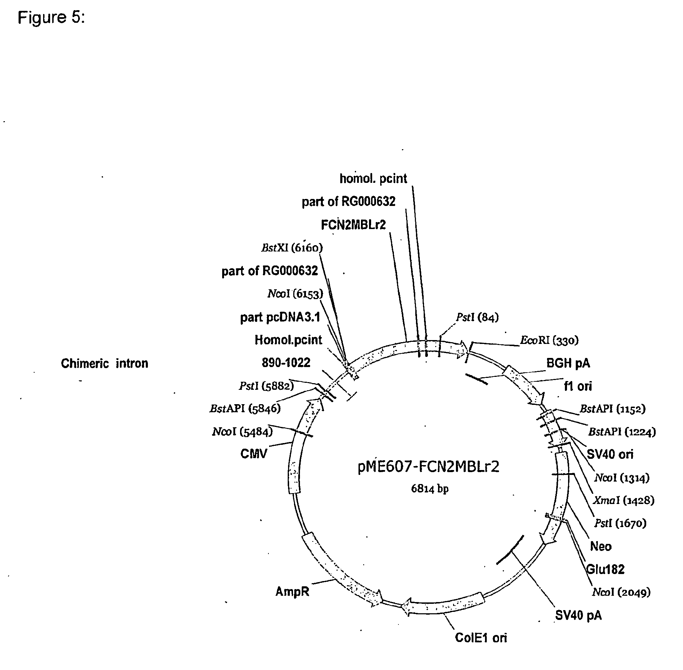

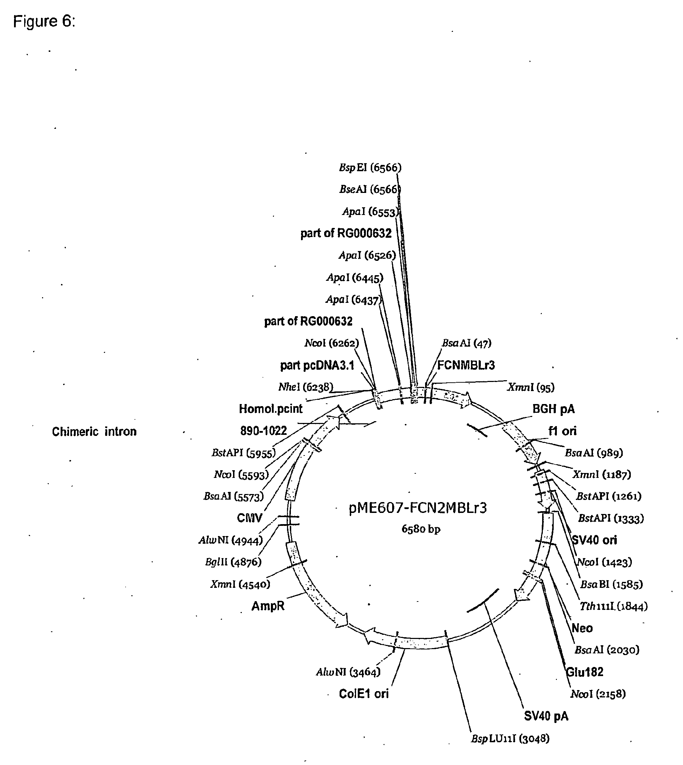

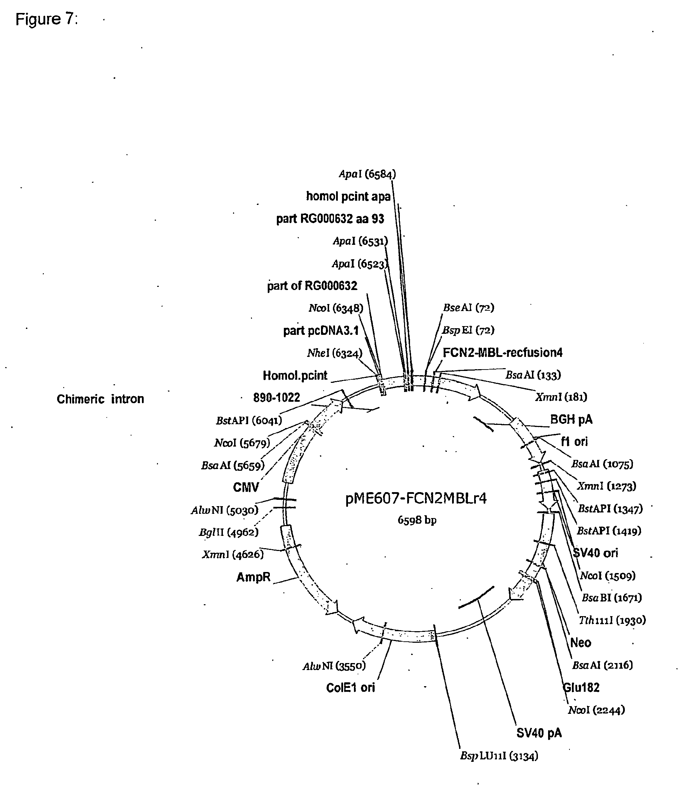

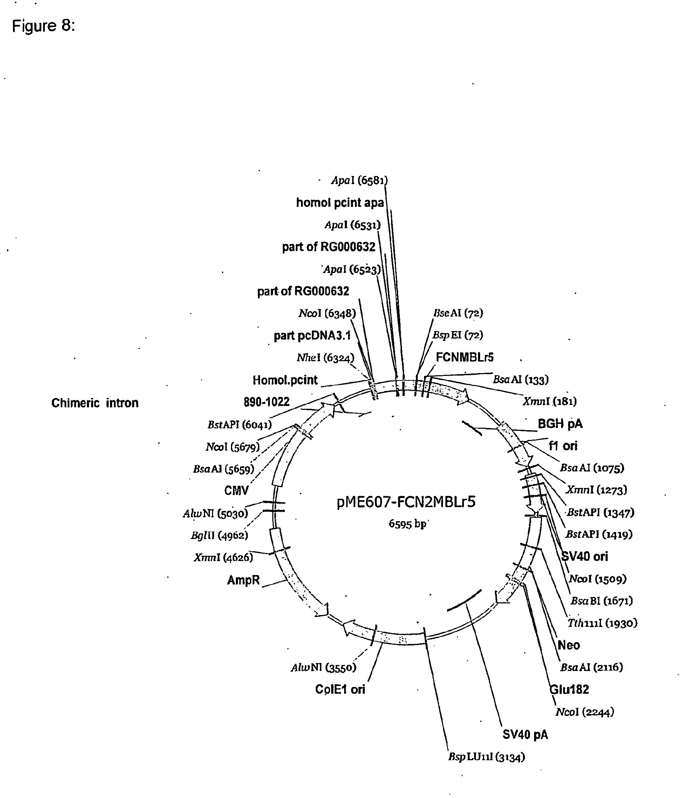

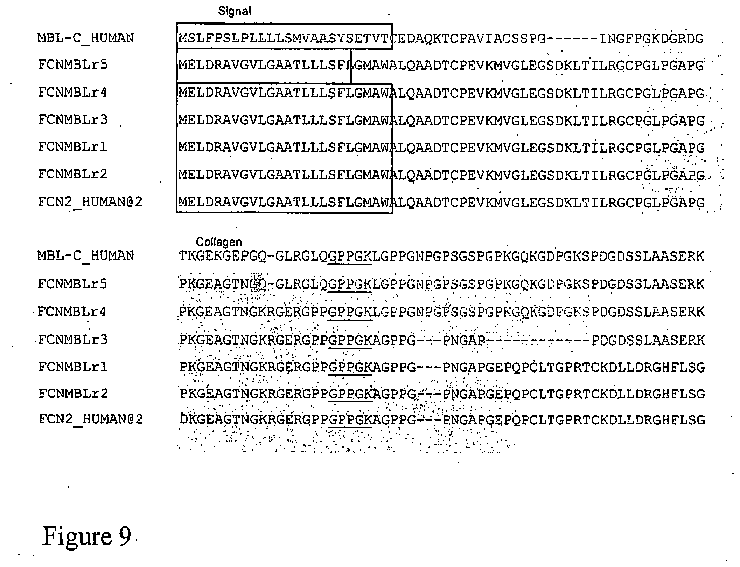

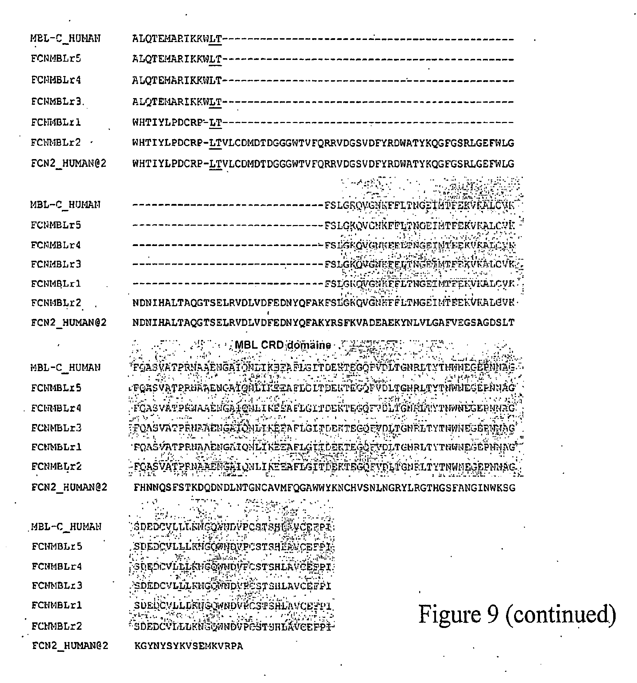

In a particular preferred embodiment the first polypeptide sequence comprises at least the amino acid residues corresponding to 1-54 of L-ficolin sequence of Figure 1, such as 1-55 of L-ficolin sequence of Figure 1, such as 1-69 of L-ficolin sequence of Figure 1, such as 1-77 of L-ficolin sequence of Figure 1, such as 1-90 of L-ficolin sequence of Figure 1, such as 1-93 of L-ficolin sequence of Figure 1, such as 1-131 of L-ficolin sequence of Figure 1, such as 1-207 of L-ficolin sequence of Figure 1. In particular the first polypeptide sequence comprises the amino acid residues selected from: 1-55 of L-ficolin sequence of Figure 1, 1-54 of L-ficolin sequence of Figure 1, 1-50, or 1-77 of L-ficolin sequence of Figure 1. In a more preferred embodiment the first polypeptide sequence has the amino acid residues selected from: 1-55 of L-ficolin sequence of Figure 1, 1-54 of L-ficolin sequence of Figure 1, 1-50, or 1-77 of L-ficolin sequence of Figure 1. In another embodiment the first polypeptide sequence comprises at least the amino acid residues corresponding to 60-90 of L-ficolin sequence of Figure 1, such as 55-90 of L-ficolin sequence of Figure 1, such as 54-92 of L-ficolin sequence of Figure 1.

It is preferred the first polypeptide sequence and the second polypeptide sequence are selected to include the motif X-X-G-X-X-G at least 5 times, such as at least 7 times, preferably in a consecutive sequence. It is more preferred to select the first polypeptide sequence and the second polypeptide sequence so that the aforementioned motif is substituted once with the motif X-X-G-X-G. In the motifs X means any amino acid different from Glycine, and G means Glycine.

### Second polypeptide sequence

The second polypeptide sequence is preferably capable of associating with one or more carbohydrates. This may be accomplished by incorporating at least the carbohydrate recognizing domain of the collectin in question. Accordingly, the second polypeptide sequence preferably comprises the CRD domain of a collectin or a homologue or a variant thereof.

Preferably the collectin is selected from the group consisting of MBL (mannose-binding lectin), SP-A (lung surfactant protein A), SP-D (lung surfactant protein D), BK (or BC, bovine conglutinin) and CL-43 (collectin-43). Most preferably the collectin is MBL.

In a particular preferred embodiment the collectin has one of the sequences listed below with reference to their database and accession No.

### Collectins

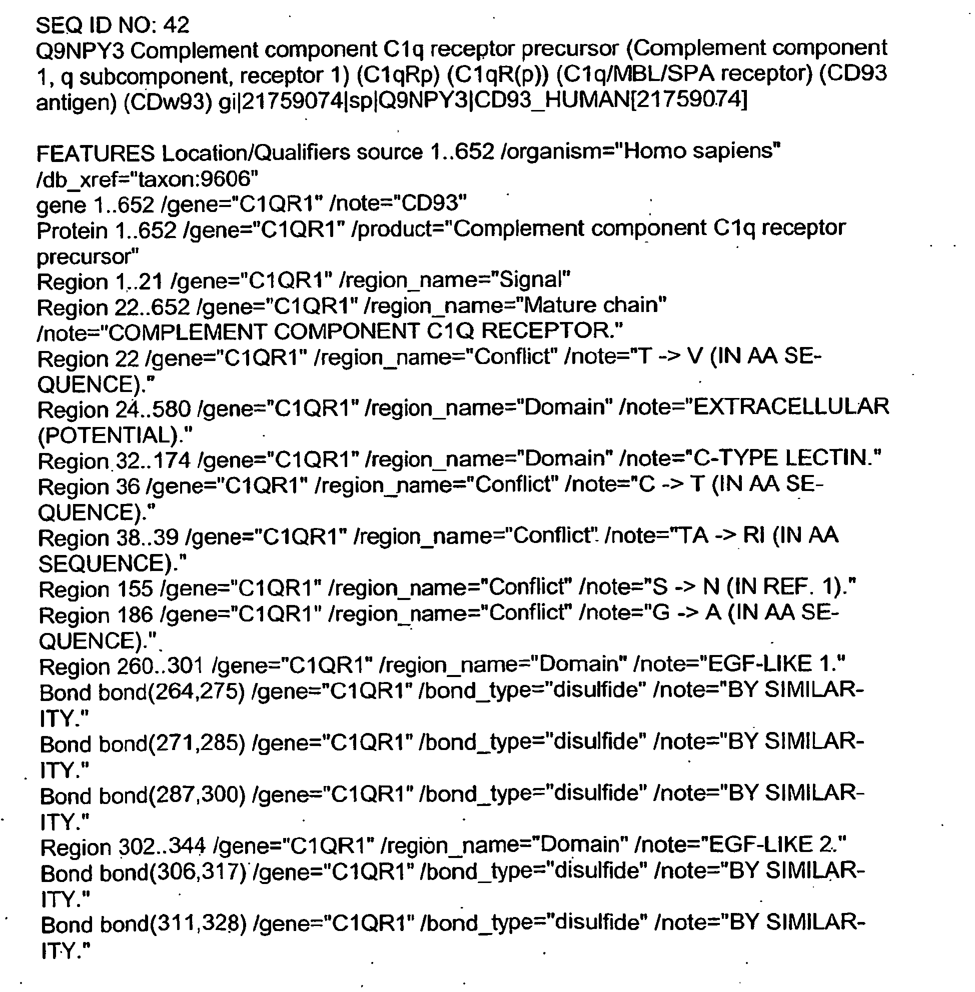

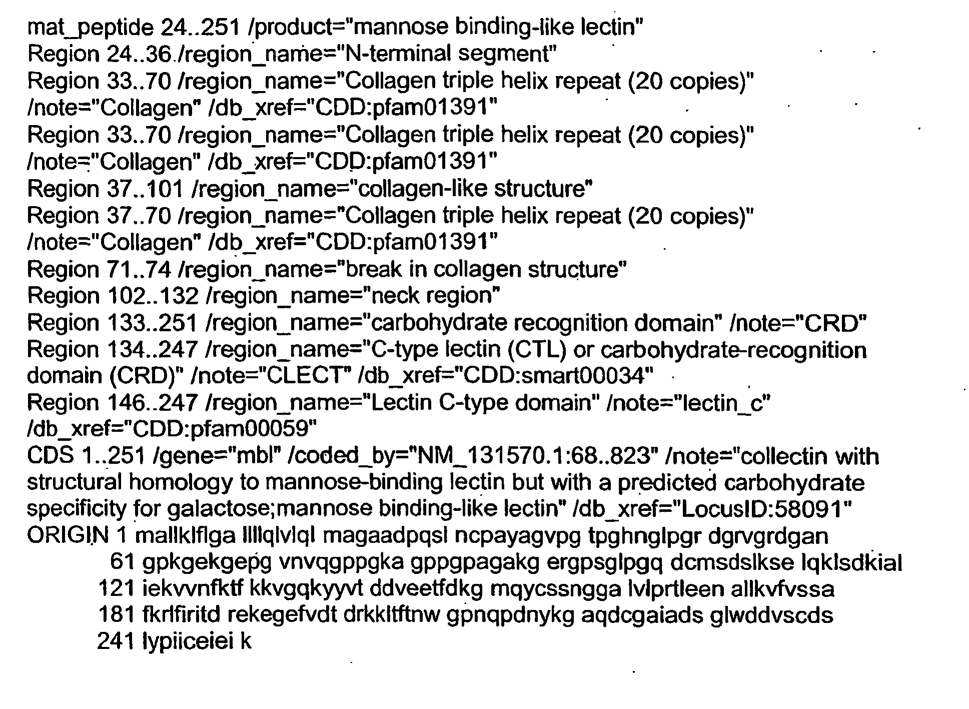

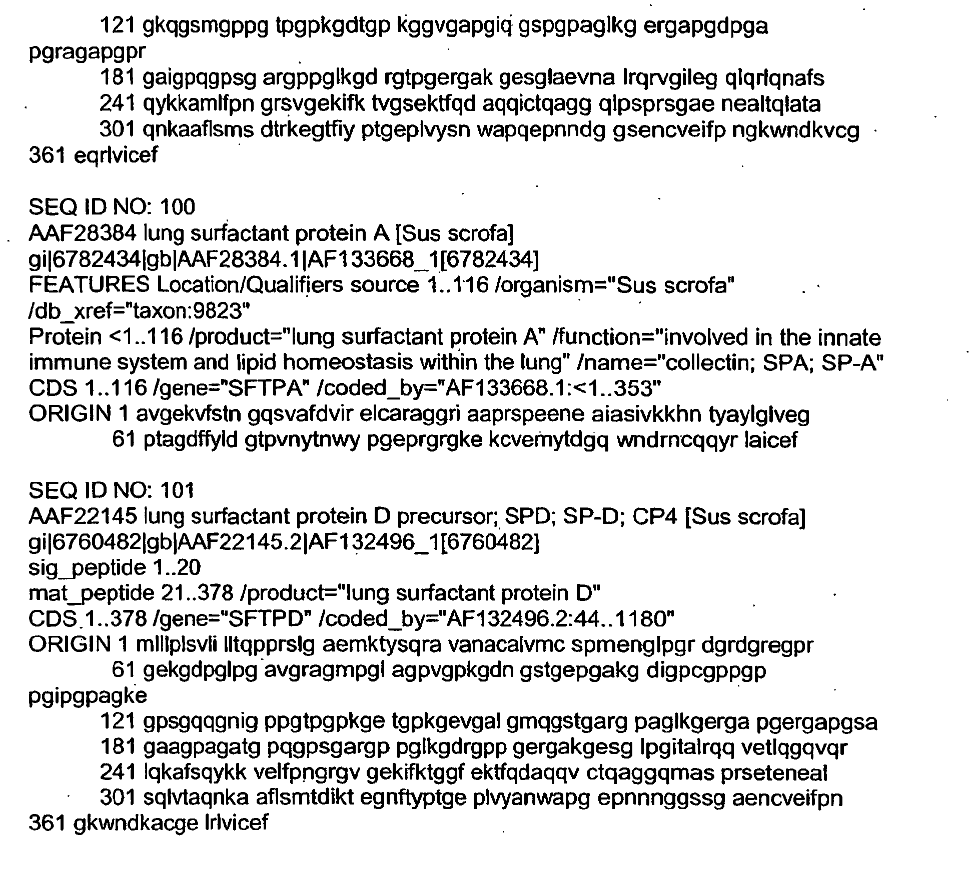

The second polypeptide preferably comprises at least 10, such as at least 12, for example at least 15, such as at least 20, for example at least 25, such as at least 30, for example at least 35, such as at least 40, for example at least 50 consecutive amino acid residues of the collectin or of a variant or a homologue to said protein. Such a variant or homologue is preferably at least 70%, such as 80%, for example 90%, such as 95% identical to the collectin.

In a preferred embodiment the second polypeptide sequence comprises the CRD domain of MBL or the neck region of MBL or the collagen-like domain of MBL. More preferably the second polypeptide comprises the neck region and the CRD domain of MBL. In a most preferred embodiment the second polypeptide sequence comprises the collagen-like domain, the neck region and the CRD domain of MBL. MBL is as defined above.

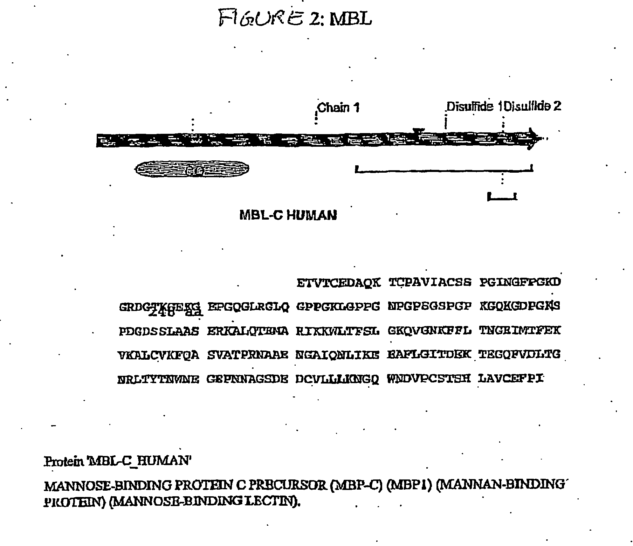

Preferably the second polypeptide sequence comprises at least amino acids 170-200 of the MBL sequence shown in Figure 2, such as at least amino acids 160-200 of the MBL sequence shown in Figure 2, such as at least amino acids 150-200 of the MBL sequence shown in Figure 2, such as at least amino acids 140-200 of the MBL sequence shown in Figure 2, such as at least amino acids 130-200 of the MBL sequence shown in Figure 2, such as at least amino acids 120-200 of the MBL sequence shown in Figure 2, such as at least amino acids 110-200 of the MBL sequence shown in Figure 2, such as at least amino acids 100-200 of the MBL sequence shown in Figure 2, such as at least amino acids 90-200 of the MBL sequence shown in Figure 2, such as at least amino acids 80-200 of the MBL sequence shown in Figure 2, such as at least amino acids 70-200 of the MBL sequence shown in Figure 2, such as at least amino acids 60-200 of the MBL sequence shown in Figure 2, such as at least amino acids 80-228 of the MBL sequence shown in Figure 2.

Preferably the second polypeptide sequence comprises amino acids 80-228 of SEQ ID. NO 2.

In a preferred embodiment the second polypeptide sequence is capable of associating with at least one MASP protein, such as a MASP protein selected from the group consisting of MASP-1, MASP-2 and MASP-3 or functional homologues or variants hereof. In particular the second polypeptide is capable of associating with said at least one MASP protein when being part of the fusion protein. Thereby the second polypeptide sequence is capable of providing the fusion protein with complement system activating activity. In a preferred embodiment the second polypeptide sequence comprises an amino acid sequence selected from: 56-228 of SEQ ID. NO 2, 55-228 of SEQ ID. NO 2, 54-228 of SEQ ID. NO 2, and 50-228 of SEQ ID.

NO 2. In a preferred embodiment the second polypeptide sequence has an amino acid sequence selected from: 56-228 of SEQ ID. NO 2, 55-228 of SEQ ID. NO 2, 54-228 of SEQ ID. NO 2, and 50-228 of SEQ ID. NO.2.

In another embodiment the second polypeptide comprises the cysteine-rich region of the collectin, such as the N-terminal region of the collectin.

### Fusion protein

The fusion protein comprises the first and the second polypeptide connected to each other, optionally through a linker region: In a preferred embodiment the first polypeptide sequence is positioned N-terminally in the fusion protein and the second polypeptide sequence is positioned C-terminally.

Specific examples of the components of the fusion protein are:
- A fusion protein comprising the cysteine-rich region and the collagen-like domain of L-ficolin and the CRD domain of MBL.
- A fusion protein comprising the cysteine-rich region of L-ficolin and the collagen-like domain, the neck region and the CRD domain of MBL.
- A fusion protein comprising the cysteine-rich region and the collagen-like domain of H-ficolin and the CRD domain of MBL.
- A fusion protein comprising the cysteine-rich region of H-ficolin and the collagen-like domain, the neck region and the CRD domain of MBL.
- A fusion protein comprising the cysteine-rich region and the collagen-like domain of M-ficolin and the CRD domain of MBL.
- A fusion protein comprising the cysteine-rich region of M-ficolin and the collagen-like domain, the neck region and the CRD domain of MBL.
- A fusion protein comprising the cysteine-rich region of MBL, and the CRD domain of ficolin.
- A fusion protein comprising the cysteine-rich region of MBL and the collagen-like domain, the neck region and the CRD domain of ficolin.
- A fusion protein comprising the cysteine-rich region and the collagen-like domain of L-ficolin and the CRD domain of Pulmonary surfactant-associated protein D.
- A fusion protein comprising the cysteine-rich region of L-ficolin and the collagen-like domain, the neck region and the CRD domain of Pulmonary surfactant-associated protein D.
- A fusion protein comprising the cysteine-rich region and the collagen-like domain of a ficolin and the CRD domain of a cbllectin-43.
- A fusion protein comprising the cysteine-rich region of a ficolin and the collagen-like domain, the neck region and the CRD domain of a collectin-43.

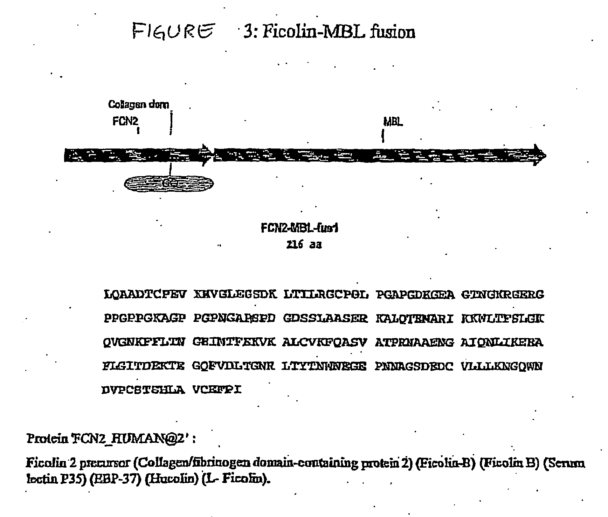

A fusion protein comprising the amino acid sequence as defined by the sequence shown in Figure 3, or a functional homologue thereof, preferably a fusion protein consisting of the amino acid sequence as shown in Figure 3. In another embodiment the fusion protein has amino acid sequence 1-50 of the amino acid shown in Figure 1 and amino acid sequence 54-228 of the amino acid sequence shown in Figure 2.

As discussed above the fusion protein is preferably capable of forming subunit complexes as well as oligomers of subunit complexes. Preferably the fusion protein forms substantially only trimeric, tetrameric, pentameric and hexameric subunit oligomers, such as trimeric, tetrameric, and pentameric subunit oligomers, such as trimeric or tetrameric subunit oligomers, more preferably substantially only tetrameric subunit oligomers, in order to obtain a more homogenous composition of fusion proteins.

### Homologues

In the present context the terms homologue or variant or functional homologues are used as synonymes, wherein a homologue of a protein exhibits one or more substituions, deletions, and/or additions of one or more amino acid residues. Fragments are a subgroup of homologues being truncations of the protein.

A homologue of the protein may comprise one or more conservative amino acid substitutions, such as at least 2 conservative amino acid substitutions, for example at least 3 conservative amino acid substitutions, such as at least 5 conservative amino acid substitutions, for example at least 10 conservative amino acid substitutions, such as at least 20 conservative amino acid substitutions, for example at least 50 conservative amino acid substitutions such as at least 75 conservative amino acid substitutions, for example at least 100 conservative amino acid substitutions. Conservative amino acid substitutions within the meaning of the present invention is substitution of one amino acid within a predetermined group of amino acids for another amino acid within the same predetermined group, exhibiting similar or substantially similar characteristics. Such predetermined groups are for example:
polar side chains (Asp, Glu, Lys, Arg, His, Asn, Gln, Ser, Thr, Tyr, and Cys,)
non-polar side chains (Gly, Ala, Val, Leu, Ile, Phe, Trp, Pro, and Met)
aliphatic side chains (Gly, Ala Val, Leu, Ile)
cyclic side chains (Phe, Tyr, Trp, His, Pro)
aromatic side chains (Phe, Tyr, Trp)
acidic side chains (Asp, Glu)
basic side chains (Lys, Arg, His)
amide side chains (Asn, Gln)
hydroxy side chains (Ser, Thr)
sulphor-containing side chains (Cys, Met), and
amino acids being monoamino-dicarboxylic acids or monoamino-monocarboxylic-monoamidocarboxylic acids (Asp, Glu, Asn, Gln).

Conservative substitutions may be introduced in any position of a preferred protein. It may however also be desirable to introduce non-conservative substitutions. A non-conservative substitution should lead to the formation of a homologue of a protein capable of exerting a function similar to the function of said protein. Such substitution could for example i) differ substantially in hydrophobicity, for example a hydrophobic residue (Val, Ile, Leu, Phe or Met) substituted for a hydrophilic residue such as Arg, Lys, Trp or Asn, or a hydrophilic residue such as Thr, Ser, His, Gln, Asn, Lys, Asp, Glu or Trp substituted for a hydrophobic residue; and/or ii) differ substantially in its effect on polypeptide backbone orientation such as substitution of or for Pro or Gly by another residue; and/or iii) differ substantially in electric charge, for example substitution of a negatively charged residue such as Glu or Asp for a positively charged residue such as Lys, His or Arg (and vice versa); and/or iv) differ substantially in steric bulk, for example substitution of a bulky residue such as His, Trp, Phe or Tyr for one having a minor side chain, e.g. Ala, Gly or Ser (and vice versa).

In a further embodiment the present invention relates to homologues of a preferred protein, wherein such homologues comprise substituted amino acids having hydrophilic or hydropathic indices that are within +/-2.5, for example within +/- 2.3, such as within +/- 2.1, for example within +/- 2.0, such as within +/-1.8, for example within +/- 1.6, such as within +/- 1.5, for example within +/- 1.4, such as within +/-1.3 for example within +/- 1.2, such as within +/- 1.1, for example within +/- 1.0, such as within +/- 0.9, for example within +/- 0.8, such as within +/- 0.7, for example within +/- 0.6, such as within +/- 0.5, for example within +/- 0.4, such as within +/-0.3, for example within +/- 0.25, such as within +/- 0.2 of the value of the amino acid it has substituted.

The importance of the hydrophilic and hydropathic amino acid indices in conferring interactive biologic function on a protein is well understood in the art (Kyte & Doolittle, 1982 and Hopp, U.S. Pat. No. 4,554,101, each incorporated herein by reference).

The amino acid hydropathic index values as used herein are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5) (Kyte & Doolittle, 1982).

The amino acid hydrophilicity values are: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4) (U.S. 4,554,101).

Substitution of amino acids can therefore in one embodiment be made based upon their hydrophobicity and hydrophilicity values and the relative similarity of the amino acid side-chain substituents, including charge, size, and the like. Exemplary amino acid substitutions which take various of the foregoing characteristics into consideration are well known to those of skill in the art and include: arginine and lysine; glutamate and aspartate; serine and threonine; glutamine and asparagine; and valine, leucine and isoleucine.

Furthermore, a homologue may comprise addition or deletion of an amino acid, for example an addition or deletion of from 2 to 100 amino acids, such as from 2 to 50 amino acids, for example from 2 to 20 amino acids, such as from 2 to 10 amino acids, for example from 2 to 5 amino acids, such as from 2 to 3 amino acids. However, additions of more than 100 amino acids, such as additions from 100 to 500 amino acids, are also comprised within the present invention.

Proteins sharing at least some homology with a preferred protein are to be considered as falling within the scope of the present invention when they are at least about 40 percent homologous, or preferably identical, with the preferred protein, such as at least about 50 percent homologous, or preferably identical, for example at least about 60 percent homologous, or preferably identical, such as at least about 70 percent homologous, or preferably identical, for example at least about 75 percent homologous, or preferably identical, such as at least about 80 percent homologous, or preferably identical, for example at least about 85 percent homologous, or preferably identical, such as at least about 90 percent homologous, or preferably identical, for example at least 92 percent homologous, or preferably identical, such as at least 94 percent homologous, or preferably identical, for example at least 95 percent homologous, or preferably identical, such as at least 96 percent homologous, or preferably identical, for example at least 97 percent homologous, s or preferably identical, uch as at least 98 percent homologous, or preferably identical, for example at least 99 percent homologous, or preferably identical, with the preferred protein.

Preferred proteins are complement activating proteins comprising collectins and lectins and homologues hereof.

### Homologues of collectins

A homologue of a collectin including MBL within the scope of the present invention should be understood as any protein capable of exerting a function similar to the function of a collectin and comprising one or more of the variations described above. In particular such function is the ability to activate complement upon binding to one or more carbohydrates.

The terms functional homologues of collectin used herein relate to functional equivalents or a fragment of collectin comprising a predetermined amino acid sequence, and such homologues are defined as:
a) A homologue comprising an amino acid sequence capable of recognising and binding to glucans, lipophosphoglycans and glycoinositol phospholipids that contain sugar with 3- and 4-hydroxyl groups in the pyranose ring (i.e. Man, Glc, Fuc or GlcNAc) either alone or when being subunit complexed as described above and/or
b) A homologue comprising an amino acid sequence capable of forming an association with a component of the Lectin/MBL pathway such as binding to the MASP-1, MASP-2, MASP-3 and/or sMAP either alone or when being subunit complexed as described above, wherein said binding result in activation of the Lectin/MBL pathway and/or
c) A homologue comprising an amino acid sequence capable of by the collagen-like domain forming an oligomeric structure of two or more subunits, where a subunit comprises three identical polypeptides of a cysteine-rich region, a collagen-like domain, a neck region and a carbohydrate recognition domain.

### Homoloques of lectins

A homologue of a lectin including ficolins within the scope of the present invention should be understood as any protein capable of exerting a function similar to the function of a lectin and comprising one or more of the variations previously described. In particular such function is the ability to activate complement upon binding to one or more carbohydrates.

The terms functional homologues of lectin used herein relate to functional equivalents of a fragment of lectin comprising a predetermined amino acid sequence, and such homologues are defined as:
a) A homologue comprising an amino acid sequence capable of recognising and binding to N-acetyl-glucosamine (GlcNAc), or N-acetyl-galactosamine (GalNAc), or elastin either alone or when being subunit complexed as described above and/or
b) A homologue comprising an amino acid sequence capable of forming an association with a component of the Lectin/MBL pathway such as binding to the MASP-1, MASP-2, MASP-3 and/or sMAP either alone or when being subunit complexed as described above, wherein said binding result in activation of the Lectin/MBL pathway and/or
c) A homologue comprising an amino acid sequence capable of by the collagen-like domain forming an oligomeric structure of two or more subunits, where a subunit comprises three identical polypeptides of a cysteine-rich region, a collagen-like domain, a neck region and a fibrinogen-like domain.

The activation of the lectin/MBL pathway, i.e. the activity of the fusion protein to activate the complement system may be assessed by assessing the C4 cleaving effect of the fusion protein or subunit complexes or oligomers of complexes thereof by the following method comprising the steps of
- applying a sample comprising a predetermined amount of fusion protein as well as a predetermined amount of MASP-1, MASP-2 or MASP-3,
- applying at least one complement factor to the sample,
- detecting the amount of cleaved complement factors,
- correlating the amount of cleaved complement factors to the amount of fusion protein , and
- determining the activity of the fusion protein.

The complement factor preferably used in the present method is a complement factor cleavable by the MBUMASP-2 complex, such as C4. However, the complement factor may also be selected from C3 and C5.

The cleaved complement factor may be detected by a variety of means, such as by of antibodies directed to the cleaved complement factor.

The assay is carried out at conditions which minimize or eliminate interference from the classical complement activation pathway and the alternative complement activation pathway.

Preferably a homologue of a collectin and/or a lectin exhibits two of the functions defined above, more preferably three of the functions defined above.

### Preparation of fusion protein

The fusion protein may be prepared by any suitable method known to the person skilled in the art. Below are described several of the methods for preparing the fusion protein, however the invention is not limited to those methods.

### Synthetic preparation

When appropriate, in particular in relation to the size of the fusion protein, the fusion protein may be produced synthetically. The methods for synthetic production of peptides are well known in art. Detailed descriptions as well as practical advice for producing synthetic peptides may be found in Synthetic Peptides: A User's Guide (Advances in Molecular Biology), Grant G. A. ed., Oxford University Press, 2002, or in: Pharmaceutical Formulation: Development of Peptides and Proteins, Frokjaer and Hovgaard eds., Taylor and Francis, 1999.

### Recombinant preparation

The fusion proteins of the invention are preferably produced by use of recombinant DNA technologies. The DNA sequence encoding each part of the fusion protein may be prepared by fragmentation of the DNA sequences encoding the full-length protein, (genomic DNA or cDNA) which the fusion protein part is derived from, using DNAase I according to a standard protocol (Sambrook et al., Molecular cloning: A Laboratory manual. 2 rd ed., CSHL Press, Cold Spring Harbor, NY, 1989). The obtained DNA sequences encoding the individual parts of the fusion protein may then be fused together.

The DNA sequence may also be prepared by polymerase chain reaction using specific primers, for instance as described in US 4,683,202 or Saiki et al., 1988, Science 239:487-491.

The DNA sequence encoding a fusion protein of the invention may be prepared synthetically by established standard methods, e.g. the phosphoamidine method described by Beaucage and Caruthers, 1981, Tetrahedron Lett. 22:1859-1869, or the method described by Matthes et al., 1984, EMBO J. 3:801-805. According to the phosphoamidine method, oligonucleotides are synthesized, e.g. in an automatic DNA synthesizer, purified, annealed, ligated and cloned in suitable vectors.

The DNA sequence is then inserted into a recombinant expression vector, which may be any vector, which may conveniently be subjected to recombinant DNA procedures. The choice of vector will often depend on the host cell into which it is to be introduced. Thus, the vector may be an autonomously replicating vector, i.e. a vector that exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding a fusion protein should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the coding DNA sequence in mammalian cells are the SV 40 promoter (Subramani et al., 1981, Mol. Cell Biol. 1:854-864), the MT-1 (metallothionein gene) promoter (Palmiter et al., 1983, Science 222: 809-814) or the adenovirus 2 major late promoter. A suitable promoter for use in insect cells is the polyhedrin promoter (Vasuvedan et al., 1992, FEBS Lett. 311:7-11). Suitable promoters for use in yeast host cells include promoters from yeast glycolytic genes (Hitzeman et al., 1980, J. Biol. Chem. 255:12073-12080; Alber and Kawasaki, 1982, J. Mol. Appl. Gen. 1: 419-434) or alcohol dehydrogenase genes (Young et al., 1982, in Genetic Engineering of Microorganisms for Chemicals, Hollaender et al, eds., Plenum Press, New York), or the TP11 (US 4,599,311) or ADH2-4c (Russell et al., 1983, Nature 304:652-654) promoters. Suitable promoters for use in filamentous fungus host cells are, for instance, the ADH3 promoter (McKnight et al., 1985, EMBO J. 4:2093-2099) or the tpiA promoter.

The coding DNA sequence may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector may further comprise a DNA sequence enabling the vector to replicate in the host cell in question. An example of such a sequence (when the host cell is a mammalian cell) is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hydromycin or methotrexate.

The procedures used to ligate the DNA sequences coding the fusion proteins, the promoter and the terminator, respectively, and to insert them into suitable vectors containing the information necessary for replication, are well known to persons skilled in the art (cf., for instance, Sambrook et al., op.cit).

The synthesis of the recombinant fusion protein may be by use of *in vitro* or *in vivo* cultures. The host cell culture is preferably an eucaryotic host cell culture. By transformation of an eukaryotic cell culture is in this context meant introduction of recombinant DNA into the cells. The expression construct used in the process is characterised by having the encoding region selected from mammalian genes including human genes and genes with big resemblance herewith such as the genes from the chimpanzee. The expression construct used is furthermore featured by the promoter region being selected from genes of virus or eukaryotes, including mammalian cells and cells from insects.

The process for producing recombinant MBL according to the invention is characterised in that the host cell culture is preferably eukaryotic, and for example a mammalian cell culture. A preferred host cell culture is a culture of human kidney cells and in an even more preferred form the host cell culture is a culture of human embryonal kidney cells (HEK cells), such as HEK 293 cell lines for production of recombinant human MBL. By "HEK 293 cell lines" is meant any cell line derived from human embryonal kidney tissue such as, but not limited to, the cell lines deposited at the American Type Culture Collection with the numbers CRL-1573 and CRL-10852.

Other cells may be chick embryo fibroblast; hamster ovary cells, baby hamster kidney cells, human cervical carcinoma cells, human melanoma cells, human kidney cells, human umbilical vascular endothelium cells, human brain endothelium cells, human oral cavity tumor cells, monkey kidney cells, mouse fibroblast, mouse kidney cells, mouse connective tissue cells, mouse oligodendritic cells, mouse macrophage, mouse fibroblast, mouse neuroblastoma cells, mouse pre-B cell, mouse B lymphoma cells, mouse plasmacytoma cells, mouse teratocacinoma cells, rat astro-cytoma cells, rat mammary epithelium cells, COS, CHO, BHK, VERO, HeLa, MDCK, WI38, and NIH 3T3 cells.

Alternatively, fungal cells (including yeast cells) may be used as host cells. Examples of suitable yeast cells include cells of *Saccharomyces spp. or Schizosaccharomyces spp.,* in particular strains of *Saccharomyces cerevisiae.* Examples of other fungal cells are cells of filamentous fungi, e.g. *Aspergillus spp.* or *Neurospora spp.,* in particular strains of *Aspergillus oryzae* or *Aspergillus niger.* The use of *Aspergillus spp*. for the expression of proteins is described in, e.g., EP 238 023.

In addition, a host cell strain may be chosen which modulates the expression of the inserted sequences, or modifies and processes the gene product in the specific fashion desired. Such modifications (for example, glycosylation) and processing (for example, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. The mammalian cell types listed above are among those that could serve as suitable host cells.

Methods of transfecting mammalian cells and expressing DNA sequences introduced in the cells are described in e.g. Kaufman and Sharp, J. Mol. Biol. 159, 1982, pp. 601-621; Southern and Berg, 1982, J. Mol. Appl. Genet. 1:327-341; Loyter et al., 1982, Proc. Natl. Acad. Sci. USA 79: 422-426; Wigler et al., 1978, Cell 14:725; Corsaro and Pearson, 1981, in Somatic Cell Genetics 7, p. 603; Graham and van der Eb, 1973, Virol. 52:456; and Neumann et al., 1982, EMBO J. 1:841-845.

Other eucaryotic production systems are also envisaged by the present invention, such as the production of the fusion protein in a transgenic plant or non-human animal.

In another aspect the present invention provides a method for producing a fusion protein by
- preparing a gene expression construct as defined above encoding a fusion protein,
- transforming a host cell culture with the construct,
- cultivating the host cell culture, thereby obtaining expression and secretion of the polypeptide into the culture medium, followed by
obtaining a culture medium comprising recombinant fusion protein, and
purifying the fusion protein.

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g. in catalogues of the American Type Culture Collection). Example of culture medium are RPMI-1640 or DMEM supplemented with, e.g., insulin, transferrin, selenium, and foetal bovine serum

The fusion proteins recombinantly produced by the cells may then be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. HPLC, ion exchange chromatography, affinity chromatography, or the like.

In a preferred embodiment the fusion protein is purified by use of carbohydrate affinity chromatography as described above. In a preferred embodiment of the invention the affinity chromatography is performed by means of matrices of mannose, hexose or N-acetyl-glucoseamin derivatized matrices, which are suitable for affinity chromatography. In particular, an affinity chromatography is used, in which the matrices have been derivatizised with mannose.

Purified recombinant fusion protein is in this context to be understood as recombinant fusion protein purified from cell culture supernatants or body fluids or tissue from transgenic animals purified by use of for example carbohydrate affinity chomatography.

After application of the culture media the column is washed, preferably by using non-denaturing buffers, having a composition, pH and ionic strength resulting in elimination of proteins, without eluting the fusion protein. Such a buffer may be TBS. Elution of fusion protein is performed with a selective desorbing agent, capable of efficient elution of fusion protein, such as TBS containing a desorbing agent, such as EDTA (5 mM for example) or mannose (50 mM for example), and fusion proteins are collected.

### Pharmaceutical composition and treatment

The fusion protein obtained by the present invention may be used for the preparation of a pharmaceutical composition for the prevention and/or treatment of various diseases or conditions. In the present context the term pharmaceutical composition is used synonymously with the wording medicament.

In addition to the fusion protein, the pharmaceutical composition may comprise a pharmaceutically acceptable carrier substance and/or vehicles.

In particular, a stabilising agent may be added to stabilise the fusion proteins. The stabilising agent may be a sugar alcohol, saccharide, protein and/or amino-acids. An example of a stabilising agent may be albumin or maltose.

Other conventional additives may be added to the pharmaceutical composition depending on administration form for example.

In one embodiment the pharmaceutical composition is in a form suitable for injections. Conventional carrier substances, such as isotonic saline, may be used.

In another embodiment the pharmaceutical composition is in a form suitable for pulmonal administration, such as in the form of a powder for inhalation or creme or fluid for topical application.

A treatment in this context may comprise cure and/or prophylaxis of e.g. the immune system and reproductive system by humans and by animals having said functional units acting in this respect like those in humans. By conditions to be treated are not necessarily meant conditions presently known to be in a need of treatment, but comprise generally any condition in connection with current and/or expected need or in connection with an improvement of a normal condition. In particular, the treatment is a treatment of a condition of deficiency of lectins, such as MBL deficiency.

In another aspect of the present invention the manufacture is provided of a medicament consisting of said pharmaceutical compositions of fusion protein intended for treatment of conditions comprising cure and/or prophylaxis of conditions of diseases and disorders of e.g. the immune system and reproductive system by humans and by animals having said functional units acting like those in humans.

Said diseases, disorders and/or conditions in need of treatment with the compounds of the invention comprise eg treatment of conditions of deficiency of MBL, treatment of cancer and of infections in connection with immunosuppressive chemotherapy including in particular those infections which are seen in connection with conditions during cancer treatment or in connection with implantation and/or transplantation of organs. The invention also comprises treatment of conditions in connection with recurrent miscarriage.

Thus, in particular the pharmaceutical composition may be used for the treatment and/or prevention of clinical conditions selected from infections, MBL deficiency, cancer, disorders associated with chemotherapy, such as infections, diseases associated with human immunodeficiency virus (HIV), diseases related with congenital or acquired immunodeficiency. More particularly, chronic inflammatory demyelinating polyneuropathy (CIDP, Multifocal motoric neuropathy, Multiple scelrosis, Myasthenia Gravis, Eaton-Lambert's syndrome, Opticus Neuritis, Epilepsy; Primary antiphosholipid syndrome; Rheumatoid arthritis, Systemic Lupus erythematosus, Systemic scleroderma, Vasculitis, Wegner's granulomatosis, Sjogren's syndrome, Juvenile rheumatiod arthritis; Autoimmune neutropenia, Autoimmune haemolytic anaemia, Neutropenia; Crohn's disease, Colitis ulcerous, Coeliac disease; Asthma, Septic shock syndrome, Chronic fatigue syndrome, Psoriasis, Toxic shock syndrome, Diabetes, Sinuitis, Dilated cardiomyopathy, Endocarditis, Atherosclerosis, Primary hypo/agammaglobulinaemia including common variable immunodeficiency, Wiskot-Aldrich syndrome and serve combined immunodefiency (SCID), Secondary hypo/agammaglobulinaemia in patients with chronic lymphatic leukaemia (CLL) and multiple myeloma, Acute and chronic idiopathic thrombocytopenic purpura (ITP), Allogenic bone marrow transplantation (BTM), Kawasaki's disease, and Guillan-Barre's syndrome.

The route of administration may be any suitable route, such as intravenously, intra-musculary, subcutanously or intradermally. Also, pulmonal or topical administration is envisaged by the present invention.

In particular the fusion protein may be administered to prevent and/or treat infections in patients having clinical symptoms associated with congenital or acquired MBL deficiency or being at risk of developing such symptoms. A wide variety of conditions may lead to increased susceptibility to infections in MBL-deficient individuals; such as chemotherapy or other therapeutic cell toxic treatments, cancer, AIDS, genetic disposition, chronic infections, and neutropenia.

The pharmaceutical composition may thus be administered for a period before the onset of administration of chemotherapy or the like and during at least a part of the chemotherapy.

The fusion protein may be administered as a general "booster" before chemotherapy, or it may be administered to those only being at risk of developing MBL deficiency. The group of patients being at risk may be determined be measuring the MBL level before treatment and only subjecting those to treatment whose MBL level is below a predetermined level.

The fusion protein is administered in suitable dosage regimes, in particularly it is usually administered at suitable intervals, eg. once or twice a week during chemotherapy.

Normally from 1-100 mg is administered per dosage, such as from 2-10 mg, mostly from 5-10 mg per dosage. Mostly about 0.1 mg/kg body weight is administered.

Furthermore, an aspect of the present invention is the use of a recombinant composition according to the present invention in a kit-of-parts further comprising another medicament. In particular the other medicament may be an anti-microbial medicament, such as antibiotics.

Concerning miscarriage, it has been reported that the frequency of low plasma levels of MBL is increased in patients with otherwise not explained recurrent miscarriages, which is the background for lowering of the susceptibility to miscarriage by a reconstitution of the MBL level by administration of recombinant MBL in these cases.

As to the nature of compounds of the invention, it appears, that in its broad aspect, the present invention relates to compounds which are able to act as opsonins, that is, able to enhance uptake by macrophages either through direct interaction between the compound and the macrophage or through mediating complement deposition on the target surface.

### Examples

### Example 1

### Plasmidcloning of FCNMBL-r1, -r2, -r3,-r4,-r5,-r6 and-r7.

### 1.1 Summary

A series of plasmids were constructed for the expression in mammalian cells of protein fusions between recombinant human mannose-binding lectin 2 gene (rhMBL) and human ficolin 2 (FCN2). The vector is derived from a high-copy-number ColE1-based plasmid and is designed to allow protein expression in mammalian systems. The fusion protein expressions are driven by the human cytomegalovirus (CMV) immediate early promoter to promote constitutive expression. Selection is made possible in bacteria by the ampicillin-resistance gene under control of the prokaryotic β-lactamase promoter. The neomycin-resistance gene is driven by the SV40 early promoter, which provides stable selection with G418 in mammalian cells.

### 1.2 Constructs and experimental work

In order to express fusion proteins between Ficolin2 and MBL we have designed and constructed a series of plasmids. The new recombinant plasmids are based on the previously cloned pcDNA2001-cintMBLcDNA. This plasmid contains a synthetic intron together with the cDNA for human MBL. The following fusions were designed (underlined font indicates FCN2 part - *italics* indicate MBL part of the fusion protein.)

### FCN2MBLr1 (SEQ ID NO:118):

FCN2 (signalseq+ collagen+"hinge" to ficolin dom aa131) MBL (from aa129 carbohyd.bind dom.)
MELDRAVGVLGAATLLLSFLGMAWALQAADTCPEVKMVGLEGSDKLTILRGCP-GLPGAPGDKGEAGTNGKRGERG
PPGPPGKAGPPGPNGAPGEPQPCLTGPRTCKDLLDRGHFLSGWHTIYLPDCR-P*LTFSLGKQVGNKFFLTNGEIMT*
*FEKVKALCVKFQASVATPRNAAENGAIQNLIKEEAFLGITDEKTEGQFVDLTGN-RLTYTNWNEGEPNNAGSDEDC*
*VLLLKNGQWNDVPCSTSHLAVCEFPI*

### FCN2MBLr2 (SEQ ID NO: 119):

FCN2 (signalseq+ collagen+"hinge"+part of ficolin-dom. containing pred. coil-coil to aa207) MBL (from aa129 carbohyd.bind dom.)
MELDRAVGVLGAATLLLSFLGMAWALQAADTCPEVKMVGLEGSDKLTILRGCP-GLPGAPGDKGEAGTNGKRGERG
PPGPPGKAGPPGPNGAPGEPQPCLTGPRTCKDLLDRGHFLSGWHTIYLPDCR-PLTVLCDMDTDGGGWTVFQRRVD
GSVDFYRDWATYKQGFGSRLGEFWLGNDNIHALTAQGTSELRVDLVDFEDNY-QFAK*LTFSLGKQVGNKFFLTNGE*
*IMTFEKVKALCVKFQASVATPRNAAENGAIQNLIKEEAFLGITDEKTEGQFVDLT-GNRLTYTNWNEGEPNNAGSD*
*EDCVLLLKNGQWNDVPCSTSHLAVCEFPI*

### FCN2MBLr3 (SEQ ID NO: 120):

FCN2 (signalseq+ collagen to aa92) MBL (from aa101 coil-coil + carbohyd.bind dom.)
MELDRAVGVLGAATLLLSFLGMAWALQAADTCPEVKMVGLEGSDKLTILRGCP-GLPGAPGDKGEAGTNGKRGERG
PPGPPGKAGPPGPNGAP*PDGDSSLAASERKALQTEMARIKKWLTFSLG-KQVGNKFFLTNGEIMTFEKVKALCVKF*
*QASVATPRNAAENGAIQNLIKEEAFLGI TDEKTEGQFVDLTGNRLTYTN-WNEGEPNNAGSDEDCVLLLKNGQWND*
*VPCSTSHLAVCEFPI*

### FCN2MBLr4 (SEQ ID NO: 121):

FCN2 (signalseq+ part of collagen to cons.K at aa93) MBL (from cons.K at aa77 rest of collagen+coil-coil + carbohyd.bind dom.)
MELDRAVGVLGAATLLLSFLGMAWALQAADTCPEVKMVGLEGSDKLTILRGCP-GLPGAPGDKGEAGTNGKRGERG
PPGPPGKLGPPGNPGPSGSPGPKGQKGDPGKSPDGDSSLAASERKALQTEMA-*RIKKINLTFSLGKQVGNKFFLTNG*
*EIMTFEKVKALCVKFQASVATPRNAAENGAIQNLIKEEAFLGITDEKTEGQFVDLT-GNRLTYTNWNEGEPNNAGS*
*DEDCVLLLKNGQWNDVPCSTSHLAVCEFPI*

### FCN2MBLr5 (SEQ ID NO: 122):

FCN2 (signalseq+ part of collagen to cons.G at aa69) MBL (from cons.G at aa.64 rest of collagen(containing "kick")+coil-coil + carbohyd.bind dom.)
MELDRAVGVLGAATLLLSFLGMAWALQAADTCPEVKMVGLEGSDKLTILRGCP-GLPGAPGDKGEAGTNGQG*LRGL*
*QGPPGKLGPPGNPGPSGSPGPKGQKGDPGKSPDGDSSLAASERKALQTEMA-RIKKWLTFSLGKQVGNKFFLTNGE*
*IMTFEKVKALCVKFQASVATPRNAAENGAIQNLIKEEAFLGITDEKTEGQFVDLT-GNRLTYTNWNEGEPNNAGSD*
*EDCVLLLKNGQWNDVPCSTSHLAVCEFPI*

### FCN2MBLr6 (SEQ ID NO: 123):

MBL (replaced MBLcollagen(aa.41 to aa 99)+coil-coil + carbohyd.bind dom.) FCN2 (inserted collagen aa.54 to aa.92)
*MSLFPSLPLLLLSMVAASYSETVTCEDAQKTCPAVIACSSP**GCPGLPGAPGDK-*GEAGTNGKRGERGPPGPPGKAG
PPGPNGAP*SPDGDSSLAASERKALQTEMARIKKWLTFSLGKQVGNKFFLT-NGEIMTFEKVKALCVKFQASVATPR*
*NAAENGAIQNLIKEEAFLGITDEKTEGQFVDLTGNRLTYTNWNEGEPNNAGSDED-CVLLLKNGQWNDVPCSTSHL*
*AVCEFPI*

### FCN2MBLr7 (SEQ ID NO: 124):

MBL (signal seq. to aa.25)FCN2 (collagen to aa93) MBL (from aa100 coil-coil + carbohyd.bind dom.)
*MSLFPSLPLLLLSMVAASYS**ALQAADTCPEVKMVGLEGSDKLTILRGCPGLPGAP-*GDKGEAGTNGKRGERGPPGP
PGKAGPPGPNGAP*SPDGDSSLAASERKALQTEMARIKKWLTFSLGKQVGNKF-FLTNGEIMTFEKVKALCVKFQAS*
*VATPRNAAENGAIQNLIKEEAFLGITDEKTEGQFVDLTGNRLTYTNWNEGEPN-NAGSDEDCVLLLKNGQWNDVPC*
*STSHLAVCEFPI*

### Parental plasmids used for all constructions :

- pcDNA2003-cintMBLcDNA
- Invitrogen Genestorm clone RG000632 (Cat. No. H-K1000 Invitrogen). Constructions were done by recombination using the BD In-Fusion^{™} PCR Cloning Kit form BD (Cat. No. 631774). The BD In-Fusion Kit allows the cloning of PCR products based only on 2 x 15 bp homology between vector and end of the PCR product. Ligase, or phosphatase are unnecessary when cloning with this kit. The In-Fusion enzyme captures the DNA fragment ends and fuses the insert to the vector.

### Primers used for the PCR reactions are shown in table 1.

### PCR reactions and linearization of vector for recombination

PCR reactions were done on plasmid "Genestorm RG000632" batch N135-15C digested with Bstz17I (N135-20B). Primers pairs were used as described below. Kit for PCR reactions : PfuUltra^{™} Hotstart PCR Master Mix Stratagene #600630. The PCR reaction tubes were run on the BioRAD i-cycler using the temperature profile shown in table 2.

For the recombination reactions the vector pcDNA2001-cintMBLcDNA was linearized by restriction enzyme digestion with the enzymes listed below.

### FCN2MBLr1:

PCR using primers : Pr1-xho-MBLFCN + Pr4-Xmn-FCNMBL-rev (product 463 bp) Digest of pcDNA2001-cintMBLcDNA : Xhol + Xmnl (partial)

### FCN2MBLr2:

PCR USING PRIMERS : Pr1-xho-MBLFCN + Pr5-Xmn-FCNMBL-rev
Digest of pcDNA2001-cintMBLcDNA : Xhol + Xmnl (partial)

### FCN2MBLr3:

PCR USING PRIMERS : Pr1-xho-MBLFCN + Pr6-b-Bsp-FCNMBL-rev
Digest of pcDNA2001-cintMBLcDNA : Xhol + BspEl

### FCN2MBLr4:

PCR USING PRIMERS : Pr1-xho-MBLFCN + Pr2-apa-FCNNIBL-rev
Digest of pcDNA2001-cintMBLcDNA: Xhol + Apal

### FCN2MBLr5:

PCR USING PRIMERS : Pr1-xho-MBLFCN + Pr3-apa-FCNMBL-rev
Digest of pcDNA2001-cintMBLcDNA : Xhol + Apal

### FCN2MBLr6:

PCR USING PRIMERS : Pr8-BstAP-MBLFCN + Pr6-b-Bsp-FCNMBL-rev
Digest of pcDNA2001-cintMBLcDNA : partial BstAPI + BspEl

### FCN2MBLr7:

PCR USING PRIMERS : Pr7-Alw-MBLFCN + Pr6-b-Bsp-FCNMBL-rev
Digest of pcDNA2001-cintMBLcDNA :partial AlwNI + BspEl

### In-Fusion PCR recombination reactions

In-Fusion PCR recombination reactions were set up using approx. 50-100 ng of Quiagen Minelute purified PCR products together with 50-100 ng of Quiagen Minelute purified linearized pcDNA2001-cintMBLcDNA.
1/10 of the recombination reactions were transformed into MAX efficiency DH5α Competent Cells (Invitrogen Cat. No. 18258-012). 1/10 and 9/10 from each transformation were spread on separate LB plates containing 200 ug/ml ampicillin. Plates were incubated at 37°C overnight. Screening for positive clones : At least 6 colonies from each experimental plate were picked for miniprep plasmid DNA isolation. To determine the presence of insert, DNA was analyzed by restriction digest analysis with the enzyme *Pst*I. Three individual positive clones from each reaction were chosen for further work.

### Restriction Analysis

In order to verify the selected individual recombinant plasmids after the primary screen we performed an intensive restriction enzyme digestion analysis on the plasmid DNA isolated.
Plasmid DNA of the recombinants were digested with the enzyme shown in table 3. The expected fragments are also listed in the table. All recombinant clones tested exhibited the expected pattern. Digestion with EcoRl was not as predicted. An additional fragment was observed both in digestion of the recombinant as well of the parental plasmid. The discrepancy can be explained by an additional EcoR1 site on the parental plasmid.

### Results

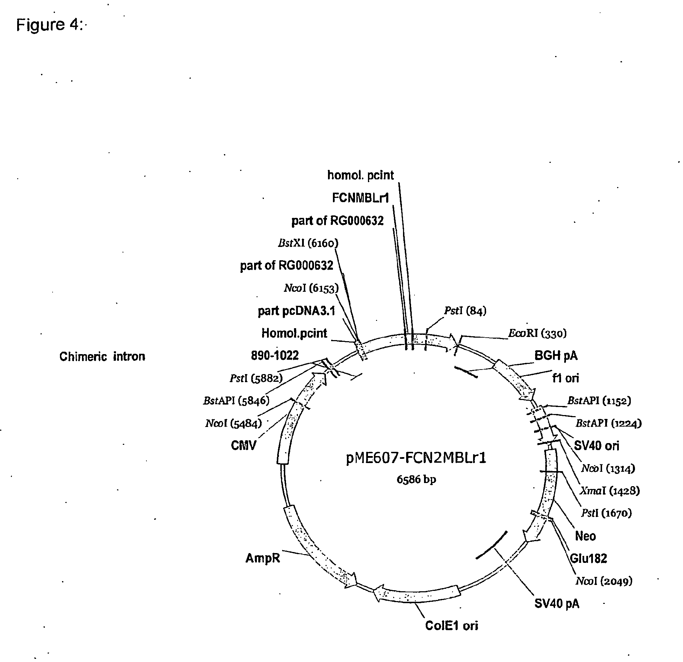

Recombinant plasmids obtained are shown schematically in figures 4-8 for constructs r1, -r2, -r3,-r4, and -r5.

### Example 2

### Experiments with transient expression of recombinant fusion proteins of human MBL and human FCN2

### 2.1 Summary

We report the expression of recombinant human fusion proteins FCNMBLr1, FCNMBLr4, FCNMBLr5 and MBL in HEK293 and Per.C6 cells. We found that the cell lines in the transient transfection experiment were able to produce at least the fusion proteins FCNMBLr4 and FCNMBLr5 assembled in active oligomeres with a structure primarly similar to MBL oligomer forms 3 and 4. The fusion proteins FCNMBLr4 and FCNMBLr5 behaved like MBL upon binding to a carbohydrate surface and upon activating the complement cascade.

### 2.2 Introduction

The aim of the studies was to elucidate the possibility of creating a hybrid protein consisting of the collagen part of human ficolin 2 and the human mannose binding lectin (MBL). Furthermore we wished to clarify if such molecules would still posses the ability to bind to complex carbohydrate structures and still are able to activate complement.
Two eukaryotic cell lines of human origin HEK293 and Per.C6 were used as host cell lines for transient transfections with the respective expression plasmids. Transcription was driven by the-CMV-IE promoter enhancer.

### 2.3 Experimental

### Material and Methods

### Plasmids used for the transfection experiments

pME607-FCNMBL-r1, -r2, -r3,-r4,-r5,-r6 and-r7 (described in example 1)

### Origin of Cells used

PerC6 cells were obtained from Crucell.
HEK 293 Freestyle cells were obtained from InVitrogen.

### Culture media

PerC6 cells were cultured at 37°C in 10% (vol/vol) CO₂ maintained as monolayers in serum free medium.
HEK 293 Freestyle were cultured at 37°C in 8% (vol/vol) CO₂ maintained as suspension in an InVitrogen Freestyle medium.

### Transfections and harvest of media

Per.C6 cells in serum containing medium were transfected with the DNA using the transfection reagent Lipofectamine. One day after transfection the medium was replaced with serum free medium.
HEK293 cells in serum free Freestyle medium were transfected with the DNA using the transfection reagent 293fect. The medium was collected after approximately 4 days of incubation after transfection.

### Quantification of MBL

Recombinant MBL assay (TRIFMA) using Mannan coated plates or mAb-131-01 coated plates. For quantification of MBL, time-resolved immunofluorometry was carried out.

### SDS-PAGE and Western blot analysis

SDS-PAGE with subsequent electrophoretic transfer of proteins to polyvinylidene diflouride membranes and detection of MBL using monoclonal anti-MBL antibody was carried out.

### C4 assay

The assay is designed to measure MBL and rMBL abilities to initiate the MBL Lectin-pathway of the complement system. MBL associated serine protease (MASP 2) associated with MBL cleaves the complement factor C4 releasing C4a and C4b.
The C4b deposition on the Mannan coated ELISA plates is detected with biotin labelled antibodies against C4b and Europium labelled Streptavidin.

### 2.4 RESULTS

In the experiments described herein we were able to express FCN2MBLr4, FCN2MBLr5 and MBL transiently in both HEK293 and Per.C6 cells under serum free conditions.

### Oligomeric form of the fusion proteins

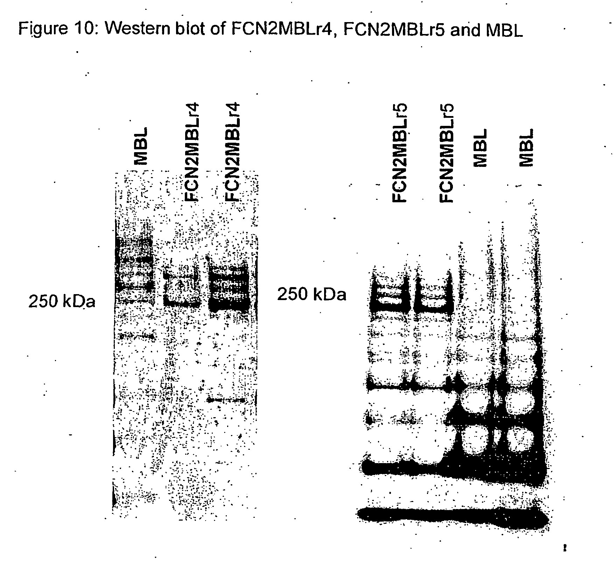

The oligomeric forms of the fusion protein were examined by non-reducing denaturizing SDS PAGE followed by a western blot. The detecting antibody recognizes the CDR part of MBL (and maybe part of the coil-coil region). The results are shown in figure 10. It is evident from the figure that the most prominent form of the fusion proteins FCN2MBLr4 and FCN2MBLr5 is approximately 250 kDa corresponding to a 3- or 4-mere of subunits consisting of 3 single protein chains (24 kDa). The appearance of the oligomeric form was independent of the host cells used. MBL was produced in a wide range of oligomeric forms.

### Binding properties

The fusion proteins were tested for functionality of the MBL carbohydrate binding domain by binding to a mannan surface and detection with an antibody that recognizes the CDR part of MBL (and maybe part of the coil-coil region). The results are shown in table 4. It can be concluded that FCN2MBLr4 and FCN2MBLr5 were expressed just as well as MBL in the host cells and that the fusion proteins bind to a mannan surface.

### MASP-2 binding and C4 cleavge

The fusion proteins were further tested for the capacity to bind MASP-2 and for activating the serine protease of MASP-2. This was done by measuring cleavage of the MASP-2 substrate complement factor C4 upon binding of the fusion protein to a mannan surface. Results are shown in table 5. It can be concluded from these results that the fusion proteins FCN2MBLr4 and FCN2MBLr5 preserved the ability to bind and activate MASP-2.

### Discussion

The results described herein clearly demonstrate that it is possible to construct fusion proteins of FCN 2 and MBL with the following properties:
1. The oligomeric structure of the fusion proteins is more simple than that of the MBL protein.
2. The fusion proteins keep the essential property of MBL activation of the complement cascade upon binding to a dense carbohydrate structure.

**Table 1. Primers used for the PCR reactions**

| **Sequence typed in bold shows the 15 bp homology needed for the recombination into the vector.** | | | |
|---|---|---|---|
| Primer name | DNA Sequence of Primer | Primer part of pcDNA2001-cintMBLcdna | Primer part of RG000632 |
| **Pr1-xho-MBLFCN** | | **ataggctagcctcga** | |
| **Pr2-apa-FCNMBL-rev** | | | |
| **Pr3-apa-FCNMBL-rev** | | | |
| **Pr4-Xmn-FCNMBL-rev** | | | |
| **Pr5-Xmn-FCNMBL-rev** | | | |
| **Pr6-b-Bsp-FCNMBL-rev** | | **ccggatggtgatagt** | |
| **Pr7-Alw-MBLFCN** | | **cagcgtcttactcag** | |
| **Pr8-BstAP-MBLFCN** | | | |

**Table 2:**

| Cycle | times | step | Temp | Time |
|---|---|---|---|---|
| 1 | 1x | 1 | 95° | 21 min |
| 2 | 30x | 1 | 95° | 0 min 30 sec |
| | | 2 | 72° (67.5° for r2) | 0 min 30 sec |
| | | 3 | 72° | 1 min |
| 3 | 1x | 1 | 72° | 10 min |
| 4 | 1x | 1 | 4° | ∞ |

**Table 3:**

| pcDNA200 1-cintMBLcD NA | pME607-FCN2M BLr1 | pME607-FCN2M BL r2 | pME607-FCN2M BL r3 | pME607-FCN2M BL r4 | pME607-FCN2M BL r5 |
|---|---|---|---|---|---|
| ***PstI*** | ***PstI*** | ***PstI*** | ***PstI*** | ***PstI*** | ***PstI*** |
| 4212 | 4212 | 4212 | 4212 | 4212 | 4212 |
| 1586 | 1586 | 1586 | 1486 | 1586 | 1586 |
| **405** | **788** | 1016 | 782 | 800 | 797 |
| 375 | | | | | |
| ***EcoRI*** | ***EcoRI*** | ***EcoRI*** | ***EcoRI*** | ***EcoRI*** | ***EcoRI*** |
| **5810** | 6586 | 6814 | 6580 | 6598 | 6595 |
| **768** | | | | | |
| ***XmaI*** | ***XmaI*** | ***XmaI*** | ***XmaI*** | ***XmaI*** | ***XmaI*** |
| 6578: | 6586 | 6814 | 6580 | 6589 | 6595 |
| ***BstXI*** | ***BstXI*** | ***BstXI*** | ***BstXI*** | ***BstXI*** | ***BstXI*** |
| undigested | **6586** | 6814 | 6580 | 6598 | 6595 |
| ***BstAPI*** | ***BstAPI*** | ***BstAPI*** | ***BstAPI*** | ***BstAPI*** | ***BstAPI*** |
| 4622 | 4622 | 4622 | 4622 | 4622 | 4622 |
| **1469** | **1892** | 2120 | 1886 | 1904 | 1901 |
| **415** | 72 | 72 | 72 | 72 | 72 |
| 72 | | | | | |
| ***Ncol*** | ***Ncol*** | ***Ncol*** | ***Ncol*** | ***Ncol*** | ***Ncol*** |
| 3435 | 3435 | 3435 | 3435 | 3435 | 3435 |
| **2408** | **1747** | 1975 | 1741 | 1759 | 1756 |
| 735 | 735 | 735 | 735 | 735 | 735 |
| | **669** | 669 | 669 | 669 | 669 |

**Table 4 MBL binding to mannan measured by TRIFMA**

| | | µg MBL equivalents /ml |
|---|---|---|
| FCN2MBLr5 | HEK293 | **0,689** |
| FCN2MBLr5 | HEK293 | **0,764** |
| FCN2MBLr1 | HEK293 | **0,874** |
| MBL | HEK293 | **0,457** |
| FCN2MBLr4 | HEK293 | **0,851** |
| MBL | HEK293 | **1,885** |
| FCN2MBLr5 | Per.C6 | **0,296** |
| MBL | Per.C6 | **0,271** |
| FCN2MBLr4 | Per.C6 | **0,077** |
| FCN2MBLr4 | Per.C6 | **0,091** |
| FCN2MBLr4 | Per.C6 | **0,089** |
| FCN2MBLr4 | Per.C6 | **0,035** |
| MBL | Per.C6 | **0,092** |

**Table 5 C4 activity of the fusion proteins**

| | **Cells** | **Aktivitet+/-** |
|---|---|---|
| pME607-FCNMBLr5 clone 1 | HEK293 | **+** |
| pME607-FCNMBLr5 clone 5 | HEK293 | **+** |
| pME607-FCNMBLr4 clone 2 | HEK293 | **+/+ (after purification)** |
| pcDNA2001-cintMBLcDNA | HEK293 | **+** |
| pME607-FCNMBLr5 clone 5 | Per.C6 | **+** |
| pME607-FCNMBLr4 clone 2 (maxi | Per.C6 | **-/(+) (after purification)** |

## Claims

1. A fusion protein comprising
i) A first polypeptide sequence derived from a lectin-complement pathway activating protein or a functional homologue at least 70% identical to said lectin-complement pathway activating protein, wherein said first polypeptide sequence is capable of activating the lectin-complement pathway; and
ii) A second polypeptide sequence derived from a collectin or a functional homologue at least 70% identical to said collectin, wherein said second polypeptide sequence is capable of associating with one or more carbohydrates;
wherein said complement activating protein is not a collectin.

2. The fusion protein according to claim 1, wherein said first polypeptide sequence is capable of associating with at least one MASP protein.

3. The fusion protein according to claim 1, wherein said first polypeptide sequence is capable of associating with a MASP protein selected from the group consisting of MASP-1, MASP-2 and MASP-3 or functional homologues or variants hereof.

4. The fusion protein according to claim 1, wherein the complement activating protein is a ficolin.

5. The fusion protein according to claim 4, wherein the ficolin is selected from the group consisting of L-ficolin, H-ficolin and M-ficolin.

6. The fusion protein according to claim 4, wherein the ficolin is L-ficolin.

7. The fusion protein according to claim 1, wherein the first polypeptide sequence comprises the collagen-like domain of a ficolin or a functional homologue or variant thereof.

8. The fusion protein according to claim 1, wherein the first polypeptide sequence comprises the cysteine-rich region of a ficolin or a functional homologue thereof.

9. The fusion protein according to claim 1, wherein the first polypeptide sequence comprises the cysteine-rich region and the collagen-like domain of a ficolin or a functional homologue or variant thereof.

10. The fusion protein according to claim 1, wherein the first polypeptide sequence comprises amino acids 1-77 of the L-ficolin sequence of figure 1 (SEQ ID. NO 125).

11. The fusion protein according to claim 1, wherein the collectin is selected from the group consisting of MBL (mannose-binding lectin), SP-A (lung surfactant protein A), SP-D (lung surfactant protein D), BK (or BC, bovine conglutinin) and CL-43 (collectin-43).

12. The fusion protein according to claim 15, wherein the collectin is MBL.

13. The fusion protein according to claim 1, wherein the second polypeptide sequence comprises the CRD domain of a collectin or a functional homologue or vaiant thereof.

14. The fusion protein according to claim 1, wherein the second polypeptide sequence comprises the CRD domain of MBL.

15. The fusion protein according to claim 1, wherein the second polypeptide sequence comprises the neck region of MBL.

16. The fusion protein according to claim 1, wherein the second polypeptide sequence comprises the collagen-like domain of MBL.

17. The fusion protein according to claim 1, wherein the second polypeptide sequence comprises the neck region and the CRD domain of MBL.

18. The fusion protein according to claim 1, wherein the second polypeptide sequence comprises the collagen-like domain, the neck region and the CRD domain of MBL.

19. The fusion protein according to claim 1, wherein the second polypeptide sequence comprises amino acids 80-228 of the MBL sequence shown in figure 2 (SEQ ID. NO 126).

20. The fusion protein according to claim 1, wherein the fusion protein comprises the the cysteine-rich region and the collagen-like domain of L-ficolin and the CRD domain of MBL.

21. The fusion protein according to claim 1, wherein the fusion protein comprises the cysteine-rich region of L-ficolin and the collagen-like domain, the neck region and the CRD domain of MBL.

22. The fusion protein according to claim 1, wherein the fusion protein comprises the amino acid sequence as defined by the sequence shown in figure 3 (SEQ ID. NO. 127), or a functional homologue at least 70% identical thereto.

23. The fusion protein according to claim 1, wherein the fusion protein consists of the amino acid sequence as defined by the sequence shown in figure 3 (SEQ ID. NO. 127).

24. An isolated nucleic acid comprising a nucleotide sequence encoding the fusion protein according to any of claims 1 to 28.

25. A vector comprising the nucleic acid sequence according to claim 29.

26. A cell comprising the vector according to claim 30.

27. A fusion protein according to any of claims 1 to 28 for use as a medicament.

28. Use of the fusion protein according to any of claims 1 to 28 for the preparation of a medicament for the prevention and/or treatment of an infection in an individual in need thereof.

29. The use according to claim 40, wherein the individual is a human being.

30. The use according to claim 40, wherein the individual is a human being suffering from an increased risk of acquiring an infection.

31. The use according to claim 40, wherein the individual is a human being with subnormal serum MBL level.

32. The use according to claim 40, wherein the individual is a human being with normal serum MBL level.

33. A medicament for the treatment or prevention of a clinical condition in an individual in need thereof, comprising the fusion protein according to any of claims 1 to 28.

34. The medicament according to claim 45, wherein the clinical condition is an infection.

35. The medicament according to claim 45, wherein the individual is a human being.

## Patentansprüche

1. Fusionsprotein umfassend:
i) eine von einem Aktivierungsprotein eines Lectin-Komplement-Übertragungsweges abgeleitete, erste Polypeptid-Sequenz, oder ein funktionelles Homolog, welches zu mindestens 70 % identisch ist zu dem Aktivierungsprotein des Lectin-Komplement-Übertragungsweges, worin die erste Polypeptid-Sequenz den Lectin-Komplement-Übertragungsweg aktivieren kann; und
ii) eine von einem Collectin abgeleitete, zweite Polypeptid-Sequenz, oder ein funktionelles Homolog, welches zu mindestens 70 % identisch ist zu dem Collectin, worin die zweite Polypeptid-Sequenz mit einem oder mehreren Kohlenhydraten assoziieren kann,
wobei das das Komplement aktivierende Protein kein Collectin ist.

2. Fusionsprotein nach Anspruch 1, worin die erste Polypeptid-Sequenz mit mindestens einem MASP-Protein assoziieren kann.

3. Fusionsprotein nach Anspruch 1, worin die erste Polypeptid-Sequenz mit einem MASP-Protein ausgewählt aus der Gruppe bestehend aus MASP-1, MASP-2 und MASP-3, oder funktionellen Homologen oder Varianten davon assoziieren kann.

4. Fusionsprotein nach Anspruch 1, worin das das Komplement aktivierende Protein ein Ficolin ist.

5. Fusionsprotein nach Anspruch 4, worin das Ficolin ausgewählt ist aus der Gruppe bestehend aus L-Ficolin, H-Ficolin und M-Ficolin.

6. Fusionsprotein nach Anspruch 4, worin das Ficolin L-Ficolin ist.

7. Fusionsprotein nach Anspruch 1, worin die erste Polypeptid-Sequenz die Kollagen-ähnliche Domäne eines Ficolins oder ein funktionelles Homolog oder eine Variante davon umfasst.

8. Fusionsprotein nach Anspruch 1, worin die erste Polypeptid-Sequenz die Cysteinreiche Region eines Ficolins oder ein funktionelles Homolog davon umfasst.

9. Fusionsprotein nach Anspruch 1, worin die erste Polypeptid-Sequenz die Cysteinreiche Region und die Kollagen-ähnliche Domäne eines Ficolins oder ein funktionelles Homolog oder eine Variante davon umfasst.

10. Fusionsprotein nach Anspruch 1, worin die erste Polypeptid-Sequenz die Aminosäuren 1-77 der L-Ficolin-Sequenz von Figur 1 (SEQ ID NO: 125) umfasst.

11. Fusionsprotein nach Anspruch 1, worin das Collectin ausgewählt ist aus der Gruppe bestehend aus MBL (Mannose bindendes Lectin), SP-A (Lungen-Oberflächenprotein A), SP-D (Lungen-Oberflächenprotein D), BK (oder BC, bovines Conglutinin) und CL-43 (Collectin-43).

12. Fusionsprotein nach Anspruch 11, worin das Collectin MBL ist.

13. Fusionsprotein nach Anspruch 1, worin die zweite Polypeptid-Sequenz die CRD-Domäne eines Collectins oder ein funktionelles Homolog oder eine Variante davon umfasst.

14. Fusionsprotein nach Anspruch 1, worin die zweite Polypeptid-Sequenz die CRD-Domäne von MBL umfasst.

15. Fusionsprotein nach Anspruch 1, worin die zweite Polypeptid-Sequenz den Rückgrat-Bereich von MBL umfasst.

16. Fusionsprotein nach Anspruch 1, worin die zweite Polypeptid-Sequenz die Collagen-ähnliche Domäne von MBL umfasst.

17. Fusionsprotein nach Anspruch 1, worin die zweite Polypeptid-Sequenz den Rückgrat-Bereich und die CRD-Domäne von MBL umfasst.

18. Fusionsprotein nach Anspruch 1, worin die zweite Polypeptid-Sequenz die Collagen-ähnliche Domäne, den Rückgrat-Bereich und die CRD-Domäne von MBL umfasst.

19. Fusionsprotein nach Anspruch 1, worin die zweite Polypeptid-Sequenz die Aminosäuren 80-228 der in Figur 2 gezeigten MBL-Sequenz (SEQ ID NO: 126) umfasst.

20. Fusionsprotein nach Anspruch 1, worin das Fusionsprotein den Cystein-reichen Bereich und die Collagen-ähnliche Domäne von L-Ficolin und die CRD-Domäne von MBL umfasst.

21. Fusionsprotein nach Anspruch 1, worin das Fusionsprotein den Cystein-reichen Bereich von L-Ficolin und die Collagen-ähnliche Domäne, den Rückgrat-Bereich und die CRD-Domäne von MBL umfasst.

22. Fusionsprotein nach Anspruch 1, worin das Fusionsprotein die Aminosäure-Sequenz, die durch die in Figur 3 gezeigte Sequenz (SEQ ID NO: 127) definiert ist, umfasst, oder ein funktionelles Homolog mit mindestens 70 % Identität dazu.

23. Fusionsprotein nach Anspruch 1, worin das Fusionsprotein aus der Aminosäure-Sequenz, die durch die in Figur 3 gezeigte Sequenz (SEQ ID NO: 127) definiert ist, besteht.

24. Isolierte Nukleinsäure, welche eine Nukleotid-Sequenz umfasst, die das Fusionsprotein nach einem der Ansprüche 1-23 codiert.

25. Vektor, welcher die Nukleinsäure-Sequenz nach Anspruch 24 umfasst.

26. Zelle, welche den Vektor nach Anspruch 25 enthält.

27. Fusionsprotein nach einem der Ansprüche 1-23 zur Verwendung als Medikament.

28. Verwendung des Fusionsproteins nach einem der Ansprüche 1-23 zur Herstellung eines Medikaments zur Verhinderung und/oder Behandlung einer Infektion in einem Individuum, der dies bedarf.

29. Verwendung nach Anspruch 28, wobei das Individuum ein Mensch ist.

30. Verwendung nach Anspruch 28, wobei das Individuum ein Mensch ist, der an einem erhöhten Risiko eine Infektion zu bekommen leidet.

31. Verwendung nach Anspruch 28, wobei das Individuum ein Mensch mit einem subnormalen Serumlevel an MBL ist.

32. Verwendung nach Anspruch 28, wobei das Individuum ein Mensch mit einem normalen Serumlevel an MBL ist.

33. Medikament zur Behandlung oder Verhinderung eines klinischen Zustandes in einem Individuum, welches dieses bedarf, welches das Fusionsprotein nach einem der Ansprüche 1-23 enthält.

34. Medikament nach Anspruch 33, wobei der klinische Zustand eine Infektion ist.

35. Medikament nach Anspruch 33, wobei das Individuum ein Mensch ist.

## Revendications

1. Protéine hybride comprenant :
i) une première séquence de polypeptide dérivée d'une protéine d'activation de la voie lectine-complément ou un homologue fonctionnel identique à au moins 70 % à ladite protéine d'activation de la voie lectine-complément, dans laquelle ladite première séquence de polypeptide est capable d'activer la voie lectine-complément ; et
ii) une seconde séquence de polypeptide dérivée d'une collectine ou un fonctionnel homologue identique à au moins 70 % à ladite collectine, dans laquelle ladite seconde séquence de polypeptide est capable de s'associer à un ou plusieurs glucides ;
dans laquelle ladite protéine d'activation du complément n'est pas une collectine.

2. Protéine hybride selon la revendication 1, dans laquelle ladite première séquence de polypeptide est capable de s'associer à au moins une protéine MASP.

3. Protéine hybride selon la revendication 1, dans laquelle ladite première séquence de polypeptide est capable de s'associer à une protéine MASP choisie dans le groupe constitué de MASP-1, MASP-2 et MASP-3 ou des homologues ou variants fonctionnels de celles-ci.

4. Protéine hybride selon la revendication 1, dans laquelle la protéine d'activation du complément est une ficoline.

5. Protéine hybride selon la revendication 4, dans laquelle la ficoline est choisie dans le groupe constitué de la L-ficoline, la H-ficoline et la M-ficoline.

6. Protéine hybride selon la revendication 4, dans laquelle la ficoline est la L-ficoline.

7. Protéine hybride selon la revendication 1, dans laquelle la première séquence de polypeptide comprend le domaine similaire au collagène d'une ficoline ou un homologue ou variant fonctionnel de celui-ci.

8. Protéine hybride selon la revendication 1, dans laquelle la première séquence de polypeptide comprend la région riche en cystéine d'une ficoline ou un homologue fonctionnel de celle-ci.

9. Protéine hybride selon la revendication 1, dans laquelle la première séquence de polypeptide comprend la région riche en cystéine et le domaine similaire au collagène d'une ficoline ou un homologue ou variant fonctionnel de ceux-ci.

10. Protéine hybride selon la revendication 1, dans laquelle la première séquence de polypeptide comprend les acides aminés 1 à 77 de la séquence de L-ficoline de la figure 1 (SEQ ID n° 125).

11. Protéine hybride selon la revendication 1, dans laquelle la collectine est choisie dans le groupe constitué de MBL (lectine liant le mannose), SP-A (protéine A du surfactant pulmonaire), SP-D (protéine D du surfactant pulmonaire), BK (ou BC, conglutinine bovine) et CL-43 (collectine 43).

12. Protéine hybride selon la revendication 15, dans laquelle la collectine est MBL.

13. Protéine hybride selon la revendication 1, dans laquelle la seconde séquence de polypeptide comprend le domaine CRD d'une collectine ou un homologue ou variant fonctionnel de celui-ci.

14. Protéine hybride selon la revendication 1, dans laquelle la seconde séquence de polypeptide comprend le domaine CRD de MBL.

15. Protéine hybride selon la revendication 1, dans laquelle la seconde séquence de polypeptide comprend la région de col de MBL.

16. Protéine hybride selon la revendication 1, dans laquelle la seconde séquence de polypeptide comprend le domaine similaire au collagène de MBL.

17. Protéine hybride selon la revendication 1, dans laquelle la seconde séquence de polypeptide comprend la région de col et le domaine CRD de MBL.

18. Protéine hybride selon la revendication 1, dans laquelle la seconde séquence de polypeptide comprend le domaine similaire au collagène, la région de col et le domaine CRD de MBL.

19. Protéine hybride selon la revendication 1, dans laquelle la seconde séquence de polypeptide comprend les acides aminés 80 à 228 de la séquence MBL représentée sur la figure 2 (SEQIDn° 126).

20. Protéine hybride selon la revendication 1, dans laquelle la protéine hybride comprend la région riche en cystéine et le domaine similaire au collagène de la L-ficoline et le domaine CRD de MBL.

21. Protéine hybride selon la revendication 1, dans laquelle la protéine hybride comprend la région riche en cystéine de la L-ficoline et le domaine similaire au collagène, la région de col et le domaine CRD de MBL.

22. Protéine hybride selon la revendication 1, dans laquelle la protéine hybride comprend la séquence d'acides aminés comme définie par la séquence représentée sur la figure 3 (SEQ ID n° 127), ou un homologue fonctionnel identique à au moins 70 % à celle-ci.

23. Protéine hybride selon la revendication 1, dans laquelle la protéine hybride est constituée de la séquence d'acides aminés comme définie par la séquence représentée sur la figure 3 (SEQ ID n° 127).

24. Acide nucléique isolé comprenant une séquence de nucléotides codant pour la protéine hybride selon l'une quelconque des revendications 1 à 23.

25. Vecteur comprenant la séquence d'acide nucléique selon la revendication 24.

26. Cellule comprenant le vecteur selon la revendication 25.

27. Protéine hybride selon l'une quelconque des revendications 1 à 23 pour utilisation en tant que médicament.

28. Utilisation de la protéine hybride selon l'une quelconque des revendications 1 à 23 pour la préparation d'un médicament pour la prévention et/ou le traitement d'une infection chez un individu nécessitant ceux-ci.

29. Utilisation selon la revendication 28, dans laquelle l'individu est un être humain.

30. Utilisation selon la revendication 28, dans laquelle l'individu est un humain ayant un risque croissant de contracter une infection.

31. Utilisation selon la revendication 28, dans laquelle l'individu est un être humain ayant un taux sérique de MBL au-dessous de la normale.

32. Utilisation selon la revendication 28, dans laquelle l'individu est un être humain ayant un taux sérique de MBL normal.

33. Médicament pour le traitement ou la prévention d'une pathologie clinique chez un individu nécessitant ceux-ci, comprenant la protéine hybride selon l'une quelconque des revendications 1 à 28.

34. Médicament selon la revendication 33, **caractérisé en ce que** la pathologie clinique est une infection.

35. Médicament selon la revendication 33, **caractérisé en ce que** l'individu est un être humain.


### SEQUENCE LISTING

<110> NatImmune A/S
   Weilguny, Dietmar
   Kongerslev, Leif
   Matthiesen, Finn
<120> Collectin-complement activating protein chimeras
<130> P 703 PC00
<160> 127
<170> PatentIn version 3.1
<210> 1
   <211> 185
   <212> PRT
   <213> Mus musculus
<400> 1
   Met Arg Leu Leu Ile Phe Leu Gly Leu Leu Trp Ser Leu Val Ala Thr
   1               5                   10                  15
   Leu Leu Gly Ser Lys Trp Pro Glu Pro Val Phe Gly Arg Leu Val Ser
               20                  25                  30
   Pro Gly Phe Pro Glu Lys Tyr Ala Asp His Gln Asp Arg Ser Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser Tyr Arg Cys Glu Tyr Asp Phe Val Lys Leu Ser
   65                  70                  75                  80
   Ser Gly Thr Lys Val Leu Ala Thr Leu Cys Gly Gln Glu Ser Thr Asp
                   85                  90                  95
   Thr Glu Gln Ala Pro Gly Asn Asp Thr Phe Tyr Ser Leu Gly Pro Ser
               100                 105                 110
   Leu Lys Val Thr Phe His Ser Asp Tyr Ser Asn Glu Lys Pro Phe Thr
           115                 120                 125
   Gly Phe Glu Ala Phe Tyr Ala Ala Glu Asp Val Asp Glu Cys Arg Val
       130                 135                 140
   Ser Leu Gly Asp Ser Val Pro Cys Asp His Tyr Cys His Asn Tyr Leu
   145                 150                 155                 160
   Gly Gly Tyr Tyr Cys Ser Cys Arg Ala Gly Tyr Val Leu His Gln Asn
                   165                 170                 175
   Lys His Thr Cys Ser Glu Gln Ser Leu
               180                 185
<210> 2
   <211> 244
   <212> PRT
   <213> Mus musculus
<400> 2
   Met Ser Ile Phe Thr Ser Phe Leu Leu Leu Cys Val Val Thr Val Val
   1               5                   10                  15
   Tyr Ala Glu Thr Leu Thr Glu Gly Val Gln Asn Ser Cys Pro Val Val
               20                  25                  30
   Thr Cys Ser Ser Pro Gly Leu Asn Gly Phe Pro Gly Lys Asp Gly Arg
           35                  40                  45
   Asp Gly Ala Lys Gly Glu Lys Gly Glu Pro Gly Gln Gly Leu Arg Gly
       50                  55                  60
   Leu Gln Gly Pro Pro Gly Lys Val Gly Pro Thr Gly Pro Pro Gly Asn
   65                  70                  75                  80
   Pro Gly Leu Lys Gly Ala Val Gly Pro Lys Gly Asp Arg Gly Asp Arg
                   85                  90                  95
   Ala Glu Phe Asp Thr Ser Glu Ile Asp Ser Glu Ile Ala Ala Leu Arg
   100 105 110
   Ser Glu Leu Arg Ala Leu Arg Asn Trp Val Leu Phe Ser Leu Ser Glu
   115 120 125
   Lys Val Gly Lys Lys Tyr Phe Val Ser Ser Val Lys Lys Met Ser Leu
   130 135 140
   Asp Arg Val Lys Ala Leu Cys Ser Glu Phe Gln Gly Ser Val Ala Thr
   145 150 155 160
   Pro Arg Asn Ala Glu Glu Asn Ser Ala Ile Gln Lys Val Ala Lys Asp
   165 170 175
   Ile Ala Tyr Leu Gly Ile Thr Asp Val Arg Val Glu Gly Ser Phe Glu
   180 185 190
   Asp Leu Thr Gly Asn Arg Val Arg Tyr Thr Asn Trp Asn Asp Gly Glu
   195 200 205
   Pro Asn Asn Thr Gly Asp Gly Glu Asp Cys Val Val Ile Leu Gly Asn
   210 215 220
   Gly Lys Trp Asn Asp Val Pro Cys Ser Asp Ser Phe Leu Ala Ile Cys
   225 230 235 240
   Glu Phe Ser Asp
<210> 3
   <211> 239
   <212> PRT
   <213> Mus musculus
<400> 3
   Met Leu Leu Leu Pro Leu Leu Pro Val Leu Leu Cys Val Val Ser Val
   1 5 10 15
   Ser Ser Ser Gly Ser Gln Thr Cys Glu Asp Thr Leu Lys Thr Cys Ser
   20 25 30
   Val Ile Ala Cys Gly Arg Asp Gly Arg Asp Gly Pro Lys Gly Glu Lys
   35 40 45
   Gly Glu Pro Gly Gln Gly Leu Arg Gly Leu Gln Gly Pro Pro Gly Lys
   50 55 60
   Leu Gly Pro Pro Gly Ser Val Gly Ser Pro Gly Ser Pro Gly Pro Lys
   65 70 75 80
   Gly Gln Lys Gly Asp His Gly Asp Asn Arg Ala Ile Glu Glu Lys Leu
   85 90 95
   Ala Asn Met Glu Ala Glu Ile Arg Ile Leu Lys Ser Lys Leu Gln Leu
   100 105 110
   Thr Asn Lys Leu His Ala Phe Ser Met Gly Lys Lys Ser Gly Lys Lys
   115 120 125
   Leu Phe Val Thr Asn His Glu Lys Met Pro Phe Ser Lys Val Lys Ser
   130 135 140
   Leu Cys Thr Glu Leu Gln Gly Thr Val Ala Ile Pro Arg Asn Ala Glu
   145 150 155 160
   Glu Asn Lys Ala Ile Gln Glu Val Ala Thr Gly Ile Ala Phe Leu Gly
   165 170 175
   Ile Thr Asp Glu Ala Thr Glu Gly Gln Phe Met Tyr Val Thr Gly Gly
   180 185 190
   Arg Leu Thr Tyr Ser Asn Trp Lys Lys Asp Glu Pro Asn Asn His Gly
   195 200 205
   Ser Gly Glu Asp Cys Val Ile Ile Leu Asp Asn Gly Leu Trp Asn Asp
   210 215 220
   Ile Ser Cys Gln Ala Ser Phe Lys Ala Val Cys Glu Phe Pro Ala
   225 230 235
<210> 4
   <211> 652
   <212> PRT
   <213> Homo sapiens
<400> 4
   Met Ala Thr Ser Met Gly Leu Leu Leu Leu Leu Leu Leu Leu Leu Thr
   1 5 10 15
   Gln Pro Gly Ala Gly Thr Gly Ala Asp Thr Glu Ala Val Val Cys Val
   20 25 30
   Gly Thr Ala Cys Tyr Thr Ala His Ser Gly Lys Leu Ser Ala Ala Glu
   35 40 45
   Ala Gln Asn His Cys Asn Gln Asn Gly Gly Asn Leu Ala Thr Val Lys
   50 55 60
   Ser Lys Glu Glu Ala Gln His Val Gln Arg Val Leu Ala Gln Leu Leu
   65 70 75 80
   Arg Arg Glu Ala Ala Leu Thr Ala Arg Met Ser Lys Phe Trp Ile Gly
   85 90 95
   Leu Gln Arg Glu Lys Gly Lys Cys Leu Asp Pro Ser Leu Pro Leu Lys
   100 105 110
   Gly Phe Ser Trp Val Gly Gly Gly Glu Asp Thr Pro Tyr Ser Asn Trp
   115 120 125
   His Lys Glu Leu Arg Asn Ser Cys Ile Ser Lys Arg Cys Val Ser Leu
   130 135 140
   Leu Leu Asp Leu Ser Gln Pro Leu Leu Pro Ser Arg Leu Pro Lys Trp
   145 150 155 160
   Ser Glu Gly Pro Cys Gly Ser Pro Gly Ser Pro Gly Ser Asn Ile Glu
   165 170 175
   Gly Phe Val Cys Lys Phe Ser Phe Lys Gly Met Cys Arg Pro Leu Ala
   180 185 190
   Leu Gly Gly Pro Gly Gln Val Thr Tyr Thr Thr Pro Phe Gln Thr Thr
   195 200 205
   Ser Ser Ser Leu Glu Ala Val Pro Phe Ala Ser Ala Ala Asn Val Ala
   210 215 220
   Cys Gly Glu Gly Asp Lys Asp Glu Thr Gln Ser His Tyr Phe Leu Cys
   225 230 235 240
   Lys Glu Lys Ala Pro Asp Val Phe Asp Trp Gly Ser Ser Gly Pro Leu
   245 250 255
   Cys Val Ser Pro Lys Tyr Gly Cys Asn Phe Asn Asn Gly Gly Cys His
   260 265 270
   Gln Asp Cys Phe Glu Gly Gly Asp Gly Ser Phe Leu Cys Gly Cys Arg
   275 280 285
   Pro Gly Phe Arg Leu Leu Asp Asp Leu Val Thr Cys Ala Ser Arg Asn
   290 295 300
   Pro Cys Ser Ser Ser Pro Cys Arg Gly Gly Ala Thr Cys Val Leu Gly
   305 310 315 320
   Pro His Gly Lys Asn Tyr Thr Cys Arg Cys Pro Gln Gly Tyr Gln Leu
   325 330 335
   Asp Ser Ser Gln Leu Asp Cys Val Asp Val Asp Glu Cys Gln Asp Ser
   340 345 350
   Pro Cys Ala Gln Glu Cys Val Asn Thr Pro Gly Gly Phe Arg Cys Glu
   355 360 365
   Cys Trp Val Gly Tyr Glu Pro Gly Gly Pro Gly Glu Gly Ala Cys Gln
   370 375 380
   Asp Val Asp Glu Cys Ala Leu Gly Arg Ser Pro Cys Ala Gln Gly Cys
   385 390 395 400
   Thr Asn Thr Asp Gly Ser Phe His Cys Ser Cys Glu Glu Gly Tyr Val
   405 410 415
   Leu Ala Gly Glu Asp Gly Thr Gln Cys Gln Asp Val Asp Glu Cys Val
   420 425 430
   Gly Pro Gly Gly Pro Leu Cys Asp Ser Leu Cys Phe Asn Thr Gln Gly
   435 440 445
   Ser Phe His Cys Gly Cys Leu Pro Gly Trp Val Leu Ala Pro Asn Gly
   450 455 460
   Val Ser Cys Thr Met Gly Pro Val Ser Leu Gly Pro Pro Ser Gly Pro
   465 470 475 480
   Pro Asp Glu Glu Asp Lys Gly Glu Lys Glu Gly Ser Thr Val Pro Arg
   485 490 495
   Ala Ala Thr Ala Ser Pro Thr Arg Gly Pro Glu Gly Thr Pro Lys Ala
   500 505 510
   Thr Pro Thr Thr Ser Arg Pro Ser Leu Ser Ser Asp Ala Pro Ile Thr
   515 520 525
   Ser Ala Pro Leu Lys Met Leu Ala Pro Ser Gly Ser Pro Gly Val Trp
   530 535 540
   Arg Glu Pro Ser Ile His His Ala Thr Ala Ala Ser Gly Pro Gln Glu
   545 550 555 560
   Pro Ala Gly Gly Asp Ser Ser Val Ala Thr Gln Asn Asn Asp Gly Thr
   565 570 575
   Asp Gly Gln Lys Leu Leu Leu Phe Tyr Ile Leu Gly Thr Val Val Ala
   580 585 590
   Ile Leu Leu Leu Leu Ala Leu Ala Leu Gly Leu Leu Val Tyr Arg Lys
   595 600 605
   Arg Arg Ala Lys Arg Glu Glu Lys Lys Glu Lys Lys Pro Gln Asn Ala
   610 615 620
   Ala Asp Ser Tyr Ser Trp Val Pro Glu Arg Ala Glu Ser Arg Ala Met
   625 630 635 640
   Glu Asn Gln Tyr Ser Pro Thr Pro Gly Thr Asp Cys
   645 650
<210> 5
   <211> 644
   <212> PRT
   <213> Mus musculus
<400> 5
   Met Ala Ile Ser Thr Gly Leu Phe Leu Leu Leu Gly Leu Leu Gly Gln
   1 5 10 15
   Pro Trp Ala Gly Ala Ala Ala Asp Ser Gln Ala Val Val Cys Glu Gly
   20 25 30
   Thr Ala Cys Tyr Thr Ala His Trp Gly Lys Leu Ser Ala Ala Glu Ala
   35 40 45
   Gln His Arg Cys Asn Glu Asn Gly Gly Asn Leu Ala Thr Val Lys Ser
   50 55 60
   Glu Glu Glu Ala Arg His Val Gln Gln Ala Leu Thr Gln Leu Leu Lys
   65 70 75 80
   Thr Lys Ala Pro Leu Glu Ala Lys Met Gly Lys Phe Trp Ile Gly Leu
   85 90 95
   Gln Arg Glu Lys Gly Asn Cys Thr Tyr His Asp Leu Pro Met Arg Gly
   100 105 110
   Phe Ser Trp Val Gly Gly Gly Glu Asp Thr Ala Tyr Ser Asn Trp Tyr
   115 120 125
   Lys Ala Ser Lys Ser Ser Cys Ile Phe Lys Arg Cys Val Ser Leu Ile
   130 135 140
   Leu Asp Leu Ser Leu Thr Pro His Pro Ser His Leu Pro Lys Trp His
   145 150 155 160
   Glu Ser Pro Cys Gly Thr Pro Glu Ala Pro Gly Asn Ser Ile Glu Gly
   165 170 175
   Phe Leu Cys Lys Phe Asn Phe Lys Gly Met Cys Arg Pro Leu Ala Leu
   180 185 190
   Gly Gly Pro Gly Arg Val Thr Tyr Thr Thr Pro Phe Gln Ala Thr Thr
   195 200 205
   Ser Ser Leu Glu Ala Val Pro Phe Ala Ser Val Ala Asn Val Ala Cys
   210 215 220
   Gly Asp Glu Ala Lys Ser Glu Thr His Tyr Phe Leu Cys Asn Glu Lys
   225 230 235 240
   Thr Pro Gly Ile Phe His Trp Gly Ser Ser Gly Pro Leu Cys Val Ser
   245 250 255
   Pro Lys Phe Gly Cys Ser Phe Asn Asn Gly Gly Cys Gln Gln Asp Cys
   260 265 270
   Phe Glu Gly Gly Asp Gly Ser Phe Arg Cys Gly Cys Arg Pro Gly Phe
   275 280 285
   Arg Leu Leu Asp Asp Leu Val Thr Cys Ala Ser Arg Asn Pro Cys Ser
   290 295 300
   Ser Asn Pro Cys Thr Gly Gly Gly Met Cys His Ser Val Pro Leu Ser
   305 310 315 320
   Glu Asn Tyr Thr Cys Arg Cys Pro Ser Gly Tyr Gln Leu Asp Ser Ser
   325 330 335
   Gln Val His Cys Val Asp Ile Asp Glu Cys Gln Asp Ser Pro Cys Ala
   340 345 350
   Gln Asp Cys Val Asn Thr Leu Gly Ser Phe His Cys Glu Cys Trp Val
   355 360 365
   Gly Tyr Gln Pro Ser Gly Pro Lys Glu Glu Ala Cys Glu Asp Val Asp
   370 375 380
   Glu Cys Ala Ala Ala Asn Ser Pro Cys Ala Gln Gly Cys Ile Asn Thr
   385 390 395 400
   Asp Gly Ser Phe Tyr Cys Ser Cys Lys Glu Gly Tyr Ile Val Ser Gly
   405 410 415
   Glu Asp Ser Thr Gln Cys Glu Asp Ile Asp Glu Cys Ser Asp Ala Arg
   420 425 430
   Gly Asn Pro Cys Asp Ser Leu Cys Phe Asn Thr Asp Gly Ser Phe Arg
   435 440 445
   Cys Gly Cys Pro Pro Gly Trp Glu Leu Ala Pro Asn Gly Val Phe Cys
   450 455 460
   Ser Arg Gly Thr Val Phe Ser Glu Leu Pro Ala Arg Pro Pro Gln Lys
   465 470 475 480
   Glu Asp Asn Asp Asp Arg Lys Glu Ser Thr Met Pro Pro Thr Glu Met
   485 490 495
   Pro Ser Ser Pro Ser Gly Ser Lys Asp Val Ser Asn Arg Ala Gln Thr
   500 505 510
   Thr Gly Leu Phe Val Gln Ser Asp Ile Pro Thr Ala Ser Val Pro Leu
   515 520 525
   Glu Ile Glu Ile Pro Ser Glu Val Ser Asp Val Trp Phe Glu Leu Gly
   530 535 540
   Thr Tyr Leu Pro Thr Thr Ser Gly His Ser Lys Pro Thr His Glu Asp
   545 550 555 560
   Ser Val Ser Ala His Ser Asp Thr Asp Gly Gln Asn Leu Leu Leu Phe
   565 570 575
   Tyr Ile Leu Gly Thr Val Val Ala Ile Ser Leu Leu Leu Val Leu Ala
   580 585 590
   Leu Gly Ile Leu Ile Tyr His Lys Arg Arg Ala Lys Lys Glu Glu Ile
   595 600 605
   Lys Glu Lys Lys Pro Gln Asn Ala Ala Asp Ser Tyr Ser Trp Val Pro
   610 615 620
   Glu Arg Ala Glu Ser Gln Ala Pro Glu Asn Gln Tyr Ser Pro Thr Pro
   625 630 635 640
   Gly Thr Asp Cys
<210> 6
   <211> 688
   <212> PRT
   <213> Homo sapiens
<400> 6
<210> 7
   <211> 652
   <212> PRT
   <213> Homo sapiens
<400> 7
   Met Ala Thr Ser Met Gly Leu Leu Leu Leu Leu Leu Leu Leu Leu Thr
   1 5 10 15
   Gln Pro Gly Ala Gly Thr Gly Ala Asp Thr Glu Ala Val Val Cys Val
   20 25 30
   Gly Thr Ala Cys Tyr Thr Ala His Ser Gly Lys Leu Ser Ala Ala Glu
   35 40 45
   Ala Gln Asn His Cys Asn Gln Asn Gly Gly Asn Leu Ala Thr Val Lys
   50 55 60
   Ser Lys Glu Glu Ala Gln His Val Gln Arg Val Leu Ala Gln Leu Leu
   65 70 75 80
   Arg Arg Glu Ala Ala Leu Thr Ala Arg Met Ser Lys Phe Trp Ile Gly
   85 90 95
   Leu Gln Arg Glu Lys Gly Lys Cys Leu Asp Pro Ser Leu Pro Leu Lys
   100 105 110
   Gly Phe Ser Trp Val Gly Gly Gly Glu Asp Thr Pro Tyr Ser Asn Trp
   115 120 125
   His Lys Glu Leu Arg Asn Ser Cys Ile Ser Lys Arg Cys Val Ser Leu
   130 135 140
   Leu Leu Asp Leu Ser Gln Pro Leu Leu Pro Asn Arg Leu Pro Lys Trp
   145 150 155 160
   Ser Glu Gly Pro Cys Gly Ser Pro Gly Ser Pro Gly Ser Asn Ile Glu
   165 170 175
   Gly Phe Val Cys Lys Phe Ser Phe Lys Gly Met Cys Arg Pro Leu Ala
   180 185 190
   Leu Gly Gly Pro Gly Gln Val Thr Tyr Thr Thr Pro Phe Gln Thr Thr
   195 200 205
   Ser Ser Ser Leu Glu Ala Val Pro Phe Ala Ser Ala Ala Asn Val Ala
   210 215 220
   Cys Gly Glu Gly Asp Lys Asp Glu Thr Gln Ser His Tyr Phe Leu Cys
   225 230 235 240
   Lys Glu Lys Ala Pro Asp Val Phe Asp Trp Gly Ser Ser Gly Pro Leu
   245 250 255
   Cys Val Ser Pro Lys Tyr Gly Cys Asn Phe Asn Asn Gly Gly Cys His
   260 265 270
   Gln Asp Cys Phe Glu Gly Gly Asp Gly Ser Phe Leu Cys Gly Cys Arg
   275 280 285
   Pro Gly Phe Arg Leu Leu Asp Asp Leu Val Thr Cys Ala Ser Arg Asn
   290 295 300
   Pro Cys Ser Ser Ser Pro Cys Arg Gly Gly Ala Thr Cys Val Leu Gly
   305 310 315 320
   Pro His Gly Lys Asn Tyr Thr Cys Arg Cys Pro Gln Gly Tyr Gln Leu
   325 330 335
   Asp Ser Ser Gln Leu Asp Cys Val Asp Val Asp Glu Cys Gln Asp Ser
   340 345 350
   Pro Cys Ala Gln Glu Cys Val Asn Thr Pro Gly Gly Phe Arg Cys Glu
   355 360 365
   Cys Trp Val Gly Tyr Glu Pro Gly Gly Pro Gly Glu Gly Ala Cys Gln
   370 375 380
   Asp Val Asp Glu Cys Ala Leu Gly Arg Ser Pro Cys Ala Gln Gly Cys
   385 390 395 400
   Thr Asn Thr Asp Gly Ser Phe His Cys Ser Cys Glu Glu Gly Tyr Val
   405 410 415
   Leu Ala Gly Glu Asp Gly Thr Gln Cys Gln Asp Val Asp Glu Cys Val
   420 425 430
   Gly Pro Gly Gly Pro Leu Cys Asp Ser Leu Cys Phe Asn Thr Gln Gly
   435 440 445
   Ser Phe His Cys Gly Cys Leu Pro Gly Trp Val Leu Ala Pro Asn Gly
   450 455 460
   Val Ser Cys Thr Met Gly Pro Val Ser Leu Gly Pro Pro Ser Gly Pro
   465 470 475 480
   Pro Asp Glu Glu Asp Lys Gly Glu Lys Glu Gly Ser Thr Val Pro Arg
   485 490 495
   Ala Ala Thr Ala Ser Pro Thr Arg Gly Pro Glu Gly Thr Pro Lys Ala
   500 505 510
   Thr Pro Thr Thr Ser Arg Pro Ser Leu Ser Ser Asp Ala Pro Ile Thr
   515 520 525
   Ser Ala Pro Leu Lys Met Leu Ala Pro Ser Gly Ser Ser Gly Val Trp
   530 535 540
   Arg Glu Pro Ser Ile His His Ala Thr Ala Ala Ser Gly Pro Gln Glu
   545 550 555 560
   Pro Ala Gly Gly Asp Ser Ser Val Ala Thr Gln Asn Asn Asp Gly Thr
   565 570 575
   Asp Gly Gln Lys Leu Leu Leu Phe Tyr  Ile Leu Gly Thr Val Val Ala
               580                 585                  590
   Ile Leu Leu Leu Leu Ala Leu Ala Leu Gly Leu Leu Val Tyr Arg Lys
           595                 600                 605
   Arg Arg Ala Lys Arg Glu Glu Lys Lys Glu Lys Lys Pro Gln Asn Ala
       610                 615                 620
   Ala Asp Ser Tyr Ser Trp Val Pro Glu Arg Ala Glu Ser Arg Ala Met
   625                 630                 635                 640
   Glu Asn Gln Tyr Ser Pro Thr Pro Gly Thr Asp Cys
                   645                 650
<210> 8
   <211> 248
   <212> PRT
   <213> Homo sapiens
<400> 8
   Met Ser Leu Phe Pro Ser Leu Pro Leu Leu Leu Leu Ser Met Val Ala
   1               5                   10                  15
   Ala Ser Tyr Ser Glu Thr Val Thr Cys Glu Asp Ala Gln Lys Thr Cys
               20                  25                  30
   Pro Ala Val Ile Ala Cys Ser Ser Pro Gly Ile Asn Gly Phe Pro Gly
           35                  40                  45
   Lys Asp Gly Arg Asp Gly Thr Lys Gly Glu Lys Gly Glu Pro Gly Gln
       50                  55                  60
   Gly Leu Arg Gly Leu Gln Gly Pro Pro Gly Lys Leu Gly Pro Pro Gly
   65                  70                  75                  80
   Asn Pro Gly Pro Ser Gly Ser Pro Gly Pro Lys Gly Gln Lys Gly Asp
                   85                  90                  95
   Pro Gly Lys Ser Pro Asp Gly Asp Ser Ser Leu Ala Ala Ser Glu Arg
               100                 105                 110
   Lys Ala Leu Gln Thr Glu Met Ala Arg Ile Lys Lys Trp Leu Thr Phe
           115                 120                 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 9
   <211> 248
   <212> PRT
   <213> Homo sapiens
<400> 9
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 10
   <211> 643
   <212> PRT
   <213> Rattus norvegicus
<400> 10
   Met Val Thr Ser Thr Gly Leu Leu Leu Leu Leu Gly Leu Leu Gly Gln
   1 5 10 15
   Leu Trp Ala Gly Ala Ala Ala Asp Ser Glu Ala Val Val Cys Glu Gly
               20                  25                  30
   Thr Ala Cys Tyr Thr Ala His Trp Gly Lys Leu Ser Ala Ala Glu Ala
           35                  40                  45
   Gln His Arg Cys Asn Glu Asn Gly Gly Asn Leu Ala Thr Val Lys Ser
       50                  55                  60
   Glu Glu Glu Ala Arg His Val Gln Glu Ala Leu Ala Gln Leu Leu Lys
   65                  70                  75                  80
   Thr Lys Ala Pro Ser Glu Thr Lys Ile Gly Lys Phe Trp Ile Gly Leu
                   85                  90                  95
   Gln Arg Glu Lys Gly Lys Cys Thr Tyr His Asp Leu Pro Met Lys Gly
               100                 105                 110
   Phe Ser Trp Val Gly Gly Gly Glu Asp Thr Thr Tyr Ser Asn Trp Tyr
           115                 120                 125
   Lys Ala Ser Lys Ser Ser Cys Ile Ser Lys Arg Cys Val Ser Leu Ile
       130                 135                 140
   Leu Asp Leu Ser Leu Lys Pro His Pro Ser His Leu Pro Lys Trp His
   145                 150                 155                 160
   Glu Ser Pro Cys Gly Thr Pro Asp Ala Pro Gly Asn Ser Ile Glu Gly
                   165                 170                 175
   Phe Leu Cys Lys Phe Asn Phe Lys Gly Met Cys Ser Pro Leu Ala Leu
               180                 185                 190
   Gly Gly Pro Gly Gln Leu Thr Tyr Thr Thr Pro Phe Gln Ala Thr Thr
           195                 200                 205
   Ser Ser Leu Lys Ala Val Pro Phe Ala Ser Val Ala Asn Val Val Cys
       210                 215                 220
   Gly Asp Glu Ala Glu Ser Lys Thr Asn Tyr Tyr Leu Cys Lys Glu Thr
   225                 230                 235                 240
   Thr Ala Gly Val Phe His Trp Gly Ser Ser Gly Pro Leu Cys Val Ser
                   245                 250                 255
   Pro Lys Phe Gly Cys Ser Phe Asn Asn Gly Gly Cys Gln Gln Asp Cys
   260 265 270
   Phe Glu Gly Gly Asp Gly Ser Phe Arg Cys Gly Cys Arg Pro Gly Phe
   275 280 285
   Arg Leu Leu Asp Asp Leu Val Thr Cys Ala Ser Arg Asn Pro Cys Ser
   290 295 300
   Ser Asn Pro Cys Thr Gly Gly Gly Met Cys His Ser Val Pro Leu Ser
   305 310 315 320
   Glu Asn Tyr Thr Cys His Cys Pro Arg Gly Tyr Gln Leu Asp Ser Ser
   325 330 335
   Gln Val His Cys Val Asp Ile Asp Glu Cys Glu Asp Ser Pro Cys Asp
   340 345 350
   Gln Glu Cys Ile Asn Thr Pro Gly Gly Phe His Cys Glu Cys Trp Val
   355 360 365
   Gly Tyr Gln Ser Ser Gly Ser Lys Glu Glu Ala Cys Glu Asp Val Asp
   370 375 380
   Glu Cys Thr Ala Ala Tyr Ser Pro Cys Ala Gln Gly Cys Thr Asn Thr
   385 390 395 400
   Asp Gly Ser Phe Tyr Cys Ser Cys Lys Glu Gly Tyr Ile Met Ser Gly
   405 410 415
   Glu Asp Ser Thr Gln Cys Glu Asp Ile Asp Glu Cys Leu Gly Asn Pro
   420 425 430
   Cys Asp Thr Leu Cys Ile Asn Thr Asp Gly Ser Phe Arg Cys Gly Cys
   435 440 445
   Pro Ala Gly Phe Glu Leu Ala Pro Asn Gly Val Ser Cys Thr Arg Gly
   450 455 460
   Ser Met Phe Ser Glu Leu Pro Ala Arg Pro Pro Gln Lys Glu Asp Lys
   465 470 475 480
   Gly Asp Gly Lys Glu Ser Thr Val Pro Leu Thr Glu Met Pro Gly Ser
   485 490 495
   Leu Asn Gly Ser Lys Asp Val Ser Asn Arg Ala Gln Thr Thr Asp Leu
   500 505 510
   Ser Ile Gln Ser Asp Ser Ser Thr Ala Ser Val Pro Leu Glu Ile Glu
   515 520 525
   Val Ser Ser Glu Ala Ser Asp Val Trp Leu Asp Leu Gly Thr Tyr Leu
   530 535 540
   Pro Thr Thr Ser Gly His Ser Gln Pro Thr His Glu Asp Ser Val Pro
   545 550 555 560
   Ala His Ser Asp Ser Asp Thr Asp Gly Gln Lys Leu Leu Leu Phe Tyr
   565 570 575
   Ile Leu Gly Thr Val Val Ala Ile Ser Leu Leu Leu Ala Leu Ala Leu
   580 585 590
   Gly Leu Leu Ile Tyr Leu Lys Arg Lys Ala Lys Lys Glu Glu Ile Lys
   595 600 605
   Glu Lys Lys Ala Gln Asn Ala Ala Asp Ser Tyr Ser Trp Ile Pro Glu
   610 615 620
   Arg Ala Glu Ser Arg Ala Pro Glu Asn Gln Tyr Ser Pro Thr Pro Gly
   625 630 635 640
   Thr Asp Cys
<210> 11
   <211> 686
   <212> PRT
   <213> Homo sapiens
<400> 11
   Met Arg Leu Leu Thr Leu Leu Gly Leu Leu Cys Gly Ser Val Ala Thr
   1 5 10 15
   Pro Leu Gly Pro Lys Trp Pro Glu Pro Val Phe Gly Arg Leu Ala Ser
   20 25 30
   Pro Gly Phe Pro Gly Glu Tyr Ala Asn Asp Gln Glu Arg Arg Trp Thr
   35 40 45
   Leu Thr Ala Pro Pro Gly Tyr Arg Leu Arg Leu Tyr Phe Thr His Phe
   50 55 60
   Asp Leu Glu Leu Ser His Leu Cys Glu Tyr Asp Phe Val Lys Leu Ser
   65 70 75 80
   Ser Gly Ala Lys Val Leu Ala Thr Leu Cys Gly Gln Glu Ser Thr Asp
   85 90 95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala Glu Asp Ile Asp Glu Cys Gln Val
   130 135 140
   Ala Pro Gly Glu Ala Pro Thr Cys Asp His His Cys His Asn His Leu
   145 150 155 160
   Gly Gly Phe Tyr Cys Ser Cys Arg Ala Gly Tyr Val Leu His Arg Asn
   165 170 175
   Lys Arg Thr Cys Ser Ala Leu Cys Ser Gly Gln Val Phe Thr Gln Arg
   180 185 190
   Ser Gly Glu Leu Ser Ser Pro Glu Tyr Pro Arg Pro Tyr Pro Lys Leu
   195 200 205
   Ser Ser Cys Thr Tyr Ser Ile Ser Leu Glu Glu Gly Phe Ser Val Ile
   210 215 220
   Leu Asp Phe Val Glu Ser Phe Asp Val Glu Thr His Pro Glu Thr Leu
   225 230 235 240
   Cys Pro Tyr Asp Phe Leu Lys Ile Gln Thr Asp Arg Glu Glu His Gly
   245 250 255
   Pro Phe Cys Gly Lys Thr Leu Pro His Arg Ile Glu Thr Lys Ser Asn
   260 265 270
   Thr Val Thr Ile Thr Phe Val Thr Asp Glu Ser Gly Asp His Thr Gly
   275 280 285
   Trp Lys Ile His Tyr Thr Ser Thr Ala Gln Pro Cys Pro Tyr Pro Met
   290 295 300
   Ala Pro Pro Asn Gly His Val Ser Pro Val Gln Ala Lys Tyr Ile Leu
   305 310 315 320
   Lys Asp Ser Phe Ser Ile Phe Cys Glu Thr Gly Tyr Glu Leu Leu Gln
   325 330 335
   Gly His Leu Pro Leu Lys Ser Phe Thr Ala Val Cys Gln Lys Asp Gly
   340 345 350
   Ser Trp Asp Arg Pro Met Pro Ala Cys Ser Ile Val Asp Cys Gly Pro
   355 360 365
   Pro Asp Asp Leu Pro Ser Gly Arg Val Glu Tyr Ile Thr Gly Pro Gly
   370 375 380
   Val Thr Thr Tyr Lys Ala Val Ile Gln Tyr Ser Cys Glu Glu Thr Phe
   385 390 395 400
   Tyr Thr Met Lys Val Asn Asp Gly Lys Tyr Val Cys Glu Ala Asp Gly
   405 410 415
   Phe Trp Thr Ser Ser Lys Gly Glu Lys Ser Leu Pro Val Cys Glu Pro
   420 425 430
   Val Cys Gly Leu Ser Ala Arg Thr Thr Gly Gly Arg Ile Tyr Gly Gly
   435 440 445
   Gln Lys Ala Lys Pro Gly Asp Phe Pro Trp Gln Val Leu Ile Leu Gly
   450 455 460
   Gly Thr Thr Ala Ala Gly Ala Leu Leu Tyr Asp Asn Trp Val Leu Thr
   465 470 475 480
   Ala Ala His Ala Val Tyr Glu Gln Lys His Asp Ala Ser Ala Leu Asp
   485 490 495
   Ile Arg Met Gly Thr Leu Lys Arg Leu Ser Pro His Tyr Thr Gln Ala
   500 505 510
   Trp Ser Glu Ala Val Phe Ile His Glu Gly Tyr Thr His Asp Ala Gly
   515 520 525
   Phe Asp Asn Asp Ile Ala Leu Ile Lys Leu Asn Asn Lys Val Val Ile
   530 535 540
   Asn Ser Asn Ile Thr Pro Ile Cys Leu Pro Arg Lys Glu Ala Glu Ser
   545 550 555 560
   Phe Met Arg Thr Asp Asp Ile Gly Thr Ala Ser Gly Trp Gly Leu Thr
   565 570 575
   Gln Arg Gly Phe Leu Ala Arg Asn Leu Met Tyr Val Asp Ile Pro Ile
   580 585 590
   Val Asp His Gln Lys Cys Thr Ala Ala Tyr Glu Lys Pro Pro Tyr Pro
   595 600 605
   Arg Gly Ser Val Thr Ala Asn Met Leu Cys Ala Gly Leu Glu Ser Gly
   610 615 620
   Gly Lys Asp Ser Cys Arg Gly Asp Ser Gly Gly Ala Leu Val Phe Leu
   625 630 635 640
   Asp Ser Glu Thr Glu Arg Trp Phe Val Gly Gly Ile Val Ser Trp Gly
   645 650 655
   Ser Met Asn Cys Gly Glu Ala Gly Gln Tyr Gly Val Tyr Thr Lys Val
   660 665 670
   Ile Asn Tyr Ile Pro Trp Ile Glu Asn Ile Ile Ser Asp Phe
   675 680 685
<210> 12
   <211> 185
   <212> PRT
   <213> Homo sapiens
<400> 12
   Met Arg Leu Leu Thr Leu Leu Gly Leu Leu Cys Gly Ser Val Ala Thr
   1 5 10 15
   Pro Leu Gly Pro Lys Trp Pro Glu Pro Val Phe Gly Arg Leu Ala Ser
   20 25 30
   Pro Gly Phe Pro Gly Glu Tyr Ala Asn Asp Gln Glu Arg Arg Trp Thr
   35 40 45
   Leu Thr Ala Pro Pro Gly Tyr Arg Leu Arg Leu Tyr Phe Thr His Phe
   50 55 60
   Asp Leu Glu Leu Ser His Leu Cys Glu Tyr Asp Phe Val Lys Leu Ser
   65 70 75 80
   Ser Gly Ala Lys Val Leu Ala Thr Leu Cys Gly Gln Glu Ser Thr Asp
   85 90 95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala Glu Asp Ile Asp Glu Cys Gln Val
   130 135 140
   Ala Pro Gly Glu Ala Pro Thr Cys Asp His His Cys His Asn His Leu
   145 150 155 160
   Gly Gly Phe Tyr Cys Ser Cys Arg Ala Gly Tyr Val Leu His Arg Asn
   165 170 175
   Lys Arg Thr Cys Ser Glu Gln Ser Leu
   180 185
<210> 13
   <211> 728
   <212> PRT
   <213> Homo sapiens
<400> 13
   Met Arg Trp Leu Leu Leu Tyr Tyr Ala Leu Cys Phe Ser Leu Ser Lys
   1               5                   10                  15
   Ala Ser Ala His Thr Val Glu Leu Asn Asn Met Phe Gly Gln Ile Gln
               20                  25                  30
   Ser Pro Gly Tyr Pro Asp Ser Tyr Pro Ser Asp Ser Glu Val Thr Trp
           35                  40                  45
   Asn Ile Thr Val Pro Asp Gly Phe Arg Ile Lys Leu Tyr Phe Met His
       50                  55                  60
   Phe Asn Leu Glu Ser Ser Tyr Leu Cys  Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
           275                 280                 285
   Gly Trp Arg Leu Ser Tyr Arg Ala Ala Gly Asn Glu Cys Pro Glu Leu
       290                 295                 300
   Gln Pro Pro Val His Gly Lys Ile Glu Pro Ser Gln Ala Lys Tyr Phe
   305                 310                 315                 320
   Phe Lys Asp Gln Val Leu Val Ser Cys Asp Thr Gly Tyr Lys Val Leu
                   325                 330                 335
   Lys Asp Asn Val Glu Met Asp Thr Phe Gln Ile Glu Cys Leu Lys Asp
               340                 345                 350
   Gly Thr Trp Ser Asn Lys Ile Pro Thr Cys Lys Ile Val Asp Cys Arg
           355                 360                 365
   Ala Pro Gly Glu Leu Glu His Gly Leu Ile Thr Phe Ser Thr Arg Asn
       370                 375                 380
   Asn Leu Thr Thr Tyr Lys Ser Glu Ile Lys Tyr Ser Cys Gln Glu Pro
   385                 390                 395                 400
   Tyr Tyr Lys Met Leu Asn Asn Asn Thr Gly Ile Tyr Thr Cys Ser Ala
                   405                 410                 415
   Gln Gly Val Trp Met Asn Lys Val Leu Gly Arg Ser Leu Pro Thr Cys
               420                 425                 430
   Leu Pro Glu Cys Gly Gln Pro Ser Arg Ser Leu Pro Ser Leu Val Lys
           435                 440                 445
   Arg Ile Ile Gly Gly Arg Asn Ala Glu Pro Gly Leu Phe Pro Trp Gln
       450                 455                 460
   Ala Leu Ile Val Val Glu Asp Thr Ser Arg Val Pro Asn Asp Lys Trp
   465                 470                 475                 480
   Phe Gly Ser Gly Ala Leu Leu Ser Ala Ser Trp Ile Leu Thr Ala Ala
                   485                 490                 495
   His Val Leu Arg Ser Gln Arg Arg Asp Thr Thr Val Ile Pro Val Ser
               500                 505                 510
   Lys Glu His Val Thr Val Tyr Leu Gly Leu His Asp Val Arg Asp Lys
           515                 520                 525
   Ser Gly Ala Val Asn Ser Ser Ala Ala Arg Val Val Leu His Pro Asp
       530                 535                 540
   Phe Asn Ile Gln Asn Tyr Asn His Asp Ile Ala Leu Val Gln Leu Gln
   545                 550                 555                 560
   Glu Pro Val Pro Leu Gly Pro His Val  Met Pro Val Cys Leu Pro Arg
                   565                  570                 575
   Leu Glu Pro Glu Gly Pro Ala Pro His Met Leu Gly Leu Val Ala Gly
               580                 585                 590
   Trp Gly Ile Ser Asn Pro Asn Val Thr Val Asp Glu Ile Ile Ser Ser
           595                 600                 605
   Gly Thr Arg Thr Leu Ser Asp Val Leu Gln Tyr Val Lys Leu Pro Val
       610                 615                 620
   Val Pro His Ala Glu Cys Lys Thr Ser Tyr Glu Ser Arg Ser Gly Asn
   625                 630                 635                 640
   Tyr Ser Val Thr Glu Asn Met Phe Cys Ala Gly Tyr Tyr Glu Gly Gly
                   645                 650                 655
   Lys Asp Thr Cys Leu Gly Asp Ser Gly Gly Ala Phe Val Ile Phe Asp
               660                 665                 670
   Asp Leu Ser Gln Arg Trp Val Val Gln Gly Leu Val Ser Trp Gly Gly
           675                 680                 685
   Pro Glu Glu Cys Gly Ser Lys Gln Val Tyr Gly Val Tyr Thr Lys Val
       690                 695                 700
   Ser Asn Tyr Val Asp Trp Val Trp Glu Gln Met Gly Leu Pro Gln Ser
   705                 710                 715                 720
   Val Val Glu Pro Gln Val Glu Arg
                   725
<210> 14
   <211> 699
   <212> PRT
   <213> Homo sapiens
<400> 14
   Met Arg Trp Leu Leu Leu Tyr Tyr Ala Leu Cys Phe Ser Leu Ser Lys
   1 5 10 15
   Ala Ser Ala His Thr Val Glu Leu Asn Asn Met Phe Gly Gln Ile Gln
   20 25 30
   Ser Pro Gly Tyr Pro Asp Ser Tyr Pro Ser Asp Ser Glu Val Thr Trp
           35                  40                  45
   Asn Ile Thr Val Pro Asp Gly Phe Arg Ile Lys Leu Tyr Phe Met His
       50                  55                  60
   Phe Asn Leu Glu Ser Ser Tyr Leu Cys Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
   275 280 285
   Gly Trp Arg Leu Ser Tyr Arg Ala Ala Gly Asn Glu Cys Pro Glu Leu
   290 295 300
   Gln Pro Pro Val His Gly Lys Ile Glu Pro Ser Gln Ala Lys Tyr Phe
   305 310 315 320
   Phe Lys Asp Gln Val Leu Val Ser Cys Asp Thr Gly Tyr Lys Val Leu
   325 330 335
   Lys Asp Asn Val Glu Met Asp Thr Phe Gln Ile Glu Cys Leu Lys Asp
   340 345 350
   Gly Thr Trp Ser Asn Lys Ile Pro Thr Cys Lys Ile Val Asp Cys Arg
   355 360 365
   Ala Pro Gly Glu Leu Glu His Gly Leu Ile Thr Phe Ser Thr Arg Asn
   370 375 380
   Asn Leu Thr Thr Tyr Lys Ser Glu Ile Lys Tyr Ser Cys Gln Glu Pro
   385 390 395 400
   Tyr Tyr Lys Met Leu Asn Asn Asn Thr Gly Ile Tyr Thr Cys Ser Ala
   405 410 415
   Gln Gly Val Trp Met Asn Lys Val Leu Gly Arg Ser Leu Pro Thr Cys
   420 425 430
   Leu Pro Val Cys Gly Leu Pro Lys Phe Ser Arg Lys Leu Met Ala Arg
   435 440 445
   Ile Phe Asn Gly Arg Pro Ala Gln Lys Gly Thr Thr Pro Trp Ile Ala
   450 455 460
   Met Leu Ser His Leu Asn Gly Gln Pro Phe Cys Gly Gly Ser Leu Leu
   465 470 475 480
   Gly Ser Ser Trp Ile Val Thr Ala Ala His Cys Leu His Gln Ser Leu
   485 490 495
   Asp Pro Glu Asp Pro Thr Leu Arg Asp Ser Asp Leu Leu Ser Pro Ser
   500 505 510
   Asp Phe Lys Ile Ile Leu Gly Lys His Trp Arg Leu Arg Ser Asp Glu
   515 520 525
   Asn Glu Gln His Leu Gly Val Lys His Thr Thr Leu His Pro Gln Tyr
   530 535 540
   Asp Pro Asn Thr Phe Glu Asn Asp Val Ala Leu Val Glu Leu Leu Glu
   545 550 555 560
   Ser Pro Val Leu Asn Ala Phe Val Met Pro Ile Cys Leu Pro Glu Gly
   565 570 575
   Pro Gln Gln Glu Gly Ala Met Val Ile Val Ser Gly Trp Gly Lys Gln
   580 585 590
   Phe Leu Gln Arg Phe Pro Glu Thr Leu Met Glu Ile Glu Ile Pro Ile
   595 600 605
   Val Asp His Ser Thr Cys Gln Lys Ala Tyr Ala Pro Leu Lys Lys Lys
   610 615 620
   Val Thr Arg Asp Met Ile Cys Ala Gly Glu Lys Glu Gly Gly Lys Asp
   625 630 635 640
   Ala Cys Ala Gly Asp Ser Gly Gly Pro Met Val Thr Leu Asn Arg Glu
   645 650 655
   Arg Gly Gln Trp Tyr Leu Val Gly Thr Val Ser Trp Gly Asp Asp Cys
   660 665 670
   Gly Lys Lys Asp Arg Tyr Gly Val Tyr Ser Tyr Ile His His Asn Lys
   675 680 685
   Asp Trp Ile Gln Arg Val Thr Gly Val Arg Asn
   690 695
<210> 15
   <211> 239
   <212> PRT
   <213> Mus musculus
<220>
   <221> MISC_FEATURE
   <222> (91)..(116)
   <223> Unknown
<400> 15
   Met Leu Leu Leu Pro Leu Leu Pro Val Leu Leu Cys Val Val Ser Val
   1               5                   10                  15
   Ser Ser Ser Gly Ser Gln Thr Cys Glu Asp Thr Leu Lys Thr Cys Ser
               20                  25                  30
   Val Ile Ala Cys Gly Arg Asp Gly Arg Asp Gly Pro Lys Gly Glu Lys
           35                  40                  45
   Gly Glu Pro Gly Gln Gly Leu Arg Gly Leu Gln Gly Pro Pro Gly Lys
       50                  55                  60
   Leu Gly Pro Pro Gly Ser Val Gly Ser Pro Gly Ser Pro Gly Pro Lys
   65                  70                  75                  80
   Gly Gln Lys Gly Asp His Gly Asp Asn Arg Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa His Ala Phe Ser Met Gly Lys Lys Ser Gly Lys Lys
           115                 120                 125
   Leu Phe Val Thr Asn His Glu Lys Met Pro Phe Ser Lys Val Lys Ser
       130                 135                 140
   Leu Cys Thr Glu Leu Gln Gly Thr Val Ala Ile Pro Arg Asn Ala Glu
   145                 150                 155                 160
   Glu Asn Lys Ala Ile Gln Glu Val Ala Thr Gly Ile Ala Phe Leu Gly
                   165                 170                 175
   Ile Thr Asp Glu Ala Thr Glu Gly Gln Phe Met Tyr Val Thr Gly Gly
               180                 185                 190
   Arg Leu Thr Tyr Ser Asn Trp Lys Lys Asp Glu Pro Asn Asn His Gly
           195                 200                 205
   Ser Gly Glu Asp Cys Val Ile Ile Leu Asp Asn Gly Leu Trp Asn Asp
       210                 215                 220
   Ile Ser Cys Gln Ala Ser Phe Lys Ala Val Cys Glu Phe Pro Ala
   225 230 235
<210> 16
   <211> 185
   <212> PRT
   <213> Oncorhynchus mykiss
<400> 16
   Met Glu Lys Leu Ala Ile Leu Leu Leu Leu Ser Ala Ser Ile Ala Leu
   1 5 10 15
   Gly Asp Ala Asn Leu Thr Gln Leu Leu Gly Leu Glu Pro Leu Leu Lys
   20 25 30
   Thr Lys Val Glu Gln Thr Thr Pro Glu Ala Gln Val Glu Ala Val Gln
   35 40 45
   Glu Gly Ile Lys Glu Gly Ser Cys Pro Ser Asp Trp Tyr Thr Tyr Gly
   50 55 60
   Ser His Cys Phe Lys Phe Val Ser Ile Gln Gln Ser Phe Val Asp Ser
   65 70 75 80
   Glu Gln Asn Cys Leu Ala Leu Gly Gly Asn Leu Ala Ser Val His Ser
   85 90 95
   Leu Leu Glu Tyr Gln Phe Met Gln Ala Leu Thr Lys Asp Ala Asn Gly
   100 105 110
   His Leu His Ser Thr Trp Leu Gly Gly Phe Asp Ala Ile Lys Glu Gly
   115 120 125
   Thr Trp Met Trp Ser Asp Gly Ser Arg Phe Asp Tyr Thr Asn Trp Asp
   130 135 140
   Thr Asp Glu Pro Asn Asn Ala Gly Glu Gly Glu Asp Cys Leu His Met
   145 150 155 160
   Asn Ala Ala Ser Ala Lys Leu Trp Phe Asp Val Pro Cys Glu Trp Lys
   165 170 175
   Phe Ala Ser Leu Cys Ser Arg Arg Met
   180 185
<210> 17
   <211> 240
   <212> PRT
   <213> Sus scrofa
<400> 17
   Met Ser Leu Phe Pro Ser Leu His Leu Leu Leu Leu Ile Val Met Thr
   1               5                   10                  15
   Ala Ser His Thr Glu Thr Glu Asn Cys Glu Asp Ile Gln Asn Thr Cys
               20                  25                  30
   Leu Val Ile Ser Cys Asp Ser Pro Gly Ile Asn Gly Leu Pro Gly Lys
           35                  40                  45
   Asp Gly Leu Asp Gly Ala Lys Gly Glu Lys Gly Glu Pro Gly Gln Gly
       50                  55                  60
   Leu Ile Gly Leu Gln Gly Leu Pro Gly Met Val Gly Pro Gln Gly Ser
   65                  70                  75                  80
   Pro Gly Ile Pro Gly Leu Pro Gly Leu Lys Gly Gln Lys Gly Asp Ser
                   85                  90                  95
   Gly Ile Asp Pro Gly Asn Ser Leu Ala Asn Leu Arg Ser Glu Leu Asp
               100                 105                 110
   Asn Ile Lys Lys Trp Leu Ile Phe Ala Gln Gly Lys Gln Val Gly Lys
           115                 120                 125
   Lys Leu Tyr Leu Thr Asn Gly Lys Lys Met Ser Phe Asn Gly Val Lys
       130                 135                 140
   Ala Leu Cys Ala Gln Phe Gln Ala Ser Val Ala Thr Pro Thr Asn Ser
   145                 150                 155                 160
   Arg Glu Asn Gln Ala Ile Gln Glu Leu Ala Gly Thr Glu Ala Phe Leu
                   165                 170                 175
   Gly Ile Thr Asp Glu Tyr Thr Glu Gly Gln Phe Val Asp Leu Thr Gly
               180                 185                 190
   Lys Arg Val Arg Tyr Gln Asn Trp Asn Asp Gly Glu Pro Asn Asn Ala
   195 200 205
   Asp Ser Ala Glu His Cys Val Glu Ile Leu Lys Asp Gly Lys Trp Asn
   210 215 220
   Asp Ile Phe Cys Ser Ser Gln Leu Ser Ala Val Cys Glu Phe Pro Ala
   225 230 235 240
<210> 18
   <211> 239
   <212> PRT
   <213> Mus musculus
<400> 18
   Met Leu Leu Leu Pro Leu Leu Pro Val Leu Leu Cys Val Val Ser Val
   1 5 10 15
   Ser Ser Ser Gly Ser Gln Thr Cys Glu Asp Thr Leu Lys Thr Cys Ser
   20 25 30
   Val Ile Ala Cys Gly Arg Asp Gly Arg Asp Gly Pro Lys Gly Glu Lys
   35 40 45
   Gly Glu Pro Gly Gln Gly Leu Arg Gly Leu Gln Gly Pro Pro Gly Lys
   50 55 60
   Leu Gly Pro Pro Gly Ser Val Gly Ser Pro Gly Ser Pro Gly Pro Lys
   65 70 75 80
   Gly Gln Lys Gly Asp His Gly Asp Asn Arg Ala Ile Glu Glu Lys Leu
   85 90 95
   Ala Asn Met Glu Ala Glu Ile Arg Ile Leu Lys Ser Lys Leu Gln Leu
   100 105 110
   Thr Asn Lys Leu His Ala Phe Ser Met Gly Lys Lys Ser Gly Lys Lys
   115 120 125
   Leu Phe Val Thr Asn His Glu Lys Met Pro Phe Ser Lys Val Lys Ser
   130 135 140
   Leu Cys Thr Glu Leu Gln Gly Thr Val Ala Ile Pro Arg Asn Ala Glu
   145 150 155 160
   Glu Asn Lys Ala Ile Gln Glu Val Ala Thr Gly Ile Ala Phe Leu Gly
   165 170 175
   Ile Thr Asp Glu Ala Thr Glu Gly Gln Phe Met Tyr Val Thr Gly Gly
   180 185 190
   Arg Leu Thr Tyr Ser Asn Trp Lys Lys Asp Glu Pro Asn Asn His Gly
   195 200 205
   Ser Gly Glu Asp Cys Val Ile Ile Leu Asp Asn Gly Leu Trp Asn Asp
   210 215 220
   Ile Ser Cys Gln Ala Ser Phe Lys Ala Val Cys Glu Phe Pro Ala
   225 230 235
<210> 19
   <211> 244
   <212> PRT
   <213> Mus musculus
<400> 19
   Met Ser Ile Phe Thr Ser Phe Leu Leu Leu Cys Val Val Thr Val Val
   1 5 10 15
   Tyr Ala Glu Thr Leu Thr Glu Gly Val Gln Asn Ser Cys Pro Val Val
   20 25 30
   Thr Cys Ser Ser Pro Gly Leu Asn Gly Phe Pro Gly Lys Asp Gly Arg
   35 40 45
   Asp Gly Ala Lys Gly Glu Lys Gly Glu Pro Gly Gln Gly Leu Arg Gly
   50 55 60
   Leu Gln Gly Pro Pro Gly Lys Val Gly Pro Thr Gly Pro Pro Gly Asn
   65 70 75 80
   Pro Gly Leu Lys Gly Ala Val Gly Pro Lys Gly Asp Arg Gly Asp Arg
   85 90 95
   Ala Glu Phe Asp Thr Ser Glu Ile Asp Ser Glu Ile Ala Ala Leu Arg
   100 105 110
   Ser Glu Leu Arg Ala Leu Arg Asn Trp Val Leu Phe Ser Leu Ser Glu
   115 120 125
   Lys Val Gly Lys Lys Tyr Phe Val Ser Ser Val Lys Lys Met Ser Leu
   130 135 140
   Asp Arg Val Lys Ala Leu Cys Ser Glu Phe Gln Gly Ser Val Ala Thr
   145 150 155 160
   Pro Arg Asn Ala Glu Glu Asn Ser Ala Ile Gln Lys Val Ala Lys Asp
   165 170 175
   Ile Ala Tyr Leu Gly Ile Thr Asp Val Arg Val Glu Gly Ser Phe Glu
   180 185 190
   Asp Leu Thr Gly Asn Arg Val Arg Tyr Thr Asn Trp Asn Asp Gly Glu
   195 200 205
   Pro Asn Asn Thr Gly Asp Gly Glu Asp Cys Val Val Ile Leu Gly Asn
   210 215 220
   Gly Lys Trp Asn Asp Val Pro Cys Ser Asp Ser Phe Leu Ala Ile Cys
   225 230 235 240
   Glu Phe Ser Asp
<210> 20
   <211> 381
   <212> PRT
   <213> Macaca nemestrina
<400> 20
   Met Ser Asp Ser Lys Glu Pro Arg Leu Gln Gln Leu Asp Leu Leu Glu
   1 5 10 15
   Glu Glu Gln Leu Gly Gly Val Gly Phe Arg Gln Thr Arg Gly Tyr Lys
   20 25 30
   Ser Leu Ala Gly Cys Leu Gly His Gly Pro Leu Val Leu Gln Leu Leu
   35 40 45
   Ser Phe Thr Leu Leu Ala Gly Leu Leu Val Gln Val Ser Lys Val Pro
   50 55 60
   Ser Ser Leu Ser Gln Gly Gln Ser Lys Gln Asp Ala Ile Tyr Gln Asn
   65 70 75 80
   Leu Thr Gln Leu Lys Val Ala Val Ser Glu Leu Ser Glu Lys Ser Lys
   85 90 95
   Gln Gln Glu Ile Tyr Gln Glu Leu Thr Arg Leu Lys Ala Ala Val Gly
   100 105 110
   Glu Leu Pro Glu Lys Ser Lys Gln Gln Glu Ile Tyr Glu Glu Leu Thr
   115 120 125
   Arg Leu Arg Ala Ala Val Gly Glu Leu Pro Glu Lys Ser Lys Leu Gln
   130 135 140
   Glu Ile Tyr Gln Glu Leu Thr Arg Leu Lys Ala Ala Val Gly Glu Leu
   145 150 155 160
   Pro Glu Lys Ser Lys Gln Gln Glu Ile Tyr Gln Glu Leu Ser Arg Leu
   165 170 175
   Lys Ala Ala Val Gly Asp Leu Pro Glu Lys Ser Lys Gln Gln Glu Ile
   180 185 190
   Tyr Gln Lys Leu Thr Gln Leu Lys Ala Ala Val Asp Gly Leu Pro Asp
   195 200 205
   Arg Ser Lys Gln Gln Glu Ile Tyr Gln Glu Leu Ile Gln Leu Lys Ala
   210 215 220
   Ala Val Glu Arg Leu Cys His Pro Cys Pro Trp Glu Trp Thr Phe Phe
   225 230 235 240
   Gln Gly Asn Cys Tyr Phe Met Ser Asn Ser Gln Arg Asn Trp His Asp
   245 250 255
   Ser Ile Thr Ala Cys Gln Glu Val Gly Ala Gln Leu Val Val Ile Lys
   260 265 270
   Ser Ala Glu Glu Gln Asn Phe Leu Gln Leu Gln Ser Ser Arg Ser Asn
   275 280 285
   Arg Phe Thr Trp Met Gly Leu Ser Asp Leu Asn His Glu Gly Thr Trp
   290 295 300
   Gln Trp Val Asp Gly Ser Pro Leu Leu Pro Ser Phe Lys Gln Tyr Trp
   305 310 315 320
   Asn Lys Gly Glu Pro Asn Asn Val Gly Glu Glu Asp Cys Ala Glu Phe
   325 330 335
   Ser Gly Asn Gly Trp Asn Asp Asp Lys Cys Asn Leu Ala Lys Phe Trp
   340 345 350
   Ile Cys Lys Lys Ser Ala Ala Ser Cys Ser Gly Asp Glu Glu Arg Leu
   355 360 365
   Leu Ser Pro Ala Pro Thr Thr Pro Asn Pro Pro Pro Ala
   370 375 380
<210> 21
   <211> 246
   <212> PRT
   <213> Carassius auratus
<400> 21
   Leu Leu Leu Leu Gln Phe Ala Leu Gln Leu Leu Asp Gly Ala Glu Pro
   1 5 10 15
   Gln Asn Leu Asn Cys Pro Ala Tyr Gly Gly Val Pro Gly Thr Pro Gly
   20 25 30
   His Asn Gly Leu Pro Gly Arg Asp Gly Arg Asp Gly Lys Asp Gly Ala
   35 40 45
   Ile Gly Pro Lys Gly Glu Lys Gly Glu Ser Gly Val Ser Val Gln Gly
   50 55 60
   Pro Pro Gly Lys Ala Gly Pro Pro Gly Thr Ala Gly Glu Lys Gly Glu
   65 70 75 80
   Arg Gly Pro Ser Gly Pro Gln Gly Ser Pro Gly Ser Glu Ser Val Leu
   85 90 95
   Glu Ser Leu Lys Ser Glu Ile Gln Gln Leu Lys Ala Lys Ile Ala Thr
   100 105 110
   Phe Glu Lys Val Ser Ser Val Cys His Phe Arg Lys Val Gly Gln Lys
   115 120 125
   Tyr Tyr Ile Thr Asp Gly Val Val Gly Asn Phe Asp Gln Gly Leu Lys
   130 135 140
   Ser Cys Met Glu Phe Gly Gly Thr Met Val Ser Pro Arg Thr Ser Ala
   145 150 155 160
   Glu Asn Gln Ala Leu Leu Lys Leu Val Val Ser Ser Gly Leu Gly Ser
   165 170 175
   Lys Lys Pro Tyr Ile Gly Val Thr Asp Arg Lys Thr Glu Gly Gln Phe
   180 185 190
   Val Asp Thr Glu Gly Lys Gln Leu Thr Phe Thr Asn Trp Gly Pro Gly
   195 200 205
   Gln Pro Asp Asp Tyr Lys Gly Leu Gln Asp Cys Gly Val Ile Glu Asp
   210 215 220
   Thr Gly Leu Trp Asp Asp Gly Gly Cys Gly Asp Ile Arg Pro Ile Met
   225 230 235 240
   Cys Glu Ile Asp Ile Lys
   245
<210> 22
   <211> 251
   <212> PRT
   <213> Danio rerio
<400> 22
   Met Ala Leu Leu Lys Leu Phe Leu Gly Ala Leu Leu Leu Leu Gln Leu
   1 5 10 15
   Val Leu Gln Leu Met Ala Gly Ala Ala Asp Pro Gln Ser Leu Asn Cys
   20 25 30
   Pro Ala Tyr Ala Gly Val Pro Gly Thr Pro Gly His Asn Gly Leu Pro
   35 40 45
   Gly Arg Asp Gly Arg Val Gly Arg Asp Gly Ala Asn Gly Pro Lys Gly
   50 55 60
   Glu Lys Gly Glu Pro Gly Val Asn Val  Gln Gly Pro Pro Gly Lys Ala
   65                  70                  75                  80
   Gly Pro Pro Gly Pro Ala Gly Ala Lys Gly Glu Arg Gly Pro Ser Gly
                   85                  90                  95
   Leu Pro Gly Gln Asp Cys Met Ser Asp Ser Leu Lys Ser Glu Leu Gln
               100                 105                 110
   Lys Leu Ser Asp Lys Ile Ala Leu Ile Glu Lys Val Val Asn Phe Lys
           115                 120                 125
   Thr Phe Lys Lys Val Gly Gln Lys Tyr Tyr Val Thr Asp Asp Val Glu
       130                 135                 140
   Glu Thr Phe Asp Lys Gly Met Gln Tyr Cys Ser Ser Asn Gly Gly Ala
   145                 150                 155                 160
   Leu Val Leu Pro Arg Thr Leu Glu Glu Asn Ala Leu Leu Lys Val Phe
                   165                 170                 175
   Val Ser Ser Ala Phe Lys Arg Leu Phe Ile Arg Ile Thr Asp Arg Glu
               180                 185                 190
   Lys Glu Gly Glu Phe Val Asp Thr Asp Arg Lys Lys Leu Thr Phe Thr
           195                 200                 205
   Asn Trp Gly Pro Asn Gln Pro Asp Asn Tyr Lys Gly Ala Gln Asp Cys
       210                 215                 220
   Gly Ala Ile Ala Asp Ser Gly Leu Trp Asp Asp Val Ser Cys Asp Ser
   225                 230                 235                 240
   Leu Tyr Pro Ile Ile Cys Glu Ile Glu Ile Lys
                   245                 250
<210> 23
   <211> 256
   <212> PRT
   <213> Cyprinus carpio
<400> 23
   Met Ala Leu Phe Lys Leu Phe Leu Gly Thr Leu Leu Leu Leu Gln Phe
   1               5                   10                  15
   Ala Leu Gln Leu Leu Asp Gly Ala Glu Pro Gln Asn Leu Asn Cys Pro
   20 25 30
   Ala Tyr Gly Gly Val Pro Gly Thr Pro Gly His Asn Gly Leu Pro Gly
   35 40 45
   Arg Asp Gly Arg Asp Gly Lys Asp Gly Ala Ile Gly Pro Lys Gly Glu
   50 55 60
   Lys Gly Glu Ser Gly Val Ser Val Gln Gly Pro Pro Gly Lys Ala Gly
   65 70 75 80
   Pro Pro Gly Pro Ala Gly Glu Lys Gly Glu Arg Gly Pro Thr Gly Ser
   85 90 95
   Gln Gly Ser Pro Gly Ser Glu Ser Val Leu Glu Ser Leu Lys Ser Glu
   100 105 110
   Ile Gln Gln Leu Lys Ala Lys Ile Ala Thr Phe Glu Lys Val Ala Ser
   115 120 125
   Val Gly His Phe Arg Gln Val Gly Gln Lys Tyr Tyr Ile Thr Asp Gly
   130 135 140
   Val Val Gly Thr Phe Asp Gln Gly Leu Lys Phe Cys Lys Asp Phe Gly
   145 150 155 160
   Gly Thr Met Val Phe Pro Arg Thr Ser Ala Glu Asn Gln Ala Leu Leu
   165 170 175
   Lys Leu Val Val Ser Ser Gly Leu Ser Ser Lys Lys Pro Tyr Ile Gly
   180 185 190
   Val Thr Asp Arg Glu Thr Glu Gly Arg Phe Val Asn Thr Glu Gly Lys
   195 200 205
   Gln Leu Thr Phe Thr Asn Trp Gly Pro Gly Gln Pro Asp Asp Tyr Lys
   210 215 220
   Gly Leu Gln Asp Cys Gly Val Ile Glu Asp Ser Gly Leu Trp Asp Asp
   225 230 235 240
   Gly Ser Cys Gly Asp Ile Arg Pro Ile Met Cys Glu Ile Asp Asn Lys
   245 250 255
<210> 24
   <211> 31
   <212> PRT
   <213> Sus scrofa
<400> 24
   Thr Lys Gly Glu Lys Gly Glu Pro Gly Pro Gly Phe Arg Gly Ser Gln
   1               5                   10                  15
   Gly Pro Pro Gly Lys Met Gly Pro Pro Gly Asn Ile Gly Glu Thr
               20                  25                  30
<210> 25
   <211> 254
   <212> PRT
   <213> Gallus gallus
<400> 25
   Met Thr Leu Leu Gln Pro Phe Ser Ala Leu Leu Leu Cys Leu Ser Leu
   1               5                   10                  15
   Met Met Ala Thr Ser Leu Leu Thr Thr Asp Lys Pro Glu Glu Lys Met
               20                  25                  30
   Tyr Ser Cys Pro Ile Ile Gln Cys Ser Ala Pro Ala Val Asn Gly Leu
           35                  40                  45
   Pro Gly Arg Asp Gly Arg Asp Gly Pro Lys Gly Glu Lys Gly Asp Pro
       50                  55                  60
   Gly Glu Gly Leu Arg Gly Leu Gln Gly Leu Pro Gly Lys Ala Gly Pro
   65                  70                  75                  80
   Gln Gly Leu Lys Gly Glu Val Gly Pro Gln Gly Glu Lys Gly Gln Lys
                   85                  90                  95
   Gly Glu Arg Gly Ile Val Val Thr Asp Asp Leu His Arg Gln Ile Thr
               100                 105                 110
   Asp Leu Glu Ala Lys Ile Arg Val Leu Glu Asp Asp Leu Ser Arg Tyr
           115                 120                 125
   Lys Lys Ala Leu Ser Leu Lys Asp Val Val Asn Val Gly Lys Lys Met
   130 135 140
   Phe Val Ser Thr Gly Lys Lys Tyr Asn Phe Glu Lys Gly Lys Ser Leu
   145 150 155 160
   Cys Ala Lys Ala Gly Ser Val Leu Ala Ser Pro Arg Asn Glu Ala Glu
   165 170 175
   Asn Thr Ala Leu Lys Asp Leu Ile Asp Pro Ser Ser Gln Ala Tyr Ile
   180 185 190
   Gly Ile Ser Asp Ala Gln Thr Glu Gly Arg Phe Met Tyr Leu Ser Gly
   195 200 205
   Gly Pro Leu Thr Tyr Ser Asn Trp Lys Pro Gly Glu Pro Asn Asn His
   210 215 220
   Lys Asn Glu Asp Cys Ala Val Ile Glu Asp Ser Gly Lys Trp Asn Asp
   225 230 235 240
   Leu Asp Cys Ser Asn Ser Asn Ile Phe Ile Ile Cys Glu Leu
   245 250
<210> 26
   <211> 244
   <212> PRT
   <213> Mus musculus
<400> 26
   Met Ser Ile Phe Thr Ser Phe Leu Leu Leu Cys Val Val Thr Val Val
   1 5 10 15
   Tyr Ala Glu Thr Leu Thr Glu Gly Val Gln Asn Ser Cys Pro Val Val
   20 25 30
   Thr Cys Ser Ser Pro Gly Leu Asn Gly Phe Pro Gly Lys Asp Gly Arg
   35 40 45
   Asp Gly Ala Lys Gly Glu Lys Gly Glu Pro Gly Gln Gly Leu Arg Gly
   50 55 60
   Leu Gln Gly Pro Pro Gly Lys Val Gly Pro Thr Gly Pro Pro Gly Asn
   65 70 75 80
   Pro Gly Leu Lys Gly Ala Val Gly Pro Lys Gly Asp Arg Gly Asp Arg
   85 90 95
   Ala Glu Phe Asp Thr Ser Glu Ile Asp Ser Glu Ile Ala Ala Leu Arg
   100 105 110
   Ser Glu Leu Arg Ala Leu Arg Asn Trp Val Leu Phe Ser Leu Ser Glu
   115 120 125
   Lys Val Gly Lys Lys Tyr Phe Val Ser Ser Val Lys Lys Met Ser Leu
   130 135 140
   Asp Arg Val Lys Ala Leu Cys Ser Glu Phe Gln Gly Ser Val Ala Thr
   145 150 155 160
   Pro Arg Asn Ala Glu Glu Asn Ser Ala Ile Gln Lys Val Ala Lys Asp
   165 170 175
   Ile Ala Tyr Leu Gly Ile Thr Asp Val Arg Val Glu Gly Ser Phe Glu
   180 185 190
   Asp Leu Thr Gly Asn Arg Val Arg Tyr Thr Asn Trp Asn Asp Gly Glu
   195 200 205
   Pro Asn Asn Thr Gly Asp Gly Glu Asp Cys Val Val Ile Leu Gly Asn
   210 215 220
   Gly Lys Trp Asn Asp Val Pro Cys Ser Asp Ser Phe Leu Ala Ile Cys
   225 230 235 240
   Glu Phe Ser Asp
<210> 27
   <211> 239
   <212> PRT
   <213> Mus musculus
<400> 27
   Met Leu Leu Leu Pro Leu Leu Pro Val Leu Leu Cys Val Val Ser Val
   1 5 10 15
   Ser Ser Ser Gly Ser Gln Thr Cys Glu Asp Thr Leu Lys Thr Cys Ser
   20 25 30
   Val Ile Ala Cys Gly Arg Asp Gly Arg Asp Gly Pro Lys Gly Glu Lys
   35 40 45
   Gly Glu Pro Gly Gln Gly Leu Arg Gly Leu Gln Gly Pro Pro Gly Lys
   50 55 60
   Leu Gly Pro Pro Gly Ser Val Gly Ser Pro Gly Ser Pro Gly Pro Lys
   65 70 75 80
   Gly Gln Lys Gly Asp His Gly Asp Asn Arg Ala Ile Glu Glu Lys Leu
   85 90 95
   Ala Asn Met Glu Ala Glu Ile Arg Ile Leu Lys Ser Lys Leu Gln Leu
   100 105 110
   Thr Asn Lys Leu His Ala Phe Ser Met Gly Lys Lys Ser Gly Lys Lys
   115 120 125
   Leu Phe Val Thr Asn His Glu Lys Met Pro Phe Ser Lys Val Lys Ser
   130 135 140
   Leu Cys Thr Glu Leu Gln Gly Thr Val Ala Ile Pro Arg Asn Ala Glu
   145 150 155 160
   Glu Asn Lys Ala Ile Gln Glu Val Ala Thr Gly Ile Ala Phe Leu Gly
   165 170 175
   Ile Thr Asp Glu Ala Thr Glu Gly Gln Phe Met Tyr Val Thr Gly Gly
   180 185 190
   Arg Leu Thr Tyr Ser Asn Trp Lys Lys Asp Glu Pro Asn Asn His Gly
   195 200 205
   Ser Gly Glu Asp Cys Val Ile Ile Leu Asp Asn Gly Leu Trp Asn Asp
   210 215 220
   Ile Ser Cys Gln Ala Ser Phe Lys Ala Val Cys Glu Phe Pro Ala
   225 230 235
<210> 28
   <211> 238
   <212> PRT
   <213> Rattus norvegicus
<400> 28
   Met Leu Leu Leu Pro Leu Leu Val Leu Leu Cys Val Val Ser Val Ser
   1 5 10 15
   Ser Ser Gly Ser Gln Thr Cys Glu Glu Thr Leu Lys Thr Cys Ser Val
   20 25 30
   Ile Ala Cys Gly Arg Asp Gly Arg Asp Gly Pro Lys Gly Glu Lys Gly
   35 40 45
   Glu Pro Gly Gln Gly Leu Arg Gly Leu Gln Gly Pro Pro Gly Lys Leu
   50 55 60
   Gly Pro Pro Gly Ser Val Gly Ala Pro Gly Ser Gln Gly Pro Lys Gly
   65 70 75 80
   Gln Lys Gly Asp Arg Gly Asp Ser Arg Ala Ile Glu Val Lys Leu Ala
   85 90 95
   Asn Met Glu Ala Glu Ile Asn Thr Leu Lys Ser Lys Leu Glu Leu Thr
   100 105 110
   Asn Lys Leu His Ala Phe Ser Met Gly Lys Lys Ser Gly Lys Lys Phe
   115 120 125
   Phe Val Thr Asn His Glu Arg Met Pro Phe Ser Lys Val Lys Ala Leu
   130 135 140
   Cys Ser Glu Leu Arg Gly Thr Val Ala Ile Pro Arg Asn Ala Glu Glu
   145 150 155 160
   Asn Lys Ala Ile Gln Glu Val Ala Lys Thr Ser Ala Phe Leu Gly Ile
   165 170 175
   Thr Asp Glu Val Thr Glu Gly Gln Phe Met Tyr Val Thr Gly Gly Arg
   180 185 190
   Leu Thr Tyr Ser Asn Trp Lys Lys Asp Glu Pro Asn Asp His Gly Ser
   195 200 205
   Gly Glu Asp Cys Val Thr Ile Val Asp Asn Gly Leu Trp Asn Asp Ile
   210 215 220
   Ser Cys Gln Ala Ser His Thr Ala Val Cys Glu Phe Pro Ala
   225 230 235
<210> 29
   <211> 244
   <212> PRT
   <213> Rattus norvegicus
<400> 29
   Met Ser Leu Phe Thr Ser Phe Leu Leu Leu Cys Val Leu Thr Ala Val
   1 5 10 15
   Tyr Ala Glu Thr Leu Thr Glu Gly Ala Gln Ser Ser Cys Pro Val Ile
   20 25 30
   Ala Cys Ser Ser Pro Gly Leu Asn Gly Phe Pro Gly Lys Asp Gly His
   35 40 45
   Asp Gly Ala Lys Gly Glu Lys Gly Glu Pro Gly Gln Gly Leu Arg Gly
   50 55 60
   Leu Gln Gly Pro Pro Gly Lys Val Gly Pro Ala Gly Pro Pro Gly Asn
   65 70 75 80
   Pro Gly Ser Lys Gly Ala Thr Gly Pro Lys Gly Asp Arg Gly Glu Ser
   85 90 95
   Val Glu Phe Asp Thr Thr Asn Ile Asp Leu Glu Ile Ala Ala Leu Arg
   100 105 110
   Ser Glu Leu Arg Ala Met Arg Lys Trp Val Leu Leu Ser Met Ser Glu
   115 120 125
   Asn Val Gly Lys Lys Tyr Phe Met Ser Ser Val Arg Arg Met Pro Leu
   130 135 140
   Asn Arg Ala Lys Ala Leu Cys Ser Glu Leu Gln Gly Thr Val Ala Thr
   145 150 155 160
   Pro Arg Asn Ala Glu Glu Asn Arg Ala Ile Gln Asn Val Ala Lys Asp
   165 170 175
   Val Ala Phe Leu Gly Ile Thr Asp Gln Arg Thr Glu Asn Val Phe Glu
   180 185 190
   Asp Leu Thr Gly Asn Arg Val Arg Tyr Thr Asn Trp Asn Glu Gly Glu
   195 200 205
   Pro Asn Asn Val Gly Ser Gly Glu Asn Cys Val Val Leu Leu Thr Asn
   210 215 220
   Gly Lys Trp Asn Asp Val Pro Cys Ser Asp Ser Phe Leu Val Val Cys
   225 230 235 240
   Glu Phe Ser Asp
<210> 30
   <211> 248
   <212> PRT
   <213> Homo sapiens
<400> 30
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 31
   <211> 329
   <212> PRT
   <213> Homo sapiens
<400> 31
   Met Trp Cys Ile Val Leu Phe Ser Leu Leu Ala Trp Val Tyr Ala Glu
   1 5 10 15
   Pro Thr Met Tyr Gly Glu Ile Leu Ser Pro Asn Tyr Pro Gln Ala Tyr
   20 25 30
   Pro Ser Glu Val Glu Lys Ser Trp Asp Ile Glu Val Pro Glu Gly Tyr
   35 40 45
   Gly Ile His Leu Tyr Phe Thr His Leu Asp Ile Glu Leu Ser Glu Asn
   50 55 60
   Cys Ala Tyr Asp Ser Val Gln Ile Ile Ser Gly Asp Thr Glu Glu Gly
   65 70 75 80
   Arg Leu Cys Gly Gln Arg Ser Ser Asn Asn Pro His Ser Pro Ile Val
   85 90 95
   Glu Glu Phe Gln Val Pro Tyr Asn Lys Leu Gln Val Ile Phe Lys Ser
   100 105 110
   Asp Phe Ser Asn Glu Glu Arg Phe Thr Gly Phe Ala Ala Tyr Tyr Val
   115 120 125
   Ala Thr Asp Ile Asn Glu Cys Thr Asp Phe Val Asp Val Pro Cys Ser
   130 135 140
   His Phe Cys Asn Asn Phe Ile Gly Gly Tyr Phe Cys Ser Cys Pro Pro
   145 150 155 160
   Glu Tyr Phe Leu His Asp Asp Met Lys Asn Cys Gly Val Asn Cys Ser
   165 170 175
   Gly Asp Val Phe Thr Ala Leu Ile Gly Glu Ile Ala Ser Pro Asn Tyr
   180 185 190
   Pro Lys Pro Tyr Pro Glu Asn Ser Arg Cys Glu Tyr Gln Ile Arg Leu
   195 200 205
   Glu Lys Gly Phe Gln Val Val Val Thr Leu Arg Arg Glu Asp Phe Asp
   210 215 220
   Val Glu Ala Ala Asp Ser Ala Gly Asn Cys Leu Asp Ser Leu Val Phe
   225 230 235 240
   Val Ala Gly Asp Arg Gln Phe Gly Pro Tyr Cys Gly His Gly Phe Pro
   245 250 255
   Gly Pro Leu Asn Ile Glu Thr Lys Ser Asn Ala Leu Asp Ile Ile Phe
   260 265 270
   Gln Thr Asp Leu Thr Gly Gln Lys Lys Gly Trp Lys Leu Arg Tyr His
   275 280 285
   Gly Asp Pro Met Pro Cys Pro Lys Glu Asp Thr Pro Asn Ser Val Trp
   290 295 300
   Glu Pro Ala Lys Ala Lys Tyr Val Phe Arg Asp Val Val Gln Ile Thr
   305 310 315 320
   Cys Leu Asp Gly Phe Glu Val Val Glu
   325
<210> 32
   <211> 248
   <212> PRT
   <213> Homo sapiens
<400> 32
   Met Ser Leu Phe Pro Ser Leu Pro Leu Leu Leu Leu Ser Met Val Ala
   1               5                   10                  15
   Ala Ser Tyr Ser Glu Thr Val Thr Cys Glu Asp Ala Gln Lys Thr Cys
               20                  25                  30
   Pro Ala Val Ile Ala Cys Ser Ser Pro Gly Ile Asn Gly Phe Pro Gly
           35                  40                  45
   Lys Asp Gly Cys Asp Gly Thr Lys Gly Glu Lys Gly Glu Pro Gly Gln
       50                  55                  60
   Gly Leu Arg Gly Leu Gln Gly Pro Pro Gly Lys Leu Gly Pro Pro Gly
   65                  70                  75                  80
   Asn Pro Gly Pro Ser Gly Ser Pro Gly Pro Lys Gly Gln Lys Gly Asp
                   85                  90                  95
   Pro Gly Lys Ser Pro Asp Gly Asp Ser Ser Leu Ala Ala Ser Glu Arg
               100                 105                 110
   Lys Ala Leu Gln Thr Glu Met Ala Arg Ile Lys Lys Trp Leu Thr Phe
           115                 120                 125
   Ser Leu Gly Lys Gln Val Gly Asn Lys Phe Phe Leu Thr Asn Gly Glu
       130                 135                 140
   Ile Met Thr Phe Glu Lys Val Lys Ala Leu Cys Val Lys Phe Gln Ala
   145                 150                 155                 160
   Ser Val Ala Thr Pro Arg Asn Ala Ala Glu Asn Gly Ala Ile Gln Asn
                   165                 170                 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 33
   <211> 248
   <212> PRT
   <213> Homo sapiens
<400> 33
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 34
   <211> 248
   <212> PRT
   <213> Homo sapiens
<400> 34
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 35
   <211> 248
   <212> PRT
   <213> Homo sapiens
<400> 35
<210> 36
   <211> 248
   <212> PRT
   <213> Homo sapiens
<400> 36
   Met Ser Leu Phe Pro Ser Leu Pro Leu Leu Leu Leu Ser Met Val Ala
   1               5                   10                  15
   Ala Ser Tyr Ser Glu Thr Val Thr Cys Glu Asp Ala Gln Lys Thr Cys
               20                  25                  30
   Pro Ala Val Ile Ala Cys Ser Ser Pro Gly Ile Asn Gly Phe Pro Gly
           35                  40                  45
   Lys Asp Gly Arg Asp Gly Thr Lys Glu Glu Lys Gly Glu Pro Gly Gln
       50                  55                  60
   Gly Leu Arg Gly Leu Gln Gly Pro Pro Gly Lys Leu Gly Pro Pro Gly
   65                  70                  75                  80
   Asn Pro Gly Pro Ser Gly Ser Pro Gly Pro Lys Gly Gln Lys Gly Asp
                   85                  90                  95
   Pro Gly Lys Ser Pro Asp Gly Asp Ser Ser Leu Ala Ala Ser Glu Arg
               100                 105                 110
   Lys Ala Leu Gln Thr Glu Met Ala Arg Ile Lys Lys Trp Leu Thr Phe
           115                 120                 125
   Ser Leu Gly Lys Gln Val Gly Asn Lys Phe Phe Leu Thr Asn Gly Glu
       130                 135                 140
   Ile Met Thr Phe Glu Lys Val Lys Ala Leu Cys Val Lys Phe Gln Ala
   145                 150                 155                 160
   Ser Val Ala Thr Pro Arg Asn Ala Ala Glu Asn Gly Ala Ile Gln Asn
                   165                 170                 175
   Leu Ile Lys Glu Glu Ala Phe Leu Gly Ile Thr Asp Glu Lys Thr Glu
               180                 185                 190
   Gly Gln Phe Val Asp Leu Thr Gly Asn Arg Leu Thr Tyr Thr Asn Trp
           195                 200                 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 37
   <211> 248
   <212> PRT
   <213> Homo sapiens
<400> 37
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 38
   <211> 248
   <212> PRT
   <213> Homo sapiens
<400> 38
   Met Ser Leu Phe Pro Ser Leu Pro Leu Leu Leu Leu Ser Met Val Ala
   1 5 10 15
   Ala Ser Tyr Ser Glu Thr Val Thr Cys Glu Asp Ala Gln Lys Thr Cys
   20 25 30
   Pro Ala Val Ile Ala Cys Ser Ser Pro Gly Ile Asn Gly Phe Pro Gly
   35 40 45
   Lys Asp Gly Arg Asp Gly Thr Lys Gly Glu Lys Gly Glu Pro Gly Gln
   50 55 60
   Gly Leu Arg Gly Leu Gln Gly Pro Pro Gly Lys Leu Gly Pro Pro Gly
   65 70 75 80
   Asn Pro Gly Pro Ser Gly Ser Pro Gly Pro Lys Gly Gln Lys Gly Asp
   85 90 95
   Pro Gly Lys Ser Pro Asp Gly Asp Ser Ser Leu Ala Ala Ser Glu Arg
   100 105 110
   Lys Ala Leu Gln Thr Glu Met Ala Arg Ile Lys Lys Trp Leu Thr Phe
   115 120 125
   Ser Leu Gly Lys Gln Val Gly Asn Lys Phe Phe Leu Thr Asn Gly Glu
   130 135 140
   Ile Met Thr Phe Glu Lys Val Lys Ala Leu Cys Val Lys Phe Gln Ala
   145 150 155 160
   Ser Val Ala Thr Pro Arg Asn Ala Ala Glu Asn Gly Ala Ile Gln Asn
   165 170 175
   Leu Ile Lys Glu Glu Ala Phe Leu Gly Ile Thr Asp Glu Lys Thr Glu
   180 185 190
   Gly Gln Phe Val Asp Leu Thr Gly Asn Arg Leu Thr Tyr Thr Asn Trp
   195 200 205
   Asn Glu Gly Glu Pro Asn Asn Ala Gly Ser Asp Glu Asp Cys Val Leu
   210 215 220
   Leu Leu Lys Asn Gly Gln Trp Asn Asp Val Pro Cys Ser Thr Ser His
   225 230 235 240
   Leu Ala Val Cys Glu Phe Pro Ile
   245
<210> 39
   <211> 652
   <212> PRT
   <213> Homo sapiens
<400> 39
   Met Ala Thr Ser Met Gly Leu Leu Leu Leu Leu Leu Leu Leu Leu Thr
   1 5 10 15
   Gln Pro Gly Ala Gly Thr Gly Ala Asp Thr Glu Ala Val Val Cys Val
   20 25 30
   Gly Thr Ala Cys Tyr Thr Ala His Ser Gly Lys Leu Ser Ala Ala Glu
   35 40 45
   Ala Gln Asn His Cys Asn Gln Asn Gly Gly Asn Leu Ala Thr Val Lys
   50 55 60
   Ser Lys Glu Glu Ala Gln His Val Gln Arg Val Leu Ala Gln Leu Leu
   65 70 75 80
   Arg Arg Glu Ala Ala Leu Thr Ala Arg Met Ser Lys Phe Trp Ile Gly
   85 90 95
   Leu Gln Arg Glu Lys Gly Lys Cys Leu Asp Pro Ser Leu Pro Leu Lys
   100 105 110
   Gly Phe Ser Trp Val Gly Gly Gly Glu Asp Thr Pro Tyr Ser Asn Trp
   115 120 125
   His Lys Glu Leu Arg Asn Ser Cys Ile Ser Lys Arg Cys Val Ser Leu
   130 135 140
   Leu Leu Asp Leu Ser Gln Pro Leu Leu Pro Asn Arg Leu Pro Lys Trp
   145 150 155 160
   Ser Glu Gly Pro Cys Gly Ser Pro Gly Ser Pro Gly Ser Asn Ile Glu
   165 170 175
   Gly Phe Val Cys Lys Phe Ser Phe Lys Gly Met Cys Arg Pro Leu Ala
   180 185 190
   Leu Gly Gly Pro Gly Gln Val Thr Tyr Thr Thr Pro Phe Gln Thr Thr
   195 200 205
   Ser Ser Ser Leu Glu Ala Val Pro Phe Ala Ser Ala Ala Asn Val Ala
   210 215 220
   Cys Gly Glu Gly Asp Lys Asp Glu Thr Gln Ser His Tyr Phe Leu Cys
   225 230 235 240
   Lys Glu Lys Ala Pro Asp Val Phe Asp Trp Gly Ser Ser Gly Pro Leu
   245 250 255
   Cys Val Ser Pro Lys Tyr Gly Cys Asn Phe Asn Asn Gly Gly Cys His
   260 265 270
   Gln Asp Cys Phe Glu Gly Gly Asp Gly Ser Phe Leu Cys Gly Cys Arg
   275 280 285
   Pro Gly Phe Arg Leu Leu Asp Asp Leu Val Thr Cys Ala Ser Arg Asn
   290 295 300
   Pro Cys Ser Ser Ser Pro Cys Arg Gly Gly Ala Thr Cys Val Leu Gly
   305 310 315 320
   Pro His Gly Lys Asn Tyr Thr Cys Arg Cys Pro Gln Gly Tyr Gln Leu
   325 330 335
   Asp Ser Ser Gln Leu Asp Cys Val Asp Val Asp Glu Cys Gln Asp Ser
   340 345 350
   Pro Cys Ala Gln Glu Cys Val Asn Thr Pro Gly Gly Phe Arg Cys Glu
   355 360 365
   Cys Trp Val Gly Tyr Glu Pro Gly Gly Pro Gly Glu Gly Ala Cys Gln
   370 375 380
   Asp Val Asp Glu Cys Ala Leu Gly Arg Ser Pro Cys Ala Gln Gly Cys
   385 390 395 400
   Thr Asn Thr Asp Gly Ser Phe His Cys Ser Cys Glu Glu Gly Tyr Val
   405 410 415
   Leu Ala Gly Glu Asp Gly Thr Gln Cys Gln Asp Val Asp Glu Cys Val
   420 425 430
   Gly Pro Gly Gly Pro Leu Cys Asp Ser Leu Cys Phe Asn Thr Gln Gly
   435 440 445
   Ser Phe His Cys Gly Cys Leu Pro Gly Trp Val Leu Ala Pro Asn Gly
   450 455 460
   Val Ser Cys Thr Met Gly Pro Val Ser Leu Gly Pro Pro Ser Gly Pro
   465 470 475 480
   Pro Asp Glu Glu Asp Lys Gly Glu Lys Glu Gly Ser Thr Val Pro Arg
   485 490 495
   Ala Ala Thr Ala Ser Pro Thr Arg Gly Pro Glu Gly Thr Pro Lys Ala
   500 505 510
   Thr Pro Thr Thr Ser Arg Pro Ser Leu Ser Ser Asp Ala Pro Ile Thr
   515 520 525
   Ser Ala Pro Leu Lys Met Leu Ala Pro Ser Gly Ser Ser Gly Val Trp
   530 535 540
   Arg Glu Pro Ser Ile His His Ala Thr Ala Ala Ser Gly Pro Gln Glu
   545 550 555 560
   Pro Ala Gly Gly Asp Ser Ser Val Ala Thr Gln Asn Asn Asp Gly Thr
   565 570 575
   Asp Gly Gln Lys Leu Leu Leu Phe Tyr Ile Leu Gly Thr Val Val Ala
   580 585 590
   Ile Leu Leu Leu Leu Ala Leu Ala Leu Gly Leu Leu Val Tyr Arg Lys
   595 600 605
   Arg Arg Ala Lys Arg Glu Glu Lys Lys Glu Lys Lys Pro Gln Asn Ala
   610 615 620
   Ala Asp Ser Tyr Ser Trp Val Pro Glu Arg Ala Glu Ser Arg Ala Met
   625 630 635 640
   Glu Asn Gln Tyr Ser Pro Thr Pro Gly Thr Asp Cys
   645 650
<210> 40
   <211> 251
   <212> PRT
   <213> Danio rerio
<400> 40
   Met Ala Leu Leu Lys Leu Phe Leu Gly Ala Leu Leu Leu Leu Gln Leu
   1 5 10 15
   Val Leu Gln Leu Met Ala Gly Ala Ala Asp Pro Gln Ser Leu Asn Cys
   20 25 30
   Pro Ala Tyr Ala Gly Val Pro Gly Thr Pro Gly His Asn Gly Leu Pro
   35 40 45
   Gly Arg Asp Gly Arg Val Gly Arg Asp Gly Ala Asn Gly Pro Lys Gly
   50 55 60
   Glu Lys Gly Glu Pro Gly Val Asn Val Gln Gly Pro Pro Gly Lys Ala
   65 70 75 80
   Gly Pro Pro Gly Pro Ala Gly Ala Lys Gly Glu Arg Gly Pro Ser Gly
   85 90 95
   Leu Pro Gly Gln Asp Cys Met Ser Asp Ser Leu Lys Ser Glu Leu Gln
   100 105 110
   Lys Leu Ser Asp Lys Ile Ala Leu Ile Glu Lys Val Val Asn Phe Lys
   115 120 125
   Thr Phe Lys Lys Val Gly Gln Lys Tyr Tyr Val Thr Asp Asp Val Glu
   130 135 140
   Glu Thr Phe Asp Lys Gly Met Gln Tyr Cys Ser Ser Asn Gly Gly Ala
   145 150 155 160
   Leu Val Leu Pro Arg Thr Leu Glu Glu Asn Ala Leu Leu Lys Val Phe
   165 170 175
   Val Ser Ser Ala Phe Lys Arg Leu Phe Ile Arg Ile Thr Asp Arg Glu
   180 185 190
   Lys Glu Gly Glu Phe Val Asp Thr Asp Arg Lys Lys Leu Thr Phe Thr
   195 200 205
   Asn Trp Gly Pro Asn Gln Pro Asp Asn Tyr Lys Gly Ala Gln Asp Cys
   210 215 220
   Gly Ala Ile Ala Asp Ser Gly Leu Trp Asp Asp Val Ser Cys Asp Ser
   225 230 235 240
   Leu Tyr Pro Ile Ile Cys Glu Ile Glu Ile Lys
   245 250
<210> 41
   <211> 118
   <212> PRT
   <213> Cyprinus carpio
<400> 41
   Lys Ile Gln Thr Gly Ser Asn Thr Val Ser Ile Leu Phe His Ser Asp
   1 5 10 15
   Asn Ser Gly Asp Asn Leu Gly Trp Lys Leu Thr Tyr Thr Ser Thr Gly
   20 25 30
   Ser Glu Cys Ser Pro Leu Ala Ala Pro Leu Asn Gly His Leu Glu Pro
   35 40 45
   Leu Gln Ser Asn Tyr Ile Phe Lys Asp His Ile Met Leu Thr Cys Asp
   50 55 60
   Pro Gly Tyr Ser Leu Arg Gln Gly Asp Lys Glu Phe Glu His Tyr Gln
   65 70 75 80
   Ile Glu Cys Gln Arg Asp Gly Lys Trp Ser Ser Asp Val Pro Leu Cys
   85 90 95
   Lys Lys Lys Glu Ser Gln Arg Arg His Arg Ser Leu Pro Ser Ile Leu
   100 105 110
   Thr Asn Gln Ile Leu Ser
   115
<210> 42
   <211> 652
   <212> PRT
   <213> Homo sapiens
<400> 42
   Met Ala Thr Ser Met Gly Leu Leu Leu Leu Leu Leu Leu Leu Leu Thr
   1 5 10 15
   Gln Pro Gly Ala Gly Thr Gly Ala Asp Thr Glu Ala Val Val Cys Val
   20 25 30
   Gly Thr Ala Cys Tyr Thr Ala His Ser Gly Lys Leu Ser Ala Ala Glu
   35 40 45
   Ala Gln Asn His Cys Asn Gln Asn Gly Gly Asn Leu Ala Thr Val Lys
   50 55 60
   Ser Lys Glu Glu Ala Gln His Val Gln Arg Val Leu Ala Gln Leu Leu
   65 70 75 80
   Arg Arg Glu Ala Ala Leu Thr Ala Arg Met Ser Lys Phe Trp Ile Gly
   85 90 95
   Leu Gln Arg Glu Lys Gly Lys Cys Leu Asp Pro Ser Leu Pro Leu Lys
   100 105 110
   Gly Phe Ser Trp Val Gly Gly Gly Glu Asp Thr Pro Tyr Ser Asn Trp
   115 120 125
   His Lys Glu Leu Arg Asn Ser Cys Ile Ser Lys Arg Cys Val Ser Leu
   130 135 140
   Leu Leu Asp Leu Ser Gln Pro Leu Leu Pro Ser Arg Leu Pro Lys Trp
   145 150 155 160
   Ser Glu Gly Pro Cys Gly Ser Pro Gly Ser Pro Gly Ser Asn Ile Glu
   165 170 175
   Gly Phe Val Cys Lys Phe Ser Phe Lys Gly Met Cys Arg Pro Leu Ala
   180 185 190
   Leu Gly Gly Pro Gly Gln Val Thr Tyr Thr Thr Pro Phe Gln Thr Thr
   195 200 205
   Ser Ser Ser Leu Glu Ala Val Pro Phe Ala Ser Ala Ala Asn Val Ala
   210 215 220
   Cys Gly Glu Gly Asp Lys Asp Glu Thr Gln Ser His Tyr Phe Leu Cys
   225 230 235 240
   Lys Glu Lys Ala Pro Asp Val Phe Asp Trp Gly Ser Ser Gly Pro Leu
   245 250 255
   Cys Val Ser Pro Lys Tyr Gly Cys Asn Phe Asn Asn Gly Gly Cys His
   260 265 270
   Gln Asp Cys Phe Glu Gly Gly Asp Gly Ser Phe Leu Cys Gly Cys Arg
   275 280 285
   Pro Gly Phe Arg Leu Leu Asp Asp Leu Val Thr Cys Ala Ser Arg Asn
   290 295 300
   Pro Cys Ser Ser Ser Pro Cys Arg Gly Gly Ala Thr Cys Val Leu Gly
   305 310 315 320
   Pro His Gly Lys Asn Tyr Thr Cys Arg Cys Pro Gln Gly Tyr Gln Leu
   325 330 335
   Asp Ser Ser Gln Leu Asp Cys Val Asp Val Asp Glu Cys Gln Asp Ser
   340 345 350
   Pro Cys Ala Gln Glu Cys Val Asn Thr Pro Gly Gly Phe Arg Cys Glu
   355 360 365
   Cys Trp Val Gly Tyr Glu Pro Gly Gly Pro Gly Glu Gly Ala Cys Gln
   370 375 380
   Asp Val Asp Glu Cys Ala Leu Gly Arg Ser Pro Cys Ala Gln Gly Cys
   385 390 395 400
   Thr Asn Thr Asp Gly Ser Phe His Cys Ser Cys Glu Glu Gly Tyr Val
   405 410 415
   Leu Ala Gly Glu Asp Gly Thr Gln Cys Gln Asp Val Asp Glu Cys Val
   420 425 430
   Gly Pro Gly Gly Pro Leu Cys Asp Ser Leu Cys Phe Asn Thr Gln Gly
   435 440 445
   Ser Phe His Cys Gly Cys Leu Pro Gly Trp Val Leu Ala Pro Asn Gly
   450 455 460
   Val Ser Cys Thr Met Gly Pro Val Ser Leu Gly Pro Pro Ser Gly Pro
   465 470 475 480
   Pro Asp Glu Glu Asp Lys Gly Glu Lys Glu Gly Ser Thr Val Pro Arg
   485 490 495
   Ala Ala Thr Ala Ser Pro Thr Arg Gly Pro Glu Gly Thr Pro Lys Ala
   500 505 510
   Thr Pro Thr Thr Ser Arg Pro Ser Leu Ser Ser Asp Ala Pro Ile Thr
   515 520 525
   Ser Ala Pro Leu Lys Met Leu Ala Pro Ser Gly Ser Pro Gly Val Trp
   530 535 540
   Arg Glu Pro Ser Ile His His Ala Thr Ala Ala Ser Gly Pro Gln Glu
   545 550 555 560
   Pro Ala Gly Gly Asp Ser Ser Val Ala Thr Gln Asn Asn Asp Gly Thr
   565 570 575
   Asp Gly Gln Lys Leu Leu Leu Phe Tyr Ile Leu Gly Thr Val Val Ala
   580 585 590
   Ile Leu Leu Leu Leu Ala Leu Ala Leu Gly Leu Leu Val Tyr Arg Lys
   595 600 605
   Arg Arg Ala Lys Arg Glu Glu Lys Lys Glu Lys Lys Pro Gln Asn Ala
   610 615 620
   Ala Asp Ser Tyr Ser Trp Val Pro Glu Arg Ala Glu Ser Arg Ala Met
   625 630 635 640
   Glu Asn Gln Tyr Ser Pro Thr Pro Gly Thr Asp Cys
   645 650
<210> 43
   <211> 742
   <212> PRT
   <213> Mus musculus
<400> 43
   Met Lys Asp Asp Phe Ala Glu Glu Glu Glu Val Gln Ser Phe Gly Tyr
   1 5 10 15
   Lys Arg Phe Gly Ile Gln Glu Gly Thr Gln Cys Thr Lys Cys Lys Asn
   20 25 30
   Asn Trp Ala Leu Lys Phe Ser Ile Val Leu Leu Tyr Ile Leu Cys Ala
   35 40 45
   Leu Leu Thr Ile Thr Val Ala Ile Leu Gly Tyr Lys Val Val Glu Lys
   50 55 60
   Met Asp Asn Val Thr Asp Gly Met Glu Thr Ser His Gln Thr Tyr Asp
   65 70 75 80
   Asn Lys Leu Thr Ala Val Glu Ser Asp Leu Lys Lys Leu Gly Asp Gln
   85 90 95
   Ala Gly Lys Lys Ala Leu Ser Thr Asn Ser Glu Leu Ser Thr Phe Arg
   100 105 110
   Ser Asp Ile Leu Asp Leu Arg Gln Gln Leu Gln Glu Ile Thr Glu Lys
   115 120 125
   Thr Ser Lys Asn Lys Asp Thr Leu Glu Lys Leu Gln Ala Asn Gly Asp
   130 135 140
   Ser Leu Val Asp Arg Gln Ser Gln Leu Lys Glu Thr Leu Gln Asn Asn
   145 150 155 160
   Ser Phe Leu Ile Thr Thr Val Asn Lys Thr Leu Gln Ala Tyr Asn Gly
   165 170 175
   Tyr Val Thr Asn Leu Gln Gln Asp Thr Ser Val Leu Gln Gly Asn Leu
   180 185 190
   Gln Ser Gln Met Tyr Ser Gln Ser Val Val Ile Met Asn Leu Asn Asn
   195 200 205
   Leu Asn Leu Thr Gln Val Gln Gln Arg Asn Leu Ile Ser Asn Leu Gln
   210 215 220
   Gln Ser Val Asp Asp Thr Ser Leu Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Ser Ser Phe Thr Gly Gln Met Asp Asn Ile Thr Thr Ile Ser
   290 295 300
   Gln Ala Asn Glu Gln Ser Leu Lys Asp Leu Gln Asp Leu His Lys Asp
   305 310 315 320
   Thr Glu Asn Arg Thr Ala Val Lys Phe Ser Gln Leu Glu Glu Arg Phe
   325 330 335
   Gln Val Phe Glu Thr Asp Ile Val Asn Ile Ile Ser Asn Ile Ser Tyr
   340 345 350
   Thr Ala His His Leu Arg Thr Leu Thr Ser Asn Leu Asn Asp Val Arg
   355 360 365
   Thr Thr Cys Thr Asp Thr Leu Thr Arg His Thr Asp Asp Leu Thr Ser
   370 375 380
   Leu Asn Asn Thr Leu Val Asn Ile Arg Leu Asp Ser Ile Ser Leu Arg
   385 390 395 400
   Met Gln Gln Asp Met Met Arg Ser Lys Leu Asp Thr Glu Val Ala Asn
   405 410 415
   Leu Ser Val Val Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Lys Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Thr Ile Gly Pro Val Gly Pro Pro Gly Glu Arg
   485 490 495
   Gly Ser Lys Gly Ser Lys Gly Ser Gln Gly Pro Lys Gly Ser Arg Gly
   500 505 510
   Ser Pro Gly Lys Pro Gly Pro Gln Gly Pro Ser Gly Asp Pro Gly Pro
   515 520 525
   Pro Gly Pro Pro Gly Lys Asp Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Met Glu Pro Leu
   580 585 590
   Ala Leu Gln Asn Glu Pro Thr Pro Ala Ser Glu Val Asn Gly Cys Pro
   595 600 605
   Pro His Trp Lys Asn Phe Thr Asp Lys Cys Tyr Tyr Phe Ser Leu Glu
   610 615 620
   Lys Glu Ile Phe Glu Asp Ala Lys Leu Phe Cys Glu Asp Lys Ser Ser
   625 630 635 640
   His Leu Val Phe Ile Asn Ser Arg Glu Glu Gln Gln Trp Ile Lys Lys
   645 650 655
   His Thr Val Gly Arg Glu Ser His Trp Ile Gly Leu Thr Asp Ser Glu
   660 665 670
   Gln Glu Ser Glu Trp Lys Trp Leu Asp Gly Ser Pro Val Asp Tyr Lys
   675 680 685
   Asn Trp Lys Ala Gly Gln Pro Asp Asn Trp Gly Ser Gly His Gly Pro
   690 695 700
   Gly Glu Asp Cys Ala Gly Leu Ile Tyr Ala Gly Gln Trp Asn Asp Phe
   705 710 715 720
   Gln Cys Asp Glu Ile Asn Asn Phe Ile Cys Glu Lys Glu Arg Glu Ala
   725 730 735
   Val Pro Ser Ser Ile Leu
   740
<210> 44
   <211> 321
   <212> PRT
   <213> Bos taurus
<400> 44
   Met Leu Pro Leu Pro Leu Ser Ile Leu Leu Leu Leu Thr Gln Ser Gln
   1 5 10 15
   Ser Phe Leu Gly Glu Glu Met Asp Val Tyr Ser Glu Lys Thr Leu Thr
   20 25 30
   Asp Pro Cys Thr Leu Val Val Cys Ala Pro Pro Ala Asp Ser Leu Arg
   35 40 45
   Gly His Asp Gly Arg Asp Gly Lys Glu Gly Pro Gln Gly Glu Lys Gly
   50 55 60
   Asp Pro Gly Pro Pro Gly Met Pro Gly Pro Ala Gly Arg Glu Gly Pro
   65 70 75 80
   Ser Gly Arg Gln Gly Ser Met Gly Pro Pro Gly Thr Pro Gly Pro Lys
                   85                  90                  95
   Gly Glu Pro Gly Pro Glu Gly Gly Val Gly Ala Pro Gly Met Pro Gly
               100                 105                 110
   Ser Pro Gly Pro Ala Gly Leu Lys Gly Glu Arg Gly Thr Pro Gly Pro
           115                 120                 125
   Gly Gly Ala Ile Gly Pro Gln Gly Pro Ser Gly Ala Met Gly Pro Pro
       130                 135                 140
   Gly Leu Lys Gly Asp Arg Gly Asp Pro Gly Glu Lys Gly Ala Arg Gly
   145                 150                 155                 160
   Glu Thr Ser Val Leu Glu Val Asp Thr Leu Arg Gln Arg Met Arg Asn
                   165                 170                 175
   Leu Glu Gly Glu Val Gln Arg Leu Gln Asn Ile Val Thr Gln Tyr Arg
               180                 185                 190
   Lys Ala Val Leu Phe Pro Asp Gly Gln Ala Val Gly Glu Lys Ile Phe
           195                 200                 205
   Lys Thr Ala Gly Ala Val Lys Ser Tyr Ser Asp Ala Glu Gln Leu Cys
       210                 215                 220
   Arg Glu Ala Lys Gly Gln Leu Ala Ser Pro Arg Ser Ser Ala Glu Asn
   225                 230                 235                 240
   Glu Ala Val Thr Gln Leu Val Arg Ala Lys Asn Lys His Ala Tyr Leu
                   245                 250                 255
   Ser Met Asn Asp Ile Ser Lys Glu Gly Lys Phe Thr Tyr Pro Thr Gly
               260                 265                 270
   Gly Ser Leu Asp Tyr Ser Asn Trp Ala Pro Gly Glu Pro Asn Asn Arg
           275                 280                 285
   Ala Lys Asp Glu Gly Pro Glu Asn Cys Leu Glu Ile Tyr Ser Asp Gly
       290                 295                 300
   Asn Trp Asn Asp Ile Glu Cys Arg Glu Glu Arg Leu Val Ile Cys Glu
   305                 310                 315                 320
   Phe
<210> 45
   <211> 371
   <212> PRT
   <213> Bos taurus
<400> 45
   Met Leu Leu Leu Pro Leu Ser Val Leu Leu Leu Leu Thr Gln Pro Trp
   1               5                   10                  15
   Arg Ser Leu Gly Ala Glu Met Lys Ile Tyr Ser Gln Lys Thr Leu Ala
               20                  25                  30
   Asn Gly Cys Thr Leu Val Val Cys Arg Pro Pro Glu Gly Gly Leu Pro
           35                  40                  45
   Gly Arg Asp Gly Gln Asp Gly Arg Glu Gly Pro Gln Gly Glu Lys Gly
       50                  55                  60
   Asp Pro Gly Ser Pro Gly Pro Ala Gly Arg Ala Gly Arg Pro Gly Pro
   65                  70                  75                  80
   Ala Gly Pro Ile Gly Pro Lys Gly Asp Asn Gly Ser Ala Gly Glu Pro
                   85                  90                  95
   Gly Pro Lys Gly Asp Thr Gly Pro Pro Gly Pro Pro Gly Met Pro Gly
               100                 105                 110
   Pro Ala Gly Arg Glu Gly Pro Ser Gly Lys Gln Gly Ser Met Gly Pro
           115                 120                 125
   Pro Gly Thr Pro Gly Pro Lys Gly Asp Thr Gly Pro Lys Gly Gly Met
       130                 135                 140
   Gly Ala Pro Gly Met Gln Gly Ser Pro Gly Pro Ala Gly Leu Lys Gly
   145                 150                 155                 160
   Glu Arg Gly Ala Pro Gly Glu Leu Gly Ala Pro Gly Ser Ala Gly Val
                   165                 170                 175
   Ala Gly Pro Ala Gly Ala Ile Gly Pro Gln Gly Pro Ser Gly Ala Arg
               180                 185                 190
   Gly Pro Pro Gly Leu Lys Gly Asp Arg Gly Asp Pro Gly Glu Arg Gly
   195 200 205
   Ala Lys Gly Glu Ser Gly Leu Ala Asp Val Asn Ala Leu Lys Gln Arg
   210 215 220
   Val Thr Ile Leu Glu Gly Gln Leu Gln Arg Leu Gln Asn Ala Phe Ser
   225 230 235 240
   Arg Tyr Lys Lys Ala Val Leu Phe Pro Asp Gly Gln Ala Val Gly Lys
   245 250 255
   Lys Ile Phe Lys Thr Ala Gly Ala Val Lys Ser Tyr Ser Asp Ala Gln
   260 265 270
   Gln Leu Cys Arg Glu Ala Lys Gly Gln Leu Ala Ser Pro Arg Ser Ala
   275 280 285
   Ala Glu Asn Glu Ala Val Ala Gln Leu Val Arg Ala Lys Asn Asn Asp
   290 295 300
   Ala Phe Leu Ser Met Asn Asp Ile Ser Thr Glu Gly Lys Phe Thr Tyr
   305 310 315 320
   Pro Thr Gly Glu Ser Leu Val Tyr Ser Asn Trp Ala Ser Gly Glu Pro
   325 330 335
   Asn Asn Asn Asn Ala Gly Gln Pro Glu Asn Cys Val Gln Ile Tyr Arg
   340 345 350
   Glu Gly Lys Trp Asn Asp Val Pro Cys Ser Glu Pro Leu Leu Val Ile
   355 360 365
   Cys Glu Phe
   370
<210> 46
   <211> 272
   <212> PRT
   <213> Mus musculus
<400> 46
   Met Met Met Arg Asp Leu Ala Leu Ala Gly Met Leu Ile Ser Leu Ala
   1 5 10 15
   Phe Leu Ser Leu Leu Pro Ser Gly Cys Pro Gln Gln Thr Thr Glu Asp
   20 25 30
   Ala Cys Ser Val Gln Ile Leu Val Pro Gly Leu Lys Gly Asp Ala Gly
   35 40 45
   Glu Lys Gly Asp Lys Gly Ala Pro Gly Arg Pro Gly Arg Val Gly Pro
   50 55 60
   Thr Gly Glu Lys Gly Asp Met Gly Asp Lys Gly Gln Lys Gly Thr Val
   65 70 75 80
   Gly Arg His Gly Lys Ile Gly Pro Ile Gly Ala Lys Gly Glu Lys Gly
   85 90 95
   Asp Ser Gly Asp Ile Gly Pro Pro Gly Pro Ser Gly Glu Pro Gly Ile
   100 105 110
   Pro Cys Glu Cys Ser Gln Leu Arg Lys Ala Ile Gly Glu Met Asp Asn
   115 120 125
   Gln Val Thr Gln Leu Thr Thr Glu Leu Lys Phe Ile Lys Asn Ala Val
   130 135 140
   Ala Gly Val Arg Glu Thr Glu Ser Lys Ile Tyr Leu Leu Val Lys Glu
   145 150 155 160
   Glu Lys Arg Tyr Ala Asp Ala Gln Leu Ser Cys Gln Ala Arg Gly Gly
   165 170 175
   Thr Leu Ser Met Pro Lys Asp Glu Ala Ala Asn Gly Leu Met Ala Ser
   180 185 190
   Tyr Leu Ala Gln Ala Gly Leu Ala Arg Val Phe Ile Gly Ile Asn Asp
   195 200 205
   Leu Glu Lys Glu Gly Ala Phe Val Tyr Ser Asp Arg Ser Pro Met Gln
   210 215 220
   Thr Phe Asn Lys Trp Arg Ser Gly Glu Pro Asn Asn Ala Tyr Asp Glu
   225 230 235 240
   Glu Asp Cys Val Glu Met Val Ala Ser Gly Gly Trp Asn Asp Val Ala
   245 250 255
   Cys His Ile Thr Met Tyr Phe Met Cys Glu Phe Asp Lys Glu Asn Leu
   260 265 270
<210> 47
   <211> 420
   <212> PRT
   <213> Mus musculus
<400> 47
   Met Asn Gly Phe Arg Val Leu Leu Arg Ser Asn Leu Ser Met Leu Leu
   1 5 10 15
   Leu Leu Ala Leu Leu His Phe Gln Ser Leu Gly Leu Asp Val Asp Ser
   20 25 30
   Arg Ser Ala Ala Glu Val Cys Ala Thr His Thr Ile Ser Pro Gly Pro
   35 40 45
   Lys Gly Asp Asp Gly Glu Arg Gly Asp Thr Gly Glu Glu Gly Lys Asp
   50 55 60
   Gly Lys Val Gly Arg Gln Gly Pro Lys Gly Val Lys Gly Glu Leu Gly
   65 70 75 80
   Asp Met Gly Ala Gln Gly Asn Ile Gly Lys Ser Gly Pro Ile Gly Lys
   85 90 95
   Lys Gly Asp Lys Gly Glu Lys Gly Leu Leu Gly Ile Pro Gly Glu Lys
   100 105 110
   Gly Lys Ala Gly Thr Ile Cys Asp Cys Gly Arg Tyr Arg Lys Val Val
   115 120 125
   Gly Gln Leu Asp Ile Ser Val Ala Arg Leu Lys Thr Ser Met Lys Phe
   130 135 140
   Ile Lys Asn Val Ile Ala Gly Ile Arg Glu Thr Glu Glu Lys Phe Tyr
   145 150 155 160
   Tyr Ile Val Gln Glu Glu Lys Asn Tyr Arg Glu Ser Leu Thr His Cys
   165 170 175
   Arg Ile Arg Gly Gly Met Leu Ala Met Pro Lys Asp Glu Val Val Asn
   180 185 190
   Thr Leu Ile Ala Asp Tyr Val Ala Lys Ser Gly Phe Phe Arg Val Phe
   195 200 205
   Ile Gly Val Asn Asp Leu Glu Arg Glu Gly Gln Tyr Val Phe Thr Asp
   210 215 220
   Asn Thr Pro Leu Gln Asn Tyr Ser Asn Trp Lys Glu Glu Glu Pro Ser
   225 230 235 240
   Asp Pro Ser Gly His Glu Asp Cys Val Glu Met Leu Ser Ser Gly Arg
   245 250 255
   Trp Asn Asp Thr Glu Cys His Leu Thr Met Tyr Phe Val Ser Ser Leu
   260 265 270
   Gln Glu Asp Leu Ile Glu Asp Cys Leu Arg Glu Gln Gly Leu Leu Val
   275 280 285
   Gln Val Thr Pro Ala Asn Gln Glu Leu Leu Phe Gly Ile Asp Thr Phe
   290 295 300
   Leu Gly Pro Met Ser Cys Val Tyr Gln Arg Thr Gly Thr Lys Gln Lys
   305 310 315 320
   Leu Tyr Ser Gln Cys Arg Leu Trp Asp Gly Leu Ala Lys Lys Gln Thr
   325 330 335
   Asn Glu Thr Ala Asn Ile Ala Thr Phe Cys Lys Gly Ala Glu Pro Asn
   340 345 350
   Arg Gly Ser Arg Pro Cys Gly Gln Lys Gln Glu Met Met Thr Leu Met
   355 360 365
   Met Ser Gly Asn Lys Gly Ile Thr Thr Phe Pro Glu Ser Asp Asn Leu
   370 375 380
   Phe Lys Trp Val Gly Thr Met Leu Gly Ala Ala Gly Thr Ile Asp Glu
   385 390 395 400
   Asp Leu Lys Tyr Lys Leu Ser Leu Asn Ser Pro Val Val Thr Leu Ile
   405 410 415
   Ile His Pro Gln
   420
<210> 48
   <211> 742
   <212> PRT
   <213> Mus musculus
<400> 48
   Met Lys Asp Asp Phe Ala Glu Glu Glu Glu Val Gln Ser Phe Gly Tyr
   1               5                   10                  15
   Lys Arg Phe Gly Ile Gln Glu Gly Thr Gln Cys Thr Lys Cys Lys Asn
               20                  25                  30
   Asn Trp Ala Leu Lys Phe Ser Ile Val Leu Leu Tyr Ile Leu Cys Ala
           35                  40                  45
   Leu Leu Thr Ile Thr Val Ala Ile Leu Gly Tyr Lys Val Val Glu Lys
       50                  55                  60
   Met Asp Asn Val Thr Asp Gly Met Glu Thr Ser His Gln Thr Tyr Asp
   65                  70                  75                  80
   Asn Lys Leu Thr Ala Val Glu Ser Asp Leu Lys Lys Leu Gly Asp Gln
                   85                  90                  95
   Ala Gly Lys Lys Ala Leu Ser Thr Asn Ser Glu Leu Ser Thr Phe Arg
               100                 105                 110
   Ser Asp Ile Leu Asp Leu Arg Gln Gln Leu Gln Glu Ile Thr Glu Lys
           115                 120                 125
   Thr Ser Lys Asn Lys Asp Thr Leu Glu Lys Leu Gln Ala Asn Gly Asp
       130                 135                 140
   Ser Leu Val Asp Arg Gln Ser Gln Leu Lys Glu Thr Leu Gln Asn Asn
   145                 150                 155                 160
   Ser Phe Leu Ile Thr Thr Val Asn Lys Thr Leu Gln Ala Tyr Asn Gly
                   165                 170                 175
   Tyr Val Thr Asn Leu Gln Gln Asp Thr Ser Val Leu Gln Gly Asn Leu
               180                 185                 190
   Gln Ser Gln Met Tyr Ser Gln Ser Val Val Ile Met Asn Leu Asn Asn
   195 200 205
   Leu Asn Leu Thr Gln Val Gln Gln Arg Asn Leu Ile Ser Asn Leu Gln
   210 215 220
   Gln Ser Val Asp Asp Thr Ser Leu Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Ser Ser Phe Thr Gly Gln Met Asp Asn Ile Thr Thr Ile Ser
   290 295 300
   Gln Ala Asn Glu Gln Ser Leu Lys Asp Leu Gln Asp Leu His Lys Asp
   305 310 315 320
   Thr Glu Asn Arg Thr Ala Val Lys Phe Ser Gln Leu Glu Glu Arg Phe
   325 330 335
   Gln Val Phe Glu Thr Asp Ile Val Asn Ile Ile Ser Asn Ile Ser Tyr
   340 345 350
   Thr Ala His His Leu Arg Thr Leu Thr Ser Asn Leu Asn Asp Val Arg
   355 360 365
   Thr Thr Cys Thr Asp Thr Leu Thr Arg His Thr Asp Asp Leu Thr Ser
   370 375 380
   Leu Asn Asn Thr Leu Val Asn Ile Arg Leu Asp Ser Ile Ser Leu Arg
   385 390 395 400
   Met Gln Gln Asp Met Met Arg Ser Lys Leu Asp Thr Glu Val Ala Asn
   405 410 415
   Leu Ser Val Val Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Lys Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Thr Ile Gly Pro Val Gly Pro Pro Gly Glu Arg
   485 490 495
   Gly Ser Lys Gly Ser Lys Gly Ser Gln Gly Pro Lys Gly Ser Arg Gly
   500 505 510
   Ser Pro Gly Lys Pro Gly Pro Gln Gly Pro Ser Gly Asp Pro Gly Pro
   515 520 525
   Pro Gly Pro Pro Gly Lys Asp Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Met Glu Pro Leu
   580 585 590
   Ala Leu Gln Asn Glu Pro Thr Pro Ala Ser Glu Val Asn Gly Cys Pro
   595 600 605
   Pro His Trp Lys Asn Phe Thr Asp Lys Cys Tyr Tyr Phe Ser Leu Glu
   610 615 620
   Lys Glu Ile Phe Glu Asp Ala Lys Leu Phe Cys Glu Asp Lys Ser Ser
   625 630 635 640
   His Leu Val Phe Ile Asn Ser Arg Glu Glu Gln Gln Trp Ile Lys Lys
   645 650 655
   His Thr Val Gly Arg Glu Ser His Trp Ile Gly Leu Thr Asp Ser Glu
   660 665 670
   Gln Glu Ser Glu Trp Lys Trp Leu Asp Gly Ser Pro Val Asp Tyr Lys
   675 680 685
   Asn Trp Lys Ala Gly Gln Pro Asp Asn Trp Gly Ser Gly His Gly Pro
   690 695 700
   Gly Glu Asp Cys Ala Gly Leu Ile Tyr Ala Gly Gln Trp Asn Asp Phe
   705 710 715 720
   Gln Cys Asp Glu Ile Asn Asn Phe Ile Cys Glu Lys Glu Arg Glu Ala
   725 730 735
   Val Pro Ser Ser Ile Leu
   740
<210> 49
   <211> 251
   <212> PRT
   <213> Danio rerio
<400> 49
   Met Ala Leu Leu Lys Leu Phe Leu Gly Ala Leu Leu Leu Leu Gln Leu
   1 5 10 15
   Val Leu Gln Leu Met Ala Gly Ala Ala Asp Pro Gln Ser Leu Asn Cys
   20 25 30
   Pro Ala Tyr Ala Gly Val Pro Gly Thr Pro Gly His Asn Gly Leu Pro
   35 40 45
   Gly Arg Asp Gly Arg Val Gly Arg Asp Gly Ala Asn Gly Pro Lys Gly
   50 55 60
   Glu Lys Gly Glu Pro Gly Val Asn Val Gln Gly Pro Pro Gly Lys Ala
   65 70 75 80
   Gly Pro Pro Gly Pro Ala Gly Ala Lys Gly Glu Arg Gly Pro Ser Gly
   85 90 95
   Leu Pro Gly Gln Asp Cys Met Ser Asp Ser Leu Lys Ser Glu Leu Gln
   100 105 110
   Lys Leu Ser Asp Lys Ile Ala Leu Ile Glu Lys Val Val Asn Phe Lys
   115 120 125
   Thr Phe Lys Lys Val Gly Gln Lys Tyr Tyr Val Thr Asp Asp Val Glu
   130 135 140
   Glu Thr Phe Asp Lys Gly Met Gln Tyr Cys Ser Ser Asn Gly Gly Ala
   145 150 155 160
   Leu Val Leu Pro Arg Thr Leu Glu Glu Asn Ala Leu Leu Lys Val Phe
   165 170 175
   Val Ser Ser Ala Phe Lys Arg Leu Phe Ile Arg Ile Thr Asp Arg Glu
   180 185 190
   Lys Glu Gly Glu Phe Val Asp Thr Asp Arg Lys Lys Leu Thr Phe Thr
   195 200 205
   Asn Trp Gly Pro Asn Gln Pro Asp Asn Tyr Lys Gly Ala Gln Asp Cys
   210 215 220
   Gly Ala Ile Ala Asp Ser Gly Leu Trp Asp Asp Val Ser Cys Asp Ser
   225 230 235 240
   Leu Tyr Pro Ile Ile Cys Glu Ile Glu Ile Lys
   245 250
<210> 50
   <211> 742
   <212> PRT
   <213> Homo sapiens
<400> 50
   Met Lys Asp Asp Phe Ala Glu Glu Glu Glu Val Gln Ser Phe Gly Tyr
   1 5 10 15
   Lys Arg Phe Gly Ile Gln Glu Gly Thr Gln Cys Thr Lys Cys Lys Asn
   20 25 30
   Asn Trp Ala Leu Lys Phe Ser Ile Ile Leu Leu Tyr Ile Leu Cys Ala
   35 40 45
   Leu Leu Thr Ile Thr Val Ala Ile Leu Gly Tyr Lys Val Val Glu Lys
   50 55 60
   Met Asp Asn Val Thr Gly Gly Met Glu Thr Ser Arg Gln Thr Tyr Asp
   65 70 75 80
   Asp Lys Leu Thr Ala Val Glu Ser Asp Leu Lys Lys Leu Gly Asp Gln
   85 90 95
   Thr Gly Lys Lys Ala Ile Ser Thr Asn Ser Glu Leu Ser Thr Phe Arg
   100 105 110
   Ser Asp Ile Leu Asp Leu Arg Gln Gln Leu Arg Glu Ile Thr Glu Lys
   115 120 125
   Thr Ser Lys Asn Lys Asp Thr Leu Glu Lys Leu Gln Ala Ser Gly Asp
   130 135 140
   Ala Leu Val Asp Arg Gln Ser Gln Leu Lys Glu Thr Leu Glu Asn Asn
   145 150 155 160
   Ser Phe Leu Ile Thr Thr Val Asn Lys Thr Leu Gln Ala Tyr Asn Gly
   165 170 175
   Tyr Val Thr Asn Leu Gln Gln Asp Thr Ser Val Leu Gln Gly Asn Leu
   180 185 190
   Gln Asn Gln Met Tyr Ser His Asn Val Val Ile Met Asn Leu Asn Asn
   195 200 205
   Leu Asn Leu Thr Gln Val Gln Gln Arg Asn Leu Ile Thr Asn Leu Gln
   210 215 220
   Arg Ser Val Asp Asp Thr Ser Gln Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Asn Ser Phe Thr Gly Gln Met Glu Asn Ile Thr Thr Ile Ser
   290 295 300
   Gln Ala Asn Glu Gln Asn Leu Lys Asp Leu Gln Asp Leu His Lys Asp
   305 310 315 320
   Ala Glu Asn Arg Thr Ala Ile Lys Phe Asn Gln Leu Glu Glu Arg Phe
   325 330 335
   Gln Leu Phe Glu Thr Asp Ile Val Asn Ile Ile Ser Asn Ile Ser Tyr
   340 345 350
   Thr Ala His His Leu Arg Thr Leu Thr Ser Asn Leu Asn Glu Val Arg
   355 360 365
   Thr Thr Cys Thr Asp Thr Leu Thr Lys His Thr Asp Asp Leu Thr Ser
   370 375 380
   Leu Asn Asn Thr Leu Ala Asn Ile Arg Leu Asp Ser Val Ser Leu Arg
   385 390 395 400
   Met Gln Gln Asp Leu Met Arg Ser Arg Leu Asp Thr Glu Val Ala Asn
   405 410 415
   Leu Ser Val Ile Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Arg Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Pro Ile Gly Pro Ala Gly Pro Pro Gly Glu Arg
   485 490 495
   Gly Gly Lys Gly Ser Lys Gly Ser Gln Gly Pro Lys Gly Ser Arg Gly
   500 505 510
   Ser Pro Gly Lys Pro Gly Pro Gln Gly Pro Ser Gly Asp Pro Gly Pro
   515 520 525
   Pro Gly Pro Pro Gly Lys Glu Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Val Val Pro Leu
   580 585 590
   Ala Leu Gln Asn Glu Pro Thr Pro Ala Pro Glu Asp Asn Gly Cys Pro
   595 600 605
   Pro His Trp Lys Asn Phe Thr Asp Lys Cys Tyr Tyr Phe Ser Val Glu
   610 615 620
   Lys Glu Ile Phe Glu Asp Ala Lys Leu Phe Cys Glu Asp Lys Ser Ser
   625 630 635 640
   His Leu Val Phe Ile Asn Thr Arg Glu Glu Gln Gln Trp Ile Lys Lys
   645 650 655
   Gln Met Val Gly Arg Glu Ser His Trp Ile Gly Leu Thr Asp Ser Glu
   660 665 670
   Arg Glu Asn Glu Trp Lys Trp Leu Asp Gly Thr Ser Pro Asp Tyr Lys
   675 680 685
   Asn Trp Lys Ala Gly Gln Pro Asp Asn Trp Gly His Gly His Gly Pro
   690 695 700
   Gly Glu Asp Cys Ala Gly Leu Ile Tyr Ala Gly Gln Trp Asn Asp Phe
   705 710 715 720
   Gln Cys Glu Asp Val Asn Asn Phe Ile Cys Glu Lys Asp Arg Glu Thr
   725 730 735
   Val Leu Ser Ser Ala Leu
   740
<210> 51
   <211> 622
   <212> PRT
   <213> Homo sapiens
<400> 51
   Met Lys Asp Asp Phe Ala Glu Glu Glu Glu Val Gln Ser Phe Gly Tyr
   1 5 10 15
   Lys Arg Phe Gly Ile Gln Glu Gly Thr Gln Cys Thr Lys Cys Lys Asn
   20 25 30
   Asn Trp Ala Leu Lys Phe Ser Ile Ile Leu Leu Tyr Ile Leu Cys Ala
           35                  40                  45
   Leu Leu Thr Ile Thr Val Ala Ile Leu Gly Tyr Lys Val Val Glu Lys
       50                  55                  60
   Met Asp Asn Val Thr Gly Gly Met Glu Thr Ser Arg Gln Thr Tyr Asp
   65                  70                  75                  80
   Asp Lys Leu Thr Ala Val Glu Ser Asp Leu Lys Lys Leu Gly Asp Gln
                   85                  90                  95
   Thr Gly Lys Lys Ala Ile Ser Thr Asn Ser Glu Leu Ser Thr Phe Arg
               100                 105                 110
   Ser Asp Ile Leu Asp Leu Arg Gln Gln Leu Arg Glu Ile Thr Glu Lys
           115                 120                 125
   Thr Ser Lys Asn Lys Asp Thr Leu Glu Lys Leu Gln Ala Ser Gly Asp
       130                 135                 140
   Ala Leu Val Asp Arg Gln Ser Gln Leu Lys Glu Thr Leu Glu Asn Asn
   145                 150                 155                 160
   Ser Phe Leu Ile Thr Thr Val Asn Lys Thr Leu Gln Ala Tyr Asn Gly
                   165                 170                 175
   Tyr Val Thr Asn Leu Gln Gln Asp Thr Ser Val Leu Gln Gly Asn Leu
               180                 185                 190
   Gln Asn Gln Met Tyr Ser His Asn Val Val Ile Met Asn Leu Asn Asn
           195                 200                 205
   Leu Asn Leu Thr Gln Val Gln Gln Arg Asn Leu Ile Thr Asn Leu Gln
       210                 215                 220
   Arg Ser Val Asp Asp Thr Ser Gln Ala Ile Gln Arg Ile Lys Asn Asp
   225                 230                 235                 240
   Phe Gln Asn Leu Gln Gln Val Phe Leu Gln Ala Lys Lys Asp Thr Asp
                   245                 250                 255
   Trp Leu Lys Glu Lys Val Gln Ser Leu Gln Thr Leu Ala Ala Asn Asn
               260                 265                 270
   Ser Ala Leu Ala Lys Ala Asn Asn Asp Thr Leu Glu Asp Met Asn Ser
   275 280 285
   Gln Leu Asn Ser Phe Thr Gly Gln Met Glu Asn Ile Thr Thr Ile Ser
   290 295 300
   Gln Ala Asn Glu Gln Asn Leu Lys Asp Leu Gln Asp Leu His Lys Asp
   305 310 315 320
   Ala Glu Asn Arg Thr Ala Ile Lys Phe Asn Gln Leu Glu Glu Arg Phe
   325 330 335
   Gln Leu Phe Glu Thr Asp Ile Val Asn Ile Ile Ser Asn Ile Ser Tyr
   340 345 350
   Thr Ala His His Leu Arg Thr Leu Thr Ser Asn Leu Asn Glu Val Arg
   355 360 365
   Thr Thr Cys Thr Asp Thr Leu Thr Lys His Thr Asp Asp Leu Thr Ser
   370 375 380
   Leu Asn Asn Thr Leu Ala Asn Ile Arg Leu Asp Ser Val Ser Leu Arg
   385 390 395 400
   Met Gln Gln Asp Leu Met Arg Ser Arg Leu Asp Thr Glu Val Ala Asn
   405 410 415
   Leu Ser Val Ile Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Arg Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Pro Ile Gly Pro Ala Gly Pro Pro Gly Glu Arg
   485 490 495
   Gly Gly Lys Gly Ser Lys Gly Ser Gln Gly Pro Lys Gly Ser Arg Gly
   500 505 510
   Ser Pro Gly Lys Pro Gly Pro Gln Gly Pro Ser Gly Asp Pro Gly Pro
   515 520 525
   Pro Gly Pro Pro Gly Lys Glu Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Val Val Pro Leu
   580 585 590
   Ala Leu Gln Asn Glu Pro Thr Pro Ala Pro Glu Asp Asn Ser Lys Ser
   595 600 605
   Lys Pro Ser Leu Gln Pro Gly Gly Gln Gly Ser Ala Cys Ala
   610 615 620
<210> 52
   <211> 742
   <212> PRT
   <213> Mus musculus
<400> 52
   Met Lys Asp Asp Phe Ala Glu Glu Glu Glu Val Gln Ser Phe Gly Tyr
   1 5 10 15
   Lys Arg Phe Gly Ile His Glu Gly Thr Gln Cys Thr Lys Cys Ile Asn
   20 25 30
   Asn Trp Ala Leu Lys Phe Ser Ile Val Leu Leu Tyr Ile Leu Cys Ala
   35 40 45
   Leu Leu Thr Ile Thr Val Ala Ile Leu Gly Tyr Lys Val Val Glu Lys
   50 55 60
   Met Asp Asn Val Ser Asp Gly Met Glu Thr Ser His Gln Thr Tyr Asp
   65 70 75 80
   Asn Lys Leu Thr Ala Val Glu Ser Asp Leu Lys Lys Leu Gly Asp Gln
   85 90 95
   Ala Gly Lys Lys Ala Leu Ser Thr Asn Ser Glu Leu Ser Thr Phe Arg
   100 105 110
   Ser Asp Ile Leu Asp Leu Arg Gln Gln Leu Gln Glu Ile Thr Glu Lys
   115 120 125
   Thr Ser Lys Asn Lys Asp Thr Leu Glu Lys Leu Gln Ala Asn Gly Asp
   130 135 140
   Ser Leu Val Asp Arg Gln Ser Gln Leu Lys Glu Thr Leu Gln Asn Asn
   145 150 155 160
   Ser Phe Leu Ile Thr Thr Val Asn Lys Thr Leu Gln Ala Tyr Asn Gly
   165 170 175
   Tyr Val Thr Asn Leu Gln Gln Asp Thr Asn Val Leu Gln Gly Asn Leu
   180 185 190
   Gln Ser Gln Met Tyr Ser Gln Ser Val Val Ile Met Asn Leu Asn Asn
   195 200 205
   Leu Asn Leu Thr Gln Val Gln Gln Arg Asn Leu Ile Ser Asn Leu Gln
   210 215 220
   Gln Ser Val Asp Asp Thr Ser Leu Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Ser Ser Phe Thr Gly Gln Met Asp Asn Ile Thr Thr Ile Ser
   290 295 300
   Gln Ala Asn Glu Gln Ser Leu Lys Asp Leu Gln Asp Leu His Lys Asp
   305 310 315 320
   Thr Glu Asn Arg Thr Ala Val Lys Phe Ser Gln Leu Glu Glu Arg Phe
   325 330 335
   Gln Val Phe Glu Thr Asp Ile Val Asn Ile Ile Ser Asn Ile Ser Tyr
   340 345 350
   Thr Ala His His Leu Arg Thr Leu Thr Ser Asn Leu Asn Asp Val Trp
   355 360 365
   Thr Thr Cys Thr Asp Thr Leu Thr Arg His Thr Asp Asp Leu Thr Ser
   370 375 380
   Leu Asn Asn Thr Leu Val Asn Ile Arg Leu Asp Ser Ile Ser Leu Arg
   385 390 395 400
   Met Gln Gln Asp Met Met Arg Ser Lys Leu Asp Thr Glu Val Ala Asn
   405 410 415
   Leu Ser Val Val Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Lys Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Thr Ile Gly Pro Val Gly Pro Pro Gly Glu Arg
   485 490 495
   Gly Ser Lys Gly Ser Lys Gly Ser Gln Gly Pro Lys Gly Ser Arg Gly
   500 505 510
   Ser Pro Gly Lys Pro Gly Pro Gln Gly Pro Ser Gly Asp Pro Gly Pro
   515 520 525
   Pro Gly Pro Pro Gly Lys Asp Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Met Glu Pro Leu
   580 585 590
   Ala Leu Gln Asn Glu Pro Thr Pro Ala Ser Glu Val Asn Gly Cys Pro
   595 600 605
   Pro His Trp Lys Asn Phe Thr Asp Lys Cys Tyr Tyr Phe Ser Leu Glu
   610 615 620
   Lys Glu Ile Leu Glu Asp Ala Lys Leu Phe Cys Glu Asp Lys Ser Ser
   625 630 635 640
   His Leu Val Phe Ile Asn Ser Arg Glu Glu Gln Gln Trp Ile Lys Lys
   645 650 655
   His Thr Val Gly Arg Glu Ser His Trp Ile Gly Leu Thr Asp Ser Glu
   660 665 670
   Gln Glu Ser Glu Trp Lys Trp Leu Asp Gly Ser Pro Val Asp Tyr Lys
   675 680 685
   Asn Trp Lys Ala Gly Gln Pro Asp Asn Trp Gly Ser Gly His Gly Pro
   690 695 700
   Gly Glu Asp Cys Ala Gly Leu Ile Tyr Ala Gly Gln Trp Asn Asp Phe
   705 710 715 720
   Gln Cys Asp Glu Ile Asn Asn Phe Ile Cys Glu Lys Glu Arg Glu Ala
   725 730 735
   Val Pro Ser Ser Ile Leu
   740
<210> 53
   <211> 321
   <212> PRT
   <213> Bos taurus
<400> 53
   Met Leu Pro Leu Pro Leu Ser Ile Leu Leu Leu Leu Thr Gln Ser Gln
   1 5 10 15
   Ser Phe Leu Gly Glu Glu Met Asp Val Tyr Ser Glu Lys Thr Leu Thr
   20 25 30
   Asp Pro Cys Thr Leu Val Val Cys Ala Pro Pro Ala Asp Ser Leu Arg
   35 40 45
   Gly His Asp Gly Arg Asp Gly Lys Glu Gly Pro Gln Gly Glu Lys Gly
   50 55 60
   Asp Pro Gly Pro Pro Gly Met Pro Gly Pro Ala Gly Arg Glu Gly Pro
   65 70 75 80
   Ser Gly Arg Gln Gly Ser Met Gly Pro Pro Gly Thr Pro Gly Pro Lys
   85 90 95
   Gly Glu Pro Gly Pro Glu Gly Gly Val Gly Ala Pro Gly Met Pro Gly
   100 105 110
   Ser Pro Gly Pro Ala Gly Leu Lys Gly Glu Arg Gly Thr Pro Gly Pro
   115 120 125
   Gly Gly Ala Ile Gly Pro Gln Gly Pro Ser Gly Ala Met Gly Pro Pro
   130 135 140
   Gly Leu Lys Gly Asp Arg Gly Asp Pro Gly Glu Lys Gly Ala Arg Gly
   145 150 155 160
   Glu Thr Ser Val Leu Glu Val Asp Thr Leu Arg Gln Arg Met Arg Asn
   165 170 175
   Leu Glu Gly Glu Val Gln Arg Leu Gln Asn Ile Val Thr Gln Tyr Arg
   180 185 190
   Lys Ala Val Leu Phe Pro Asp Gly Gln Ala Val Gly Glu Lys Ile Phe
   195 200 205
   Lys Thr Ala Gly Ala Val Lys Ser Tyr Ser Asp Ala Glu Gln Leu Cys
   210 215 220
   Arg Glu Ala Lys Gly Gln Leu Ala Ser Pro Arg Ser Ser Ala Glu Asn
   225 230 235 240
   Glu Ala Val Thr Gln Leu Val Arg Ala Lys Asn Lys His Ala Tyr Leu
   245 250 255
   Ser Met Asn Asp Ile Ser Lys Glu Gly Lys Phe Thr Tyr Pro Thr Gly
   260 265 270
   Gly Ser Leu Asp Tyr Ser Asn Trp Ala Pro Gly Glu Pro Asn Asn Arg
   275 280 285
   Ala Lys Asp Glu Gly Pro Glu Asn Cys Leu Glu Ile Tyr Ser Asp Gly
   290 295 300
   Asn Trp Asn Asp Ile Glu Cys Arg Glu Glu Arg Leu Val Ile Cys Glu
   305 310 315 320
   Phe
<210> 54
   <211> 239
   <212> PRT
   <213> Mus musculus
<400> 54
   Met Leu Leu Leu Pro Leu Leu Pro Val Leu Leu Cys Val Val Ser Val
   1 5 10 15
   Ser Ser Ser Gly Ser Gln Thr Cys Glu Asp Thr Leu Lys Thr Cys Ser
   20 25 30
   Val Ile Ala Cys Gly Arg Asp Gly Arg Asp Gly Pro Lys Gly Glu Lys
   35 40 45
   Gly Glu Pro Gly Gln Gly Leu Arg Gly Leu Gln Gly Pro Pro Gly Lys
   50 55 60
   Leu Gly Pro Pro Gly Ser Val Gly Ser Pro Gly Ser Pro Gly Pro Lys
   65 70 75 80
   Gly Gln Lys Gly Asp His Gly Asp Asn Arg Ala Ile Glu Glu Lys Leu
   85 90 95
   Ala Asn Met Glu Ala Glu Ile Arg Ile Leu Lys Ser Lys Leu Gln Leu
   100 105 110
   Thr Asn Lys Leu His Ala Phe Ser Met Gly Lys Lys Ser Gly Lys Lys
   115 120 125
   Leu Phe Val Thr Asn His Glu Lys Met Pro Phe Ser Lys Val Lys Ser
   130 135 140
   Leu Cys Thr Glu Leu Gln Gly Thr Val Ala Ile Pro Arg Asn Ala Glu
   145 150 155 160
   Glu Asn Lys Ala Ile Gln Glu Val Ala Thr Gly Ile Ala Phe Leu Gly
                   165                 170                 175
   Ile Thr Asp Glu Ala Thr Glu Gly Gln Phe Met Tyr Val Thr Gly Gly
               180                 185                 190
   Arg Leu Thr Tyr Ser Asn Trp Lys Lys Asp Glu Pro Asn Asn His Gly
           195                 200                 205
   Ser Gly Glu Asp Cys Val Ile Ile Leu Asp Asn Gly Leu Trp Asn Asp
       210                 215                 220
   Ile Ser Cys Gln Ala Ser Phe Lys Ala Val Cys Glu Phe Pro Ala
   225                 230                 235
<210> 55
   <211> 244
   <212> PRT
   <213> Mus musculus
<400> 55
   Met Ser Ile Phe Thr Ser Phe Leu Leu Leu Cys Val Val Thr Val Val
   1               5                   10                  15
   Tyr Ala Glu Thr Leu Thr Glu Gly Val Gln Asn Ser Cys Pro Val Val
               20                  25                  30
   Thr Cys Ser Ser Pro Gly Leu Asn Gly Phe Pro Gly Lys Asp Gly Arg
           35                  40                  45
   Asp Gly Ala Lys Gly Glu Lys Gly Glu Pro Gly Gln Gly Leu Arg Gly
       50                  55                  60
   Leu Gln Gly Pro Pro Gly Lys Val Gly Pro Thr Gly Pro Pro Gly Asn
   65                  70                  75                  80
   Pro Gly Leu Lys Gly Ala Val Gly Pro Lys Gly Asp Arg Gly Asp Arg
                   85                  90                  95
   Ala Glu Phe Asp Thr Ser Glu Ile Asp Ser Glu Ile Ala Ala Leu Arg
               100                 105                 110
   Ser Glu Leu Arg Ala Leu Arg Asn Trp Val Leu Phe Ser Leu Ser Glu
   115 120 125
   Lys Val Gly Lys Lys Tyr Phe Val Ser Ser Val Lys Lys Met Ser Leu
   130 135 140
   Asp Arg Val Lys Ala Leu Cys Ser Glu Phe Gln Gly Ser Val Ala Thr
   145 150 155 160
   Pro Arg Asn Ala Glu Glu Asn Ser Ala Ile Gln Lys Val Ala Lys Asp
   165 170 175
   Ile Ala Tyr Leu Gly Ile Thr Asp Val Arg Val Glu Gly Ser Phe Glu
   180 185 190
   Asp Leu Thr Gly Asn Arg Val Arg Tyr Thr Asn Trp Asn Asp Gly Glu
   195 200 205
   Pro Asn Asn Thr Gly Asp Gly Glu Asp Cys Val Val Ile Leu Gly Asn
   210 215 220
   Gly Lys Trp Asn Asp Val Pro Cys Ser Asp Ser Phe Leu Ala Ile Cys
   225 230 235 240
   Glu Phe Ser Asp
<210> 56
   <211> 277
   <212> PRT
   <213> Homo sapiens
<400> 56
   Met Asn Gly Phe Ala Ser Leu Leu Arg Arg Asn Gln Phe Ile Leu Leu
   1 5 10 15
   Val Leu Phe Leu Leu Gln Ile Gln Ser Leu Gly Leu Asp Ile Asp Ser
   20 25 30
   Arg Pro Thr Ala Glu Val Cys Ala Thr His Thr Ile Ser Pro Gly Pro
   35 40 45
   Lys Gly Asp Asp Gly Glu Lys Gly Asp Pro Gly Glu Glu Gly Lys His
   50 55 60
   Gly Lys Val Gly Arg Met Gly Pro Lys Gly Ile Lys Gly Glu Leu Gly
   65 70 75 80
   Asp Met Gly Asp Arg Gly Asn Ile Gly Lys Thr Gly Pro Ile Gly Lys
   85 90 95
   Lys Gly Asp Lys Gly Glu Lys Gly Leu Leu Gly Ile Pro Gly Glu Lys
   100 105 110
   Gly Lys Ala Gly Thr Val Cys Asp Cys Gly Arg Tyr Arg Lys Phe Val
   115 120 125
   Gly Gln Leu Asp Ile Ser Ile Ala Arg Leu Lys Thr Ser Met Lys Phe
   130 135 140
   Val Lys Asn Val Ile Ala Gly Ile Arg Glu Thr Glu Glu Lys Phe Tyr
   145 150 155 160
   Tyr Ile Val Gln Glu Glu Lys Asn Tyr Arg Glu Ser Leu Thr His Cys
   165 170 175
   Arg Ile Arg Gly Gly Met Leu Ala Met Pro Lys Asp Glu Ala Ala Asn
   180 185 190
   Thr Leu Ile Ala Asp Tyr Val Ala Lys Ser Gly Phe Phe Arg Val Phe
   195 200 205
   Ile Gly Val Asn Asp Leu Glu Arg Glu Gly Gln Tyr Met Phe Thr Asp
   210 215 220
   Asn Thr Pro Leu Gln Asn Tyr Ser Asn Trp Asn Glu Gly Glu Pro Ser
   225 230 235 240
   Asp Pro Tyr Gly His Glu Asp Cys Val Glu Met Leu Ser Ser Gly Arg
   245 250 255
   Trp Asn Asp Thr Glu Cys His Leu Thr Met Tyr Phe Val Cys Glu Phe
   260 265 270
   Ile Lys Lys Lys Lys
   275
<210> 57
   <211> 742
   <212> PRT
   <213> Homo sapiens
<400> 57
   Met Lys Asp Asp Phe Ala Glu Glu Glu Glu Val Gln Ser Phe Gly Tyr
   1               5                   10                  15
   Lys Arg Phe Gly Ile Gln Glu Gly Thr Gln Cys Thr Lys Cys Lys Asn
               20                  25                  30
   Asn Trp Ala Leu Lys Phe Ser Ile Ile Leu Leu Tyr Ile Leu Cys Ala
           35                  40                  45
   Leu Leu Thr Ile Thr Val Ala Ile Leu Gly Tyr Lys Val Val Glu Lys
       50                  55                  60
   Met Asp Asn Val Thr Gly Gly Met Glu Thr Ser Arg Gln Thr Tyr Asp
   65                  70                  75                  80
   Asp Lys Leu Thr Ala Val Glu Ser Asp Leu Lys Lys Leu Gly Asp Gln
                   85                  90                  95
   Thr Gly Lys Lys Ala Ile Ser Thr Asn Ser Glu Leu Ser Thr Phe Arg
               100                 105                 110
   Ser Asp Ile Leu Asp Leu Arg Gln Gln Leu Arg Glu Ile Thr Glu Lys
           115                 120                 125
   Thr Ser Lys Asn Lys Asp Thr Leu Glu Lys Leu Gln Ala Ser Gly Asp
       130                 135                 140
   Ala Leu Val Asp Arg Gln Ser Gln Leu Lys Glu Thr Leu Glu Asn Asn
   145                 150                 155                 160
   Ser Phe Leu Ile Thr Thr Val Asn Lys Thr Leu Gln Ala Tyr Asn Gly
                   165                 170                 175
   Tyr Val Thr Asn Leu Gln Gln Asp Thr Ser Val Leu Gln Gly Asn Leu
               180                 185                 190
   Gln Asn Gln Met Tyr Ser His Asn Val Val Ile Met Asn Leu Asn Asn
           195                 200                 205
   Leu Asn Leu Thr Gln Val Gln Gln Arg Asn Leu Ile Thr Asn Leu Gln
       210                 215                 220
   Arg Ser Val Asp Asp Thr Ser Gln Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Asn Ser Phe Thr Gly Gln Met Glu Asn Ile Thr Thr Ile Ser
   290 295 300
   Gln Ala Asn Glu Gln Asn Leu Lys Asp Leu Gln Asp Leu His Lys Asp
   305 310 315 320
   Ala Glu Asn Arg Thr Ala Ile Lys Phe Asn Gln Leu Glu Glu Arg Phe
   325 330 335
   Gln Leu Phe Glu Thr Asp Ile Val Asn Ile Ile Ser Asn Ile Ser Tyr
   340 345 350
   Thr Ala His His Leu Arg Thr Leu Thr Ser Asn Leu Asn Glu Val Arg
   355 360 365
   Thr Thr Cys Thr Asp Thr Leu Thr Lys His Thr Asp Asp Leu Thr Ser
   370 375 380
   Leu Asn Asn Thr Leu Ala Asn Ile Arg Leu Asp Ser Val Ser Leu Arg
   385 390 395 400
   Met Gln Gln Asp Leu Met Arg Ser Arg Leu Asp Thr Glu Val Ala Asn
   405 410 415
   Leu Ser Val Ile Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Arg Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465                 470                 475                 480
   Ala Gly Glu Arg Gly Pro Ile Gly Pro Ala Gly Pro Pro Gly Glu Arg
                   485                 490                 495
   Gly Gly Lys Gly Ser Lys Gly Ser Gln Gly Pro Lys Gly Ser Arg Gly
               500                 505                 510
   Ser Pro Gly Lys Pro Gly Pro Gln Gly Pro Ser Gly Asp Pro Gly Pro
           515                 520                 525
   Pro Gly Pro Pro Gly Lys Glu Gly Leu Pro Gly Pro Gln Gly Pro Pro
       530                 535                 540
   Gly Phe Gln Gly Leu Gln Gly Thr Val Gly Glu Pro Gly Val Pro Gly
   545                 550                 555                 560
   Pro Arg Gly Leu Pro Gly Leu Pro Gly Val Pro Gly Met Pro Gly Pro
                   565                 570                 575
   Lys Gly Pro Pro Gly Pro Pro Gly Pro Ser Gly Ala Val Val Pro Leu
               580                 585                 590
   Ala Leu Gln Asn Glu Pro Thr Pro Ala Pro Glu Asp Asn Gly Cys Pro
           595                 600                 605
   Pro His Trp Lys Asn Phe Thr Asp Lys Cys Tyr Tyr Phe Ser Val Glu
       610                 615                 620
   Lys Glu Ile Phe Glu Asp Ala Lys Leu Phe Cys Glu Asp Lys Ser Ser
   625                 630                 635                 640
   His Leu Val Phe Ile Asn Thr Arg Glu Glu Gln Gln Trp Ile Lys Lys
                   645                 650                 655
   Gln Met Val Gly Arg Glu Ser His Trp Ile Gly Leu Thr Asp Ser Glu
               660                 665                 670
   Arg Glu Asn Glu Trp Lys Trp Leu Asp Gly Thr Ser Pro Asp Tyr Lys
           675                 680                 685
   Asn Trp Lys Ala Gly Gln Pro Asp Asn Trp Gly His Gly His Gly Pro
       690                 695                 700
   Gly Glu Asp Cys Ala Gly Leu Ile Tyr Ala Gly Gln Trp Asn Asp Phe
   705                 710                 715                 720
   Gln Cys Glu Asp Val Asn Asn Phe Ile Cys Glu Lys Asp Arg Glu Thr
   725 730 735
   Val Leu Ser Ser Ala Leu
   740
<210> 58
   <211> 246
   <212> PRT
   <213> Carassius auratus
<400> 58
   Leu Leu Leu Leu Gln Phe Ala Leu Gln Leu Leu Asp Gly Ala Glu Pro
   1 5 10 15
   Gln Asn Leu Asn Cys Pro Ala Tyr Gly Gly Val Pro Gly Thr Pro Gly
   20 25 30
   His Asn Gly Leu Pro Gly Arg Asp Gly Arg Asp Gly Lys Asp Gly Ala
   35 40 45
   Ile Gly Pro Lys Gly Glu Lys Gly Glu Ser Gly Val Ser Val Gln Gly
   50 55 60
   Pro Pro Gly Lys Ala Gly Pro Pro Gly Thr Ala Gly Glu Lys Gly Glu
   65 70 75 80
   Arg Gly Pro Ser Gly Pro Gln Gly Ser Pro Gly Ser Glu Ser Val Leu
   85 90 95
   Glu Ser Leu Lys Ser Glu Ile Gln Gln Leu Lys Ala Lys Ile Ala Thr
   100 105 110
   Phe Glu Lys Val Ser Ser Val Cys His Phe Arg Lys Val Gly Gln Lys
   115 120 125
   Tyr Tyr Ile Thr Asp Gly Val Val Gly Asn Phe Asp Gln Gly Leu Lys
   130 135 140
   Ser Cys Met Glu Phe Gly Gly Thr Met Val Ser Pro Arg Thr Ser Ala
   145 150 155 160
   Glu Asn Gln Ala Leu Leu Lys Leu Val Val Ser Ser Gly Leu Gly Ser
   165 170 175
   Lys Lys Pro Tyr Ile Gly Val Thr Asp Arg Lys Thr Glu Gly Gln Phe
   180 185 190
   Val Asp Thr Glu Gly Lys Gln Leu Thr Phe Thr Asn Trp Gly Pro Gly
   195 200 205
   Gln Pro Asp Asp Tyr Lys Gly Leu Gln Asp Cys Gly Val Ile Glu Asp
   210 215 220
   Thr Gly Leu Trp Asp Asp Gly Gly Cys Gly Asp Ile Arg Pro Ile Met
   225 230 235 240
   Cys Glu Ile Asp Ile Lys
   245
<210> 59
   <211> 251
   <212> PRT
   <213> Danio rerio
<400> 59
   Met Ala Leu Leu Lys Leu Phe Leu Gly Ala Leu Leu Leu Leu Gln Leu
   1 5 10 15
   Val Leu Gln Leu Met Ala Gly Ala Ala Asp Pro Gln Ser Leu Asn Cys
   20 25 30
   Pro Ala Tyr Ala Gly Val Pro Gly Thr Pro Gly His Asn Gly Leu Pro
   35 40 45
   Gly Arg Asp Gly Arg Val Gly Arg Asp Gly Ala Asn Gly Pro Lys Gly
   50 55 60
   Glu Lys Gly Glu Pro Gly Val Asn Val Gln Gly Pro Pro Gly Lys Ala
   65 70 75 80
   Gly Pro Pro Gly Pro Ala Gly Ala Lys Gly Glu Arg Gly Pro Ser Gly
   85 90 95
   Leu Pro Gly Gln Asp Cys Met Ser Asp Ser Leu Lys Ser Glu Leu Gln
   100 105 110
   Lys Leu Ser Asp Lys Ile Ala Leu Ile Glu Lys Val Val Asn Phe Lys
   115 120 125
   Thr Phe Lys Lys Val Gly Gln Lys Tyr Tyr Val Thr Asp Asp Val Glu
   130 135 140
   Glu Thr Phe Asp Lys Gly Met Gln Tyr Cys Ser Ser Asn Gly Gly Ala
   145 150 155 160
   Leu Val Leu Pro Arg Thr Leu Glu Glu Asn Ala Leu Leu Lys Val Phe
   165 170 175
   Val Ser Ser Ala Phe Lys Arg Leu Phe Ile Arg Ile Thr Asp Arg Glu
   180 185 190
   Lys Glu Gly Glu Phe Val Asp Thr Asp Arg Lys Lys Leu Thr Phe Thr
   195 200 205
   Asn Trp Gly Pro Asn Gln Pro Asp Asn Tyr Lys Gly Ala Gln Asp Cys
   210 215 220
   Gly Ala Ile Ala Asp Ser Gly Leu Trp Asp Asp Val Ser Cys Asp Ser
   225 230 235 240
   Leu Tyr Pro Ile Ile Cys Glu Ile Glu Ile Lys
   245 250
<210> 60
   <211> 256
   <212> PRT
   <213> Cyprinus carpio
<400> 60
   Met Ala Leu Phe Lys Leu Phe Leu Gly Thr Leu Leu Leu Leu Gln Phe
   1 5 10 15
   Ala Leu Gln Leu Leu Asp Gly Ala Glu Pro Gln Asn Leu Asn Cys Pro
   20 25 30
   Ala Tyr Gly Gly Val Pro Gly Thr Pro Gly His Asn Gly Leu Pro Gly
   35 40 45
   Arg Asp Gly Arg Asp Gly Lys Asp Gly Ala Ile Gly Pro Lys Gly Glu
   50 55 60
   Lys Gly Glu Ser Gly Val Ser Val Gln Gly Pro Pro Gly Lys Ala Gly
   65 70 75 80
   Pro Pro Gly Pro Ala Gly Glu Lys Gly Glu Arg Gly Pro Thr Gly Ser
   85 90 95
   Gln Gly Ser Pro Gly Ser Glu Ser Val Leu Glu Ser Leu Lys Ser Glu
   100 105 110
   Ile Gln Gln Leu Lys Ala Lys Ile Ala Thr Phe Glu Lys Val Ala Ser
   115 120 125
   Val Gly His Phe Arg Gln Val Gly Gln Lys Tyr Tyr Ile Thr Asp Gly
   130 135 140
   Val Val Gly Thr Phe Asp Gln Gly Leu Lys Phe Cys Lys Asp Phe Gly
   145 150 155 160
   Gly Thr Met Val Phe Pro Arg Thr Ser Ala Glu Asn Gln Ala Leu Leu
   165 170 175
   Lys Leu Val Val Ser Ser Gly Leu Ser Ser Lys Lys Pro Tyr Ile Gly
   180 185 190
   Val Thr Asp Arg Glu Thr Glu Gly Arg Phe Val Asn Thr Glu Gly Lys
   195 200 205
   Gln Leu Thr Phe Thr Asn Trp Gly Pro Gly Gln Pro Asp Asp Tyr Lys
   210 215 220
   Gly Leu Gln Asp Cys Gly Val Ile Glu Asp Ser Gly Leu Trp Asp Asp
   225 230 235 240
   Gly Ser Cys Gly Asp Ile Arg Pro Ile Met Cys Glu Ile Asp Asn Lys
   245 250 255
<210> 61
   <211> 222
   <212> PRT
   <213> Gallus gallus
<400> 61
   Met Leu Ser Tyr Ser Phe Cys Met Ile Ala Ala Ala Val Ala Leu Leu
   1               5                   10                  15
   Thr Pro Cys His Ala Gln Asn Cys Ala Gly Ala Pro Glu Leu Pro Ser
               20                  25                  30
   Ile Pro Gly Val Ser Gly Leu Leu Gly Leu Gly Ala Leu Lys Arg Tyr
           35                  40                  45
   Phe Gly Ser Leu Leu Trp Pro Tyr Gly Glu Glu Lys Leu Pro Glu Cys
       50                  55                  60
   Gln Trp Leu Gln Arg Gln Gln Asp Leu Ser Thr Ser Ser Asp Asp Glu
   65                  70                  75                  80
   Leu Gly Asn Val Leu Leu Asn Leu Arg Gln Arg Ile Leu Gln Leu Glu
                   85                  90                  95
   Gly Val Leu Ala Leu Asp Gly Lys Ile Thr Lys Val Gly Glu Lys Ile
               100                 105                 110
   Phe Ala Ser Asn Gly Lys Glu Val Asn Phe Ser Ser Ala Leu Glu Ser
           115                 120                 125
   Cys Glu Glu Thr Gly Gly Thr Leu Ala Thr Pro Met Asn Glu Glu Glu
       130                 135                 140
   Asn Lys Ala Ile Met Gly Ile Val Lys Gln Tyr Asn Arg Tyr Ala Tyr
   145                 150                 155                 160
   Leu Gly Ile Lys Glu Ser Asp Thr Ala Gly Gln Phe Lys Tyr Val Asn
                   165                 170                 175
   Asn Gln Pro Leu Asn Tyr Thr Ser Trp Gln Gln Tyr Glu Pro Asn Gly
               180                 185                 190
   Lys Gly Thr Glu Lys Cys Val Glu Met Tyr Thr Asp Gly Asn Trp Lys
           195                 200                 205
   Asp Arg Lys Cys Asn Leu Tyr Arg Leu Thr Val Cys Glu Tyr
       210                 215                 220
<210> 62
   <211> 371
   <212> PRT
   <213> Bos taurus
<400> 62
   Met Leu Leu Leu Pro Leu Ser Val Leu Leu Leu Leu Thr Gln Pro Trp
   1               5                   10                  15
   Arg Ser Leu Gly Ala Glu Met Thr Thr Phe Ser Gln Lys Ile Leu Ala
               20                  25                  30
   Asn Ala Cys Thr Leu Val Met Cys Ser Pro Leu Glu Ser Gly Leu Pro
           35                  40                  45
   Gly His Asp Gly Gln Asp Gly Arg Glu Cys Pro His Gly Glu Lys Gly
       50                  55                  60
   Asp Pro Gly Ser Pro Gly Pro Ala Gly Arg Ala Gly Arg Pro Gly Trp
   65                  70                  75                  80
   Val Gly Pro Ile Gly Pro Lys Gly Asp Asn Gly Phe Val Gly Glu Pro
                   85                  90                  95
   Gly Pro Lys Gly Asp Thr Gly Pro Arg Gly Pro Pro Gly Met Pro Gly
               100                 105                 110
   Pro Ala Gly Arg Glu Gly Pro Ser Gly Lys Gln Gly Ser Met Gly Pro
           115                 120                 125
   Pro Gly Thr Pro Gly Pro Lys Gly Glu Thr Gly Pro Lys Gly Gly Val
       130                 135                 140
   Gly Ala Pro Gly Ile Gln Gly Phe Pro Gly Pro Ser Gly Leu Lys Gly
   145                 150                 155                 160
   Glu Lys Gly Ala Pro Gly Glu Thr Gly Ala Pro Gly Arg Ala Gly Val
                   165                 170                 175
   Thr Gly Pro Ser Gly Ala Ile Gly Pro Gln Gly Pro Ser Gly Ala Arg
               180                 185                 190
   Gly Pro Pro Gly Leu Lys Gly Asp Arg Gly Asp Pro Gly Glu Thr Gly
           195                 200                 205
   Ala Lys Gly Glu Ser Gly Leu Ala Glu Val Asn Ala Leu Lys Gln Arg
       210                 215                 220
   Val Thr Ile Leu Asp Gly His Leu Arg Arg Phe Gln Asn Ala Phe Ser
   225 230 235 240
   Gln Tyr Lys Lys Ala Val Leu Phe Pro Asp Gly Gln Ala Val Gly Glu
   245 250 255
   Lys Ile Phe Lys Thr Ala Gly Ala Val Lys Ser Tyr Ser Asp Ala Glu
   260 265 270
   Gln Leu Cys Arg Glu Ala Lys Gly Gln Leu Ala Ser Pro Arg Ser Ser
   275 280 285
   Ala Glu Asn Glu Ala Val Thr Gln Met Val Arg Ala Gln Glu Lys Asn
   290 295 300
   Ala Tyr Leu Ser Met Asn Asp Ile Ser Thr Glu Gly Arg Phe Thr Tyr
   305 310 315 320
   Pro Thr Gly Glu Ile Leu Val Tyr Ser Asn Trp Ala Asp Gly Glu Pro
   325 330 335
   Asn Asn Ser Asp Glu Gly Gln Pro Glu Asn Cys Val Glu Ile Phe Pro
   340 345 350
   Asp Gly Lys Trp Asn Asp Val Pro Cys Ser Lys Gln Leu Leu Val Ile
   355 360 365
   Cys Glu Phe
   370
<210> 63
   <211> 30
   <212> PRT
   <213> Gallus gallus
<400> 63
   Leu Leu Thr Cys Asp Lys Pro Glu Glu Lys Met Tyr Ser Cys Pro Ile
   1 5 10 15
   Ile Gln Cys Ser Ala Pro Ala Val Asn Gly Leu Pro Gly Asp
   20 25 30
<210> 64
   <211> 301
   <212> PRT
   <213> Bos taurus
<400> 64
   Glu Glu Met Asp Val Tyr Ser Glu Lys Thr Leu Thr Asp Pro Cys Thr
   1               5                   10                  15
   Leu Val Val Cys Ala Pro Pro Ala Asp Ser Leu Arg Gly His Asp Gly
               20                  25                  30
   Arg Asp Gly Lys Glu Gly Pro Gln Gly Glu Lys Gly Asp Pro Gly Pro
           35                  40                  45
   Pro Gly Met Pro Gly Pro Ala Gly Arg Glu Gly Pro Ser Gly Arg Gln
       50                  55                  60
   Gly Ser Met Gly Pro Pro Gly Thr Pro Gly Pro Lys Gly Glu Pro Gly
   65                  70                  75                  80
   Pro Glu Gly Gly Val Gly Ala Pro Gly Met Pro Gly Ser Pro Gly Pro
                   85                  90                  95
   Ala Gly Leu Lys Gly Glu Arg Gly Ala Pro Gly Pro Gly Gly Ala Ile
               100                 105                 110
   Gly Pro Gln Gly Pro Ser Gly Ala Met Gly Pro Pro Gly Leu Lys Gly
           115                 120                 125
   Asp Arg Gly Asp Pro Gly Glu Lys Gly Ala Arg Gly Glu Thr Ser Val
       130                 135                 140
   Leu Glu Val Asp Thr Leu Arg Gln Arg Met Arg Asn Leu Glu Gly Glu
   145                 150                 155                 160
   Val Gln Arg Leu Gln Asn Ile Val Thr Gln Tyr Arg Lys Ala Val Leu
                   165                 170                 175
   Phe Pro Asp Gly Gln Ala Val Gly Glu Lys Ile Phe Lys Thr Ala Gly
               180                 185                 190
   Ala Val Lys Ser Tyr Ser Asp Ala Glu Gln Leu Cys Arg Glu Ala Lys
           195                 200                 205
   Gly Gln Leu Ala Ser Pro Arg Ser Ser Ala Glu Asn Glu Ala Val Thr
       210                 215                 220
   Gln Leu Val Arg Ala Lys Asn Lys His Ala Tyr Leu Ser Met Asn Asp
   225                 230                 235                 240
   Ile Ser Lys Glu Gly Lys Phe Thr Tyr Pro Thr Gly Gly Ser Leu Asp
                   245                 250                 255
   Tyr Ser Asn Trp Ala Pro Gly Glu Pro Asn Asn Arg Ala Lys Asp Glu
               260                 265                 270
   Gly Pro Glu Asn Cys Leu Glu Ile Tyr Ser Asp Gly Asn Trp Asn Asp
           275                 280                 285
   Ile Glu Cys Arg Glu Glu Arg Leu Val Ile Cys Glu Phe
       290                 295                 300
<210> 65
   <211> 116
   <212> PRT
   <213> Sus scrofa
<400> 65
   Ala Val Gly Glu Lys Val Phe Ser Thr Asn Gly Gln Ser Val Ala Phe
   1               5                   10                  15
   Asp Val Ile Arg Glu Leu Cys Ala Arg Ala Gly Gly Arg Ile Ala Ala
               20                  25                  30
   Pro Arg Ser Pro Glu Glu Asn Glu Ala Ile Ala Ser Ile Val Lys Lys
           35                  40                  45
   His Asn Thr Tyr Ala Tyr Leu Gly Leu Val Glu Gly Pro Thr Ala Gly
       50                  55                  60
   Asp Phe Phe Tyr Leu Asp Gly Thr Pro Val Asn Tyr Thr Asn Trp Tyr
   65                  70                  75                  80
   Pro Gly Glu Pro Arg Gly Arg Gly Lys Glu Lys Cys Val Glu Met Tyr
                   85                  90                  95
   Thr Asp Gly Gln Trp Asn Asp Arg Asn Cys Gln Gln Tyr Arg Leu Ala
   100 105 110
   Ile Cys Glu Phe
   115
<210> 66
   <211> 378
   <212> PRT
   <213> Sus scrofa
<400> 66
   Met Leu Leu Leu Pro Leu Ser Val Leu Ile Leu Leu Thr Gln Pro Pro
   1 5 10 15
   Arg Ser Leu Gly Ala Glu Met Lys Thr Tyr Ser Gln Arg Ala Val Ala
   20 25 30
   Asn Ala Cys Ala Leu Val Met Cys Ser Pro Met Glu Asn Gly Leu Pro
   35 40 45
   Gly Arg Asp Gly Arg Asp Gly Arg Glu Gly Pro Arg Gly Glu Lys Gly
   50 55 60
   Asp Pro Gly Leu Pro Gly Ala Val Gly Arg Ala Gly Met Pro Gly Leu
   65 70 75 80
   Ala Gly Pro Val Gly Pro Lys Gly Asp Asn Gly Ser Thr Gly Glu Pro
   85 90 95
   Gly Ala Lys Gly Asp Ile Gly Pro Cys Gly Pro Pro Gly Pro Pro Gly
   100 105 110
   Ile Pro Gly Pro Ala Gly Lys Glu Gly Pro Ser Gly Gln Gln Gly Asn
   115 120 125
   Ile Gly Pro Pro Gly Thr Pro Gly Pro Lys Gly Glu Thr Gly Pro Lys
   130 135 140
   Gly Glu Val Gly Ala Leu Gly Met Gln Gly Ser Thr Gly Ala Arg Gly
   145 150 155 160
   Pro Ala Gly Leu Lys Gly Glu Arg Gly Ala Pro Gly Glu Arg Gly Ala
   165 170 175
   Pro Gly Ser Ala Gly Ala Ala Gly Pro Ala Gly Ala Thr Gly Pro Gln
   180 185 190
   Gly Pro Ser Gly Ala Arg Gly Pro Pro Gly Leu Lys Gly Asp Arg Gly
   195 200 205
   Pro Pro Gly Glu Arg Gly Ala Lys Gly Glu Ser Gly Leu Pro Gly Ile
   210 215 220
   Thr Ala Leu Arg Gln Gln Val Glu Thr Leu Gln Gly Gln Val Gln Arg
   225 230 235 240
   Leu Gln Lys Ala Phe Ser Gln Tyr Lys Lys Val Glu Leu Phe Pro Asn
   245 250 255
   Gly Arg Gly Val Gly Glu Lys Ile Phe Lys Thr Gly Gly Phe Glu Lys
   260 265 270
   Thr Phe Gln Asp Ala Gln Gln Val Cys Thr Gln Ala Gly Gly Gln Met
   275 280 285
   Ala Ser Pro Arg Ser Glu Thr Glu Asn Glu Ala Leu Ser Gln Leu Val
   290 295 300
   Thr Ala Gln Asn Lys Ala Ala Phe Leu Ser Met Thr Asp Ile Lys Thr
   305 310 315 320
   Glu Gly Asn Phe Thr Tyr Pro Thr Gly Glu Pro Leu Val Tyr Ala Asn
   325 330 335
   Trp Ala Pro Gly Glu Pro Asn Asn Asn Gly Gly Ser Ser Gly Ala Glu
   340 345 350
   Asn Cys Val Glu Ile Phe Pro Asn Gly Lys Trp Asn Asp Lys Ala Cys
   355 360 365
   Gly Glu Leu Arg Leu Val Ile Cys Glu Phe
   370 375
<210> 67
   <211> 244
   <212> PRT
   <213> Mus musculus
<400> 67
   Met Ser Ile Phe Thr Ser Phe Leu Leu Leu Cys Val Val Thr Val Val
   1               5                   10                  15
   Tyr Ala Glu Thr Leu Thr Glu Gly Val Gln Asn Ser Cys Pro Val Val
               20                  25                  30
   Thr Cys Ser Ser Pro Gly Leu Asn Gly Phe Pro Gly Lys Asp Gly Arg
           35                  40                  45
   Asp Gly Ala Lys Gly Glu Lys Gly Glu Pro Gly Gln Gly Leu Arg Gly
       50                  55                  60
   Leu Gln Gly Pro Pro Gly Lys Val Gly Pro Thr Gly Pro Pro Gly Asn
   65                  70                  75                  80
   Pro Gly Leu Lys Gly Ala Val Gly Pro Lys Gly Asp Arg Gly Asp Arg
                   85                  90                  95
   Ala Glu Phe Asp Thr Ser Glu Ile Asp Ser Glu Ile Ala Ala Leu Arg
               100                 105                 110
   Ser Glu Leu Arg Ala Leu Arg Asn Trp Val Leu Phe Ser Leu Ser Glu
           115                 120                 125
   Lys Val Gly Lys Lys Tyr Phe Val Ser Ser Val Lys Lys Met Ser Leu
       130                 135                 140
   Asp Arg Val Lys Ala Leu Cys Ser Glu Phe Gln Gly Ser Val Ala Thr
   145                 150                 155                 160
   Pro Arg Asn Ala Glu Glu Asn Ser Ala Ile Gln Lys Val Ala Lys Asp
                   165                 170                 175
   Ile Ala Tyr Leu Gly Ile Thr Asp Val Arg Val Glu Gly Ser Phe Glu
               180                 185                 190
   Asp Leu Thr Gly Asn Arg Val Arg Tyr Thr Asn Trp Asn Asp Gly Glu
           195                 200                 205
   Pro Asn Asn Thr Gly Asp Gly Glu Asp Cys Val Val Ile Leu Gly Asn
       210                 215                 220
   Gly Lys Trp Asn Asp Val Pro Cys Ser Asp Ser Phe Leu Ala Ile Cys
   225                 230                 235                 240
   Glu Phe Ser Asp
<210> 68
   <211> 239
   <212> PRT
   <213> Mus musculus
<400> 68
   Met Leu Leu Leu Pro Leu Leu Pro Val Leu Leu Cys Val Val Ser Val
   1               5                   10                  15
   Ser Ser Ser Gly Ser Gln Thr Cys Glu Asp Thr Leu Lys Thr Cys Ser
               20                  25                  30
   Val Ile Ala Cys Gly Arg Asp Gly Arg Asp Gly Pro Lys Gly Glu Lys
           35                  40                  45
   Gly Glu Pro Gly Gln Gly Leu Arg Gly Leu Gln Gly Pro Pro Gly Lys
       50                  55                  60
   Leu Gly Pro Pro Gly Ser Val Gly Ser Pro Gly Ser Pro Gly Pro Lys
   65                  70                  75                  80
   Gly Gln Lys Gly Asp His Gly Asp Asn Arg Ala Ile Glu Glu Lys Leu
                   85                  90                  95
   Ala Asn Met Glu Ala Glu Ile Arg Ile Leu Lys Ser Lys Leu Gln Leu
               100                 105                 110
   Thr Asn Lys Leu His Ala Phe Ser Met Gly Lys Lys Ser Gly Lys Lys
           115                 120                 125
   Leu Phe Val Thr Asn His Glu Lys Met Pro Phe Ser Lys Val Lys Ser
       130                 135                 140
   Leu Cys Thr Glu Leu Gln Gly Thr Val Ala Ile Pro Arg Asn Ala Glu
   145                 150                 155                 160
   Glu Asn Lys Ala Ile Gln Glu Val Ala Thr Gly Ile Ala Phe Leu Gly
                   165                 170                 175
   Ile Thr Asp Glu Ala Thr Glu Gly Gln Phe Met Tyr Val Thr Gly Gly
   180 185 190
   Arg Leu Thr Tyr Ser Asn Trp Lys Lys Asp Glu Pro Asn Asn His Gly
   195 200 205
   Ser Gly Glu Asp Cys Val Ile Ile Leu Asp Asn Gly Leu Trp Asn Asp
   210 215 220
   Ile Ser Cys Gln Ala Ser Phe Lys Ala Val Cys Glu Phe Pro Ala
   225 230 235
<210> 69
   <211> 301
   <212> PRT
   <213> Bos taurus
<400> 69
   Glu Glu Met Asp Val Tyr Ser Glu Lys Thr Leu Thr Asp Pro Cys Thr
   1 5 10 15
   Leu Val Val Cys Ala Pro Pro Ala Asp Ser Leu Arg Gly His Asp Gly
   20 25 30
   Arg Asp Gly Lys Glu Gly Pro Gln Gly Glu Lys Gly Asp Pro Gly Pro
   35 40 45
   Pro Gly Met Pro Gly Pro Ala Gly Arg Glu Gly Pro Ser Gly Arg Gln
   50 55 60
   Gly Ser Met Gly Pro Pro Gly Thr Pro Gly Pro Lys Gly Glu Pro Gly
   65 70 75 80
   Pro Glu Gly Gly Val Gly Ala Pro Gly Met Pro Gly Ser Pro Gly Pro
   85 90 95
   Ala Gly Leu Lys Gly Glu Arg Gly Ala Pro Gly Pro Gly Gly Ala Ile
   100 105 110
   Gly Pro Gln Gly Pro Ser Gly Ala Met Gly Pro Pro Gly Leu Lys Gly
   115 120 125
   Asp Arg Gly Asp Pro Gly Glu Lys Gly Ala Arg Gly Glu Thr Ser Val
   130 135 140
   Leu Glu Val Asp Thr Leu Arg Gln Arg Met Arg Asn Leu Glu Gly Glu
   145 150 155 160
   Val Gln Arg Leu Gln Asn Ile Val Thr Gln Tyr Arg Lys Ala Val Leu
   165 170 175
   Phe Pro Asp Gly Gln Ala Val Gly Glu Lys Ile Phe Lys Thr Ala Gly
   180 185 190
   Ala Val Lys Ser Tyr Ser Asp Ala Glu Gln Leu Cys Arg Glu Ala Lys
   195 200 205
   Gly Gln Leu Ala Ser Pro Arg Ser Ser Ala Glu Asn Glu Ala Val Thr
   210 215 220
   Gln Leu Val Arg Ala Lys Asn Lys His Ala Tyr Leu Ser Met Asn Asp
   225 230 235 240
   Ile Ser Lys Glu Gly Lys Phe Thr Tyr Pro Thr Gly Gly Ser Leu Asp
   245 250 255
   Tyr Ser Asn Trp Ala Pro Gly Glu Pro Gly Asn Arg Ala Lys Asp Glu
   260 265 270
   Gly Pro Glu Asn Cys Leu Glu Ile Tyr Ser Asp Gly Asn Trp Asn Asp
   275 280 285
   Ile Glu Cys Arg Glu Glu Arg Leu Val Ile Cys Glu Phe
   290 295 300
<210> 70
   <211> 2412
   <212> PRT
   <213> Homo sapiens
<400> 70
<210> 71
   <211> 277
   <212> PRT
   <213> Homo sapiens
<400> 71
   Met Asn Gly Phe Ala Ser Leu Leu Arg Arg Asn Gln Phe Ile Leu Leu
   1               5                   10                  15
   Val Leu Phe Leu Leu Gln Ile Gln Ser Leu Gly Leu Asp Ile Asp Ser
               20                  25                  30
   Arg Pro Thr Ala Glu Val Cys Ala Thr His Thr Ile Ser Pro Gly Pro
           35                  40                  45
   Lys Gly Asp Asp Gly Glu Lys Gly Asp Pro Gly Glu Glu Gly Lys His
       50                  55                  60
   Gly Lys Val Gly Arg Met Gly Pro Lys Gly Ile Lys Gly Glu Leu Gly
   65                  70                  75                  80
   Asp Met Gly Asp Arg Gly Asn Ile Gly Lys Thr Gly Pro Ile Gly Lys
                   85                  90                  95
   Lys Gly Asp Lys Gly Glu Lys Gly Leu Leu Gly Ile Pro Gly Glu Lys
               100                 105                 110
   Gly Lys Ala Gly Thr Val Cys Asp Cys Gly Arg Tyr Arg Lys Phe Val
           115                 120                 125
   Gly Gln Leu Asp Ile Ser Ile Ala Arg Leu Lys Thr Ser Met Lys Phe
       130                 135                 140
   Val Lys Asn Val Ile Ala Gly Ile Arg Glu Thr Glu Glu Lys Phe Tyr
   145                 150                 155                 160
   Tyr Ile Val Gln Glu Glu Lys Asn Tyr Arg Glu Ser Leu Thr His Cys
                   165                 170                 175
   Arg Ile Arg Gly Gly Met Leu Ala Met Pro Lys Asp Glu Ala Ala Asn
               180                 185                 190
   Thr Leu Ile Ala Asp Tyr Val Ala Lys Ser Gly Phe Phe Arg Val Phe
           195                 200                 205
   Ile Gly Val Asn Asp Leu Glu Arg Glu Gly Gln Tyr Met Phe Thr Asp
   210 215 220
   Asn Thr Pro Leu Gln Asn Tyr Ser Asn Trp Asn Glu Gly Glu Pro Ser
   225 230 235 240
   Asp Pro Tyr Gly His Glu Asp Cys Val Glu Met Leu Ser Ser Gly Arg
   245 250 255
   Trp Asn Asp Thr Glu Cys His Leu Thr Met Tyr Phe Val Cys Glu Phe
   260 265 270
   Ile Lys Lys Lys Lys
   275
<210> 72
   <211> 238
   <212> PRT
   <213> Gallus gallus
<400> 72
   Met Met Ala Thr Ser Leu Leu Thr Thr Asp Lys Pro Glu Glu Lys Met
   1 5 10 15
   Tyr Ser Cys Pro Ile Ile Gln Cys Ser Ala Pro Ala Val Asn Gly Leu
   20 25 30
   Pro Gly Arg Asp Gly Arg Asp Gly Pro Lys Gly Glu Lys Gly Asp Pro
   35 40 45
   Gly Glu Gly Leu Arg Gly Leu Gln Gly Leu Pro Gly Lys Ala Gly Pro
   50 55 60
   Gln Gly Leu Lys Gly Glu Val Gly Pro Gln Gly Glu Lys Gly Gln Lys
   65 70 75 80
   Gly Glu Arg Gly Ile Val Val Thr Asp Asp Leu His Arg Gln Ile Thr
   85 90 95
   Asp Leu Glu Ala Lys Ile Arg Val Leu Glu Asp Asp Leu Ser Arg Tyr
   100 105 110
   Lys Lys Ala Leu Ser Leu Lys Asp Val Val Asn Ile Gly Lys Lys Met
   115 120 125
   Phe Val Ser Thr Gly Lys Lys Tyr Asn Phe Glu Lys Gly Lys Ser Leu
   130 135 140
   Cys Ala Lys Ala Gly Ser Val Leu Ala Ser Pro Arg Asn Glu Ala Glu
   145 150 155 160
   Asn Thr Ala Leu Lys Asp Leu Ile Asp Pro Ser Ser Gln Ala Tyr Ile
   165 170 175
   Gly Ile Ser Asp Ala Gln Thr Glu Gly Arg Phe Met Tyr Leu Ser Gly
   180 185 190
   Gly Pro Leu Thr Tyr Ser Asn Trp Lys Pro Gly Glu Pro Asn Asn His
   195 200 205
   Lys Asn Glu Asp Cys Ala Val Ile Glu Asp Ser Gly Lys Trp Asn Asp
   210 215 220
   Leu Asp Cys Ser Asn Ser Asn Ile Phe Ile Ile Cys Glu Leu
   225 230 235
<210> 73
   <211> 30
   <212> PRT
   <213> Gallus gallus
<400> 73
   Leu Leu Thr Cys Asp Lys Pro Glu Glu Lys Met Tyr Ser Cys Pro Ile
   1 5 10 15
   Ile Gln Cys Ser Ala Pro Ala Val Asn Gly Leu Pro Gly Asp
   20 25 30
<210> 74
   <211> 60
   <212> PRT
   <213> Bos taurus
<400> 74
   Ala Glu Met Thr Thr Phe Ser Gln Lys Ile Leu Ala Asn Ala Cys Thr
   1 5 10 15
   Leu Val Met Cys Ser Pro Leu Glu Ser Gly Leu Pro Gly His Asp Gly
   20 25 30
   Gln Asp Gly Arg Glu Cys Pro His Gly Glu Lys Gly Asp Pro Gly Ser
   35 40 45
   Pro Gly Pro Ala Gly Arg Ala Gly Arg Pro Gly Trp
   50 55 60
<210> 75
   <211> 301
   <212> PRT
   <213> Bos taurus
<220>
   <221> MISC_FEATURE
   <222> (7)..()
   <223> Unknown
<220>
   <221> MISC_FEATURE
   <222> (7)..(7)
   <223> Unknown
<400> 75
   Glu Glu Met Asp Val Tyr Xaa Glu Lys Thr Leu Thr Asp Pro Cys Thr
   1 5 10 15
   Leu Val Val Cys Ala Pro Pro Ala Asp Ser Leu Arg Gly His Asp Gly
   20 25 30
   Arg Asp Gly Lys Glu Gly Pro Gln Gly Glu Lys Gly Asp Pro Gly Pro
   35 40 45
   Pro Gly Met Pro Gly Pro Ala Gly Arg Glu Gly Pro Ser Gly Arg Gln
   50 55 60
   Gly Ser Met Gly Pro Pro Gly Thr Pro Gly Pro Lys Gly Glu Pro Gly
   65 70 75 80
   Pro Glu Gly Gly Val Gly Ala Pro Gly Met Pro Gly Ser Pro Gly Pro
   85 90 95
   Ala Gly Leu Lys Gly Glu Arg Gly Ala Pro Gly Pro Gly Gly Ala Ile
   100 105 110
   Gly Pro Gln Gly Pro Ser Gly Ala Met Gly Pro Pro Gly Leu Lys Gly
   115 120 125
   Asp Arg Gly Asp Pro Gly Glu Lys Gly Ala Arg Gly Glu Thr Ser Val
   130 135 140
   Leu Glu Val Asp Thr Leu Arg Gln Arg Met Arg Asn Leu Glu Gly Glu
   145 150 155 160
   Val Gln Arg Leu Gln Asn Ile Val Thr Gln Tyr Arg Lys Ala Val Leu
   165 170 175
   Phe Pro Asp Gly Gln Ala Val Gly Glu Lys Ile Phe Lys Thr Ala Gly
   180 185 190
   Ala Val Lys Ser Tyr Ser Asp Ala Glu Gln Leu Cys Arg Glu Ala Lys
   195 200 205
   Gly Gln Leu Ala Ser Pro Arg Ser Ser Ala Glu Asn Glu Ala Val Thr
   210 215 220
   Gln Leu Val Arg Ala Lys Asn Lys His Ala Tyr Leu Ser Met Asn Asp
   225 230 235 240
   Ile Ser Lys Glu Gly Lys Phe Thr Tyr Pro Thr Gly Gly Ser Leu Asp
   245 250 255
   Tyr Ser Asn Trp Ala Pro Gly Glu Pro Gly Asn Arg Ala Lys Asp Glu
   260 265 270
   Gly Pro Glu Asn Cys Leu Glu Ile Tyr Ser Asp Gly Asn Trp Asn Asp
   275 280 285
   Ile Glu Cys Arg Glu Glu Arg Leu Val Ile Cys Glu Phe
   290 295 300
<210> 76
   <211> 244
   <212> PRT
   <213> Mus musculus
<400> 76
   Met Ser Ile Phe Thr Ser Phe Leu Leu Leu Cys Val Val Thr Val Val
   1               5                   10                  15
   Tyr Ala Glu Thr Leu Thr Glu Gly Val Gln Asn Ser Cys Pro Val Val
               20                  25                  30
   Thr Cys Ser Ser Pro Gly Leu Asn Gly Phe Pro Gly Lys Asp Gly Arg
           35                  40                  45
   Asp Gly Ala Lys Gly Glu Lys Gly Glu Pro Gly Gln Gly Leu Arg Gly
       50                  55                  60
   Leu Gln Gly Pro Pro Gly Lys Val Gly Pro Thr Gly Pro Pro Gly Asn
   65                  70                  75                  80
   Pro Gly Leu Lys Gly Ala Val Gly Pro Lys Gly Asp Arg Gly Asp Arg
                   85                  90                  95
   Ala Glu Phe Asp Thr Ser Glu Ile Asp Ser Glu Ile Ala Ala Leu Arg
               100                 105                 110
   Ser Glu Leu Arg Ala Leu Arg Asn Trp Val Leu Phe Ser Leu Ser Glu
           115                 120                 125
   Lys Val Gly Lys Lys Tyr Phe Val Ser Ser Val Lys Lys Met Ser Leu
       130                 135                 140
   Asp Arg Val Lys Ala Leu Cys Ser Glu Phe Gln Gly Ser Val Ala Thr
   145                 150                 155                 160
   Pro Arg Asn Ala Glu Glu Asn Ser Ala Ile Gln Lys Val Ala Lys Asp
                   165                 170                 175
   Ile Ala Tyr Leu Gly Ile Thr Asp Val Arg Val Glu Gly Ser Phe Glu
               180                 185                 190
   Asp Leu Thr Gly Asn Arg Val Arg Tyr Thr Asn Trp Asn Asp Gly Glu
   195 200 205
   Pro Asn Asn Thr Gly Asp Gly Glu Asp Cys Val Val Ile Leu Gly Asn
   210 215 220
   Gly Lys Trp Asn Asp Val Pro Cys Ser Asp Ser Phe Leu Ala Ile Cys
   225 230 235 240
   Glu Phe Ser Asp
<210> 77
   <211> 239
   <212> PRT
   <213> Mus musculus
<400> 77
   Met Leu Leu Leu Pro Leu Leu Pro Val Leu Leu Cys Val Val Ser Val
   1 5 10 15
   Ser Ser Ser Gly Ser Gln Thr Cys Glu Asp Thr Leu Lys Thr Cys Ser
   20 25 30
   Val Ile Ala Cys Gly Arg Asp Gly Arg Asp Gly Pro Lys Gly Glu Lys
   35 40 45
   Gly Glu Pro Gly Gln Gly Leu Arg Gly Leu Gln Gly Pro Pro Gly Lys
   50 55 60
   Leu Gly Pro Pro Gly Ser Val Gly Ser Pro Gly Ser Pro Gly Pro Lys
   65 70 75 80
   Gly Gln Lys Gly Asp His Gly Asp Asn Arg Ala Ile Glu Glu Lys Leu
   85 90 95
   Ala Asn Met Glu Ala Glu Ile Arg Ile Leu Lys Ser Lys Leu Gln Leu
   100 105 110
   Thr Asn Lys Leu His Ala Phe Ser Met Gly Lys Lys Ser Gly Lys Lys
   115 120 125
   Leu Phe Val Thr Asn His Glu Lys Met Pro Phe Ser Lys Val Lys Ser
   130 135 140
   Leu Cys Thr Glu Leu Gln Gly Thr Val Ala Ile Pro Arg Asn Ala Glu
   145 150 155 160
   Glu Asn Lys Ala Ile Gln Glu Val Ala Thr Gly Ile Ala Phe Leu Gly
   165 170 175
   Ile Thr Asp Glu Ala Thr Glu Gly Gln Phe Met Tyr Val Thr Gly Gly
   180 185 190
   Arg Leu Thr Tyr Ser Asn Trp Lys Lys Asp Glu Pro Asn Asn His Gly
   195 200 205
   Ser Gly Glu Asp Cys Val Ile Ile Leu Asp Asn Gly Leu Trp Asn Asp
   210 215 220
   Ile Ser Cys Gln Ala Ser Phe Lys Ala Val Cys Glu Phe Pro Ala
   225 230 235
<210> 78
   <211> 375
   <212> PRT
   <213> Homo sapiens
<400> 78
   Met Leu Leu Phe Leu Leu Ser Ala Leu Val Leu Leu Thr Gln Pro Leu
   1 5 10 15
   Gly Tyr Leu Glu Ala Glu Met Lys Thr Tyr Ser His Arg Thr Met Pro
   20 25 30
   Ser Ala Cys Thr Leu Val Met Cys Ser Ser Val Glu Ser Gly Leu Pro
   35 40 45
   Gly Arg Asp Gly Arg Asp Gly Arg Glu Gly Pro Arg Gly Glu Lys Gly
   50 55 60
   Asp Pro Gly Leu Pro Gly Ala Ala Gly Gln Ala Gly Met Pro Gly Gln
   65 70 75 80
   Ala Gly Pro Val Gly Pro Lys Gly Asp Asn Gly Ser Val Gly Glu Pro
   85 90 95
   Gly Pro Lys Gly Asp Thr Gly Pro Ser Gly Pro Pro Gly Pro Pro Gly
   100 105 110
   Val Pro Gly Pro Ala Gly Arg Glu Gly Ala Leu Gly Lys Gln Gly Asn
   115 120 125
   Ile Gly Pro Gln Gly Lys Pro Gly Pro Lys Gly Glu Ala Gly Pro Lys
   130 135 140
   Gly Glu Val Gly Ala Pro Gly Met Gln Gly Ser Ala Gly Ala Arg Gly
   145 150 155 160
   Leu Ala Gly Pro Lys Gly Glu Arg Gly Val Pro Gly Glu Arg Gly Val
   165 170 175
   Pro Gly Asn Thr Gly Ala Ala Gly Ser Ala Gly Ala Met Gly Pro Gln
   180 185 190
   Gly Ser Pro Gly Ala Arg Gly Pro Pro Gly Leu Lys Gly Asp Lys Gly
   195 200 205
   Ile Pro Gly Asp Lys Gly Ala Lys Gly Glu Ser Gly Leu Pro Asp Val
   210 215 220
   Ala Ser Leu Arg Gln Gln Val Glu Ala Leu Gln Gly Gln Val Gln His
   225 230 235 240
   Leu Gln Ala Ala Phe Ser Gln Tyr Lys Lys Val Glu Leu Phe Pro Asn
   245 250 255
   Gly Gln Ser Val Gly Glu Lys Ile Phe Lys Thr Ala Gly Phe Val Lys
   260 265 270
   Pro Phe Thr Glu Ala Gln Leu Leu Cys Thr Gln Ala Gly Gly Gln Leu
   275 280 285
   Ala Ser Pro Arg Ser Ala Ala Glu Asn Ala Ala Leu Gln Gln Leu Val
   290 295 300
   Val Ala Lys Asn Glu Ala Ala Phe Leu Ser Met Thr Asp Ser Lys Thr
   305 310 315 320
   Glu Gly Lys Phe Thr Tyr Pro Thr Gly Glu Ser Leu Val Tyr Ser Asn
   325 330 335
   Trp Ala Pro Gly Glu Pro Asn Asp Asp Gly Gly Ser Glu Asp Cys Val
   340 345 350
   Glu Ile Phe Thr Asn Gly Lys Trp Asn Asp Arg Ala Cys Gly Glu Lys
   355 360 365
   Arg Leu Val Val Cys Glu Phe
   370 375
<210> 79
   <211> 255
   <212> PRT
   <213> Homo sapiens
<400> 79
   Met Lys Ala Leu Leu Ala Leu Pro Leu Leu Leu Leu Leu Ser Thr Pro
   1 5 10 15
   Pro Cys Ala Pro Gln Val Ser Gly Ile Arg Gly Asp Ala Leu Glu Arg
   20 25 30
   Phe Cys Leu Gln Gln Pro Leu Asp Cys Asp Asp Ile Tyr Ala Gln Gly
   35 40 45
   Tyr Gln Ser Asp Gly Val Tyr Leu Ile Tyr Pro Ser Gly Pro Ser Val
   50 55 60
   Pro Val Pro Val Phe Cys Asp Met Thr Thr Glu Gly Gly Lys Trp Thr
   65 70 75 80
   Val Phe Gln Lys Arg Phe Asn Gly Ser Val Ser Phe Phe Arg Gly Trp
   85 90 95
   Asn Asp Tyr Lys Leu Gly Phe Gly Arg Ala Asp Gly Glu Tyr Trp Leu
   100 105 110
   Gly Leu Gln Asn Met His Leu Leu Thr Leu Lys Gln Lys Tyr Glu Leu
   115 120 125
   Arg Val Asp Leu Glu Asp Phe Glu Asn Asn Thr Ala Tyr Ala Lys Tyr
   130 135 140
   Ala Asp Phe Ser Ile Ser Pro Asn Ala Val Ser Ala Glu Glu Asp Gly
   145 150 155 160
   Tyr Thr Leu Phe Val Ala Gly Phe Glu Asp Gly Gly Ala Gly Asp Ser
   165 170 175
   Leu Ser Tyr His Ser Gly Gln Lys Phe Ser Thr Phe Asp Arg Asp Gln
   180 185 190
   Asp Leu Phe Val Gln Asn Cys Ala Ala Leu Ser Ser Gly Ala Phe Trp
   195 200 205
   Phe Arg Ser Cys His Phe Ala Asn Leu Asn Gly Phe Tyr Leu Gly Gly
   210 215 220
   Ser His Leu Ser Tyr Ala Asn Gly Ile Asn Trp Ala Gln Trp Lys Gly
   225 230 235 240
   Phe Tyr Tyr Ser Leu Lys Arg Thr Glu Met Lys Ile Arg Arg Ala
   245 250 255
<210> 80
   <211> 15
   <212> PRT
   <213> Homo sapiens
<400> 80
   Cys Arg Ala Leu Ile Lys Arg Ile Gln Ala Met Ile Pro Lys Gly
   1 5 10 15
<210> 81
   <211> 374
   <212> PRT
   <213> Mus musculus
<400> 81
   Met Leu Pro Phe Leu Ser Met Leu Val Leu Leu Val Gln Pro Leu Gly
   1 5 10 15
   Asn Leu Gly Ala Glu Met Lys Ser Leu Ser Gln Arg Ser Val Pro Asn
   20 25 30
   Thr Cys Thr Leu Val Met Cys Ser Pro Thr Glu Asn Gly Leu Pro Gly
   35 40 45
   Arg Asp Gly Arg Asp Gly Arg Glu Gly Pro Arg Gly Glu Lys Gly Asp
       50                  55                  60
   Pro Gly Leu Pro Gly Pro Met Gly Leu Ser Gly Leu Gln Gly Pro Thr
   65                  70                  75                  80
   Gly Pro Val Gly Pro Lys Gly Glu Asn Gly Ser Ala Gly Glu Pro Gly
                   85                  90                  95
   Pro Lys Gly Glu Arg Gly Leu Ser Gly Pro Pro Gly Leu Pro Gly Ile
               100                 105                 110
   Pro Gly Pro Ala Gly Lys Glu Gly Pro Ser Gly Lys Gln Gly Asn Ile
           115                 120                 125
   Gly Pro Gln Gly Lys Pro Gly Pro Lys Gly Glu Ala Gly Pro Lys Gly
       130                 135                 140
   Glu Val Gly Ala Pro Gly Met Gln Gly Ser Thr Gly Ala Lys Gly Ser
   145                 150                 155                 160
   Thr Gly Pro Lys Gly Glu Arg Gly Ala Pro Gly Val Gln Gly Ala Pro
                   165                 170                 175
   Gly Asn Ala Gly Ala Ala Gly Pro Ala Gly Pro Ala Gly Pro Gln Gly
               180                 185                 190
   Ala Pro Gly Ser Arg Gly Pro Pro Gly Leu Lys Gly Asp Arg Gly Val
           195                 200                 205
   Pro Gly Asp Arg Gly Ile Lys Gly Glu Ser Gly Leu Pro Asp Ser Ala
       210                 215                 220
   Ala Leu Arg Gln Gln Met Glu Ala Leu Lys Gly Lys Leu Gln Arg Leu
   225                 230                 235                 240
   Glu Val Ala Phe Ser His Tyr Gln Lys Ala Ala Leu Phe Pro Asp Gly
                   245                 250                 255
   Arg Ser Val Gly Asp Lys Ile Phe Arg Thr Ala Asp Ser Glu Lys Pro
               260                 265                 270
   Phe Glu Asp Ala Gln Glu Met Cys Lys Gln Ala Gly Gly Gln Leu Ala
           275                 280                 285
   Ser Pro Arg Ser Ala Thr Glu Asn Ala Ala Ile Gln Gln Leu Ile Thr
   290 295 300
   Ala His Asn Lys Ala Ala Phe Leu Ser Met Thr Asp Val Gly Thr Glu
   305 310 315 320
   Gly Lys Phe Thr Tyr Pro Thr Gly Glu Pro Leu Val Tyr Ser Asn Trp
   325 330 335
   Ala Pro Gly Glu Pro Asn Asn Asn Gly Gly Ala Glu Asn Cys Val Glu
   340 345 350
   Ile Phe Thr Asn Gly Gln Trp Asn Asp Lys Ala Cys Gly Glu Gln Arg
   355 360 365
   Leu Val Ile Cys Glu Phe
   370
<210> 82
   <211> 248
   <212> PRT
   <213> Canis familiaris
<400> 82
   Met Trp Leu Arg Cys Leu Ala Leu Ala Leu Thr Leu Leu Met Val Ser
   1 5 10 15
   Gly Ile Glu Asn Asn Thr Lys Asp Val Cys Val Gly Asn Pro Gly Ile
   20 25 30
   Pro Gly Thr Pro Gly Ser His Gly Leu Pro Gly Arg Asp Gly Arg Asp
   35 40 45
   Gly Val Lys Gly Asp Pro Gly Pro Pro Gly Pro Leu Gly Pro Pro Gly
   50 55 60
   Gly Met Pro Gly His Pro Gly Pro Asn Gly Met Thr Gly Ala Pro Gly
   65 70 75 80
   Val Ala Gly Glu Arg Gly Glu Lys Gly Glu Pro Gly Glu Arg Gly Pro
   85 90 95
   Pro Gly Leu Pro Ala Ser Leu Asp Glu Glu Leu Gln Thr Thr Leu His
   100 105 110
   Asp Leu Arg His Gln Ile Leu Gln Thr Met Gly Val Leu Ser Leu His
   115 120 125
   Glu Ser Leu Leu Val Val Gly Arg Lys Val Phe Ser Ser Asn Ala Gln
   130 135 140
   Ser Ile Asn Phe Asn Asp Ile Gln Glu Leu Cys Ala Gly Ala Gly Gly
   145 150 155 160
   Gln Ile Ala Ala Pro Met Ser Pro Glu Glu Asn Glu Ala Val Ala Ser
   165 170 175
   Ile Val Lys Lys Tyr Asn Thr Tyr Ala Tyr Leu Gly Leu Val Glu Ser
   180 185 190
   Pro Asp Ser Gly Asp Phe Gln Tyr Met Asp Gly Ala Pro Val Asn Tyr
   195 200 205
   Thr Asn Trp Tyr Pro Gly Glu Pro Arg Gly Arg Gly Lys Glu Gln Cys
   210 215 220
   Val Glu Met Tyr Thr Asp Gly Gln Trp Asn Asn Lys Asn Cys Leu Gln
   225 230 235 240
   Tyr Arg Leu Ala Ile Cys Glu Phe
   245
<210> 83
   <211> 247
   <212> PRT
   <213> Oryctolagus cuniculus
<400> 83
   Met Leu Leu Leu Ser Leu Ala Leu Thr Leu Ile Ser Ala Pro Ala Ser
   1 5 10 15
   Asp Thr Cys Asp Thr Lys Asp Val Cys Ile Gly Ser Pro Gly Ile Pro
   20 25 30
   Gly Thr Pro Gly Ser His Gly Leu Pro Gly Arg Asp Gly Arg Asp Gly
   35 40 45
   Val Lys Gly Asp Pro Gly Pro Pro Gly Pro Met Gly Pro Pro Gly Gly
   50 55 60
   Met Pro Gly Leu Pro Gly Arg Asp Gly Leu Ile Gly Ala Pro Gly Val
   65 70 75 80
   Pro Gly Glu Arg Gly Asp Lys Gly Glu Pro Gly Glu Arg Gly Pro Pro
   85 90 95
   Gly Leu Pro Ala Tyr Leu Asp Glu Glu Leu Gln Ala Thr Leu His Glu
   100 105 110
   Leu Arg His His Ala Leu Gln Ser Ile Gly Val Leu Ser Leu Gln Gly
   115 120 125
   Ser Met Lys Ala Val Gly Glu Lys Ile Phe Ser Thr Asn Gly Gln Ser
   130 135 140
   Val Asn Phe Asp Ala Ile Arg Glu Val Cys Ala Arg Ala Gly Gly Arg
   145 150 155 160
   Ile Ala Val Pro Arg Ser Leu Glu Glu Asn Glu Ala Ile Ala Ser Ile
   165 170 175
   Val Lys Glu Arg Asn Thr Tyr Ala Tyr Leu Gly Leu Ala Glu Gly Pro
   180 185 190
   Thr Ala Gly Asp Phe Tyr Tyr Leu Asp Gly Asp Pro Val Asn Tyr Thr
   195 200 205
   Asn Trp Tyr Pro Gly Glu Pro Arg Gly Gln Gly Arg Glu Lys Cys Val
   210 215 220
   Glu Met Tyr Thr Asp Gly Lys Trp Asn Asp Lys Asn Cys Leu Gln Tyr
   225 230 235 240
   Arg Leu Val Ile Cys Glu Phe
   245
<210> 84
   <211> 374
   <212> PRT
   <213> Mus musculus
<400> 84
   Met Leu Pro Phe Leu Ser Met Leu Val Leu Leu Val Gln Pro Leu Gly
   1               5                   10                  15
   Asn Leu Gly Ala Glu Met Lys Ser Leu Ser Gln Arg Ser Val Pro Asn
               20                  25                  30
   Thr Cys Thr Leu Val Met Cys Ser Pro Thr Glu Asn Gly Leu Pro Gly
           35                  40                  45
   Arg Asp Gly Arg Asp Gly Arg Glu Gly Pro Arg Gly Glu Lys Gly Asp
       50                  55                  60
   Pro Gly Leu Pro Gly Pro Met Gly Leu Ser Gly Leu Gln Gly Pro Thr
   65                  70                  75                  80
   Gly Pro Val Gly Pro Lys Gly Glu Asn Gly Ser Ala Gly Glu Pro Gly
                   85                  90                  95
   Pro Lys Gly Glu Arg Gly Leu Ser Gly Pro Pro Gly Leu Pro Gly Ile
               100                 105                 110
   Pro Gly Pro Ala Gly Lys Glu Gly Pro Ser Gly Lys Gln Gly Asn Ile
           115                 120                 125
   Gly Pro Gln Gly Lys Pro Gly Pro Lys Gly Glu Ala Gly Pro Lys Gly
       130                 135                 140
   Glu Val Gly Ala Pro Gly Met Gln Gly Ser Thr Gly Ala Lys Gly Ser
   145                 150                 155                 160
   Thr Gly Pro Lys Gly Glu Arg Gly Ala Pro Gly Val Gln Gly Ala Pro
                   165                 170                 175
   Gly Asn Ala Gly Ala Ala Gly Pro Ala Gly Pro Ala Gly Pro Gln Gly
               180                 185                 190
   Ala Pro Gly Ser Arg Gly Pro Pro Gly Leu Lys Gly Asp Arg Gly Val
           195                 200                 205
   Pro Gly Asp Arg Gly Ile Lys Gly Glu Ser Gly Leu Pro Asp Ser Ala
       210                 215                 220
   Ala Leu Arg Gln Gln Met Glu Ala Leu Lys Gly Lys Leu Gln Arg Leu
   225                 230                 235                 240
   Glu Val Ala Phe Ser His Tyr Gln Lys Ala Ala Leu Phe Pro Asp Gly
   245 250 255
   Arg Ser Val Gly Asp Lys Ile Phe Arg Thr Ala Asp Ser Glu Lys Pro
   260 265 270
   Phe Glu Asp Ala Gln Glu Met Cys Lys Gln Ala Gly Gly Gln Leu Ala
   275 280 285
   Ser Pro Arg Ser Ala Thr Glu Asn Ala Ala Ile Gln Gln Leu Ile Thr
   290 295 300
   Ala His Asn Lys Ala Ala Phe Leu Ser Met Thr Asp Val Gly Thr Glu
   305 310 315 320
   Gly Lys Phe Thr Tyr Pro Thr Gly Glu Pro Leu Val Tyr Ser Asn Trp
   325 330 335
   Ala Pro Gly Glu Pro Asn Asn Asn Gly Gly Ala Glu Asn Cys Val Glu
   340 345 350
   Ile Phe Thr Asn Gly Gln Trp Asn Asp Lys Ala Cys Gly Glu Gln Arg
   355 360 365
   Leu Val Ile Cys Glu Phe
   370
<210> 85
   <211> 158
   <212> PRT
   <213> Homo sapiens
<400> 85
   Glu Ala Glu Ala Gly Ser Val Ala Ser Leu Arg Gln Gln Val Glu Ala
   1 5 10 15
   Leu Gln Gly Gln Val Gln His Leu Gln Ala Ala Phe Ser Gln Tyr Lys
   20 25 30
   Lys Val Glu Leu Phe Pro Asn Gly Gln Ser Val Gly Glu Lys Ile Phe
   35 40 45
   Lys Thr Ala Gly Phe Val Lys Pro Phe Thr Glu Ala Gln Leu Leu Cys
   50 55 60
   Thr Gln Ala Gly Gly Gln Leu Ala Ser Pro Arg Ser Ala Ala Glu Asn
   65 70 75 80
   Ala Ala Leu Gln Gln Leu Val Val Ala Lys Asn Glu Ala Ala Phe Leu
   85 90 95
   Ser Met Thr Asp Ser Lys Thr Glu Gly Lys Phe Thr Tyr Pro Thr Gly
   100 105 110
   Glu Ser Leu Val Tyr Ser Asn Trp Ala Pro Gly Glu Pro Asn Asp Asp
   115 120 125
   Gly Gly Ser Glu Asp Cys Val Glu Ile Phe Thr Asn Gly Lys Trp Asn
   130 135 140
   Asp Arg Ala Cys Gly Glu Lys Arg Leu Val Val Cys Glu Phe
   145 150 155
<210> 86
   <211> 158
   <212> PRT
   <213> Homo sapiens
<400> 86
   Glu Ala Glu Ala Gly Ser Val Ala Ser Leu Arg Gln Gln Val Glu Ala
   1 5 10 15
   Leu Gln Gly Gln Val Gln His Leu Gln Ala Ala Phe Ser Gln Tyr Lys
   20 25 30
   Lys Val Glu Leu Phe Pro Asn Gly Gln Ser Val Gly Glu Lys Ile Phe
   35 40 45
   Lys Thr Ala Gly Phe Val Lys Pro Phe Thr Glu Ala Gln Leu Leu Cys
   50 55 60
   Thr Gln Ala Gly Gly Gln Leu Ala Ser Pro Arg Ser Ala Ala Glu Asn
   65 70 75 80
   Ala Ala Leu Gln Gln Leu Val Val Ala Lys Asn Glu Ala Ala Phe Leu
   85 90 95
   Ser Met Thr Asp Ser Lys Thr Glu Gly Lys Phe Thr Tyr Pro Thr Gly
   100 105 110
   Glu Ser Leu Val Tyr Ser Asn Trp Ala Pro Gly Glu Pro Asn Asp Asp
   115 120 125
   Gly Gly Ser Glu Asp Cys Val Glu Ile Phe Thr Asn Gly Lys Trp Asn
   130 135 140
   Asp Arg Ala Cys Gly Glu Lys Arg Leu Val Val Cys Glu Phe
   145 150 155
<210> 87
   <211> 158
   <212> PRT
   <213> Homo sapiens
<400> 87
   Glu Ala Glu Ala Gly Ser Val Ala Ser Leu Arg Gln Gln Val Glu Ala
   1 5 10 15
   Leu Gln Gly Gln Val Gln His Leu Gln Ala Ala Phe Ser Gln Tyr Lys
   20 25 30
   Lys Val Glu Leu Phe Pro Asn Gly Gln Ser Val Gly Glu Lys Ile Phe
   35 40 45
   Lys Thr Ala Gly Phe Val Lys Pro Phe Thr Glu Ala Gln Leu Leu Cys
   50 55 60
   Thr Gln Ala Gly Gly Gln Leu Ala Ser Pro Arg Ser Ala Ala Glu Asn
   65 70 75 80
   Ala Ala Leu Gln Gln Leu Val Val Ala Lys Asn Glu Ala Ala Phe Leu
   85 90 95
   Ser Met Thr Asp Ser Lys Thr Glu Gly Lys Phe Thr Tyr Pro Thr Gly
   100 105 110
   Glu Ser Leu Val Tyr Ser Asn Trp Ala Pro Gly Glu Pro Asn Asp Asp
   115 120 125
   Gly Gly Ser Glu Asp Cys Val Glu Ile Phe Thr Asn Gly Lys Trp Asn
   130 135 140
   Asp Arg Ala Cys Gly Glu Lys Arg Leu Val Val Cys Glu Phe
   145 150 155
<210> 88
   <211> 2403
   <212> PRT
   <213> Homo sapiens
<400> 88
   Met Gly Ile Ser Thr Val Ile Leu Glu Met Cys Leu Leu Trp Gly Gln
   1 5 10 15
   Val Leu Ser Thr Gly Gly Trp Ile Pro Arg Thr Thr Asp Tyr Ala Ser
   20 25 30
   Leu Ile Pro Ser Glu Val Pro Leu Asp Thr Thr Val Ala Glu Gly Ser
   35 40 45
   Pro Phe Pro Ser Glu Leu Thr Leu Glu Ser Thr Ala Ala Glu Gly Ser
   50 55 60
   Pro Ile Ser Leu Glu Ser Thr Leu Glu Ser Thr Val Ala Glu Gly Ser
   65 70 75 80
   Leu Ile Pro Ser Glu Ser Thr Leu Glu Ser Thr Val Ala Glu Gly Ser
   85 90 95
   Asp Ser Gly Leu Ala Leu Arg Leu Val Asn Gly Asp Gly Arg Cys Gln
   100 105 110
   Gly Arg Val Glu Ile Leu Tyr Arg Gly Ser Trp Gly Thr Val Cys Asp
   115 120 125
   Asp Ser Trp Asp Thr Asn Asp Ala Asn Val Val Cys Arg Gln Leu Gly
   130 135 140
   Cys Gly Trp Ala Met Ser Ala Pro Gly Asn Ala Trp Phe Gly Gln Gly
   145 150 155 160
   Ser Gly Pro Ile Ala Leu Asp Asp Val Arg Cys Ser Gly His Glu Ser
   165 170 175
   Tyr Leu Trp Ser Cys Pro His Asn Gly Trp Leu Ser His Asn Cys Gly
   180 185 190
   His Gly Glu Asp Ala Gly Val Ile Cys Ser Ala Ala Gln Pro Gln Ser
   195 200 205
   Thr Leu Arg Pro Glu Ser Trp Pro Val Arg Ile Ser Pro Pro Val Pro
   210 215 220
   Thr Glu Gly Ser Glu Ser Ser Leu Ala Leu Arg Leu Val Asn Gly Gly
   225 230 235 240
   Asp Arg Cys Arg Gly Arg Val Glu Val Leu Tyr Arg Gly Ser Trp Gly
   245 250 255
   Thr Val Cys Asp Asp Tyr Trp Asp Thr Asn Asp Ala Asn Val Val Cys
   260 265 270
   Arg Gln Leu Gly Cys Gly Trp Ala Met Ser Ala Pro Gly Asn Ala Gln
   275 280 285
   Phe Gly Gln Gly Ser Gly Pro Ile Val Leu Asp Asp Val Arg Cys Ser
   290 295 300
   Gly His Glu Ser Tyr Leu Trp Ser Cys Pro His Asn Gly Trp Leu Thr
   305 310 315 320
   His Asn Cys Gly His Ser Glu Asp Ala Gly Val Ile Cys Ser Ala Pro
   325 330 335
   Leu Ser Arg Pro Thr Pro Ser Pro Asp Thr Trp Pro Thr Ser His Ala
   340 345 350
   Ser Thr Ala Gly Pro Glu Ser Ser Leu Ala Leu Arg Leu Val Asn Gly
   355 360 365
   Gly Asp Arg Cys Gln Gly Arg Val Glu Val Leu Tyr Arg Gly Ser Trp
   370 375 380
   Gly Thr Val Cys Asp Asp Ser Trp Asp Thr Ser Asp Ala Asn Val Val
   385 390 395 400
   Cys Arg Gln Leu Gly Cys Gly Trp Ala Thr Ser Ala Pro Gly Asn Ala
   405 410 415
   Arg Phe Gly Gln Gly Ser Gly Pro Ile Val Leu Asp Asp Val Arg Cys
   420 425 430
   Ser Gly Tyr Glu Ser Tyr Leu Trp Ser Cys Pro His Asn Gly Trp Leu
   435 440 445
   Ser His Asn Cys Gln His Ser Glu Asp Ala Gly Val Ile Cys Ser Asp
   450 455 460
   Thr Leu Pro Thr Ile Thr Leu Pro Ala Ser Thr Val Gly Ser Glu Ser
   465 470 475 480
   Ser Leu Ala Leu Arg Leu Val Asn Gly Gly Asp Arg Cys Gln Gly Arg
   485 490 495
   Val Glu Val Leu Tyr Arg Gly Ser Trp Gly Thr Val Cys Asp Asp Ser
   500 505 510
   Trp Asp Thr Asn Asp Ala Asn Val Val Cys Arg Gln Leu Gly Cys Gly
   515 520 525
   Trp Ala Met Leu Ala Pro Gly Asn Ala Arg Phe Gly Gln Gly Ser Gly
   530 535 540
   Pro Ile Val Leu Asp Asp Val Arg Cys Ser Gly Asn Glu Ser Tyr Leu
   545 550 555 560
   Trp Ser Cys Pro His Asn Gly Trp Leu Ser His Asn Cys Gly His Ser
   565 570 575
   Glu Asp Ala Gly Val Ile Cys Ser Gly Pro Glu Ser Ser Leu Ala Leu
   580 585 590
   Gly Leu Val Asn Gly Gly Asp Arg Cys Gln Gly Arg Val Glu Val Leu
   595 600 605
   Tyr Arg Gly Ser Trp Gly Thr Val Cys Asp Asp Ser Trp Asp Thr Asn
   610 615 620
   Asp Ala Asn Val Val Cys Arg Gln Leu Gly Cys Gly Trp Ala Thr Ser
   625 630 635 640
   Ala Pro Gly Asn Ala Arg Phe Gly Gln Gly Ser Gly Pro Ile Val Leu
   645 650 655
   Asp Asp Val Arg Cys Ser Gly His Glu Ser Tyr Leu Trp Ser Cys Pro
   660 665 670
   Asn Asn Gly Trp Leu Ser His Asn Cys Gly His His Glu Asp Ala Gly
   675 680 685
   Val Ile Cys Ser Ala Ala Gln Ser Arg Ser Thr Pro Arg Pro Asp Thr
   690 695 700
   Leu Ser Thr Ile Thr Leu Pro Pro Ser Thr Val Gly Ser Glu Ser Ser
   705 710 715 720
   Leu Thr Leu Arg Leu Val Asn Gly Ser Asp Arg Cys Gln Gly Arg Val
   725 730 735
   Glu Val Leu Tyr Arg Gly Ser Trp Gly Thr Val Cys Asp Asp Ser Trp
   740 745 750
   Asp Thr Asn Asp Ala Asn Val Val Cys Arg Gln Leu Gly Cys Gly Trp
   755 760 765
   Ala Thr Ser Ala Pro Gly Asn Ala Arg Phe Gly Gln Gly Ser Gly Pro
   770 775 780
   Ile Val Leu Asp Asp Val Arg Cys Ser Gly His Glu Ser Tyr Leu Trp
   785 790 795 800
   Ser Cys Pro His Asn Gly Trp Leu Ser His Asn Cys Gly His His Glu
   805 810 815
   Asp Ala Gly Val Ile Cys Ser Val Ser Gln Ser Arg Pro Thr Pro Ser
   820 825 830
   Pro Asp Thr Trp Pro Thr Ser His Ala Ser Thr Ala Gly Ser Glu Ser
   835 840 845
   Ser Leu Ala Leu Arg Leu Val Asn Gly Gly Asp Arg Cys Gln Gly Arg
   850 855 860
   Val Glu Val Leu Tyr Arg Gly Ser Trp Gly Thr Val Cys Asp Asp Ser
   865 870 875 880
   Trp Asp Thr Ser Asp Ala Asn Val Val Cys Arg Arg Leu Gly Cys Gly
   885 890 895
   Trp Ala Thr Ser Ala Pro Gly Asn Ala Arg Phe Gly Gln Gly Ser Gly
   900 905 910
   Pro Ile Val Leu Asp Asp Val Arg Cys Ser Gly Tyr Glu Ser Tyr Leu
   915 920 925
   Trp Ser Cys Pro His Asn Gly Trp Leu Ser His Asn Cys Gln His Ser
   930 935 940
   Glu Asp Ala Gly Val Ile Cys Ser Ala Ala His Ser Trp Ser Thr Pro
   945 950 955 960
   Ser Pro Asp Thr Leu Pro Thr Ile Thr Leu Pro Ala Ser Thr Val Gly
   965 970 975
   Ser Glu Ser Ser Leu Ala Leu Arg Leu Val Asn Gly Gly Asp Arg Cys
   980 985 990
   Gln Gly Arg Val Glu Val Leu Tyr Gln Gly Ser Trp Gly Thr Val Cys
   995 1000 1005
   Asp Asp Ser Trp Asp Thr Asn Asp Ala Asn Val Val Cys Arg Gln
   1010 1015 1020
   Leu Gly Cys Gly Trp Ala Met Ser Ala Pro Gly Asn Ala Arg Phe
   1025 1030 1035
   Gly Gln Gly Ser Gly Pro Ile Val Leu Asp Asp Val Arg Cys Ser
   1040 1045 1050
   Gly His Glu Ser Tyr Leu Trp Ser Cys Pro His Asn Gly Trp Leu
   1055 1060 1065
   Ser His Asn Cys Gly His Ser Glu Asp Ala Gly Val Ile Cys Ser
   1070 1075 1080
   Ala Ser Gln Ser Arg Pro Thr Pro Ser Pro Asp Thr Trp Pro Thr
   1085 1090 1095
   Ser His Ala Ser Thr Ala Gly Ser Glu Ser Ser Leu Ala Leu Arg
   1100 1105 1110
   Leu Val Asn Gly Gly Asp Arg Cys Gln Gly Arg Val Glu Val Leu
   1115 1120 1125
   Tyr Arg Gly Ser Trp Gly Thr Val Cys Asp Asp Tyr Trp Asp Thr
   1130 1135 1140
   Asn Asp Ala Asn Val Val Cys Arg Gln Leu Gly Cys Gly Trp Ala
   1145 1150 1155
   Met Ser Ala Pro Gly Asn Ala Arg Phe Gly Gln Gly Ser Gly Pro
   1160 1165 1170
   Ile Val Leu Asp Asp Val Arg Cys Ser Gly His Glu Ser Tyr Leu
   1175 1180 1185
   Trp Ser Cys Pro His Asp Gly Trp Leu Ser His Asn Cys Gly His
   1190 1195 1200
   His Glu Asp Ala Gly Val Ile Cys Ser Ala Ser Gln Ser Gln Pro
   1205 1210 1215
   Thr Pro Ser Pro Asp Thr Trp Pro Thr Ser His Ala Ser Thr Ala
   1220 1225 1230
   Gly Ser Glu Ser Ser Leu Ala Leu Arg Leu Val Asn Gly Gly Asp
   1235 1240 1245
   Arg Cys Gln Gly Arg Val Glu Val Leu Tyr Arg Gly Pro Trp Gly
   1250 1255 1260
   Thr Val Cys Asp Asp Tyr Trp Asp Thr Asn Asp Ala Asn Val Val
   1265 1270 1275
   Cys Arg Gln Leu Gly Cys Gly Trp Ala Thr Ser Ala Pro Gly Asn
   1280 1285 1290
   Ala Arg Phe Gly Gln Gly Ser Gly Pro Ile Val Leu Asp Asp Val
   1295 1300 1305
   Arg Cys Ser Gly His Glu Ser Tyr Leu Trp Ser Cys Pro His Asn
   1310 1315 1320
   Gly Trp Leu Ser His Asn Cys Gly His His Glu Asp Ala Gly Val
   1325 1330 1335
   Ile Cys Ser Ala Ser Gln Ser Gln Pro Thr Pro Ser Pro Asp Thr
   1340 1345 1350
   Trp Pro Thr Ser His Ala Ser Thr Ala Gly Ser Glu Ser Ser Leu
   1355 1360 1365
   Ala Leu Arg Leu Val Asn Gly Gly Asp Arg Cys Gln Gly Arg Val
   1370 1375 1380
   Glu Val Leu Tyr Arg Gly Ser Trp Gly Thr Val Cys Asp Asp Tyr
   1385 1390 1395
   Trp Asp Thr Asn Asp Ala Asn Val Val Cys Arg Gln Leu Gly Cys
   1400 1405 1410
   Gly Trp Ala Thr Ser Ala Pro Gly Ser Ala Arg Phe Gly Gln Gly
   1415 1420 1425
   Ser Gly Pro Ile Ala Leu Asp Asp Val Arg Cys Ser Gly His Glu
   1430 1435 1440
   Ser Tyr Leu Trp Ser Cys Pro His Asn Gly Trp Leu Ser His Asn
   1445 1450 1455
   Cys Gly His His Glu Asp Ala Gly Val Ile Cys Ser Ala Ser Gln
   1460 1465 1470
   Ser Gln Pro Thr Pro Ser Pro Asp Thr Trp Pro Thr Ser Arg Ala
   1475 1480 1485
   Ser Thr Ala Gly Ser Glu Ser Thr Leu Ala Leu Arg Leu Val Asn
   1490 1495 1500
   Gly Gly Asp Arg Cys Arg Gly Arg Val Glu Val Leu Tyr Gln Gly
   1505 1510 1515
   Ser Trp Gly Thr Val Cys Asp Asp Tyr Trp Asp Thr Asn Asp Ala
   1520 1525 1530
   Asn Val Val Cys Arg Gln Leu Gly Cys Gly Trp Ala Met Ser Ala
   1535 1540 1545
   Pro Gly Asn Ala Gln Phe Gly Gln Gly Ser Gly Pro Ile Val Leu
   1550 1555 1560
   Asp Asp Val Arg Cys Ser Gly His Glu Ser Tyr Leu Trp Ser Cys
   1565 1570 1575
   Pro His Asn Gly Trp Leu Ser His Asn Cys Gly His His Glu Asp
   1580 1585 1590
   Ala Gly Val Ile Cys Ser Ala Ala Gln Ser Gln Ser Thr Pro Arg
   1595 1600 1605
   Pro Asp Thr Trp Leu Thr Thr Asn Leu Pro Ala Leu Thr Val Gly
   1610 1615 1620
   Ser Glu Ser Ser Leu Ala Leu Arg Leu Val Asn Gly Gly Asp Arg
   1625 1630 1635
   Cys Arg Gly Arg Val Glu Val Leu Tyr Arg Gly Ser Trp Gly Thr
   1640 1645 1650
   Val Cys Asp Asp Ser Trp Asp Thr Asn Asp Ala Asn Val Val Cys
   1655 1660 1665
   Arg Gln Leu Gly Cys Gly Trp Ala Met Ser Ala Pro Gly Asn Ala
   1670 1675 1680
   Arg Phe Gly Gln Gly Ser Gly Pro Ile Val Leu Asp Asp Val Arg
   1685 1690 1695
   Cys Ser Gly Asn Glu Ser Tyr Leu Trp Ser Cys Pro His Lys Gly
   1700 1705 1710
   Trp Leu Thr His Asn Cys Gly His His Glu Asp Ala Gly Val Ile
   1715 1720 1725
   Cys Ser Ala Thr Gln Ile Asn Ser Thr Thr Thr Asp Trp Trp His
   1730 1735 1740
   Pro Thr Thr Thr Thr Thr Ala Arg Pro Ser Ser Asn Cys Gly Gly
   1745 1750 1755
   Phe Leu Phe Tyr Ala Ser Gly Thr Phe Ser Ser Pro Ser Tyr Pro
   1760 1765 1770
   Ala Tyr Tyr Pro Asn Asn Ala Lys Cys Val Trp Glu Ile Glu Val
   1775 1780 1785
   Asn Ser Gly Tyr Arg Ile Asn Leu Gly Phe Ser Asn Leu Lys Leu
   1790 1795 1800
   Glu Ala His His Asn Cys Ser Phe Asp Tyr Val Glu Ile Phe Asp
   1805 1810 1815
   Gly Ser Leu Asn Ser Ser Leu Leu Leu Gly Lys Ile Cys Asn Asp
   1820 1825 1830
   Thr Arg Gln Ile Phe Thr Ser Ser Tyr Asn Arg Met Thr Ile His
   1835 1840 1845
   Phe Arg Ser Asp Ile Ser Phe Gln Asn Thr Gly Phe Leu Ala Trp
   1850 1855 1860
   Tyr Asn Ser Phe Pro Ser Asp Ala Thr Leu Arg Leu Val Asn Leu
   1865 1870 1875
   Asn Ser Ser Tyr Gly Leu Cys Ala Gly Arg Val Glu Ile Tyr His
   1880 1885 1890
   Gly Gly Thr Trp Gly Thr Val Cys Asp Asp Ser Trp Thr Ile Gln
   1895 1900 1905
   Glu Ala Glu Val Val Cys Arg Gln Leu Gly Cys Gly Arg Ala Val
   1910 1915 1920
   Ser Ala Leu Gly Asn Ala Tyr Phe Gly Ser Gly Ser Gly Pro Ile
   1925 1930 1935
   Thr Leu Asp Asp Val Glu Cys Ser Gly Thr Glu Ser Thr Leu Trp
   1940 1945 1950
   Gln Cys Arg Asn Arg Gly Trp Phe Ser His Asn Cys Asn His Arg
   1955 1960 1965
   Glu Asp Ala Gly Val Ile Cys Ser Gly Asn His Leu Ser Thr Pro
   1970 1975 1980
   Ala Pro Phe Leu Asn Ile Thr Arg Pro Asn Thr Asp Tyr Ser Cys
   1985 1990 1995
   Gly Gly Phe Leu Ser Gln Pro Ser Gly Asp Phe Ser Ser Pro Phe
   2000 2005 2010
   Tyr Pro Gly Asn Tyr Pro Asn Asn Ala Lys Cys Val Trp Asp Ile
   2015 2020 2025
   Glu Val Gln Asn Asn Tyr Arg Val Thr Val Ile Phe Arg Asp Val
   2030 2035 2040
   Gln Leu Glu Gly Gly Cys Asn Tyr Asp Tyr Ile Glu Val Phe Asp
   2045 2050 2055
   Gly Pro Tyr Arg Ser Ser Pro Leu Ile Ala Arg Val Cys Asp Gly
   2060 2065 2070
   Ala Arg Gly Ser Phe Thr Ser Ser Ser Asn Phe Met Ser Ile Arg
   2075 2080 2085
   Phe Ile Ser Asp His Ser Ile Thr Arg Arg Gly Phe Arg Ala Glu
   2090 2095 2100
   Tyr Tyr Ser Ser Pro Ser Asn Asp Ser Thr Asn Leu Leu Cys Leu
   2105 2110 2115
   Pro Asn His Met Gln Ala Ser Val Ser Arg Ser Tyr Leu Gln Ser
   2120 2125 2130
   Leu Gly Phe Ser Ala Ser Asp Leu Val Ile Ser Thr Trp Asn Gly
   2135 2140 2145
   Tyr Tyr Glu Cys Arg Pro Gln Ile Thr Pro Asn Leu Val Ile Phe
   2150 2155 2160
   Thr Ile Pro Tyr Ser Gly Cys Gly Thr Phe Lys Gln Ala Asp Asn
   2165 2170 2175
   Asp Thr Ile Asp Tyr Ser Asn Phe Leu Thr Ala Ala Val Ser Gly
   2180 2185 2190
   Gly Ile Ile Lys Arg Arg Thr Asp Leu Arg Ile His Val Ser Cys
   2195 2200 2205
   Arg Met Leu Gln Asn Thr Trp Val Asp Thr Met Tyr Ile Ala Asn
   2210 2215 2220
   Asp Thr Ile His Val Ala Asn Asn Thr Ile Gln Val Glu Glu Val
   2225 2230 2235
   Gln Tyr Gly Asn Phe Asp Val Asn Ile Ser Phe Tyr Thr Ser Ser
   2240 2245 2250
   Ser Phe Leu Tyr Pro Val Thr Ser Arg Pro Tyr Tyr Val Asp Leu
   2255 2260 2265
   Asn Gln Asp Leu Tyr Val Gln Ala Glu Ile Leu His Ser Asp Ala
   2270 2275 2280
   Val Leu Thr Leu Phe Val Asp Thr Cys Val Ala Ser Pro Tyr Ser
   2285 2290 2295
   Asn Asp Phe Thr Ser Leu Thr Tyr Asp Leu Ile Arg Ser Gly Cys
   2300 2305 2310
   Val Arg Asp Asp Thr Tyr Gly Pro Tyr Ser Ser Pro Ser Leu Arg
   2315 2320 2325
   Ile Ala Arg Phe Arg Phe Arg Ala Phe His Phe Leu Asn Arg Phe
   2330 2335 2340
   Pro Ser Val Tyr Leu Arg Cys Lys Met Val Val Cys Arg Ala Tyr
   2345 2350 2355
   Asp Pro Ser Ser Arg Cys Tyr Arg Gly Cys Val Leu Arg Ser Lys
   2360 2365 2370
   Arg Asp Val Gly Ser Tyr Gln Glu Lys Val Asp Val Val Leu Gly
   2375 2380 2385
   Pro Ile Gln Leu Gln Thr Pro Pro Arg Arg Glu Glu Glu Pro Arg
   2390 2395 2400
<210> 89
   <211> 2413
   <212> PRT
   <213> Homo sapiens
<400> 89
   Met Gly Ile Ser Thr Val Ile Leu Glu Met Cys Leu Leu Trp Gly Gln
   1 5 10 15
   Val Leu Ser Thr Gly Gly Trp Ile Pro Arg Thr Thr Asp Tyr Ala Ser
   20 25 30
   Leu Ile Pro Ser Glu Val Pro Leu Asp Gln Thr Val Ala Glu Gly Ser
   35 40 45
   Pro Phe Pro Ser Glu Ser Thr Leu Glu Ser Thr Ala Ala Glu Gly Ser
   50 55 60
   Pro Ile Ser Leu Glu Ser Thr Leu Glu Ser Thr Val Ala Glu Gly Ser
   65 70 75 80
   Leu Ile Pro Ser Glu Ser Thr Leu Glu Ser Thr Val Ala Glu Gly Ser
   85 90 95
   Asp Ser Gly Leu Ala Leu Arg Leu Val Asn Gly Asp Gly Arg Cys Gln
   100 105 110
   Gly Arg Val Glu Ile Leu Tyr Arg Gly Ser Trp Gly Thr Val Cys Asp
   115 120 125
   Asp Ser Trp Asp Thr Asn Asp Ala Asn Val Val Cys Arg Gln Leu Gly
   130 135 140
   Cys Gly Trp Ala Met Ser Ala Pro Gly Asn Ala Trp Phe Gly Gln Gly
   145 150 155 160
   Ser Gly Pro Ile Ala Leu Asp Asp Val Arg Cys Ser Gly His Glu Ser
   165 170 175
   Tyr Leu Trp Ser Cys Pro His Asn Gly Trp Leu Ser His Asn Cys Gly
   180 185 190
   His Gly Glu Asp Ala Gly Val Ile Cys Ser Ala Ala Gln Pro Gln Ser
   195 200 205
   Thr Leu Arg Pro Glu Ser Trp Pro Val Arg Ile Ser Pro Pro Val Pro
   210 215 220
   Thr Glu Gly Ser Glu Ser Ser Leu Ala Leu Arg Leu Val Asn Gly Gly
   225 230 235 240
   Asp Arg Cys Arg Gly Arg Val Glu Val Leu Tyr Arg Gly Ser Trp Gly
   245 250 255
   Thr Val Cys Asp Asp Tyr Trp Asp Thr Asn Asp Ala Asn Val Val Cys
   260 265 270
   Arg Gln Leu Gly Cys Gly Trp Ala Met Ser Ala Pro Gly Asn Ala Gln
   275 280 285
   Phe Gly Gln Gly Ser Gly Pro Ile Val Leu Asp Asp Val Arg Cys Ser
   290 295 300
   Gly His Glu Ser Tyr Leu Trp Ser Cys Pro His Asn Gly Trp Leu Thr
   305 310 315 320
   His Asn Cys Gly His Ser Glu Asp Ala Gly Val Ile Cys Ser Ala Pro
   325 330 335
   Gln Ser Arg Pro Thr Pro Ser Pro Asp Thr Trp Pro Thr Ser His Ala
   340 345 350
   Ser Thr Ala Gly Pro Glu Ser Ser Leu Ala Leu Arg Leu Val Asn Gly
   355 360 365
   Gly Asp Arg Cys Gln Gly Arg Val Glu Val Leu Tyr Arg Gly Ser Trp
   370 375 380
   Gly Thr Val Cys Asp Asp Ser Trp Asp Thr Ser Asp Ala Asn Val Val
   385 390 395 400
   Cys Arg Gln Leu Gly Cys Gly Trp Ala Thr Ser Ala Pro Gly Asn Ala
   405 410 415
   Arg Phe Gly Gln Gly Ser Gly Pro Ile Val Leu Asp Asp Val Arg Cys
   420 425 430
   Ser Gly Tyr Glu Ser Tyr Leu Trp Ser Cys Pro His Asn Gly Trp Leu
   435 440 445
   Ser His Asn Cys Gln His Ser Glu Asp Ala Gly Val Ile Cys Ser Ala
   450 455 460
   Ala His Ser Trp Ser Thr Pro Ser Pro Asp Thr Leu Pro Thr Ile Thr
   465 470 475 480
   Leu Pro Ala Ser Thr Val Gly Ser Glu Ser Ser Leu Ala Leu Arg Leu
   485 490 495
   Val Asn Gly Gly Asp Arg Cys Gln Gly Arg Val Glu Val Leu Tyr Arg
   500 505 510
   Gly Ser Trp Gly Thr Val Cys Asp Asp Ser Trp Asp Thr Asn Asp Ala
   515 520 525
   Asn Val Val Cys Arg Gln Leu Gly Cys Gly Trp Ala Met Leu Ala Pro
   530 535 540
   Gly Asn Ala Arg Phe Gly Gln Gly Ser Gly Pro Ile Val Leu Asp Asp
   545 550 555 560
   Val Arg Cys Ser Gly Asn Glu Ser Tyr Leu Trp Ser Cys Pro His Asn
   565 570 575
   Gly Trp Leu Ser His Asn Cys Gly His Ser Glu Asp Ala Gly Val Ile
   580 585 590
   Cys Ser Gly Pro Glu Ser Ser Leu Ala Leu Arg Leu Val Asn Gly Gly
   595 600 605
   Asp Arg Cys Gln Gly Arg Val Glu Val Leu Tyr Arg Gly Ser Trp Gly
   610 615 620
   Thr Val Cys Asp Asp Ser Trp Asp Thr Asn Asp Ala Asn Val Val Cys
   625 630 635 640
   Arg Gln Leu Gly Cys Gly Trp Ala Met Ser Ala Pro Gly Asn Ala Arg
   645 650 655
   Phe Gly Gln Gly Ser Gly Pro Ile Val Leu Asp Asp Val Arg Cys Ser
   660 665 670
   Gly His Glu Ser Tyr Leu Trp Ser Cys Pro Asn Asn Gly Trp Leu Ser
   675 680 685
   His Asn Cys Gly His His Glu Asp Ala Gly Val Ile Cys Ser Ala Ala
   690 695 700
   Gln Ser Arg Ser Thr Pro Arg Pro Asp Thr Leu Ser Thr Ile Thr Leu
   705 710 715 720
   Pro Pro Ser Thr Val Gly Ser Glu Ser Ser Leu Thr Leu Arg Leu Val
   725 730 735
   Asn Gly Ser Asp Arg Cys Gln Gly Arg Val Glu Val Leu Tyr Arg Gly
   740 745 750
   Ser Trp Gly Thr Val Cys Asp Asp Ser Trp Asp Thr Asn Asp Ala Asn
   755 760 765
   Val Val Cys Arg Gln Leu Gly Cys Gly Trp Ala Met Ser Ala Pro Gly
   770 775 780
   Asn Ala Arg Phe Gly Gln Gly Ser Gly Pro Ile Val Leu Asp Asp Val
   785 790 795 800
   Arg Cys Ser Gly His Glu Ser Tyr Leu Trp Ser Cys Pro His Asn Gly
   805 810 815
   Trp Leu Ser His Asn Cys Gly His His Glu Asp Ala Gly Val Ile Cys
   820 825 830
   Ser Val Ser Gln Ser Arg Pro Thr Pro Ser Pro Asp Thr Trp Pro Thr
   835 840 845
   Ser His Ala Ser Thr Ala Gly Ser Glu Ser Ser Leu Ala Leu Arg Leu
   850 855 860
   Val Asn Gly Gly Asp Arg Cys Gln Gly Arg Val Glu Val Leu Tyr Arg
   865                 870                 875                 880
   Gly Ser Trp Gly Thr Val Cys Asp Asp Ser Trp Asp Thr Ser Asp Ala
                   885                 890                 895
   Asn Val Val Cys Arg Gln Leu Gly Cys Gly Trp Ala Thr Ser Ala Pro
               900                 905                 910
   Gly Asn Ala Arg Phe Gly Gln Gly Ser Gly Pro Ile Val Leu Asp Asp
           915                 920                 925
   Val Arg Cys Ser Gly Tyr Glu Ser Tyr Leu Trp Ser Cys Pro His Asn
       930                 935                 940
   Gly Trp Leu Ser His Asn Cys Gln His Ser Glu Asp Ala Gly Val Ile
   945                 950                 955                 960
   Cys Ser Ala Ala His Ser Trp Ser Thr Pro Ser Pro Asp Thr Leu Pro
                   965                 970                 975
   Thr Ile Thr Leu Pro Ala Ser Thr Val Gly Ser Glu Ser Ser Leu Ala
               980                 985                 990
   Leu Arg Leu Val Asn Gly Gly Asp  Arg Cys Gln Gly Arg  Val Glu Val
           995                 1000                 1005
   Leu Tyr  Gln Gly Ser Trp Gly  Thr Val Cys Asp Asp  Ser Trp Asp
       1010                 1015                 1020
   Thr Asn  Asp Ala Asn Val Val  Cys Arg Gln Pro Gly  Cys Gly Trp
       1025                 1030                 1035
   Ala Met  Ser Ala Pro Gly Asn  Ala Arg Phe Gly Gln  Gly Ser Gly
       1040                 1045                 1050
   Pro Ile  Val Leu Asp Asp Val  Arg Cys Ser Gly His  Glu Ser Tyr
       1055                 1060                 1065
   Pro Trp  Ser Cys Pro His Asn  Gly Trp Leu Ser His  Asn Cys Gly
       1070                 1075                 1080
   His Ser  Glu Asp Ala Gly Val  Ile Cys Ser Ala Ser  Gln Ser Arg
       1085                 1090                 1095
   Pro Thr  Pro Ser Pro Asp Thr  Trp Pro Thr Ser His  Ala Ser Thr
   1100 1105 1110
   Ala Gly Ser Glu Ser Ser Leu Ala Leu Arg Leu Val Asn Gly Gly
   1115 1120 1125
   Asp Arg Cys Gln Gly Arg Val Glu Val Leu Tyr Arg Gly Ser Trp
   1130 1135 1140
   Gly Thr Val Cys Asp Asp Tyr Trp Asp Thr Asn Asp Ala Asn Val
   1145 1150 1155
   Val Cys Arg Gln Leu Gly Cys Gly Trp Ala Met Ser Ala Pro Gly
   1160 1165 1170
   Asn Ala Arg Phe Gly Gln Gly Ser Gly Pro Ile Val Leu Asp Asp
   1175 1180 1185
   Val Arg Cys Ser Gly His Glu Ser Tyr Leu Trp Ser Cys Pro His
   1190 1195 1200
   Asn Gly Trp Leu Ser His Asn Cys Gly His His Glu Asp Ala Gly
   1205 1210 1215
   Val Ile Cys Ser Ala Ser Gln Ser Gln Pro Thr Pro Ser Pro Asp
   1220 1225 1230
   Thr Trp Pro Thr Ser His Ala Ser Thr Ala Gly Ser Glu Ser Ser
   1235 1240 1245
   Leu Ala Leu Arg Leu Val Asn Gly Gly Asp Arg Cys Gln Gly Arg
   1250 1255 1260
   Val Glu Val Leu Tyr Arg Gly Ser Trp Gly Thr Val Cys Asp Asp
   1265 1270 1275
   Tyr Trp Asp Thr Asn Asp Ala Asn Val Val Cys Arg Gln Leu Gly
   1280 1285 1290
   Cys Gly Trp Ala Thr Ser Ala Pro Gly Asn Ala Arg Phe Gly Gln
   1295 1300 1305
   Gly Ser Gly Pro Ile Val Leu Asp Asp Val Arg Cys Ser Gly His
   1310 1315 1320
   Glu Ser Tyr Leu Trp Ser Cys Pro His Asn Gly Trp Leu Ser His
   1325 1330 1335
   Asn Cys Gly His His Glu Asp Ala Gly Val Ile Cys Ser Ala Ser
   1340 1345 1350
   Gln Ser Gln Pro Thr Pro Ser Pro Asp Thr Trp Pro Thr Ser His
   1355 1360 1365
   Ala Ser Thr Ala Gly Ser Glu Ser Ser Leu Ala Leu Arg Leu Val
   1370 1375 1380
   Asn Gly Gly Asp Arg Cys Gln Gly Arg Val Glu Val Leu Tyr Arg
   1385 1390 1395
   Gly Ser Trp Gly Thr Val Cys Asp Asp Tyr Trp Asp Thr Asn Asp
   1400 1405 1410
   Ala Asn Val Val Cys Arg Gln Leu Gly Cys Gly Trp Ala Thr Ser
   1415 1420 1425
   Ala Pro Gly Asn Ala Arg Phe Gly Gln Gly Ser Gly Pro Ile Val
   1430 1435 1440
   Leu Asp Asp Val Arg Cys Ser Gly His Glu Ser Tyr Leu Trp Ser
   1445 1450 1455
   Cys Pro His Asn Gly Trp Leu Ser His Asn Cys Gly His His Glu
   1460 1465 1470
   Asp Ala Gly Val Ile Cys Ser Ala Ser Gln Ser Gln Pro Thr Pro
   1475 1480 1485
   Ser Pro Asp Thr Trp Pro Thr Ser Arg Ala Ser Thr Ala Gly Ser
   1490 1495 1500
   Glu Ser Thr Leu Ala Leu Arg Leu Val Asn Gly Gly Asp Arg Cys
   1505 1510 1515
   Arg Gly Arg Val Glu Val Leu Tyr Gln Gly Ser Trp Gly Thr Val
   1520 1525 1530
   Cys Asp Asp Tyr Trp Asp Thr Asn Asp Ala Asn Val Val Cys Arg
   1535 1540 1545
   Gln Leu Gly Cys Gly Trp Ala Met Ser Ala Pro Gly Asn Ala Gln
   1550 1555 1560
   Phe Gly Gln Gly Ser Gly Pro Ile Val Leu Asp Asp Val Arg Cys
   1565 1570 1575
   Ser Gly His Glu Ser Tyr Leu Trp Ser Cys Pro His Asn Gly Trp
   1580 1585 1590
   Leu Ser His Asn Cys Gly His His Glu Asp Ala Gly Val Ile Cys
   1595 1600 1605
   Ser Ala Ala Gln Ser Gln Ser Thr Pro Arg Pro Asp Thr Trp Leu
   1610 1615 1620
   Thr Thr Asn Leu Pro Ala Leu Thr Val Gly Ser Glu Ser Ser Leu
   1625 1630 1635
   Ala Leu Arg Leu Val Asn Gly Gly Asp Arg Cys Arg Gly Arg Val
   1640 1645 1650
   Glu Val Leu Tyr Arg Gly Ser Trp Gly Thr Val Cys Asp Asp Ser
   1655 1660 1665
   Trp Asp Thr Asn Asp Ala Asn Val Val Cys Arg Gln Leu Gly Cys
   1670 1675 1680
   Gly Trp Ala Met Ser Ala Pro Gly Asn Ala Arg Phe Gly Gln Gly
   1685 1690 1695
   Ser Gly Pro Ile Val Leu Asp Asp Val Arg Cys Ser Gly Asn Glu
   1700 1705 1710
   Ser Tyr Leu Trp Ser Cys Pro His Lys Gly Trp Leu Thr His Asn
   1715 1720 1725
   Cys Gly His His Glu Asp Ala Gly Val Ile Cys Ser Ala Thr Gln
   1730 1735 1740
   Ile Asn Ser Thr Thr Thr Asp Trp Trp His Pro Thr Thr Thr Thr
   1745 1750 1755
   Thr Ala Arg Pro Ser Ser Asn Cys Gly Gly Phe Leu Phe Tyr Ala
   1760 1765 1770
   Ser Gly Thr Phe Ser Ser Pro Ser Tyr Pro Ala Tyr Tyr Pro Asn
   1775 1780 1785
   Asn Ala Lys Cys Val Trp Glu Ile Glu Val Asn Ser Gly Tyr Arg
   1790 1795 1800
   Ile Asn Leu Gly Phe Ser Asn Leu Lys Leu Glu Ala His His Asn
   1805 1810 1815
   Cys Ser Phe Asp Tyr Val Glu Ile Phe Asp Gly Ser Leu Asn Ser
   1820 1825 1830
   Ser Leu Leu Leu Gly Lys Ile Cys Asn Asp Thr Arg Gln Ile Phe
   1835 1840 1845
   Thr Ser Ser Tyr Asn Arg Met Thr Ile His Phe Arg Ser Asp Ile
   1850 1855 1860
   Ser Phe Gln Asn Thr Gly Phe Leu Ala Trp Tyr Asn Ser Phe Pro
   1865 1870 1875
   Ser Asp Ala Thr Leu Arg Leu Val Asn Leu Asn Ser Ser Tyr Gly
   1880 1885 1890
   Leu Cys Ala Gly Arg Val Glu Ile Tyr His Gly Gly Thr Trp Gly
   1895 1900 1905
   Thr Val Cys Asp Asp Ser Trp Thr Ile Gln Glu Ala Glu Val Val
   1910 1915 1920
   Cys Arg Gln Leu Gly Cys Gly Arg Ala Val Ser Ala Leu Gly Asn
   1925 1930 1935
   Ala Tyr Phe Gly Ser Gly Ser Gly Pro Ile Thr Leu Asp Asp Val
   1940 1945 1950
   Glu Cys Ser Gly Thr Glu Ser Thr Leu Trp Gln Cys Arg Asn Arg
   1955 1960 1965
   Gly Trp Phe Ser His Asn Cys Asn His Arg Glu Asp Ala Gly Val
   1970 1975 1980
   Ile Cys Ser Gly Asn His Leu Ser Thr Pro Ala Pro Phe Leu Asn
   1985 1990 1995
   Ile Thr Arg Pro Asn Thr Asp Tyr Ser Cys Gly Gly Phe Leu Ser
   2000 2005 2010
   Gln Pro Ser Gly Asp Phe Ser Ser Pro Phe Tyr Pro Gly Asn Tyr
   2015 2020 2025
   Pro Asn Asn Ala Lys Cys Val Trp Asp Ile Glu Val Gln Asn Asn
   2030 2035 2040
   Tyr Arg Val Thr Val Ile Phe Arg Asp Val Gln Leu Glu Gly Gly
   2045 2050 2055
   Cys Asn Tyr Asp Tyr Ile Glu Val Phe Asp Gly Pro Tyr Arg Ser
   2060 2065 2070
   Ser Pro Leu Ile Ala Arg Val Cys Asp Gly Ala Arg Gly Ser Phe
   2075 2080 2085
   Thr Ser Ser Ser Asn Phe Met Ser Ile Arg Phe Ile Ser Asp His
   2090 2095 2100
   Ser Ile Thr Arg Arg Gly Phe Arg Ala Glu Tyr Tyr Ser Ser Pro
   2105 2110 2115
   Ser Asn Asp Ser Thr Asn Leu Leu Cys Leu Pro Asn His Met Gln
   2120 2125 2130
   Ala Ser Val Ser Arg Ser Tyr Leu Gln Ser Leu Gly Phe Ser Ala
   2135 2140 2145
   Ser Asp Leu Val Ile Ser Thr Trp Asn Gly Tyr Tyr Glu Cys Arg
   2150 2155 2160
   Pro Gln Ile Thr Pro Asn Leu Val Ile Phe Thr Ile Pro Tyr Ser
   2165 2170 2175
   Gly Cys Gly Thr Phe Lys Gln Ala Asp Asn Asp Thr Ile Asp Tyr
   2180 2185 2190
   Ser Asn Phe Leu Thr Ala Ala Val Ser Gly Gly Ile Ile Lys Arg
   2195 2200 2205
   Arg Thr Asp Leu Arg Ile His Val Ser Cys Arg Met Leu Gln Asn
   2210 2215 2220
   Thr Trp Val Asp Thr Met Tyr Ile Ala Asn Asp Thr Ile His Val
   2225 2230 2235
   Ala Asn Asn Thr Ile Gln Val Glu Glu Val Gln Tyr Gly Asn Phe
   2240 2245 2250
   Asp Val Asn Ile Ser Phe Tyr Thr Ser Ser Ser Phe Leu Tyr Pro
   2255 2260 2265
   Val Thr Ser Arg Pro Tyr Tyr Val Asp Leu Asn Gln Asp Leu Tyr
   2270 2275 2280
   Val Gln Ala Glu Ile Leu His Ser Asp Ala Val Leu Thr Leu Phe
   2285 2290 2295
   Val Asp Thr Cys Val Ala Ser Pro Tyr Ser Asn Asp Phe Thr Ser
   2300 2305 2310
   Leu Thr Tyr Asp Leu Ile Arg Ser Gly Cys Val Arg Asp Asp Thr
   2315 2320 2325
   Tyr Gly Pro Tyr Ser Ser Pro Ser Leu Arg Ile Ala Arg Phe Arg
   2330 2335 2340
   Phe Arg Ala Phe His Phe Leu Asn Arg Phe Pro Ser Val Tyr Leu
   2345 2350 2355
   Arg Cys Lys Met Val Val Cys Arg Ala Tyr Asp Pro Ser Ser Arg
   2360 2365 2370
   Cys Tyr Arg Gly Cys Val Leu Arg Ser Lys Arg Asp Val Gly Ser
   2375 2380 2385
   Tyr Gln Glu Lys Val Asp Val Val Leu Gly Pro Ile Gln Leu Gln
   2390 2395 2400
   Thr Pro Pro Arg Arg Glu Glu Glu Pro Arg
   2405 2410
<210> 90
   <211> 1785
   <212> PRT
   <213> Homo sapiens
<400> 90
   Met Gly Ile Ser Thr Val Ile Leu Glu Met Cys Leu Leu Trp Gly Gln
   1 5 10 15
   Val Leu Ser Thr Gly Gly Trp Ile Pro Arg Thr Thr Asp Tyr Ala Ser
   20 25 30
   Leu Ile Pro Ser Glu Val Pro Leu Asp Gln Thr Val Ala Glu Gly Ser
   35 40 45
   Pro Phe Pro Ser Glu Ser Thr Leu Glu Ser Thr Ala Ala Glu Gly Ser
   50 55 60
   Pro Ile Ser Leu Glu Ser Thr Leu Glu Ser Thr Val Ala Glu Gly Ser
   65 70 75 80
   Leu Ile Pro Ser Glu Ser Thr Leu Glu Ser Thr Val Ala Glu Gly Ser
   85 90 95
   Asp Ser Gly Leu Ala Leu Arg Leu Val Asn Gly Asp Gly Arg Cys Gln
   100 105 110
   Gly Arg Val Glu Ile Leu Tyr Arg Gly Ser Trp Gly Thr Val Cys Asp
   115 120 125
   Asp Ser Trp Asp Thr Asn Asp Ala Asn Val Val Cys Arg Gln Leu Gly
   130 135 140
   Cys Gly Trp Ala Met Ser Ala Pro Gly Asn Ala Trp Phe Gly Gln Gly
   145 150 155 160
   Ser Gly Pro Ile Ala Leu Asp Asp Val Arg Cys Ser Gly His Glu Ser
   165 170 175
   Tyr Leu Trp Ser Cys Pro His Asn Gly Trp Leu Ser His Asn Cys Gly
   180 185 190
   His Gly Glu Asp Ala Gly Val Ile Cys Ser Ala Ala Gln Pro Gln Ser
   195 200 205
   Thr Leu Arg Pro Glu Ser Trp Pro Val Arg Ile Ser Pro Pro Val Pro
   210 215 220
   Thr Glu Gly Ser Glu Ser Ser Leu Ala Leu Arg Leu Val Asn Gly Gly
   225 230 235 240
   Asp Arg Cys Arg Gly Arg Val Glu Val Leu Tyr Arg Gly Ser Trp Gly
   245 250 255
   Thr Val Cys Asp Asp Tyr Trp Asp Thr Asn Asp Ala Asn Val Val Cys
   260 265 270
   Arg Gln Leu Gly Cys Gly Trp Ala Met Ser Ala Pro Gly Asn Ala Gln
   275 280 285
   Phe Gly Gln Gly Ser Gly Pro Ile Val Leu Asp Asp Val Arg Cys Ser
   290 295 300
   Gly His Glu Ser Tyr Leu Trp Ser Cys Pro His Asn Gly Trp Leu Thr
   305 310 315 320
   His Asn Cys Gly His Ser Glu Asp Ala Gly Val Ile Cys Ser Ala Pro
   325 330 335
   Gln Ser Arg Pro Thr Pro Ser Pro Asp Thr Trp Pro Thr Ser His Ala
   340 345 350
   Ser Thr Ala Gly Pro Glu Ser Ser Leu Ala Leu Arg Leu Val Asn Gly
   355 360 365
   Gly Asp Arg Cys Gln Gly Arg Val Glu Val Leu Tyr Arg Gly Ser Trp
   370 375 380
   Gly Thr Val Cys Asp Asp Ser Trp Asp Thr Ser Asp Ala Asn Val Val
   385 390 395 400
   Cys Arg Gln Leu Gly Cys Gly Trp Ala Thr Ser Ala Pro Gly Asn Ala
   405 410 415
   Arg Phe Gly Gln Gly Ser Gly Pro Ile Val Leu Asp Asp Val Arg Cys
   420 425 430
   Ser Gly Tyr Glu Ser Tyr Leu Trp Ser Cys Pro His Asn Gly Trp Leu
   435 440 445
   Ser His Asn Cys Gln His Ser Glu Asp Ala Gly Val Ile Cys Ser Ala
   450 455 460
   Ala His Ser Trp Ser Thr Pro Ser Pro Asp Thr Leu Pro Thr Ile Thr
   465 470 475 480
   Leu Pro Ala Ser Thr Val Gly Ser Glu Ser Ser Leu Ala Leu Arg Leu
   485 490 495
   Val Asn Gly Gly Asp Arg Cys Gln Gly Arg Val Glu Val Leu Tyr Gln
   500 505 510
   Gly Ser Trp Gly Thr Val Cys Asp Asp Ser Trp Asp Thr Asn Asp Ala
   515 520 525
   Asn Val Val Cys Arg Gln Pro Gly Cys Gly Trp Ala Met Ser Ala Pro
   530 535 540
   Gly Asn Ala Arg Phe Gly Gln Gly Ser Gly Pro Ile Val Leu Asp Asp
   545 550 555 560
   Val Arg Cys Ser Gly His Glu Ser Tyr Pro Trp Ser Cys Pro His Asn
   565 570 575
   Gly Trp Leu Ser His Asn Cys Gly His Ser Glu Asp Ala Gly Val Ile
   580 585 590
   Cys Ser Ala Ser Gln Ser Arg Pro Thr Pro Ser Pro Asp Thr Trp Pro
   595 600 605
   Thr Ser His Ala Ser Thr Ala Gly Ser Glu Ser Ser Leu Ala Leu Arg
   610 615 620
   Leu Val Asn Gly Gly Asp Arg Cys Gln Gly Arg Val Glu Val Leu Tyr
   625 630 635 640
   Arg Gly Ser Trp Gly Thr Val Cys Asp Asp Tyr Trp Asp Thr Asn Asp
   645 650 655
   Ala Asn Val Val Cys Arg Gln Leu Gly Cys Gly Trp Ala Met Ser Ala
   660 665 670
   Pro Gly Asn Ala Arg Phe Gly Gln Gly Ser Gly Pro Ile Val Leu Asp
   675 680 685
   Asp Val Arg Cys Ser Gly His Glu Ser Tyr Leu Trp Ser Cys Pro His
   690 695 700
   Asn Gly Trp Leu Ser His Asn Cys Gly His His Glu Asp Ala Gly Val
   705 710 715 720
   Ile Cys Ser Ala Ser Gln Ser Gln Pro Thr Pro Ser Pro Asp Thr Trp
   725 730 735
   Pro Thr Ser His Ala Ser Thr Ala Gly Ser Glu Ser Ser Leu Ala Leu
   740 745 750
   Arg Leu Val Asn Gly Gly Asp Arg Cys Gln Gly Arg Val Glu Val Leu
   755 760 765
   Tyr Arg Gly Ser Trp Gly Thr Val Cys Asp Asp Tyr Trp Asp Thr Asn
   770 775 780
   Asp Ala Asn Val Val Cys Arg Gln Leu Gly Cys Gly Trp Ala Thr Ser
   785 790 795 800
   Ala Pro Gly Asn Ala Arg Phe Gly Gln Gly Ser Gly Pro Ile Val Leu
   805 810 815
   Asp Asp Val Arg Cys Ser Gly His Glu Ser Tyr Leu Trp Ser Cys Pro
   820 825 830
   His Asn Gly Trp Leu Ser His Asn Cys Gly His His Glu Asp Ala Gly
   835 840 845
   Val Ile Cys Ser Ala Ser Gln Ser Gln Pro Thr Pro Ser Pro Asp Thr
   850 855 860
   Trp Pro Thr Ser Arg Ala Ser Thr Ala Gly Ser Glu Ser Thr Leu Ala
   865 870 875 880
   Leu Arg Leu Val Asn Gly Gly Asp Arg Cys Arg Gly Arg Val Glu Val
   885 890 895
   Leu Tyr Gln Gly Ser Trp Gly Thr Val Cys Asp Asp Tyr Trp Asp Thr
   900 905 910
   Asn Asp Ala Asn Val Val Cys Arg Gln Leu Gly Cys Gly Trp Ala Met
   915 920 925
   Ser Ala Pro Gly Asn Ala Gln Phe Gly Gln Gly Ser Gly Pro Ile Val
   930 935 940
   Leu Asp Asp Val Arg Cys Ser Gly His Glu Ser Tyr Leu Trp Ser Cys
   945 950 955 960
   Pro His Asn Gly Trp Leu Ser His Asn Cys Gly His His Glu Asp Ala
   965 970 975
   Gly Val Ile Cys Ser Ala Ala Gln Ser Gln Ser Thr Pro Arg Pro Asp
   980 985 990
   Thr Trp Leu Thr Thr Asn Leu Pro Ala Leu Thr Val Gly Ser Glu Ser
   995 1000 1005
   Ser Leu Ala Leu Arg Leu Val Asn Gly Gly Asp Arg Cys Arg Gly
   1010 1015 1020
   Arg Val Glu Val Leu Tyr Arg Gly Ser Trp Gly Thr Val Cys Asp
   1025 1030 1035
   Asp Ser Trp Asp Thr Asn Asp Ala Asn Val Val Cys Arg Gln Leu
   1040 1045 1050
   Gly Cys Gly Trp Ala Met Ser Ala Pro Gly Asn Ala Arg Phe Gly
   1055 1060 1065
   Gln Gly Ser Gly Pro Ile Val Leu Asp Asp Val Arg Cys Ser Gly
   1070 1075 1080
   Asn Glu Ser Tyr Leu Trp Ser Cys Pro His Lys Gly Trp Leu Thr
   1085 1090 1095
   His Asn Cys Gly His His Glu Asp Ala Gly Val Ile Cys Ser Ala
   1100 1105 1110
   Thr Gln Ile Asn Ser Thr Thr Thr Asp Trp Trp His Pro Thr Thr
   1115 1120 1125
   Thr Thr Thr Ala Arg Pro Ser Ser Asn Cys Gly Gly Phe Leu Phe
   1130 1135 1140
   Tyr Ala Ser Gly Thr Phe Ser Ser Pro Ser Tyr Pro Ala Tyr Tyr
   1145 1150 1155
   Pro Asn Asn Ala Lys Cys Val Trp Glu Ile Glu Val Asn Ser Gly
   1160 1165 1170
   Tyr Arg Ile Asn Leu Gly Phe Ser Asn Leu Lys Leu Glu Ala His
   1175 1180 1185
   His Asn Cys Ser Phe Asp Tyr Val Glu Ile Phe Asp Gly Ser Leu
   1190 1195 1200
   Asn Ser Ser Leu Leu Leu Gly Lys Ile Cys Asn Asp Thr Arg Gln
   1205 1210 1215
   Ile Phe Thr Ser Ser Tyr Asn Arg Met Thr Ile His Phe Arg Ser
   1220 1225 1230
   Asp Ile Ser Phe Gln Asn Thr Gly Phe Leu Ala Trp Tyr Asn Ser
   1235 1240 1245
   Phe Pro Ser Asp Ala Thr Leu Arg Leu Val Asn Leu Asn Ser Ser
   1250 1255 1260
   Tyr Gly Leu Cys Ala Gly Arg Val Glu Ile Tyr His Gly Gly Thr
   1265 1270 1275
   Trp Gly Thr Val Cys Asp Asp Ser Trp Thr Ile Gln Glu Ala Glu
   1280 1285 1290
   Val Val Cys Arg Gln Leu Gly Cys Gly Arg Ala Val Ser Ala Leu
   1295 1300 1305
   Gly Asn Ala Tyr Phe Gly Ser Gly Ser Gly Pro Ile Thr Leu Asp
   1310 1315 1320
   Asp Val Glu Cys Ser Gly Thr Glu Ser Thr Leu Trp Gln Cys Arg
   1325 1330 1335
   Asn Arg Gly Trp Phe Ser His Asn Cys Asn His Arg Glu Asp Ala
   1340 1345 1350
   Gly Val Ile Cys Ser Gly Asn His Leu Ser Thr Pro Ala Pro Phe
   1355 1360 1365
   Leu Asn Ile Thr Arg Pro Asn Thr Asp Tyr Ser Cys Gly Gly Phe
   1370 1375 1380
   Leu Ser Gln Pro Ser Gly Asp Phe Ser Ser Pro Phe Tyr Pro Gly
   1385 1390 1395
   Asn Tyr Pro Asn Asn Ala Lys Cys Val Trp Asp Ile Glu Val Gln
   1400 1405 1410
   Asn Asn Tyr Arg Val Thr Val Ile Phe Arg Asp Val Gln Leu Glu
   1415 1420 1425
   Gly Gly Cys Asn Tyr Asp Tyr Ile Glu Val Phe Asp Gly Pro Tyr
   1430 1435 1440
   Arg Ser Ser Pro Leu Ile Ala Arg Val Cys Asp Gly Ala Arg Gly
   1445 1450 1455
   Ser Phe Thr Ser Ser Ser Asn Phe Met Ser Ile Arg Phe Ile Ser
   1460 1465 1470
   Asp His Ser Ile Thr Arg Arg Gly Phe Arg Ala Glu Tyr Tyr Ser
   1475 1480 1485
   Ser Pro Ser Asn Asp Ser Thr Asn Leu Leu Cys Leu Pro Asn His
   1490 1495 1500
   Met Gln Ala Ser Val Ser Arg Ser Tyr Leu Gln Ser Leu Gly Phe
   1505 1510 1515
   Ser Ala Ser Asp Leu Val Ile Ser Thr Trp Asn Gly Tyr Tyr Glu
   1520 1525 1530
   Cys Arg Pro Gln Ile Thr Pro Asn Leu Val Ile Phe Thr Ile Pro
   1535 1540 1545
   Tyr Ser Gly Cys Gly Thr Phe Lys Gln Ala Asp Asn Asp Thr Ile
   1550 1555 1560
   Asp Tyr Ser Asn Phe Leu Thr Ala Ala Val Ser Gly Gly Ile Ile
   1565 1570 1575
   Lys Arg Arg Thr Asp Leu Arg Ile His Val Ser Cys Arg Met Leu
   1580 1585 1590
   Gln Asn Thr Trp Val Asp Thr Met Tyr Ile Ala Asn Asp Thr Ile
   1595 1600 1605
   His Val Ala Asn Asn Thr Ile Gln Val Glu Glu Val Gln Tyr Gly
   1610 1615 1620
   Asn Phe Asp Val Asn Ile Ser Phe Tyr Thr Ser Ser Ser Phe Leu
   1625 1630 1635
   Tyr Pro Val Thr Ser Arg Pro Tyr Tyr Val Asp Leu Asn Gln Asp
   1640 1645 1650
   Leu Tyr Val Gln Ala Glu Ile Leu His Ser Asp Ala Val Leu Thr
   1655 1660 1665
   Leu Phe Val Asp Thr Cys Val Ala Ser Pro Tyr Ser Asn Asp Phe
   1670 1675 1680
   Thr Ser Leu Thr Tyr Asp Leu Ile Arg Ser Gly Cys Val Arg Asp
   1685 1690 1695
   Asp Thr Tyr Gly Pro Tyr Ser Ser Pro Ser Leu Arg Ile Ala Arg
   1700 1705 1710
   Phe Arg Phe Arg Ala Phe His Phe Leu Asn Arg Phe Pro Ser Val
   1715 1720 1725
   Tyr Leu Arg Cys Lys Met Val Val Cys Arg Ala Tyr Asp Pro Ser
   1730 1735 1740
   Ser Arg Cys Tyr Arg Gly Cys Val Leu Arg Ser Lys Arg Asp Val
   1745 1750 1755
   Gly Ser Tyr Gln Glu Lys Val Asp Val Val Leu Gly Pro Ile Gln
   1760 1765 1770
   Leu Gln Thr Pro Pro Arg Arg Glu Glu Glu Pro Arg
   1775 1780 1785
<210> 91
   <211> 34
   <212> PRT
   <213> Bos taurus
<400> 91
   Leu Ile Pro Cys Cys Pro Val Asn Ile Lys Arg Leu Leu Ile Val Val
   1 5 10 15
   Val Val Val Val Leu Leu Val Val Val Ile Val Gly Ala Leu Leu Met
   20 25 30
   Gly Leu
<210> 92
   <211> 35
   <212> PRT
   <213> Canis familiaris
<400> 92
   Leu Gly Ile Pro Cys Phe Pro Ser Ser Leu Lys Arg Leu Leu Ile Ile
   1 5 10 15
   Val Val Val Ile Val Leu Val Val Val Val Ile Val Gly Ala Leu Leu
   20 25 30
   Met Gly Leu
<210> 93
   <211> 371
   <212> PRT
   <213> Bos taurus
<400> 93
   Met Leu Leu Leu Pro Leu Ser Val Leu Leu Leu Leu Thr Gln Pro Trp
   1               5                   10                  15
   Arg Ser Leu Gly Ala Glu Met Thr Thr Phe Ser Gln Lys Ile Leu Ala
               20                  25                  30
   Asn Ala Cys Thr Leu Val Met Cys Ser Pro Leu Glu Ser Gly Leu Pro
           35                  40                  45
   Gly His Asp Gly Gln Asp Gly Arg Glu Cys Pro His Gly Glu Lys Gly
       50                  55                  60
   Asp Pro Gly Ser Pro Gly Pro Ala Gly Arg Ala Gly Arg Pro Gly Trp
   65                  70                  75                  80
   Val Gly Pro Ile Gly Pro Lys Gly Asp Asn Gly Phe Val Gly Glu Pro
                   85                  90                  95
   Gly Pro Lys Gly Asp Thr Gly Pro Arg Gly Pro Pro Gly Met Pro Gly
               100                 105                 110
   Pro Ala Gly Arg Glu Gly Pro Ser Gly Lys Gln Gly Ser Met Gly Pro
           115                 120                 125
   Pro Gly Thr Pro Gly Pro Lys Gly Glu Thr Gly Pro Lys Gly Gly Val
       130                 135                 140
   Gly Ala Pro Gly Ile Gln Gly Phe Pro Gly Pro Ser Gly Leu Lys Gly
   145                 150                 155                 160
   Glu Lys Gly Ala Pro Gly Glu Thr Gly Ala Pro Gly Arg Ala Gly Val
                   165                 170                 175
   Thr Gly Pro Ser Gly Ala Ile Gly Pro Gln Gly Pro Ser Gly Ala Arg
               180                 185                 190
   Gly Pro Pro Gly Leu Lys Gly Asp Arg Gly Asp Pro Gly Glu Thr Gly
   195 200 205
   Ala Lys Gly Glu Ser Gly Leu Ala Glu Val Asn Ala Leu Lys Gln Arg
   210 215 220
   Val Thr Ile Leu Asp Gly His Leu Arg Arg Phe Gln Asn Ala Phe Ser
   225 230 235 240
   Gln Tyr Lys Lys Ala Val Leu Phe Pro Asp Gly Gln Ala Val Gly Glu
   245 250 255
   Lys Ile Phe Lys Thr Ala Gly Ala Val Lys Ser Tyr Ser Asp Ala Glu
   260 265 270
   Gln Leu Cys Arg Glu Ala Lys Gly Gln Leu Ala Ser Pro Arg Ser Ser
   275 280 285
   Ala Glu Asn Glu Ala Val Thr Gln Met Val Arg Ala Gln Glu Lys Asn
   290 295 300
   Ala Tyr Leu Ser Met Asn Asp Ile Ser Thr Glu Gly Arg Phe Thr Tyr
   305 310 315 320
   Pro Thr Gly Glu Ile Leu Val Tyr Ser Asn Trp Ala Asp Gly Glu Pro
   325 330 335
   Asn Asn Ser Asp Glu Gly Gln Pro Glu Asn Cys Val Glu Ile Phe Pro
   340 345 350
   Asp Gly Lys Trp Asn Asp Val Pro Cys Ser Lys Gln Leu Leu Val Ile
   355 360 365
   Cys Glu Phe
   370
<210> 94
   <211> 375
   <212> PRT
   <213> Homo sapiens
<400> 94
   Met Leu Leu Phe Leu Leu Ser Ala Leu Val Leu Leu Thr Gln Pro Leu
   1               5                   10                  15
   Gly Tyr Leu Glu Ala Glu Met Lys Thr Tyr Ser His Arg Thr Met Pro
               20                  25                  30
   Ser Ala Cys Thr Leu Val Met Cys Ser Ser Val Glu Ser Gly Leu Pro
           35                  40                  45
   Gly Arg Asp Gly Arg Asp Gly Arg Glu Gly Pro Arg Gly Glu Lys Gly
       50                  55                  60
   Asp Pro Gly Leu Pro Gly Ala Ala Gly Gln Ala Gly Met Pro Gly Gln
   65                  70                  75                  80
   Ala Gly Pro Val Gly Pro Lys Gly Asp Asn Gly Ser Val Gly Glu Pro
                   85                  90                  95
   Gly Pro Lys Gly Asp Thr Gly Pro Ser Gly Pro Pro Gly Pro Pro Gly
               100                 105                 110
   Val Pro Gly Pro Ala Gly Arg Glu Gly Ala Leu Gly Lys Gln Gly Asn
           115                 120                 125
   Ile Gly Pro Gln Gly Lys Pro Gly Pro Lys Gly Glu Ala Gly Pro Lys
       130                 135                 140
   Gly Glu Val Gly Ala Pro Gly Met Gln Gly Ser Ala Gly Ala Arg Gly
   145                 150                 155                 160
   Leu Ala Gly Pro Lys Gly Glu Arg Gly Val Pro Gly Glu Arg Gly Val
                   165                 170                 175
   Pro Gly Asn Thr Gly Ala Ala Gly Ser Ala Gly Ala Met Gly Pro Gln
               180                 185                 190
   Gly Ser Pro Gly Ala Arg Gly Pro Pro Gly Leu Lys Gly Asp Lys Gly
           195                 200                 205
   Ile Pro Gly Asp Lys Gly Ala Lys Gly Glu Ser Gly Leu Pro Asp Val
       210                 215                 220
   Ala Ser Leu Arg Gln Gln Val Glu Ala Leu Gln Gly Gln Val Gln His
   225                 230                 235                 240
   Leu Gln Ala Ala Phe Ser Gln Tyr Lys Lys Val Glu Leu Phe Pro Asn
                   245                 250                 255
   Gly Gln Ser Val Gly Glu Lys Ile Phe Lys Thr Ala Gly Phe Val Lys
   260 265 270
   Pro Phe Thr Glu Ala Gln Leu Leu Cys Thr Gln Ala Gly Gly Gln Leu
   275 280 285
   Ala Ser Pro Arg Ser Ala Ala Glu Asn Ala Ala Leu Gln Gln Leu Val
   290 295 300
   Val Ala Lys Asn Glu Ala Ala Phe Leu Ser Met Thr Asp Ser Lys Thr
   305 310 315 320
   Glu Gly Lys Phe Thr Tyr Pro Thr Gly Glu Ser Leu Val Tyr Ser Asn
   325 330 335
   Trp Ala Pro Gly Glu Pro Asn Asp Asp Gly Gly Ser Glu Asp Cys Val
   340 345 350
   Glu Ile Phe Thr Asn Gly Lys Trp Asn Asp Arg Ala Cys Gly Glu Lys
   355 360 365
   Arg Leu Val Val Cys Glu Phe
   370 375
<210> 95
   <211> 197
   <212> PRT
   <213> Homo sapiens
<400> 95
   Met Asp Val Gly Ser Lys Glu Val Leu Met Glu Ser Pro Pro Asp Tyr
   1 5 10 15
   Ser Ala Ala Pro Arg Gly Arg Phe Gly Ile Pro Cys Cys Pro Val His
   20 25 30
   Leu Lys Arg Leu Leu Ile Val Val Val Val Val Val Leu Ile Val Val
   35 40 45
   Val Ile Val Gly Ala Leu Leu Met Gly Leu His Met Ser Gln Lys His
   50 55 60
   Thr Glu Met Val Leu Glu Met Ser Ile Gly Ala Pro Glu Ala Gln Gln
   65 70 75 80
   Arg Leu Ala Leu Ser Glu His Leu Val Thr Thr Ala Thr Phe Ser Ile
   85 90 95
   Gly Ser Thr Gly Leu Val Val Tyr Asp Tyr Gln Gln Leu Leu Ile Ala
   100 105 110
   Tyr Lys Pro Ala Pro Gly Thr Cys Cys Tyr Ile Met Lys Ile Ala Pro
   115 120 125
   Glu Ser Ile Pro Ser Leu Glu Ala Leu Asn Arg Lys Val His Asn Phe
   130 135 140
   Gln Met Glu Cys Ser Leu Gln Ala Lys Pro Ala Val Pro Thr Ser Lys
   145 150 155 160
   Leu Gly Gln Ala Glu Gly Arg Asp Ala Gly Ser Ala Pro Ser Gly Gly
   165 170 175
   Asp Pro Ala Phe Leu Gly Met Ala Val Asn Thr Leu Cys Gly Glu Val
   180 185 190
   Pro Leu Tyr Tyr Ile
   195
<210> 96
   <211> 248
   <212> PRT
   <213> Canis familiaris
<400> 96
   Met Trp Leu Arg Cys Leu Ala Leu Ala Leu Thr Leu Leu Met Val Ser
   1 5 10 15
   Gly Ile Glu Asn Asn Thr Lys Asp Val Cys Val Gly Asn Pro Gly Ile
   20 25 30
   Pro Gly Thr Pro Gly Ser His Gly Leu Pro Gly Arg Asp Gly Arg Asp
   35 40 45
   Gly Val Lys Gly Asp Pro Gly Pro Pro Gly Pro Leu Gly Pro Pro Gly
   50 55 60
   Gly Met Pro Gly His Pro Gly Pro Asn Gly Met Thr Gly Ala Pro Gly
   65 70 75 80
   Val Ala Gly Glu Arg Gly Glu Lys Gly Glu Pro Gly Glu Arg Gly Pro
   85 90 95
   Pro Gly Leu Pro Ala Ser Leu Asp Glu Glu Leu Gln Thr Thr Leu His
   100 105 110
   Asp Leu Arg His Gln Ile Leu Gln Thr Met Gly Val Leu Ser Leu His
   115 120 125
   Glu Ser Leu Leu Val Val Gly Arg Lys Val Phe Ser Ser Gly Ala Gln
   130 135 140
   Ser Ile Asn Phe Asn Asp Ile Gln Glu Leu Cys Ala Gly Ala Gly Gly
   145 150 155 160
   Gln Ile Ala Ala Pro Met Ser Pro Glu Glu Asn Glu Ala Val Ala Ser
   165 170 175
   Ile Val Lys Lys Tyr Asn Thr Tyr Ala Tyr Leu Gly Leu Val Glu Ser
   180 185 190
   Pro Asp Ser Gly Asp Phe Gln Tyr Met Asp Gly Ala Pro Val Asn Tyr
   195 200 205
   Thr Asn Trp Tyr Pro Gly Glu Pro Arg Gly Arg Gly Lys Glu Gln Cys
   210 215 220
   Val Glu Met Tyr Thr Asp Gly Gln Trp Asn Asn Lys Asn Cys Leu Gln
   225 230 235 240
   Tyr Arg Leu Ala Ile Cys Glu Phe
   245
<210> 97
   <211> 248
   <212> PRT
   <213> Homo sapiens
<400> 97
<210> 98
   <211> 301
   <212> PRT
   <213> Bos taurus
<400> 98
   Glu Glu Met Asp Val Tyr Ser Glu Lys Thr Leu Thr Asp Pro Cys Thr
   1               5                   10                  15
   Leu Val Val Cys Ala Pro Pro Ala Asp Ser Leu Arg Gly His Asp Gly
               20                  25                  30
   Arg Asp Gly Lys Glu Gly Pro Gln Gly Glu Lys Gly Asp Pro Gly Pro
           35                  40                  45
   Pro Gly Met Pro Gly Pro Ala Gly Arg Glu Gly Pro Ser Gly Arg Gln
       50                  55                  60
   Gly Ser Met Gly Pro Pro Gly Thr Pro Gly Pro Lys Gly Glu Pro Gly
   65                  70                  75                  80
   Pro Glu Gly Gly Val Gly Ala Pro Gly Met Pro Gly Ser Pro Gly Pro
                   85                  90                  95
   Ala Gly Leu Lys Gly Glu Arg Gly Ala Pro Gly Pro Gly Gly Ala Ile
               100                 105                 110
   Gly Pro Gln Gly Pro Ser Gly Ala Met Gly Pro Pro Gly Leu Lys Gly
           115                 120                 125
   Asp Arg Gly Asp Pro Gly Glu Lys Gly Ala Arg Gly Glu Thr Ser Val
       130                 135                 140
   Leu Glu Val Asp Thr Leu Arg Gln Arg Met Arg Asn Leu Glu Gly Glu
   145                 150                 155                 160
   Val Gln Arg Leu Gln Asn Ile Val Thr Gln Tyr Arg Lys Ala Val Leu
                   165                 170                 175
   Phe Pro Asp Gly Gln Ala Val Gly Glu Lys Ile Phe Lys Thr Ala Gly
               180                 185                 190
   Ala Val Lys Ser Tyr Ser Asp Ala Glu Gln Leu Cys Arg Glu Ala Lys
   195 200 205
   Gly Gln Leu Ala Ser Pro Arg Ser Ser Ala Glu Asn Glu Ala Val Thr
   210 215 220
   Gln Leu Val Arg Ala Lys Asn Lys His Ala Tyr Leu Ser Met Asn Asp
   225 230 235 240
   Ile Ser Lys Glu Gly Lys Phe Thr Tyr Pro Thr Gly Gly Ser Leu Asp
   245 250 255
   Tyr Ser Asn Trp Ala Pro Gly Glu Pro Asn Asn Arg Ala Lys Asp Glu
   260 265 270
   Gly Pro Glu Asn Cys Leu Glu Ile Tyr Ser Asp Gly Asn Trp Asn Asp
   275 280 285
   Ile Glu Cys Arg Glu Glu Arg Leu Val Ile Cys Glu Phe
   290 295 300
<210> 99
   <211> 369
   <212> PRT
   <213> Bos taurus
<400> 99
   Met Leu Leu Leu Pro Leu Ser Val Leu Leu Leu Leu Thr Gln Pro Trp
   1 5 10 15
   Arg Ser Leu Gly Ala Glu Met Lys Ile Tyr Ser Gln Lys Thr Met Ala
   20 25 30
   Asn Ala Cys Thr Leu Val Met Cys Ser Pro Pro Glu Asp Gly Leu Pro
   35 40 45
   Gly Arg Asp Gly Arg Asp Gly Arg Glu Gly Pro Arg Gly Glu Lys Gly
   50 55 60
   Asp Pro Gly Ser Pro Gly Pro Ala Gly Arg Ala Gly Met Pro Gly Pro
   65 70 75 80
   Ala Gly Pro Ile Gly Leu Lys Gly Asp Asn Gly Ser Ala Gly Glu Pro
   85 90 95
   Gly Pro Lys Gly Asp Thr Gly Pro Pro Gly Pro Pro Gly Met Pro Gly
   100 105 110
   Pro Ala Gly Arg Glu Gly Pro Ser Gly Lys Gln Gly Ser Met Gly Pro
   115 120 125
   Pro Gly Thr Pro Gly Pro Lys Gly Asp Thr Gly Pro Lys Gly Gly Val
   130 135 140
   Gly Ala Pro Gly Ile Gln Gly Ser Pro Gly Pro Ala Gly Leu Lys Gly
   145 150 155 160
   Glu Arg Gly Ala Pro Gly Asp Pro Gly Ala Pro Gly Arg Ala Gly Ala
   165 170 175
   Pro Gly Pro Arg Gly Ala Ile Gly Pro Gln Gly Pro Ser Gly Ala Arg
   180 185 190
   Gly Pro Pro Gly Leu Lys Gly Asp Arg Gly Thr Pro Gly Glu Arg Gly
   195 200 205
   Ala Lys Gly Glu Ser Gly Leu Ala Glu Val Asn Ala Leu Arg Gln Arg
   210 215 220
   Val Gly Ile Leu Glu Gly Gln Leu Gln Arg Leu Gln Asn Ala Phe Ser
   225 230 235 240
   Gln Tyr Lys Lys Ala Met Leu Phe Pro Asn Gly Arg Ser Val Gly Glu
   245 250 255
   Lys Ile Phe Lys Thr Val Gly Ser Glu Lys Thr Phe Gln Asp Ala Gln
   260 265 270
   Gln Ile Cys Thr Gln Ala Gly Gly Gln Leu Pro Ser Pro Arg Ser Gly
   275 280 285
   Ala Glu Asn Glu Ala Leu Thr Gln Leu Ala Thr Ala Gln Asn Lys Ala
   290 295 300
   Ala Phe Leu Ser Met Ser Asp Thr Arg Lys Glu Gly Thr Phe Ile Tyr
   305 310 315 320
   Pro Thr Gly Glu Pro Leu Val Tyr Ser Asn Trp Ala Pro Gln Glu Pro
   325 330 335
   Asn Asn Asp Gly Gly Ser Glu Asn Cys Val Glu Ile Phe Pro Asn Gly
   340 345 350
   Lys Trp Asn Asp Lys Val Cys Gly Glu Gln Arg Leu Val Ile Cys Glu
   355 360 365
   Phe
<210> 100
   <211> 116
   <212> PRT
   <213> Sus scrofa
<400> 100
   Ala Val Gly Glu Lys Val Phe Ser Thr Asn Gly Gln Ser Val Ala Phe
   1 5 10 15
   Asp Val Ile Arg Glu Leu Cys Ala Arg Ala Gly Gly Arg Ile Ala Ala
   20 25 30
   Pro Arg Ser Pro Glu Glu Asn Glu Ala Ile Ala Ser Ile Val Lys Lys
   35 40 45
   His Asn Thr Tyr Ala Tyr Leu Gly Leu Val Glu Gly Pro Thr Ala Gly
   50 55 60
   Asp Phe Phe Tyr Leu Asp Gly Thr Pro Val Asn Tyr Thr Asn Trp Tyr
   65 70 75 80
   Pro Gly Glu Pro Arg Gly Arg Gly Lys Glu Lys Cys Val Glu Met Tyr
   85 90 95
   Thr Asp Gly Gln Trp Asn Asp Arg Asn Cys Gln Gln Tyr Arg Leu Ala
   100 105 110
   Ile Cys Glu Phe
   115
<210> 101
   <211> 378
   <212> PRT
   <213> Sus scrofa
<400> 101
   Met Leu Leu Leu Pro Leu Ser Val Leu Ile Leu Leu Thr Gln Pro Pro
   1               5                   10                  15
   Arg Ser Leu Gly Ala Glu Met Lys Thr Tyr Ser Gln Arg Ala Val Ala
               20                  25                  30
   Asn Ala Cys Ala Leu Val Met Cys Ser Pro Met Glu Asn Gly Leu Pro
           35                  40                  45
   Gly Arg Asp Gly Arg Asp Gly Arg Glu Gly Pro Arg Gly Glu Lys Gly
       50                  55                  60
   Asp Pro Gly Leu Pro Gly Ala Val Gly Arg Ala Gly Met Pro Gly Leu
   65                  70                  75                  80
   Ala Gly Pro Val Gly Pro Lys Gly Asp Asn Gly Ser Thr Gly Glu Pro
                   85                  90                  95
   Gly Ala Lys Gly Asp Ile Gly Pro Cys Gly Pro Pro Gly Pro Pro Gly
               100                 105                 110
   Ile Pro Gly Pro Ala Gly Lys Glu Gly Pro Ser Gly Gln Gln Gly Asn
           115                 120                 125
   Ile Gly Pro Pro Gly Thr Pro Gly Pro Lys Gly Glu Thr Gly Pro Lys
       130                 135                 140
   Gly Glu Val Gly Ala Leu Gly Met Gln Gly Ser Thr Gly Ala Arg Gly
   145                 150                 155                 160
   Pro Ala Gly Leu Lys Gly Glu Arg Gly Ala Pro Gly Glu Arg Gly Ala
                   165                 170                 175
   Pro Gly Ser Ala Gly Ala Ala Gly Pro Ala Gly Ala Thr Gly Pro Gln
               180                 185                 190
   Gly Pro Ser Gly Ala Arg Gly Pro Pro Gly Leu Lys Gly Asp Arg Gly
           195                 200                 205
   Pro Pro Gly Glu Arg Gly Ala Lys Gly Glu Ser Gly Leu Pro Gly Ile
       210                 215                 220
   Thr Ala Leu Arg Gln Gln Val Glu Thr Leu Gln Gly Gln Val Gln Arg
   225 230 235 240
   Leu Gln Lys Ala Phe Ser Gln Tyr Lys Lys Val Glu Leu Phe Pro Asn
   245 250 255
   Gly Arg Gly Val Gly Glu Lys Ile Phe Lys Thr Gly Gly Phe Glu Lys
   260 265 270
   Thr Phe Gln Asp Ala Gln Gln Val Cys Thr Gln Ala Gly Gly Gln Met
   275 280 285
   Ala Ser Pro Arg Ser Glu Thr Glu Asn Glu Ala Leu Ser Gln Leu Val
   290 295 300
   Thr Ala Gln Asn Lys Ala Ala Phe Leu Ser Met Thr Asp Ile Lys Thr
   305 310 315 320
   Glu Gly Asn Phe Thr Tyr Pro Thr Gly Glu Pro Leu Val Tyr Ala Asn
   325 330 335
   Trp Ala Pro Gly Glu Pro Asn Asn Asn Gly Gly Ser Ser Gly Ala Glu
   340 345 350
   Asn Cys Val Glu Ile Phe Pro Asn Gly Lys Trp Asn Asp Lys Ala Cys
   355 360 365
   Gly Glu Leu Arg Leu Val Ile Cys Glu Phe
   370 375
<210> 102
   <211> 34
   <212> PRT
   <213> Bos taurus
<400> 102
   Leu Ile Pro Cys Cys Pro Val Asn Ile Lys Arg Leu Leu Ile Val Val
   1 5 10 15
   Val Val Val Val Leu Leu Val Val Val Ile Val Gly Ala Leu Leu Met
   20 25 30
   Gly Leu
<210> 103
   <211> 369
   <212> PRT
   <213> Bos taurus
<400> 103
   Met Leu Leu Leu Pro Leu Ser Val Leu Leu Leu Leu Thr Gln Pro Trp
   1 5 10 15
   Arg Ser Leu Gly Ala Glu Met Lys Ile Tyr Ser Gln Lys Thr Met Ala
   20 25 30
   Asn Ala Cys Thr Leu Val Met Cys Ser Pro Pro Glu Asp Gly Leu Pro
   35 40 45
   Gly Arg Asp Gly Arg Asp Gly Arg Glu Gly Pro Arg Gly Glu Lys Gly
   50 55 60
   Asp Pro Gly Ser Pro Gly Pro Ala Gly Arg Ala Gly Met Pro Gly Pro
   65 70 75 80
   Ala Gly Pro Ile Gly Leu Lys Gly Asp Asn Gly Ser Ala Gly Glu Pro
   85 90 95
   Gly Pro Lys Gly Asp Thr Gly Pro Pro Gly Pro Pro Gly Met Pro Gly
   100 105 110
   Pro Ala Gly Arg Glu Gly Pro Ser Gly Lys Gln Gly Ser Met Gly Pro
   115 120 125
   Pro Gly Thr Pro Gly Pro Lys Gly Asp Thr Gly Pro Lys Gly Gly Val
   130 135 140
   Gly Ala Pro Gly Ile Gln Gly Ser Pro Gly Pro Ala Gly Leu Lys Gly
   145 150 155 160
   Glu Arg Gly Ala Pro Gly Glu Pro Gly Ala Pro Gly Arg Ala Gly Ala
   165 170 175
   Pro Gly Pro Ala Gly Ala Ile Gly Pro Gln Gly Pro Ser Gly Ala Arg
   180 185 190
   Gly Pro Pro Gly Leu Lys Gly Asp Arg Gly Thr Pro Gly Glu Arg Gly
   195 200 205
   Ala Lys Gly Glu Ser Gly Leu Ala Glu Val Asn Ala Leu Arg Gln Arg
   210 215 220
   Val Gly Ile Leu Glu Gly Gln Leu Gln Arg Leu Gln Asn Ala Phe Ser
   225 230 235 240
   Gln Tyr Lys Lys Ala Met Leu Phe Pro Asn Gly Arg Ser Val Gly Glu
   245 250 255
   Lys Ile Phe Lys Thr Val Gly Ser Glu Lys Thr Phe Gln Asp Ala Gln
   260 265 270
   Gln Ile Cys Thr Gln Ala Gly Gly Gln Leu Pro Ser Pro Arg Ser Gly
   275 280 285
   Ala Glu Asn Glu Ala Leu Thr Gln Leu Ala Thr Ala Gln Asn Lys Ala
   290 295 300
   Ala Phe Leu Ser Met Ser Asp Thr Arg Lys Glu Gly Thr Phe Ile Tyr
   305 310 315 320
   Pro Thr Gly Glu Pro Leu Val Tyr Ser Asn Trp Ala Pro Gln Glu Pro
   325 330 335
   Asn Asn Asp Gly Gly Ser Glu Asn Cys Val Glu Ile Phe Pro Asn Gly
   340 345 350
   Lys Trp Asn Asp Lys Val Cys Gly Glu Gln Arg Leu Val Ile Cys Glu
   355 360 365
   Phe
<210> 104
   <211> 301
   <212> PRT
   <213> Bos taurus
<400> 104
   Glu Glu Met Asp Val Tyr Ser Glu Lys Thr Leu Thr Asp Pro Cys Thr
   1 5 10 15
   Leu Val Val Cys Ala Pro Pro Ala Asp Ser Leu Arg Gly His Asp Gly
   20 25 30
   Arg Asp Gly Lys Glu Gly Pro Gln Gly Glu Lys Gly Asp Pro Gly Pro
   35 40 45
   Pro Gly Met Pro Gly Pro Ala Gly Arg Glu Gly Pro Ser Gly Arg Gln
   50 55 60
   Gly Ser Met Gly Pro Pro Gly Thr Pro Gly Pro Lys Gly Glu Pro Gly
   65 70 75 80
   Pro Glu Gly Gly Val Gly Ala Pro Gly Met Pro Gly Ser Pro Gly Pro
   85 90 95
   Ala Gly Leu Lys Gly Glu Arg Gly Ala Pro Gly Pro Gly Gly Ala Ile
   100 105 110
   Gly Pro Gln Gly Pro Ser Gly Ala Met Gly Pro Pro Gly Leu Lys Gly
   115 120 125
   Asp Arg Gly Asp Pro Gly Glu Lys Gly Ala Arg Gly Glu Thr Ser Val
   130 135 140
   Leu Glu Val Asp Thr Leu Arg Gln Arg Met Arg Asn Leu Glu Gly Glu
   145 150 155 160
   Val Gln Arg Leu Gln Asn Ile Val Thr Gln Tyr Arg Lys Ala Val Leu
   165 170 175
   Phe Pro Asp Gly Gln Ala Val Gly Glu Lys Ile Phe Lys Thr Ala Gly
   180 185 190
   Ala Val Lys Ser Tyr Ser Asp Ala Glu Gln Leu Cys Arg Glu Ala Lys
   195 200 205
   Gly Gln Leu Ala Ser Pro Arg Ser Ser Ala Glu Asn Glu Ala Val Thr
   210 215 220
   Gln Leu Val Arg Ala Lys Asn Lys His Ala Tyr Leu Ser Met Asn Asp
   225 230 235 240
   Ile Ser Lys Glu Gly Lys Phe Thr Tyr Pro Thr Gly Gly Ser Leu Asp
   245 250 255
   Tyr Ser Asn Trp Ala Pro Gly Glu Pro Gly Asn Arg Ala Lys Asp Glu
   260 265 270
   Gly Pro Glu Asn Cys Leu Glu Ile Tyr Ser Asp Gly Asn Trp Asn Asp
   275 280 285
   Ile Glu Cys Arg Glu Glu Arg Leu Val Ile Cys Glu Phe
   290 295 300
<210> 105
   <211> 2412
   <212> PRT
   <213> Homo sapiens
<400> 105
<210> 106
   <211> 2413
   <212> PRT
   <213> Homo sapiens
<400> 106
<210> 107
   <211> 374
   <212> PRT
   <213> Mus musculus
<400> 107
   Met Leu Pro Phe Leu Ser Met Leu Val Leu Leu Val Gln Pro Leu Gly
   1 5 10 15
   Asn Leu Gly Ala Glu Met Lys Ser Leu Ser Gln Arg Ser Val Pro Asn
   20 25 30
   Thr Cys Thr Leu Val Met Cys Ser Pro Thr Glu Asn Gly Leu Pro Gly
   35 40 45
   Arg Asp Gly Arg Asp Gly Arg Glu Gly Pro Arg Gly Glu Lys Gly Asp
   50 55 60
   Pro Gly Leu Pro Gly Pro Met Gly Leu Ser Gly Leu Gln Gly Pro Thr
   65 70 75 80
   Gly Pro Val Gly Pro Lys Gly Glu Asn Gly Ser Ala Gly Glu Pro Gly
   85 90 95
   Pro Lys Gly Glu Arg Gly Leu Ser Gly Pro Pro Gly Leu Pro Gly Ile
   100 105 110
   Pro Gly Pro Ala Gly Lys Glu Gly Pro Ser Gly Lys Gln Gly Asn Ile
   115 120 125
   Gly Pro Gln Gly Lys Pro Gly Pro Lys Gly Glu Ala Gly Pro Lys Gly
   130 135 140
   Glu Val Gly Ala Pro Gly Met Gln Gly Ser Thr Gly Ala Lys Gly Ser
   145 150 155 160
   Thr Gly Pro Lys Gly Glu Arg Gly Ala Pro Gly Val Gln Gly Ala Pro
   165 170 175
   Gly Asn Ala Gly Ala Ala Gly Pro Ala Gly Pro Ala Gly Pro Gln Gly
   180 185 190
   Ala Pro Gly Ser Arg Gly Pro Pro Gly Leu Lys Gly Asp Arg Gly Val
   195 200 205
   Pro Gly Asp Arg Gly Ile Lys Gly Glu Ser Gly Leu Pro Asp Ser Ala
   210 215 220
   Ala Leu Arg Gln Gln Met Glu Ala Leu Lys Gly Lys Leu Gln Arg Leu
   225 230 235 240
   Glu Val Ala Phe Ser His Tyr Gln Lys Ala Ala Leu Phe Pro Asp Gly
   245 250 255
   Arg Ser Val Gly Asp Lys Ile Phe Arg Thr Ala Asp Ser Glu Lys Pro
   260 265 270
   Phe Glu Asp Ala Gln Glu Met Cys Lys Gln Ala Gly Gly Gln Leu Ala
   275 280 285
   Ser Pro Arg Ser Ala Thr Glu Asn Ala Ala Ile Gln Gln Leu Ile Thr
   290 295 300
   Ala His Asn Lys Ala Ala Phe Leu Ser Met Thr Asp Val Gly Thr Glu
   305 310 315 320
   Gly Lys Phe Thr Tyr Pro Thr Gly Glu Pro Leu Val Tyr Ser Asn Trp
   325 330 335
   Ala Pro Gly Glu Pro Asn Asn Asn Gly Gly Ala Glu Asn Cys Val Glu
   340 345 350
   Ile Phe Thr Asn Gly Gln Trp Asn Asp Lys Ala Cys Gly Glu Gln Arg
   355 360 365
   Leu Val Ile Cys Glu Phe
   370
<210> 108
   <211> 35
   <212> PRT
   <213> Canis familiaris
<400> 108
   Leu Gly Ile Pro Cys Phe Pro Ser Ser Leu Lys Arg Leu Leu Ile Ile
   1 5 10 15
   Val Val Val Ile Val Leu Val Val Val Val Ile Val Gly Ala Leu Leu
   20 25 30
   Met Gly Leu
   35
<210> 109
   <211> 34
   <212> PRT
   <213> Bos taurus
<400> 109
   Leu Ile Pro Cys Cys Pro Val Asn Ile Lys Arg Leu Leu Ile Val Val
   1 5 10 15
   Val Val Val Val Leu Leu Val Val Val Ile Val Gly Ala Leu Leu Met
   20 25 30
   Gly Leu
<210> 110
   <211> 35
   <212> PRT
   <213> Homo sapiens
<400> 110
   Gly Gln Ser Ile Thr Phe Asp Ala Gly Lys Glu Gln Cys Val Glu Met
   1 5 10 15
   Tyr Thr Asp Gly Gln Trp Asn Asp Arg Asn Cys Leu Tyr Leu Thr Ile
   20 25 30
   Cys Glu Phe
   35
<210> 111
   <211> 370
   <212> PRT
   <213> Oryctolagus cuniculus
<400> 111
   Met Ala Lys Ser His Leu Pro Pro Trp Leu Leu Leu Leu Leu Leu Pro
   1 5 10 15
   Thr Leu Cys Gly Pro Gly Thr Ala Val Trp Ala Thr Ser Pro Leu Ala
   20 25 30
   Cys Ala Gln Gly Pro Glu Phe Trp Cys Gln Ser Leu Glu Gln Ala Leu
   35 40 45
   Gln Cys Lys Ala Leu Gly His Cys Leu Gln Glu Val Trp Gly His Val
   50 55 60
   Gly Ala Asp Asp Leu Cys Gln Glu Cys Gln Asp Ile Val Asn Ile Leu
   65 70 75 80
   Thr Lys Met Thr Lys Glu Ala Ile Phe Gln Asp Thr Ile Arg Lys Phe
   85 90 95
   Leu Glu His Glu Cys Asp Val Leu Pro Leu Lys Leu Leu Val Pro Gln
   100 105 110
   Cys His His Val Leu Asp Val Tyr Phe Pro Leu Thr Ile Thr Tyr Phe
   115 120 125
   Leu Ser Gln Ile Asn Ala Lys Ala Ile Cys Gln His Leu Gly Leu Cys
   130 135 140
   Gln Pro Gly Ser Pro Glu Pro Pro Leu Asp Pro Leu Pro Asp Lys Leu
   145 150 155 160
   Val Leu Pro Thr Leu Leu Gly Ala Leu Pro Ala Lys Pro Gly Pro His
   165 170 175
   Thr Gln Asp Leu Ser Ala Gln Arg Phe Pro Ile Pro Leu Pro Leu Cys
   180 185 190
   Trp Leu Cys Arg Thr Leu Leu Lys Arg Ile Gln Ala Met Ile Pro Lys
   195 200 205
   Gly Val Leu Ala Met Ala Val Ala Gln Val Cys His Val Val Pro Leu
   210 215 220
   Val Val Gly Gly Ile Cys Gln Cys Leu Ala Glu Arg Tyr Thr Val Ile
   225 230 235 240
   Leu Leu Glu Val Leu Leu Gly His Val Leu Pro Gln Leu Val Cys Gly
   245 250 255
   Leu Val Leu Arg Cys Ser Ser Val Asp Ser Ile Gly Gln Val Pro Pro
   260 265 270
   Thr Leu Glu Ala Leu Pro Gly Glu Trp Leu Pro Gln Asp Pro Glu Cys
   275 280 285
   Pro Leu Cys Met Ser Val Thr Thr Gln Ala Arg Asn Ile Ser Glu Gln
   290 295 300
   Thr Arg Pro Gln Ala Val Tyr His Ala Cys Leu Ser Ser Gln Leu Asp
   305 310 315 320
   Lys Gln Glu Cys Glu Gln Phe Val Glu Leu His Thr Pro Gln Leu Leu
   325 330 335
   Ser Leu Leu Ser Arg Gly Trp Asp Ala Arg Ala Ile Cys Gln Ala Leu
   340 345 350
   Gly Ala Cys Val Ala Thr Leu Ser Pro Leu Gln Cys Ile Gln Ser Pro
   355 360 365
   His Phe
   370
<210> 112
   <211> 247
   <212> PRT
   <213> Oryctolagus cuniculus
<400> 112
   Met Leu Leu Leu Ser Leu Ala Leu Thr Leu Ile Ser Ala Pro Ala Ser
   1 5 10 15
   Asp Thr Cys Asp Thr Lys Asp Val Cys Ile Gly Ser Pro Gly Ile Pro
   20 25 30
   Gly Thr Pro Gly Ser His Gly Leu Pro Gly Arg Asp Gly Arg Asp Gly
   35 40 45
   Val Lys Gly Asp Pro Gly Pro Pro Gly Pro Met Gly Pro Pro Gly Gly
   50 55 60
   Met Pro Gly Leu Pro Gly Arg Asp Gly Leu Ile Gly Ala Pro Gly Val
   65 70 75 80
   Pro Gly Glu Arg Gly Asp Lys Gly Glu Pro Gly Glu Arg Gly Pro Pro
   85 90 95
   Gly Leu Pro Ala Tyr Leu Asp Glu Glu Leu Gln Ala Thr Leu His Glu
   100 105 110
   Leu Arg His His Ala Leu Gln Ser Ile Gly Val Leu Ser Leu Gln Gly
   115 120 125
   Ser Met Lys Ala Val Gly Glu Lys Ile Phe Ser Thr Asn Gly Gln Ser
   130 135 140
   Val Asn Phe Asp Ala Ile Arg Glu Val Cys Ala Arg Ala Gly Gly Arg
   145 150 155 160
   Ile Ala Ile Val Lys Glu Val Pro Arg Ser Leu Glu Glu Asn Glu Ala
   165 170 175
   Ile Ala Ser Arg Asn Thr Tyr Ala Tyr Leu Gly Leu Ala Glu Gly Pro
   180 185 190
   Thr Ala Gly Asp Phe Tyr Tyr Leu Asp Gly Asp Pro Val Asn Tyr Thr
   195 200 205
   Asn Trp Tyr Pro Gly Glu Pro Arg Gly Gln Gly Arg Glu Lys Cys Val
   210 215 220
   Glu Met Tyr Thr Asp Gly Lys Trp Asn Asp Lys Asn Cys Leu Gln Tyr
   225 230 235 240
   Arg Leu Val Ile Cys Glu Phe
   245
<210> 113
   <211> 369
   <212> PRT
   <213> Bos taurus
<400> 113
   Met Leu Leu Leu Pro Leu Ser Val Leu Leu Leu Leu Thr Gln Pro Trp
   1 5 10 15
   Arg Ser Leu Gly Ala Glu Met Lys Ile Tyr Ser Gln Lys Thr Met Ala
   20 25 30
   Asn Ala Cys Thr Leu Val Met Cys Ser Pro Pro Glu Asp Gly Leu Pro
           35                  40                  45
   Gly Arg Asp Gly Arg Asp Gly Arg Glu Gly Pro Arg Gly Glu Lys Gly
       50                  55                  60
   Asp Pro Gly Ser Pro Gly Pro Ala Gly Arg Ala Gly Met Pro Gly Pro
   65                  70                  75                  80
   Ala Gly Pro Ile Gly Leu Lys Gly Asp Asn Gly Ser Ala Gly Glu Pro
                   85                  90                  95
   Gly Pro Lys Gly Asp Thr Gly Pro Pro Gly Pro Pro Gly Met Pro Gly
               100                 105                 110
   Pro Ala Gly Arg Glu Gly Pro Ser Gly Lys Gln Gly Ser Met Gly Pro
           115                 120                 125
   Pro Gly Thr Pro Gly Pro Lys Gly Asp Thr Gly Pro Lys Gly Gly Val
       130                 135                 140
   Gly Ala Pro Gly Ile Gln Gly Ser Pro Gly Pro Ala Gly Leu Lys Gly
   145                 150                 155                 160
   Glu Arg Gly Ala Pro Gly Glu Pro Gly Ala Pro Gly Arg Ala Gly Ala
                   165                 170                 175
   Pro Gly Pro Ala Gly Ala Ile Gly Pro Gln Gly Pro Ser Gly Ala Arg
               180                 185                 190
   Gly Pro Pro Gly Leu Lys Gly Asp Arg Gly Thr Pro Gly Glu Arg Gly
           195                 200                 205
   Ala Lys Gly Glu Ser Gly Leu Ala Glu Val Asn Ala Leu Arg Gln Arg
       210                 215                 220
   Val Gly Ile Leu Glu Gly Gln Leu Gln Arg Leu Gln Asn Ala Phe Ser
   225                 230                 235                 240
   Gln Tyr Lys Lys Ala Met Leu Phe Pro Asn Gly Arg Ser Val Gly Glu
                   245                 250                 255
   Lys Ile Phe Lys Thr Val Gly Ser Glu Lys Thr Phe Gln Asp Ala Gln
               260                 265                 270
   Gln Ile Cys Thr Gln Ala Gly Gly Gln Leu Pro Ser Pro Arg Ser Gly
   275 280 285
   Ala Glu Asn Glu Ala Leu Thr Gln Leu Ala Thr Ala Gln Asn Lys Ala
   290 295 300
   Ala Phe Leu Ser Met Ser Asp Thr Arg Lys Glu Gly Thr Phe Ile Tyr
   305 310 315 320
   Pro Thr Gly Glu Pro Leu Val Tyr Ser Asn Trp Ala Pro Gln Glu Pro
   325 330 335
   Asn Asn Asp Gly Gly Ser Glu Asn Cys Val Glu Ile Phe Pro Asn Gly
   340 345 350
   Lys Trp Asn Asp Lys Val Cys Gly Glu Gln Arg Leu Val Ile Cys Glu
   355 360 365
   Phe
<210> 114
   <211> 301
   <212> PRT
   <213> Bos taurus
<220>
   <221> MISC_FEATURE
   <222> (7) .. (7)
   <223> Unknown
<400> 114
   Glu Glu Met Asp Val Tyr Xaa Glu Lys Thr Leu Thr Asp Pro Cys Thr
   1 5 10 15
   Leu Val Val Cys Ala Pro Pro Ala Asp Ser Leu Arg Gly His Asp Gly
   20 25 30
   Arg Asp Gly Lys Glu Gly Pro Gln Gly Glu Lys Gly Asp Pro Gly Pro
   35 40 45
   Pro Gly Met Pro Gly Pro Ala Gly Arg Glu Gly Pro Ser Gly Arg Gln
   50 55 60
   Gly Ser Met Gly Pro Pro Gly Thr Pro Gly Pro Lys Gly Glu Pro Gly
   65 70 75 80
   Pro Glu Gly Gly Val Gly Ala Pro Gly Met Pro Gly Ser Pro Gly Pro
   85 90 95
   Ala Gly Leu Lys Gly Glu Arg Gly Ala Pro Gly Pro Gly Gly Ala Ile
   100 105 110
   Gly Pro Gln Gly Pro Ser Gly Ala Met Gly Pro Pro Gly Leu Lys Gly
   115 120 125
   Asp Arg Gly Asp Pro Gly Glu Lys Gly Ala Arg Gly Glu Thr Ser Val
   130 135 140
   Leu Glu Val Asp Thr Leu Arg Gln Arg Met Arg Asn Leu Glu Gly Glu
   145 150 155 160
   Val Gln Arg Leu Gln Asn Ile Val Thr Gln Tyr Arg Lys Ala Val Leu
   165 170 175
   Phe Pro Asp Gly Gln Ala Val Gly Glu Lys Ile Phe Lys Thr Ala Gly
   180 185 190
   Ala Val Lys Ser Tyr Ser Asp Ala Glu Gln Leu Cys Arg Glu Ala Lys
   195 200 205
   Gly Gln Leu Ala Ser Pro Arg Ser Ser Ala Glu Asn Glu Ala Val Thr
   210 215 220
   Gln Leu Val Arg Ala Lys Asn Lys His Ala Tyr Leu Ser Met Asn Asp
   225 230 235 240
   Ile Ser Lys Glu Gly Lys Phe Thr Tyr Pro Thr Gly Gly Ser Leu Asp
   245 250 255
   Tyr Ser Asn Trp Ala Pro Gly Glu Pro Gly Asn Arg Ala Lys Asp Glu
   260 265 270
   Gly Pro Glu Asn Cys Leu Glu Ile Tyr Ser Asp Gly Asn Trp Asn Asp
   275 280 285
   Ile Glu Cys Arg Glu Glu Arg Leu Val Ile Cys Glu Phe
   290 295 300
<210> 115
   <211> 375
   <212> PRT
   <213> Homo sapiens
<400> 115
   Met Leu Leu Phe Leu Leu Ser Ala Leu Val Leu Leu Thr Gln Pro Leu
   1               5                   10                  15
   Gly Tyr Leu Glu Ala Glu Met Lys Thr Tyr Ser His Arg Thr Thr Pro
               20                  25                  30
   Ser Ala Cys Thr Leu Val Met Cys Ser Ser Val Glu Ser Gly Leu Pro
           35                  40                  45
   Gly Arg Asp Gly Arg Asp Gly Arg Glu Gly Pro Arg Gly Glu Lys Gly
       50                  55                  60
   Asp Pro Gly Leu Pro Gly Ala Ala Gly Gln Ala Gly Met Pro Gly Gln
   65                  70                  75                  80
   Ala Gly Pro Val Gly Pro Lys Gly Asp Asn Gly Ser Val Gly Glu Pro
                   85                  90                  95
   Gly Pro Lys Gly Asp Thr Gly Pro Ser Gly Pro Pro Gly Pro Pro Gly
               100                 105                 110
   Val Pro Gly Pro Ala Gly Arg Glu Gly Pro Leu Gly Lys Gln Gly Asn
           115                 120                 125
   Ile Gly Pro Gln Gly Lys Pro Gly Pro Lys Gly Glu Ala Gly Pro Lys
       130                 135                 140
   Gly Glu Val Gly Ala Pro Gly Met Gln Gly Ser Ala Gly Ala Arg Gly
   145                 150                 155                 160
   Leu Ala Gly Pro Lys Gly Glu Arg Gly Val Pro Gly Glu Arg Gly Val
                   165                 170                 175
   Pro Gly Asn Ala Gly Ala Ala Gly Ser Ala Gly Ala Met Gly Pro Gln
               180                 185                 190
   Gly Ser Pro Gly Ala Arg Gly Pro Pro Gly Leu Lys Gly Asp Lys Gly
   195 200 205
   Ile Pro Gly Asp Lys Gly Ala Lys Gly Glu Ser Gly Leu Pro Asp Val
   210 215 220
   Ala Ser Leu Arg Gln Gln Val Glu Ala Leu Gln Gly Gln Val Gln His
   225 230 235 240
   Leu Gln Ala Ala Phe Ser Gln Tyr Lys Lys Val Glu Leu Phe Pro Asn
   245 250 255
   Gly Gln Ser Val Gly Glu Lys Ile Phe Lys Thr Ala Gly Phe Val Lys
   260 265 270
   Pro Phe Thr Glu Ala Gln Leu Leu Cys Thr Gln Ala Gly Gly Gln Leu
   275 280 285
   Ala Ser Pro Arg Ser Ala Ala Glu Asn Ala Ala Leu Gln Gln Leu Val
   290 295 300
   Val Ala Lys Asn Glu Ala Ala Phe Leu Ser Met Thr Asp Ser Lys Thr
   305 310 315 320
   Glu Gly Lys Phe Thr Tyr Pro Thr Gly Glu Ser Leu Val Tyr Ser Asn
   325 330 335
   Trp Ala Pro Gly Glu Pro Asn Asp Asp Gly Gly Ser Glu Asp Cys Val
   340 345 350
   Glu Ile Phe Thr Asn Gly Lys Trp Asn Asp Arg Ala Cys Gly Glu Lys
   355 360 365
   Arg Leu Val Val Cys Glu Phe
   370 375
<210> 116
   <211> 194
   <212> PRT
   <213> Rattus norvegicus
<400> 116
   Met Asp Met Gly Ser Lys Glu Val Leu Met Glu Ser Pro Pro Asp Tyr
   1 5 10 15
   Ser Thr Gly Pro Arg Ser Gln Phe Arg Ile Pro Cys Cys Pro Val His
   20 25 30
   Leu Lys Arg Leu Leu Ile Val Val Val Val Val Val Leu Val Val Val
   35 40 45
   Val Ile Val Gly Ala Leu Leu Met Gly Leu His Met Ser Gln Lys His
   50 55 60
   Thr Glu Met Val Leu Glu Met Ser Ile Gly Gly Ala Pro Glu Thr Gln
   65 70 75 80
   Lys Arg Leu Ala Leu Ser Glu His Thr Asp Thr Ile Ala Thr Phe Ser
   85 90 95
   Ile Gly Ser Thr Gly Ile Val Leu Tyr Asp Tyr Gln Arg Leu Leu Thr
   100 105 110
   Ala Tyr Lys Pro Ala Pro Gly Thr Tyr Cys Tyr Ile Met Lys Met Ala
   115 120 125
   Pro Glu Ser Ile Pro Ser Leu Glu Ala Leu Ala Arg Lys Phe Lys Asn
   130 135 140
   Phe Gln Ala Lys Ser Ser Thr Pro Thr Ser Lys Leu Gly Gln Glu Glu
   145 150 155 160
   Gly His Ser Ala Gly Ser Asp Ser Asp Ser Ser Gly Arg Asp Leu Ala
   165 170 175
   Phe Leu Gly Leu Ala Val Ser Thr Leu Cys Gly Val Leu Pro Leu Tyr
   180 185 190
   Tyr Ile
<210> 117
   <211> 247
   <212> PRT
   <213> Oryctolagus cuniculus
<400> 117
   Met Leu Leu Leu Ser Leu Ala Leu Thr Leu Ile Ser Ala Pro Ala Ser
   1               5                   10                  15
   Asp Thr Cys Asp Thr Lys Asp Val Cys Ile Gly Ser Pro Gly Ile Pro
               20                  25                  30
   Gly Thr Pro Gly Ser His Gly Leu Pro Gly Arg Asp Gly Arg Asp Gly
           35                  40                  45
   Val Lys Gly Asp Pro Gly Pro Pro Ala Pro Trp Ala Pro Pro Gly Gly
       50                  55                  60
   Met Pro Gly Leu Pro Gly Arg Asp Gly Leu Ile Gly Ala Pro Gly Val
   65                  70                  75                  80
   Pro Gly Glu Arg Gly Asp Lys Gly Glu Pro Gly Glu Arg Gly Pro Pro
                   85                  90                  95
   Gly Leu Pro Ala Tyr Leu Asp Glu Glu Leu Gln Ala Thr Leu His Glu
               100                 105                 110
   Leu Arg His His Ala Leu Gln Ser Ile Gly Val Leu Ser Leu Gln Gly
           115                 120                 125
   Ser Met Lys Ala Val Gly Glu Lys Ile Phe Ser Thr Asn Gly Gln Ser
       130                 135                 140
   Val Asn Phe Asp Ala Ile Arg Glu Val Cys Ala Arg Ala Gly Gly Arg
   145                 150                 155                 160
   Ile Ala Val Pro Arg Ser Leu Glu Glu Asn Glu Ala Ile Ala Ser Ile
                   165                 170                 175
   Val Lys Glu Arg Asn Thr Tyr Ala Tyr Leu Gly Leu Ala Glu Gly Pro
               180                 185                 190
   Thr Ala Gly Asp Phe Tyr Tyr Leu Asp Gly Asp Pro Val Asn Tyr Thr
           195                 200                 205
   Asn Trp Tyr Pro Gly Glu Pro Arg Gly Gln Gly Arg Glu Lys Cys Val
       210                 215                 220
   Glu Met Tyr Thr Asp Gly Lys Trp Asn Asp Lys Asn Cys Leu Gln Tyr
   225                 230                 235                 240
   Arg Leu Val Ile Cys Glu Phe
                   245
<210> 118
   <211> 251
   <212> PRT
   <213> Homo sapiens
<220>
   <221> MISC_FEATURE
   <222> (25)..(130)
   <223> Polypeptide sequence from ficolin dom aa131
<220>
   <221> MISC_FEATURE
   <222> (131)..(251)
   <223> Polypeptide sequence from MBL aa129
<400> 118
   Met Glu Leu Asp Arg Ala Val Gly Val Leu Gly Ala Ala Thr Leu Leu
   1               5                   10                  15
   Leu Ser Phe Leu Gly Met Ala Trp Ala Leu Gln Ala Ala Asp Thr Cys
               20                  25                  30
   Pro Glu Val Lys Met Val Gly Leu Glu Gly Ser Asp Lys Leu Thr Ile
           35                  40                  45
   Leu Arg Gly Cys Pro Gly Leu Pro Gly Ala Pro Gly Asp Lys Gly Glu
       50                  55                  60
   Ala Gly Thr Asn Gly Lys Arg Gly Glu Arg Gly Pro Pro Gly Pro Pro
   65                  70                  75                  80
   Gly Lys Ala Gly Pro Pro Gly Pro Asn Gly Ala Pro Gly Glu Pro Gln
                   85                  90                  95
   Pro Cys Leu Thr Gly Pro Arg Thr Cys Lys Asp Leu Leu Asp Arg Gly
               100                 105                 110
   His Phe Leu Ser Gly Trp His Thr Ile Tyr Leu Pro Asp Cys Arg Pro
   115 120 125
   Leu Thr Phe Ser Leu Gly Lys Gln Val Gly Asn Lys Phe Phe Leu Thr
   130 135 140
   Asn Gly Glu Ile Met Thr Phe Glu Lys Val Lys Ala Leu Cys Val Lys
   145 150 155 160
   Phe Gln Ala Ser Val Ala Thr Pro Arg Asn Ala Ala Glu Asn Gly Ala
   165 170 175
   Ile Gln Asn Leu Ile Lys Glu Glu Ala Phe Leu Gly Ile Thr Asp Glu
   180 185 190
   Lys Thr Glu Gly Gln Phe Val Asp Leu Thr Gly Asn Arg Leu Thr Tyr
   195 200 205
   Thr Asn Trp Asn Glu Gly Glu Pro Asn Asn Ala Gly Ser Asp Glu Asp
   210 215 220
   Cys Val Leu Leu Leu Lys Asn Gly Gln Trp Asn Asp Val Pro Cys Ser
   225 230 235 240
   Thr Ser His Leu Ala Val Cys Glu Phe Pro Ile
   245 250
<210> 119
   <211> 329
   <212> PRT
   <213> Homo sapiens
<220>
   <221> MISC_FEATURE
   <222> (25)..(206)
   <223> Polypeptide sequence from ficolin-dom. containing pred. coil-coil to aa207
<220>
   <221> MISC_FEATURE
   <222> (207) .. (329)
   <223> Polypeptide sequence from MBL
<400> 119
   Met Glu Leu Asp Arg Ala Val Gly Val Leu Gly Ala Ala Thr Leu Leu
   1               5                   10                  15
   Leu Ser Phe Leu Gly Met Ala Trp Ala Leu Gln Ala Ala Asp Thr Cys
               20                  25                  30
   Pro Glu Val Lys Met Val Gly Leu Glu Gly Ser Asp Lys Leu Thr Ile
           35                  40                  45
   Leu Arg Gly Cys Pro Gly Leu Pro Gly Ala Pro Gly Asp Lys Gly Glu
       50                  55                  60
   Ala Gly Thr Asn Gly Lys Arg Gly Glu Arg Gly Pro Pro Gly Pro Pro
   65                  70                  75                  80
   Gly Lys Ala Gly Pro Pro Gly Pro Asn Gly Ala Pro Gly Glu Pro Gln
                   85                  90                  95
   Pro Cys Leu Thr Gly Pro Arg Thr Cys Lys Asp Leu Leu Asp Arg Gly
               100                 105                 110
   His Phe Leu Ser Gly Trp His Thr Ile Tyr Leu Pro Asp Cys Arg Pro
           115                 120                 125
   Leu Thr Val Leu Cys Asp Met Asp Thr Asp Gly Gly Gly Trp Thr Val
       130                 135                 140
   Phe Gln Arg Arg Val Asp Gly Ser Val Asp Phe Tyr Arg Asp Trp Ala
   145                 150                 155                 160
   Thr Tyr Lys Gln Gly Phe Gly Ser Arg Leu Gly Glu Phe Trp Leu Gly
                   165                 170                 175
   Asn Asp Asn Ile His Ala Leu Thr Ala Gln Gly Thr Ser Glu Leu Arg
               180                 185                 190
   Val Asp Leu Val Asp Phe Glu Asp Asn Tyr Gln Phe Ala Lys Leu Thr
           195                 200                 205
   Phe Ser Leu Gly Lys Gln Val Gly Asn Lys Phe Phe Leu Thr Asn Gly
       210                 215                 220
   Glu Ile Met Thr Phe Glu Lys Val Lys Ala Leu Cys Val Lys Phe Gln
   225 230 235 240
   Ala Ser Val Ala Thr Pro Arg Asn Ala Ala Glu Asn Gly Ala Ile Gln
   245 250 255
   Asn Leu Ile Lys Glu Glu Ala Phe Leu Gly Ile Thr Asp Glu Lys Thr
   260 265 270
   Glu Gly Gln Phe Val Asp Leu Thr Gly Asn Arg Leu Thr Tyr Thr Asn
   275 280 285
   Trp Asn Glu Gly Glu Pro Asn Asn Ala Gly Ser Asp Glu Asp Cys Val
   290 295 300
   Leu Leu Leu Lys Asn Gly Gln Trp Asn Asp Val Pro Cys Ser Thr Ser
   305 310 315 320
   His Leu Ala Val Cys Glu Phe Pro Ile
   325
<210> 120
   <211> 240
   <212> PRT
   <213> Homo sapiens
<220>
   <221> MISC_FEATURE
   <222> (25) .. (91)
   <223> Polypeptide sequence from collagen
<220>
   <221> MISC_FEATURE
   <222> (92)..(240)
   <223> Polypeptide sequence from MBL
<400> 120
   Met Glu Leu Asp Arg Ala Val Gly Val Leu Gly Ala Ala Thr Leu Leu
   1 5 10 15
   Leu Ser Phe Leu Gly Met Ala Trp Ala Leu Gln Ala Ala Asp Thr Cys
   20 25 30
   Pro Glu Val Lys Met Val Gly Leu Glu Gly Ser Asp Lys Leu Thr Ile
   35 40 45
   Leu Arg Gly Cys Pro Gly Leu Pro Gly Ala Pro Gly Asp Lys Gly Glu
   50 55 60
   Ala Gly Thr Asn Gly Lys Arg Gly Glu Arg Gly Pro Pro Gly Pro Pro
   65 70 75 80
   Gly Lys Ala Gly Pro Pro Gly Pro Asn Gly Ala Pro Pro Asp Gly Asp
   85 90 95
   Ser Ser Leu Ala Ala Ser Glu Arg Lys Ala Leu Gln Thr Glu Met Ala
   100 105 110
   Arg Ile Lys Lys Trp Leu Thr Phe Ser Leu Gly Lys Gln Val Gly Asn
   115 120 125
   Lys Phe Phe Leu Thr Asn Gly Glu Ile Met Thr Phe Glu Lys Val Lys
   130 135 140
   Ala Leu Cys Val Lys Phe Gln Ala Ser Val Ala Thr Pro Arg Asn Ala
   145 150 155 160
   Ala Glu Asn Gly Ala Ile Gln Asn Leu Ile Lys Glu Glu Ala Phe Leu
   165 170 175
   Gly Ile Thr Asp Glu Lys Thr Glu Gly Gln Phe Val Asp Leu Thr Gly
   180 185 190
   Asn Arg Leu Thr Tyr Thr Asn Trp Asn Glu Gly Glu Pro Asn Asn Ala
   195 200 205
   Gly Ser Asp Glu Asp Cys Val Leu Leu Leu Lys Asn Gly Gln Trp Asn
   210 215 220
   Asp Val Pro Cys Ser Thr Ser His Leu Ala Val Cys Glu Phe Pro Ile
   225 230 235 240
<210> 121
   <211> 255
   <212> PRT
   <213> Homo sapiens
<220>
   <221> MISC_FEATURE
   <222> (25)..(82)
   <223> Polypeptide sequence from collagen to cons.K at aa 93
<220>
   <221> MISC_FEATURE
   <222> (83)..(255)
   <223> Polypeptide sequence from MBL
<400> 121
   Met Glu Leu Asp Arg Ala Val Gly Val Leu Gly Ala Ala Thr Leu Leu
   1               5                   10                  15
   Leu Ser Phe Leu Gly Met Ala Trp Ala Leu Gln Ala Ala Asp Thr Cys
               20                  25                  30
   Pro Glu Val Lys Met Val Gly Leu Glu Gly Ser Asp Lys Leu Thr Ile
           35                  40                  45
   Leu Arg Gly Cys Pro Gly Leu Pro Gly Ala Pro Gly Asp Lys Gly Glu
       50                  55                  60
   Ala Gly Thr Asn Gly Lys Arg Gly Glu Arg Gly Pro Pro Gly Pro Pro
   65                  70                  75                  80
   Gly Lys Leu Gly Pro Pro Gly Asn Pro Gly Pro Ser Gly Ser Pro Gly
                   85                  90                  95
   Pro Lys Gly Gln Lys Gly Asp Pro Gly Lys Ser Pro Asp Gly Asp Ser
               100                 105                 110
   Ser Leu Ala Ala Ser Glu Arg Lys Ala Leu Gln Thr Glu Met Ala Arg
           115                 120                 125
   Ile Lys Lys Trp Leu Thr Phe Ser Leu Gly Lys Gln Val Gly Asn Lys
       130                 135                 140
   Phe Phe Leu Thr Asn Gly Glu Ile Met Thr Phe Glu Lys Val Lys Ala
   145 150 155 160
   Leu Cys Val Lys Phe Gln Ala Ser Val Ala Thr Pro Arg Asn Ala Ala
   165 170 175
   Glu Asn Gly Ala Ile Gln Asn Leu Ile Lys Glu Glu Ala Phe Leu Gly
   180 185 190
   Ile Thr Asp Glu Lys Thr Glu Gly Gln Phe Val Asp Leu Thr Gly Asn
   195 200 205
   Arg Leu Thr Tyr Thr Asn Trp Asn Glu Gly Glu Pro Asn Asn Ala Gly
   210 215 220
   Ser Asp Glu Asp Cys Val Leu Leu Leu Lys Asn Gly Gln Trp Asn Asp
   225 230 235 240
   Val Pro Cys Ser Thr Ser His Leu Ala Val Cys Glu Phe Pro Ile
   245 250 255
<210> 122
   <211> 254
   <212> PRT
   <213> Homo sapiens
<220>
   <221> MISC_FEATURE
   <222> (25)..(69)
   <223> Polypeptide sequence from collagen
<220>
   <221> MISC_FEATURE
   <222> (70)..(254)
   <223> Polypeptide sequence from MBL
<400> 122
   Met Glu Leu Asp Arg Ala Val Gly Val Leu Gly Ala Ala Thr Leu Leu
   1               5                   10                  15
   Leu Ser Phe Leu Gly Met Ala Trp Ala Leu Gln Ala Ala Asp Thr Cys
               20                  25                  30
   Pro Glu Val Lys Met Val Gly Leu Glu Gly Ser Asp Lys Leu Thr Ile
           35                  40                  45
   Leu Arg Gly Cys Pro Gly Leu Pro Gly Ala Pro Gly Asp Lys Gly Glu
       50                  55                  60
   Ala Gly Thr Asn Gly Gln Gly Leu Arg Gly Leu Gln Gly Pro Pro Gly
   65                  70                  75                  80
   Lys Leu Gly Pro Pro Gly Asn Pro Gly Pro Ser Gly Ser Pro Gly Pro
                   85                  90                  95
   Lys Gly Gln Lys Gly Asp Pro Gly Lys Ser Pro Asp Gly Asp Ser Ser
               100                 105                 110
   Leu Ala Ala Ser Glu Arg Lys Ala Leu Gln Thr Glu Met Ala Arg Ile
           115                 120                 125
   Lys Lys Trp Leu Thr Phe Ser Leu Gly Lys Gln Val Gly Asn Lys Phe
       130                 135                 140
   Phe Leu Thr Asn Gly Glu Ile Met Thr Phe Glu Lys Val Lys Ala Leu
   145                 150                 155                 160
   Cys Val Lys Phe Gln Ala Ser Val Ala Thr Pro Arg Asn Ala Ala Glu
                   165                 170                 175
   Asn Gly Ala Ile Gln Asn Leu Ile Lys Glu Glu Ala Phe Leu Gly Ile
               180                 185                 190
   Thr Asp Glu Lys Thr Glu Gly Gln Phe Val Asp Leu Thr Gly Asn Arg
           195                 200                 205
   Leu Thr Tyr Thr Asn Trp Asn Glu Gly Glu Pro Asn Asn Ala Gly Ser
       210                 215                 220
   Asp Glu Asp Cys Val Leu Leu Leu Lys Asn Gly Gln Trp Asn Asp Val
   225                 230                 235                 240
   Pro Cys Ser Thr Ser His Leu Ala Val Cys Glu Phe Pro Ile
                   245                 250
<210> 123
   <211> 232
   <212> PRT
   <213> Homo sapiens
<220>
   <221> MISC_FEATURE
   <222> (21)..(41)
   <223> Polypeptide sequence from MBL collagen
<220>
   <221> MISC_FEATURE
   <222> (42)..(232)
   <223> Polypeptide sequence from collagen
<400> 123
<210> 124
   <211> 237
   <212> PRT
   <213> Homo sapiens
<220>
   <221> MISC_FEATURE
   <222> (21) .. (89)
   <223> Polypeptide sequence from collagen
<220>
   <221> MISC_FEATURE
   <222> (90)..(237)
   <223> Polypeptide sequence from MBL
<400> 124
   Met Ser Leu Phe Pro Ser Leu Pro Leu Leu Leu Leu Ser Met Val Ala
   1               5                   10                  15
   Ala Ser Tyr Ser Ala Leu Gln Ala Ala Asp Thr Cys Pro Glu Val Lys
               20                  25                  30
   Met Val Gly Leu Glu Gly Ser Asp Lys Leu Thr Ile Leu Arg Gly Cys
           35                  40                  45
   Pro Gly Leu Pro Gly Ala Pro Gly Asp Lys Gly Glu Ala Gly Thr Asn
       50                  55                  60
   Gly Lys Arg Gly Glu Arg Gly Pro Pro Gly Pro Pro Gly Lys Ala Gly
   65                  70                  75                  80
   Pro Pro Gly Pro Asn Gly Ala Pro Ser Pro Asp Gly Asp Ser Ser Leu
                   85                  90                  95
   Ala Ala Ser Glu Arg Lys Ala Leu Gln Thr Glu Met Ala Arg Ile Lys
               100                 105                 110
   Lys Trp Leu Thr Phe Ser Leu Gly Lys Gln Val Gly Asn Lys Phe Phe
           115                 120                 125
   Leu Thr Asn Gly Glu Ile Met Thr Phe Glu Lys Val Lys Ala Leu Cys
       130                 135                 140
   Val Lys Phe Gln Ala Ser Val Ala Thr Pro Arg Asn Ala Ala Glu Asn
   145                 150                 155                 160
   Gly Ala Ile Gln Asn Leu Ile Lys Glu Glu Ala Phe Leu Gly Ile Thr
                   165                 170                 175
   Asp Glu Lys Thr Glu Gly Gln Phe Val Asp Leu Thr Gly Asn Arg Leu
               180                 185                 190
   Thr Tyr Thr Asn Trp Asn Glu Gly Glu Pro Asn Asn Ala Gly Ser Asp
           195                 200                 205
   Glu Asp Cys Val Leu Leu Leu Lys Asn Gly Gln Trp Asn Asp Val Pro
       210                 215                 220
   Cys Ser Thr Ser His Leu Ala Val Cys Glu Phe Pro Ile
   225                 230                 235
<210> 125
   <211> 288
   <212> PRT
   <213> Homo sapiens
<400> 125
   Leu Gln Ala Ala Asp Thr Cys Pro Glu Val Lys Met Val Gly Leu Glu
   1               5                   10                  15
   Gly Ser Asp Lys Leu Thr Ile Leu Arg Gly Cys Pro Gly Leu Pro Gly
               20                  25                  30
   Ala Pro Gly Asp Lys Gly Glu Ala Gly Thr Asn Gly Lys Arg Gly Glu
           35                  40                  45
   Arg Gly Pro Pro Gly Pro Pro Gly Lys Ala Gly Pro Pro Gly Pro Asn
       50                  55                  60
   Gly Ala Pro Gly Glu Pro Gln Pro Cys Leu Thr Gly Pro Arg Thr Cys
   65                  70                  75                  80
   Lys Asp Leu Leu Asp Arg Gly His Phe Leu Ser Gly Trp His Thr Ile
                   85                  90                  95
   Tyr Leu Pro Asp Cys Arg Pro Leu Thr Val Leu Cys Asp Met Asp Thr
               100                 105                 110
   Asp Gly Gly Gly Trp Thr Val Phe Gln Arg Arg Val Asp Gly Ser Val
           115                 120                 125
   Asp Phe Tyr Arg Asp Trp Ala Thr Tyr Lys Gln Gly Phe Gly Ser Arg
       130                 135                 140
   Leu Gly Glu Phe Trp Leu Gly Asn Asp Asn Ile His Ala Leu Thr Ala
   145                 150                 155                 160
   Gln Gly Thr Ser Glu Leu Arg Val Asp Leu Val Asp Phe Glu Asp Asn
                   165                 170                 175
   Tyr Gln Phe Ala Lys Tyr Arg Ser Phe Lys Val Ala Asp Glu Ala Glu
               180                 185                 190
   Lys Tyr Asn Leu Val Leu Gly Ala Phe Val Glu Gly Ser Ala Gly Asp
           195                 200                 205
   Ser Leu Thr Phe His Asn Asn Gln Ser Phe Ser Thr Lys Asp Gln Asp
       210                 215                 220
   Asn Asp Leu Asn Thr Gly Asn Cys Ala Val Met Phe Gln Gly Ala Trp
   225                 230                 235                 240
   Trp Tyr Lys Asn Cys His Val Ser Asn Leu Asn Gly Arg Tyr Leu Arg
                   245                 250                 255
   Gly Thr His Gly Ser Phe Ala Asn Gly Ile Asn Trp Lys Ser Gly Lys
               260                 265                 270
   Gly Tyr Asn Tyr Ser Tyr Lys Val Ser Glu Met Lys Val Arg Pro Ala
           275                 280                 285
<210> 126
   <211> 228
   <212> PRT
   <213> Homo sapiens
<400> 126
   Glu Thr Val Thr Cys Glu Asp Ala Gln Lys Thr Cys Pro Ala Val Ile
   1               5                   10                  15
   Ala Cys Ser Ser Pro Gly Ile Asn Gly Phe Pro Gly Lys Asp Gly Arg
               20                  25                  30
   Asp Gly Thr Lys Gly Glu Lys Gly Glu Pro Gly Gln Gly Leu Arg Gly
           35                  40                  45
   Leu Gln Gly Pro Pro Gly Lys Leu Gly Pro Pro Gly Asn Pro Gly Pro
       50                  55                  60
   Ser Gly Ser Pro Gly Pro Lys Gly Gln Lys Gly Asp Pro Gly Lys Ser
   65                  70                  75                  80
   Pro Asp Gly Asp Ser Ser Leu Ala Ala Ser Glu Arg Lys Ala Leu Gln
                   85                  90                  95
   Thr Glu Met Ala Arg Ile Lys Lys Trp Leu Thr Phe Ser Leu Gly Lys
               100                 105                 110
   Gln Val Gly Asn Lys Phe Phe Leu Thr Asn Gly Glu Ile Met Thr Phe
   115 120 125
   Glu Lys Val Lys Ala Leu Cys Val Lys Phe Gln Ala Ser Val Ala Thr
   130 135 140
   Pro Arg Asn Ala Ala Glu Asn Gly Ala Ile Gln Asn Leu Ile Lys Glu
   145 150 155 160
   Glu Ala Phe Leu Gly Ile Thr Asp Glu Lys Thr Glu Gly Gln Phe Val
   165 170 175
   Asp Leu Thr Gly Asn Arg Leu Thr Tyr Thr Asn Trp Asn Glu Gly Glu
   180 185 190
   Pro Asn Asn Ala Gly Ser Asp Glu Asp Cys Val Leu Leu Leu Lys Asn
   195 200 205
   Gly Gln Trp Asn Asp Val Pro Cys Ser Thr Ser His Leu Ala Val Cys
   210 215 220
   Glu Phe Pro Ile
   225
<210> 127
   <211> 216
   <212> PRT
   <213> Homo sapiens
<400> 127
   Leu Gln Ala Ala Asp Thr Cys Pro Glu Val Lys Met Val Gly Leu Glu
   1 5 10 15
   Gly Ser Asp Lys Leu Thr Ile Leu Arg Gly Cys Pro Gly Leu Pro Gly
   20 25 30
   Ala Pro Gly Asp Lys Gly Glu Ala Gly Thr Asn Gly Lys Arg Gly Glu
   35 40 45
   Arg Gly Pro Pro Gly Pro Pro Gly Lys Ala Gly Pro Pro Gly Pro Asn
   50 55 60
   Gly Ala Pro Ser Pro Asp Gly Asp Ser Ser Leu Ala Ala Ser Glu Arg
   65 70 75 80
   Lys Ala Leu Gln Thr Glu Met Ala Arg Ile Lys Lys Trp Leu Thr Phe
                   85                  90                  95
   Ser Leu Gly Lys Gln Val Gly Asn Lys Phe Phe Leu Thr Asn Gly Glu
               100                 105                 110
   Ile Met Thr Phe Glu Lys Val Lys Ala Leu Cys Val Lys Phe Gln Ala
           115                 120                 125
   Ser Val Ala Thr Pro Arg Asn Ala Ala Glu Asn Gly Ala Ile Gln Asn
       130                 135                 140
   Leu Ile Lys Glu Glu Ala Phe Leu Gly Ile Thr Asp Glu Lys Thr Glu
   145                 150                 155                 160
   Gly Gln Phe Val Asp Leu Thr Gly Asn Arg Leu Thr Tyr Thr Asn Trp
                   165                 170                 175
   Asn Glu Gly Glu Pro Asn Asn Ala Gly Ser Asp Glu Asp Cys Val Leu
               180                 185                 190
   Leu Leu Lys Asn Gly Gln Trp Asn Asp Val Pro Cys Ser Thr Ser His
           195                 200                 205
   Leu Ala Val Cys Glu Phe Pro Ile
       210                 215